# EUROPEAN PATENT SPECIFICATION

(11) **EP 2 850 189 B1**
(45) Date of publication and mention of the grant of the patent: **07.11.2018**
(21) Application number: 13791343.0
(22) Date of filing: 16.05.2013
(51) Int. Cl.: C12N 15/113, C12N 15/11, A61K 48/00

(54) **COMPOSITIONS AND METHODS FOR MODULATING GENE EXPRESSION**
ZUSAMMENSETZUNGEN UND VERFAHREN ZUR MODULATION VON GENEXPRESSION
COMPOSITIONS ET MÉTHODES POUR MODULER L'EXPRESSION GÉNIQUE

(30) Priority: 16.05.2012 US 201261647949 P; 16.05.2012 US 201261648016 P; 16.05.2012 US 201261648021 P; 16.05.2012 US 201261647858 P; 16.05.2012 US 201261647925 P; 16.05.2012 US 201261647886 P; 16.05.2012 US 201261647901 P; 16.05.2012 US 201261648041 P; 16.05.2012 US 201261648051 P; 16.05.2012 US 201261648058 P; 27.10.2012 US 201261719394 P; 14.03.2013 US 201361785832 P; 14.03.2013 US 201361785778 P; 14.03.2013 US 201361785885 P; 14.03.2013 US 201361786232 P; 14.03.2013 US 201361785956 P; 14.03.2013 US 201361785529 P
(43) Date of publication of application: 25.03.2015
(73) Proprietor: Translate Bio MA, Inc., Cambridge, MA 02139 (US); The General Hospital Corporation d/b/a Massachusetts General Hospital, Boston, MA 02114 (US)
(72) Inventor: KRIEG, Arthur, M., Wellesley, MA 02482 (US); SUBRAMANIAN, Romesh, Framingham, MA 01701 (US); MCSWIGGEN, James, Arlington, MA 02474 (US); LEE, Jeannie, T., Boston, MA 02114 (US)
(74) Representative: Hoffmann Eitle
(86) International application number: PCT/US2013/041461
(87) International publication number: WO 2013/173652

(56) References cited:
- WO-A1-2012/065143
- WO-A1-2012/087983
- WO-A1-2012/087983
- WO-A2-2004/092356
- WO-A2-2007/131237
- WO-A2-2012/018881
- US-A1- 2011 251 261
- US-A1- 2012 004 278
- JING ZHAO ET AL: "Genome-wide Identification of Polycomb-Associated RNAs by RIP-seq", MOLECULAR CELL., vol. 40, no. 6, 1 December 2010 (2010-12-01), pages 939-953, XP055232287, US ISSN: 1097-2765, DOI: 10.1016/j.molcel.2010.12.011
- A. M. KHALIL ET AL: "Many human large intergenic noncoding RNAs associate with chromatin-modifying complexes and affect gene expression", PROCEEDINGS OF THE NATIONAL ACADEMY OF SCIENCES, vol. 106, no. 28, 14 July 2009 (2009-07-14), pages 11667-11672, XP55073351, ISSN: 0027-8424, DOI: 10.1073/pnas.0904715106
- WANG, YU ET AL.: 'Long non-coding RNA UCAla(CUDR) promotes proliferation and tumorigenesis of bladder cancer' INT. J. ONCOL. vol. 41, no. 1, 20 April 2012, pages 276 - 284, XP055177810
- NYCE JW LI Y SANDRASAGRA A KATZ E PABALAN J AGUILAR D ET AL: "Human oligonucleotide sequence", GENESEQ,, 31 October 2002 (2002-10-31), XP002751891, [retrieved on 2003-10-17]

## Description

### FIELD OF THE INVENTION

The invention relates to oligonucleotide based compositions, as well as oligonucleotides for use in methods for treating disease.

### BACKGROUND OF THE INVENTION

Transcriptome analyses have suggested that, although only 1-2% of the mammalian genome is protein coding, 70-90% is transcriptionally active. Recent discoveries argue that a subset of these non-protein coding transcripts play crucial roles in epigenetic regulation. In spite of their ubiquity, the structure and function of many of such transcripts remains uncharacterized. Recent studies indicate that some long non-coding RNAs function as an epigenetic regulator/RNA cofactor in chromatin remodeling through interactions with Polycomb repressor complex 2 (PRC2) and thus function to regulate gene expression.

WO 2012/065143 and WO 2012/087983 relate to long non-coding RNAs (lncRNAs), libraries of those ncRNAs that bind chromatin modifiers, such as Polycomb Repressive Complex 2, inhibitory nucleic acids and methods and compositions for targeting lncRNAs.

### SUMMARY OF THE INVENTION

The invention provides a method for selecting a candidate oligonucleotide for activating expression of a target gene, the method comprising:
selecting a PRC2-associated region within a first nucleotide sequence, wherein the first nucleotide sequence maps to a position in a first chromosome between 50 kilobases upstream of a 5'-end of the target gene and 50 kilobases downstream of a 3'-end of the target gene, wherein the PRC2-associated region is a region of an RNA that is protected from a nuclease in an RNA-immunoprecipitation assay that employs an antibody that targets a component of PRC2;
determining a second nucleotide sequence that is complementary with at least 8 consecutive nucleotides of the PRC2-associated region; and
selecting as the candidate oligonucleotide, a single stranded oligonucleotide comprising the second nucleotide sequence, wherein the oligonucleotide has at least one of following features:
   a) a sequence: 5'-X-Y-Z, wherein X is any nucleotide, Y is a nucleotide sequence of 6 nucleotides in length that is not a seed sequence of a human microRNA, and Z is a nucleotide sequence of 1 to 23 nucleotides in length, wherein X is anchored at the 5' end of the oligonucleotide;
   b) a sequence that does not comprise three or more consecutive guanosine nucleotides;
   c) a sequence that is complementary to a PRC2-associated region that encodes an RNA that forms a secondary structure comprising at least two single stranded loops; or
   d) a sequence that has greater than 60% G-C content;
wherein the single stranded oligonucleotide is 8 to 30 nucleotides in length.

The invention further provides a method of selecting a set of oligonucleotides that is enriched in oligonucleotides that activate expression of a target gene, the method comprising:
selecting a PRC2-associated region within a first nucleotide sequence that maps to a position in a first chromosome between 50 kilobases upstream of a 5'-end of the target gene and 50 kilobases downstream of a 3'-end of the target gene, wherein the PRC2-associated region is a region of an RNA that is protected from a nuclease in an RNA-immunoprecipitation assay that employs an antibody that targets a component of PRC2;
selecting a set of oligonucleotides, wherein each oligonucleotide in the set comprises a second nucleotide sequence that is complementary with at least 8 consecutive nucleotides of the PRC2-associated region, and has at least one of the following features:
   a) a sequence: 5'-X-Y-Z, wherein X is any nucleotide, Y is a nucleotide sequence of 6 nucleotides in length that is not a human seed sequence of a microRNA, and Z is a nucleotide sequence of 1 to 23 nucleotides in length, wherein X is anchored at the 5' end of the oligonucleotide;
   b) a sequence that does not comprise three or more consecutive guanosine nucleotides;
   c) a sequence that is complementary to a PRC2-associated region that encodes an RNA that forms a secondary structure comprising at least two single stranded loops; and/or
   d) a sequence that has greater than 60% G-C content; and
wherein the set of oligonucleotides is enriched in oligonucleotides that activate expression of a target gene;
wherein each of the oligonucleotides is 8 to 30 nucleotides in length.

The invention further provides a single stranded oligonucleotide for use in the treatment of a disease, wherein the treatment involves upregulating expression of a gene targeted by the PRC2-binding RNA and wherein the single stranded oligonucleotide comprises a region of complementarity that is complementary with at least 8 consecutive nucleotides of a PRC2-associated region located in a first chromosome between 50 kilobases upstream of a 5'-end of a target gene and 50 kilobases downstream of a 3'-end of the target gene, wherein the PRC2-associated region is a region of an RNA that is protected from a nuclease in an RNA-immunoprecipitation assay that employs an antibody that targets a component of PRC2, and wherein the oligonucleotide has at least one of:
a) a sequence comprising 5'-X-Y-Z, wherein X is any nucleotide, Y is a nucleotide sequence of 6 nucleotides in length that is not a human seed sequence of a microRNA, and Z is a nucleotide sequence of 1 to 23 nucleotides in length;
b) a sequence that does not comprise three or more consecutive guanosine nucleotides;
c) a sequence that is complementary to a PRC2-associated region that encodes an RNA that forms a secondary structure comprising at least two single stranded loops; and/or
d) a sequence that has greater than 60% G-C content;
wherein the oligonucleotide is 8 to 30 nucleotides in length.
Further aspects of the invention are also defined in the claims. The disclosure in the following description provides both information relating to subject matter covered by the definitions of the claims and additional information relating to subject matter which is not covered by the claims. In the following disclosure, where reference is made to embodiments, such embodiments are to be understood as embodiments of the disclosure in general, whereas the scope of the invention is defined in the claims. The description of any subject matter not covered by the claims is provided for information only.

Aspects of the disclosure provide methods for selecting oligonucleotides for activating or enhancing expression of target genes. The methods are particularly useful for identifying candidate oligonucleotides for activating or enhancing expression of target genes for which reduced expression or activity results in, or contributes to, disease. Further aspects of the disclosure provide methods of selecting a set of oligonucleotides that is enriched in oligonucleotides (e.g., compared with a random selection of oligonucleotides) that activate expression of a target gene. Accordingly, the methods may be used to establish large libraries of clinical candidates that are enriched in oligonucleotides that activate gene expression. Such libraries may be utilized, for example, to identify lead oligonucleotides for therapeutic development. Thus, the methods provided are useful for establishing a broad platform of candidate oligonucleotides for targeting the expression of most known genes, including protein coding genes. Further aspects provide single stranded oligonucleotides that modulate gene expression, and compositions and kits comprising the same. Methods for modulating gene expression using the single stranded oligonucleotides are also provided.

In some aspects, the disclosure is a method for selecting a candidate oligonucleotide for activating expression of a target gene by selecting a PRC2-associated region within a first nucleotide sequence, wherein the first nucleotide sequence maps to a position in a first chromosome between 50 kilobases upstream of a 5'-end of the target gene and 50 kilobases downstream of a 3'-end of the target gene; determining a second nucleotide sequence that is complementary with at least 8 consecutive nucleotides of the PRC2-associated region; and selecting as the candidate oligonucleotide, a single stranded oligonucleotide comprising the second nucleotide sequence, wherein the oligonucleotide has at least one of following features: a) a sequence comprising 5'-X-Y-Z, wherein X is any nucleotide, Y is a nucleotide sequence of 6 nucleotides in length that is not a seed sequence of a human microRNA, and Z is a nucleotide sequence of 1 to 23 nucleotides in length, wherein X is anchored at the 5' end of the oligonucleotide; b) a sequence that does not comprise three or more consecutive guanosine nucleotides; c) a sequence that has less than a threshold level of sequence identity with every sequence of nucleotides, of equivalent length to the second nucleotide sequence, that is between 50 kilobases upstream of a 5'-end of an off-target gene and 50 kilobases downstream of a 3'-end of the off-target gene; d) a sequence that is complementary to a PRC2-associated region that encodes an RNA that forms a secondary structure comprising at least two single stranded loops; and/or e) a sequence that has greater than 60% G-C content.

In some embodiments, the single stranded oligonucleotide has only one of features a), b), c), d), and e). In some embodiments, the single stranded oligonucleotide has at least two of features a), b), c), d), and e), each independently selected. In some embodiments, the single stranded oligonucleotide has at least three of features a), b), c), d), and e), each independently selected. In some embodiments, the single stranded oligonucleotide has at least four of features a), b), c), d), and e), each independently selected. In some embodiments, the single stranded oligonucleotide has each of features a), b), c), d), and e). In certain embodiments, the oligonucleotide has the sequence 5'X-Y-Z, in which the oligonucleotide is 8-50 nucleotides in length. In some embodiments, Y is a sequence selected from Table 3.

In another aspect the disclosure is a method of selecting a set of oligonucleotides that is enriched in oligonucleotides that activate expression of a target gene, by selecting a PRC2-associated region within a first nucleotide sequence that maps to a position in a first chromosome between 50 kilobases upstream of a 5'-end of the target gene and 50 kilobases downstream of a 3'-end of the target gene; selecting a set of oligonucleotides, wherein each oligonucleotide in the set comprises a second nucleotide sequence that is complementary with at least 8 consecutive nucleotides of the PRC2-associated region, and has at least one of the following features: a) a sequence: 5'-X-Y-Z, wherein X is any nucleotide, Y is a nucleotide sequence of 6 nucleotides in length that is not a human seed sequence of a microRNA, and Z is a nucleotide sequence of 1 to 23 nucleotides in length, wherein X is anchored at the 5' end of the oligonucleotide; b) a sequence that does not comprise three or more consecutive guanosine nucleotides; c) a sequence that has less than a threshold level of sequence identity with every sequence of nucleotides, of equivalent length to the second nucleotide sequence, that are between 50 kilobases upstream of a 5'-end of an off-target gene and 50 kilobases downstream of a 3'-end of the off-target gene; d) a sequence that is complementary to a PRC2-associated region that encodes an RNA that forms a secondary structure comprising at least two single stranded loops; and/or) a sequence that has greater than 60% G-C content; and wherein the set of oligonucleotides is enriched in oligonucleotides that activate expression of a target gene.

In some embodiments, each of the oligonucleotides has only one of features a), b), c), d), and e). In some embodiments, each of the oligonucleotides has at least two of features a), b), c), d), and e), each independently selected. In some embodiments, each of the oligonucleotides has at least three of features a), b), c), d), and e), each independently selected. In some embodiments, each of the oligonucleotides has at least four of features a), b), c), d), and e), each independently selected. In some embodiments, each of the oligonucleotides has each of features a), b), c), d), and e). In certain embodiments, each of the oligonucleotides has the sequence 5'X-Y-Z, in which the oligonucleotide is 8-50 nucleotides in length. In some embodiments, Y is a sequence selected from Table 3.

In some embodiments the single stranded oligonucleotide or each of the oligonucleotides is up to 100, 50, 40, 30, or 20 nucleotides in length. In other embodiments the single stranded oligonucleotide or each of the oligonucleotides is 8 to 30 nucleotides in length.

The threshold level of sequence identity in some embodiments is 50%, 60%, 70%, 80%, 85%, 90%, 95% or 99% sequence identity.

In one embodiment Y is a nucleotide sequence of 6 nucleotides in length set forth in Table 3.

In other embodiments the first chromosome is a chromosome of a first species, and wherein the method further comprises determining that the second nucleotide sequence is complementary to a second region of a second chromosome of a second species, the second region being located between 50 kilobases upstream of a 5'-end of a homolog of the target gene and 50 kilobases downstream of a 3'-end of the homolog of the target gene.

The second nucleotide sequence may be at least 80 % complementary to the second region of the second chromosome

In some embodiments the first nucleotide sequence maps to the strand of the first chromosome comprising the sense strand of the target gene. In other embodiments the first nucleotide sequence maps to the strand of the first chromosome comprising the antisense strand of the target gene.

In some embodiments the PRC2-associated region is upstream of the 5' end of the target gene and in other embodiments the PRC2-associated region is downstream of the 3' end of the target gene. Optionally, the PRC2-associated region may be within an intron or an exon of the target gene or the PRC2-associated region may traverse an intron-exon junction, a 5'-UTR-exon junction or a 3'-UTR-exon junction of the target gene.

The PRC2-associated region may encode an RNA that forms a secondary structure comprising at least two single stranded loops. Optionally the secondary structure comprises a double stranded stem between the at least two single stranded loops. In some embodiments the at least 8 consecutive nucleotides of the PRC2-associated region encode at least a portion of at least one or at least two of the loops or at least a portion of the double stranded stem.

In other aspects the disclosure is a single stranded oligonucleotide comprising a region of complementarity that is complementary with at least 8 consecutive nucleotides of a PRC2-associated region located in a first chromosome between 50 kilobases upstream of a 5'-end of a target gene and 50 kilobases downstream of a 3'-end of the target gene, wherein the oligonucleotide has at least one of: a) a sequence comprising 5'-X-Y-Z, wherein X is any nucleotide, Y is a nucleotide sequence of 6 nucleotides in length that is not a human seed sequence of a microRNA, and Z is a nucleotide sequence of 1 to 23 nucleotides in length; b) a sequence that does not comprise three or more consecutive guanosine nucleotides; c) a sequence that has less than a threshold level of sequence identity with every sequence of nucleotides, of equivalent length to the second nucleotide sequence, that are between 50 kilobases upstream of a 5'-end of an off-target gene and 50 kilobases downstream of a 3'-end of the off-target gene; d) a sequence that is complementary to a PRC2-associated region that encodes an RNA that forms a secondary structure comprising at least two single stranded loops; and/or e) a sequence that has greater than 60% G-C content. In some embodiments, the single stranded oligonucleotide has only one of features a), b), c), d), and e). In some embodiments, the single stranded oligonucleotide has at least two of features a), b), c), d), and e), each independently selected. In some embodiments, the single stranded oligonucleotide has at least three of features a), b), c), d), and e), each independently selected. In some embodiments, the single stranded oligonucleotide has at least four of features a), b), c), d), and e), each independently selected. In some embodiments, the single stranded oligonucleotide has each of features a), b), c), d), and e). In certain embodiments, the oligonucleotide has the sequence 5'X-Y-Z, in which the oligonucleotide is 8-50 nucleotides in length. In some embodiments, Y is a sequence selected from Table 3.

The first chromosome is a chromosome of a first species in some embodiments. A sequence comprising the at least 8 consecutive nucleotides is located in a second chromosome between 50 kilobases upstream of a 5'-end of a homolog of the target gene and 50 kilobases downstream of a 3'-end of the homolog of the target gene, wherein the second chromosome is a chromosome of second species. The first species may be human and the second species may be a mouse.

The invention also includes a single stranded oligonucleotide as defined by the claims of 8-30 nucleotides in length, wherein the single stranded oligonucleotide is complementary with at least 8 consecutive nucleotides of a PRC2-associated region located in a chromosome between 50 kilobases upstream of a 5'-end of a target gene and 50 kilobases downstream of a 3'-end of the target gene, wherein the nucleotide sequence of the single stranded oligonucleotide comprises one or more nucleotide sequences selected from (X)Xxxxxx, (X)xXxxxx, (X)xxXxxx, (X)xxxXxx, (X)xxxxXx and (X)xxxxxX, (X)XXxxxx, (X)XxXxxx, (X)XxxXxx, (X)XxxxXx, (X)XxxxxX, (X)xXXxxx, (X)xXxXxx, (X)xXxxXx, (X)xXxxxX, (X)xxXXxx, (X)xxXxXx, (X)xxXxxX, (X)xxxXXx, (X)xxxXxX and (X)xxxxXX, (X)XXXxxx, (X)xXXXxx, (X)xxXXXx, (X)xxxXXX, (X)XXxXxx, (X)XXxxXx, (X)XXxxxX, (X)xXXxXx, (X)xXXxxX, (X)xxXXxX, (X)XxXXxx, (X)XxxXXx (X)XxxxXX, (X)xXxXXx, (X)xXxxXX, (X)xxXxXX, (X)xXxXxX and (X)XxXxXx, (X)xxXXX, (X)xXxXXX, (X)xXXxXX, (X)xXXXxX, (X)xXXXXx, (X)XxxXXXX, (X)XxXxXX, (X)XxXXxX, (X)XxXXx, (X)XXxxXX, (X)XXxXxX, (X)XXxXXx, (X)XXXxxX, (X)XXXxXx, and (X)XXXXxx, (X)xXXXXX, (X)XxXXXX, (X)XXxXXX, (X)XXXxXX, (X)XXXXxX and (X)XXXXXx, and XXXXXX, XxXXXXX, XXxXXXX, XXXxXXX, XXXXxXX, XXXXXxX and XXXXXXx, wherein "X" denotes a nucleotide analogue, (X) denotes an optional nucleotide analogue, and "x" denotes a DNA or RNA nucleotide unit.

A single stranded oligonucleotide as defined by the claims of 8 to 30 nucleotides in length having a region of complementarity that is complementary with at least 8 contiguous nucleotides of a long non-coding RNA (lncRNA) that regulates expression of a target gene, wherein 2-19 nucleotides of the oligonucleotide are nucleotide analogues is provided in other aspects of the invention.

In other aspects, a single stranded oligonucleotide of 5 to 30 nucleotides in length having a region of complementarity that is complementary with at least 5 contiguous nucleotides of a long non- coding RNA (lncRNA) that regulates expression of a target gene, wherein the oligonucleotide is linked to a second oligonucleotide by a cleavable linker is provided. In some embodiments the oligonucleotide has the structure of any of the single stranded oligonucleotides described herein.

A single stranded single stranded oligonucleotide of 8 to 40 nucleotides in length having a region of complementarity that is complementary with at least 5 contiguous nucleotides of a PRC2-binding long non- coding RNA (lncRNA) that regulates expression of a protein-coding reference gene, wherein the lncRNA is transcribed from the opposite strand as the protein-coding reference gene in a genomic region containing the protein-coding reference gene, wherein the single stranded oligonucleotide binds to a region of the IncRNA that originates within or overlaps an exon, an intron, exon, intron-exon junction, 5' UTR, 3' UTR, a translation initiation region, or a translation termination region is provided in other aspects of the disclosure.

A single stranded oligonucleotide of 8 to 40 nucleotides in length having a region of complementarity that is complementary with at least 5 contiguous nucleotides of a long non-coding RNA (lncRNA) that regulates expression of a target gene is provided in other aspects of the disclosure. The oligonucleotide has complementarity to the IncRNA in a region of the IncRNA that is outside of the transcribed region of the target gene.

In yet other aspects of the disclosure a single stranded oligonucleotide of 8 to 30 nucleotides in length having a region of complementarity that is complementary with at least 5 contiguous nucleotides of a long non- coding RNA (lncRNA) that inhibits expression of a target gene, wherein the oligonucleotide has complementarity to the IncRNA in a region of the IncRNA that is transcribed from a non-coding portion of the target gene is provided.

In some embodiments the IncRNA is a PRC2-associated region.

The present application makes reference to the nucleotide sequences listed as SEQ ID NOs:1-193,049 in International Patent Application PCT/US2011/060493, filed on November 12, 2011, published on May 18, 2012, as WO/2012/065143, and entitled, "POLYCOMB-ASSOCIATED NON-CODING RNAS." These sequences are referred to herein by their sequence identifier number preceded by an "A". Accordingly, the set of nucleotide sequences referenced from International Patent Application PCT/US2011/060493 is referred to as "sequences A1-A193,049."

The present application also makes reference to the nucleotide sequences listed as SEQ ID NOs: 1 to 916,209, or 916,626 to 934,931 in International Patent Application PCT/US2011/65939, filed on December 19,2011, published on June 28, 2012, as WO/2012/087983, and entitled "POLYCOMB-ASSOCIATED NON-CODING RNAS." These sequences are referred to herein by their sequence identifier number preceded by an "B". Accordingly, the set of nucleotide sequences referenced from International Patent Application PCT/US2011/65939 is referred to as "sequences B1 to B916,209, or B916,626 to B934,931."

In some embodiments the PRC2-associated region has a nucleotide sequence selected from sequences A1 to A 193,049, B1 to B916,209, and B916,626 to B934,931.

In some embodiments, the PRC2-associated region has a nucleotide sequence selected from SEQ ID NO: 1-1212.

The oligonucleotide may be any length. In some embodiments the oligonucleotide is up to 100, 50, 40, 30, or 20 nucleotides in length. In other embodiments the oligonucleotide is 8 to 30 nucleotides in length. In yet other embodiments the oligonucleotide is 8 to 10 nucleotides in length and all but 1, 2, or 3 of the nucleotides of the complementary sequence of the PRC2-associated region are cytosine or guanosine nucleotides.

The at least 8 consecutive nucleotides of the PRC2-associated region in some embodiments is in the strand of the chromosome comprising the antisense strand of the target gene and in other embodiments is in the strand of the chromosome comprising the sense strand of the target gene.

In some embodiments the PRC2-associated region is upstream of the 5' end of the target gene and in other embodiments the PRC2-associated region is downstream of the 3' end of the target gene. Optionally, the PRC2-associated region may be within an intron or an exon of the target gene or the PRC2-associated region may traverse an intron-exon junction, a 5'-UTR-exon junction or a 3'-UTR-exon junction of the target gene.

The PRC2-associated region may encode an RNA that forms a secondary structure comprising at least two single stranded loops. Optionally the secondary structure comprises a double stranded stem between the at least two single stranded loops. In some embodiments the at least 8 consecutive nucleotides of the PRC2-associated region encode at least a portion of at least one or at least two of the loops or at least a portion of the double stranded stem.

In some embodiments the at least one nucleotide analogue results in an increase in Tₘ of the oligonucleotide in a range of 1 to 5 °C compared with an oligonucleotide that does not have the at least one nucleotide analogue.

In some embodiments at least one nucleotide of the oligonucleotide comprises a nucleotide analogue. In other embodiments each nucleotide of the oligonucleotide comprises a nucleotide analogue For instance the nucleotide analogue may be a 2' O-methyl or a bridged nucleotide. In other embodiments the oligonucleotide comprises at least one ribonucleotide, at least one deoxyribonucleotide, or at least one bridged nucleotide. The bridged nucleotide may be, for instance, a LNA nucleotide, a cEt nucleotide or a ENA nucleotide analogue. Optionally each nucleotide of the oligonucleotide is a LNA nucleotide.

In some embodiments the nucleotides of the oligonucleotide comprise alternating nucleotide types. For instance, in some embodiments the oligonucleotide comprises deoxyribonucleotides and 2'-fluoro-deoxyribonucleotides. In other embodiments the nucleotides of the oligonucleotide comprise alternating deoxyribonucleotides and 2'-O-methyl nucleotides. In yet other embodiments the nucleotides of the oligonucleotide comprise alternating deoxyribonucleotides and ENA nucleotide analogues or the nucleotides of the oligonucleotide comprise alternating deoxyribonucleotides and LNA nucleotides. In yet other embodiments the nucleotides of the oligonucleotide comprise alternating LNA nucleotides and 2'-O-methyl nucleotides.

The 5' nucleotide of the oligonucleotide may have different properties. For instance in some embodiments the 5' nucleotide of the oligonucleotide is a deoxyribonucleotide or a LNA nucleotide.

In some embodiments the nucleotides of the oligonucleotide comprise deoxyribonucleotides flanked by at least one LNA nucleotide on each of the 5' and 3' ends of the deoxyribonucleotides.

The single stranded oligonucleotide may also include phosphorothioate internucleotide linkages between at least two nucleotides or between all nucleotides.

In some embodiments the nucleotide at the 3' position of the oligonucleotide has a 3' hydroxyl group. In other embodiments the nucleotide at the 3' position of the oligonucleotide has a 3' thiophosphate.

Optionally the single stranded oligonucleotide has a biotin moiety conjugated to the 5' or 3' nucleotide. In some embodiments the single stranded oligonucleotide has one or more of the following conjugates to either the 5' or 3' nucleotide or both: cholesterol, Vitamin A, folate, sigma receptor ligands, aptamers, peptides, such as CPP, hydrophobic molecules, such as lipids, ASGPR or dynamic polyconjugates and variants thereof.

A composition is provided in another aspect. The composition is a single stranded oligonucleotide described herein and a carrier, a buffered solution, and/or a pharmaceutically acceptable carrier.

In some aspects the disclosure is a composition of a single stranded RNA oligonucleotide of 8 to 20 nucleotides in length having a region of complementarity that is complementary with at least 5 contiguous nucleotides of a long non-coding RNA (lncRNA) that regulates expression of a target gene, wherein 2-19 nucleotides of the oligonucleotide are nucleotide analogues, formulated in a pharmaceutically acceptable carrier, wherein a complementary RNA oligonucleotide is not present in the composition.

In some embodiments the nucleotide analogues are selected from the group consisting of a bridged nucleotide, 2' fluoro, and 2'O-methyl nucleotide. In other embodiments the bridged nucleotide is a LNA, ENA or cEt nucleotide.

The lncRNA may be transcribed from the opposite strand as the target gene in a genomic region containing the target gene.

In some embodiments the oligonucleotide has complementarity to the lncRNA in a region of the lncRNA that is transcribed from a non-coding portion of the target gene. In other embodiments the oligonucleotide has complementarity to the lncRNA in a region of the lncRNA that is outside of the transcribed region of the target gene.

A kit comprising a container housing any of the compositions is also provided.

In other aspects the disclosure is a method of increasing expression of a target gene in a cell, by delivering a single stranded oligonucleotide described herein into the cell.

A method of increasing levels of a target gene in a subject by administering a single stranded oligonucleotide described herein to the subject is provided in other aspects of the disclosure.

A method of treating a condition associated with decreased levels of a target gene in a subject by administering a single stranded oligonucleotide described herein to the subject is provided in yet other aspects of the disclosure.

A method of upregulating gene expression is provided in other aspects. The method involves contacting a cell with a single stranded RNA oligonucleotide of 8 to 30 nucleotides in length having a region of complementarity that is complementary with at least 5 contiguous nucleotides of a long non- coding RNA (lncRNA) that inhibits expression of a target gene.

The following applications are also referenced: International Patent Application: PCT/US2011/65939, filed on December 19, 2011, published on June 28, 2012, as WO/2012/087983, and entitled POLYCOMB-ASSOCIATED NON-CODING RNAS, and International Patent Application: PCT/US2011/060493, filed on November 12, 2011, published on May 18, 2012, as WO/2012/065143, and entitled POLYCOMB-ASSOCIATED NON-CODING RNAS.

The use of "including," "comprising," or "having," "containing", "involving", and variations thereof herein, is meant to encompass the items listed thereafter and equivalents thereof as well as additional items.

**Table 1: Brief Description of Sequence Listing**

| **SeqID** | **Chrom** | **gene** | **Chr.Start** | **Chr.End** | **strand** | **Organism** | **Approx. Length** |
|---|---|---|---|---|---|---|---|
| 1 | chr9 | FXN | 71638478 | 71705993 | + | Homo sapiens | 67515 |
| 2 | chr9 | FXN | 71638478 | 71705993 | - | Homo sapiens | 67515 |
| 3 | chr19 | Fxn | 24323942 | 24367076 | - | Mus musculus | 43134 |
| 4 | chr19 | Fxn | 24323942 | 24367076 | + | Mus musculus | 43134 |
| 5 | chr9 | FXN | 71651581 | 71651633 | + | Homo sapiens | 52 |
| 6 | chr9 | FXN | 71651674 | 71651733 | + | Homo sapiens | 59 |
| 7 | chr9 | FXN | 71664748 | 71664793 | + | Homo sapiens | 45 |
| 8 | chr9 | FXN | 71676243 | 71676290 | + | Homo sapiens | 47 |
| 9 | chr9 | FXN | 71677819 | 71678190 | + | Homo sapiens | 371 |
| 10 | chr9 | FXN | 71649581 | 71653633 | + | Homo sapiens | 4052 |
| 11 | chr9 | FXN | 71649674 | 71653733 | + | Homo sapiens | 4059 |
| 12 | chr9 | FXN | 71662748 | 71666793 | + | Homo sapiens | 4045 |
| 13 | chr9 | FXN | 71674243 | 71678290 | + | Homo sapiens | 4047 |
| 14 | chr9 | FXN | 71675819 | 71680190 | + | Homo sapiens | 4371 |
| 15 | chr9 | FXN | 71661444 | 71661499 | - | Homo sapiens | 55 |
| 16 | chr9 | FXN | 71679886 | 71679910 | - | Homo sapiens | 24 |
| 17 | chr9 | FXN | 71659444 | 71663499 | - | Homo sapiens | 4055 |
| 18 | chr9 | FXN | 71677886 | 71681910 | - | Homo sapiens | 4024 |
| 19 | chr5 | SMN1 | 70208768 | 70260842 | + | Homo sapiens | 52070 |
| 20 | chr5 | SMN2 | 69333350 | 69385422 | + | Homo sapiens | 52073 |
| 21 | chr9 | SMNP | 20319406 | 20344375 | + | Homo sapiens | 24970 |
| 22 | chr5 | SMN1 | 70208768 | 70260838 | - | Homo sapiens | 52071 |
| 23 | chr5 | SMN2 | 69333350 | 69385422 | - | Homo sapiens | 52073 |
| 24 | chr9 | SMNP | 20319406 | 20344375 | - | Homo sapiens | 24970 |
| 25 | chr13 | Smn1 | 100881160 | 100919653 | + | Mus musculus | 38494 |
| 26 | chr13 | Smn1 | 100881160 | 100919653 | - | Mus musculus | 38494 |
| 27 | chr5 | SMN1 | 70240095 | 70240127 | + | Homo sapiens | 32 |
| 27 | chr5 | SMN2 | 69364672 | 69364704 | + | Homo sapiens | 32 |
| 28 | chr5 | SMN1 | 70214393 | 70214822 | + | Homo sapiens | 429 |
| 28 | chr5 | SMN2 | 69338976 | 69339405 | + | Homo sapiens | 429 |
| 29 | chr5 | SMN1 | 70214064 | 70214108 | + | Homo sapiens | 44 |
| 29 | chr5 | SMN2 | 69338647 | 69338691 | + | Homo sapiens | 44 |
| 30 | chr5 | SMN1 | 70214276 | 70214317 | + | Homo sapiens | 41 |
| 30 | chr5 | SMN2 | 69338859 | 69338900 | + | Homo sapiens | 41 |
| 31 | chr5 | SMN1 | 70214445 | 70214472 | + | Homo sapiens | 27 |
| 31 | chr5 | SMN2 | 69339028 | 69339055 | + | Homo sapiens | 27 |
| 32 | chr5 | SMN1 | 70238095 | 70242127 | + | Homo sapiens | 4032 |
| 32 | chr5 | SMN2 | 69362672 | 69366704 | + | Homo sapiens | 4032 |
| 33 | chr5 | SMN1 | 70212393 | 70216822 | + | Homo sapiens | 4429 |
| 33 | chr5 | SMN2 | 69336976 | 69341405 | + | Homo sapiens | 4429 |
| 34 | chr5 | SMN1 | 70212064 | 70216108 | + | Homo sapiens | 4044 |
| 34 | chr5 | SMN2 | 69336647 | 69340691 | + | Homo sapiens | 4044 |
| 35 | chr5 | SMN1 | 70212276 | 70216317 | + | Homo sapiens | 4041 |
| 35 | chr5 | SMN2 | 69336859 | 69340900 | + | Homo sapiens | 4041 |
| 36 | chr5 | SMN1 | 70212445 | 70216472 | + | Homo sapiens | 4027 |
| 36 | chr5 | SMN2 | 69337028 | 69341055 | + | Homo sapiens | 4027 |
| 37 | chr5 | SMN1 | 70240510 | 70240551 | - | Homo sapiens | 41 |
| 37 | chr5 | SMN2 | 69365087 | 69365128 | - | Homo sapiens | 41 |
| 38 | chr5 | SMN1 | 70241924 | 70241968 | - | Homo sapiens | 44 |
| 38 | chr5 | SMN2 | 69366499 | 69366543 | - | Homo sapiens | 44 |
| 39 | chr5 | SMN1 | 70238510 | 70242551 | - | Homo sapiens | 4041 |
| 39 | chr5 | SMN2 | 69363087 | 69367128 | - | Homo sapiens | 4041 |
| 40 | chr5 | SMN1 | 70239924 | 70243968 | - | Homo sapiens | 4044 |
| 40 | chr5 | SMN2 | 69364499 | 69368543 | - | Homo sapiens | 4044 |
| 41 | chr5 | SMN1 | 70247831 | 70247845 | + | Homo sapiens | 14 |
| 41 | chr5 | SMN2 | 69372411 | 69372425 | + | Homo sapiens | 14 |
| 42 | chr5 | SMN2 | 69372402 | 69372845 | + | Homo sapiens | 443 |
| 43 | chr6 | UTRN | 144600872 | 145186170 | + | Homo sapiens | 585298 |
| 44 | chr6 | UTRN | 144600872 | 145186170 | - | Homo sapiens | 585298 |
| 45 | chr10 | Utrn | 12089985 | 12593533 | - | Mus musculus | 503548 |
| 46 | chr10 | Utrn | 12089985 | 12593533 | + | Mus musculus | 503548 |
| 47 | chr6 | UTRN | 144610489 | 144610535 | + | Homo sapiens | 46 |
| 48 | chr6 | UTRN | 144612994 | 144613040 | + | Homo sapiens | 46 |
| 49 | chr6 | UTRN | 144614120 | 144614162 | + | Homo sapiens | 42 |
| 50 | chr6 | UTRN | 144614968 | 144615021 | + | Homo sapiens | 53 |
| 51 | chr6 | UTRN | 144618862 | 144618901 | + | Homo sapiens | 39 |
| 52 | chr6 | UTRN | 144621690 | 144621714 | + | Homo sapiens | 24 |
| 53 | chr6 | UTRN | 144625028 | 144625096 | + | Homo sapiens | 68 |
| 54 | chr6 | UTRN | 144625129 | 144625174 | + | Homo sapiens | 45 |
| 55 | chr6 | UTRN | 144625319 | 144625379 | + | Homo sapiens | 60 |
| 56 | chr6 | UTRN | 144628965 | 144629011 | + | Homo sapiens | 46 |
| 57 | chr6 | UTRN | 144633818 | 144633869 | + | Homo sapiens | 51 |
| 58 | chr6 | UTRN | 144633933 | 144633968 | + | Homo sapiens | 35 |
| 59 | chr6 | UTRN | 144658267 | 144658302 | + | Homo sapiens | 35 |
| 60 | chr6 | UTRN | 144685087 | 144685140 | + | Homo sapiens | 53 |
| 61 | chr6 | UTRN | 144695039 | 144695063 | + | Homo sapiens | 24 |
| 62 | chr6 | UTRN | 144699670 | 144699699 | + | Homo sapiens | 29 |
| 63 | chr6 | UTRN | 144704043 | 144704398 | + | Homo sapiens | 355 |
| 64 | chr6 | UTRN | 144706312 | 144706345 | + | Homo sapiens | 33 |
| 65 | chr6 | UTRN | 144706654 | 144706704 | + | Homo sapiens | 50 |
| 66 | chr6 | UTRN | 144721683 | 144721738 | + | Homo sapiens | 55 |
| 67 | chr6 | UTRN | 144722580 | 144722627 | + | Homo sapiens | 47 |
| 68 | chr6 | UTRN | 144724848 | 144724889 | + | Homo sapiens | 41 |
| 69 | chr6 | UTRN | 144727897 | 144727947 | + | Homo sapiens | 50 |
| 70 | chr6 | UTRN | 144746408 | 144746448 | + | Homo sapiens | 40 |
| 71 | chr6 | UTRN | 144749102 | 144749152 | + | Homo sapiens | 50 |
| 72 | chr6 | UTRN | 144749948 | 144750026 | + | Homo sapiens | 78 |
| 73 | chr6 | UTRN | 144750728 | 144750789 | + | Homo sapiens | 61 |
| 74 | chr6 | UTRN | 144750789 | 144750853 | + | Homo sapiens | 64 |
| 75 | chr6 | UTRN | 144757162 | 144757214 | + | Homo sapiens | 52 |
| 76 | chr6 | UTRN | 144757214 | 144757274 | + | Homo sapiens | 60 |
| 77 | chr6 | UTRN | 144758752 | 144758807 | + | Homo sapiens | 55 |
| 78 | chr6 | UTRN | 144758807 | 144758864 | + | Homo sapiens | 57 |
| 79 | chr6 | UTRN | 144761513 | 144761579 | + | Homo sapiens | 66 |
| 80 | chr6 | UTRN | 144765456 | 144765506 | + | Homo sapiens | 50 |
| 81 | chr6 | UTRN | 144768420 | 144768461 | + | Homo sapiens | 41 |
| 82 | chr6 | UTRN | 144768737 | 144768764 | + | Homo sapiens | 27 |
| 83 | chr6 | UTRN | 144772549 | 144772627 | + | Homo sapiens | 78 |
| 84 | chr6 | UTRN | 144774960 | 144775001 | + | Homo sapiens | 41 |
| 85 | chr6 | UTRN | 144779914 | 144779960 | + | Homo sapiens | 46 |
| 86 | chr6 | UTRN | 144780026 | 144780070 | + | Homo sapiens | 44 |
| 87 | chr6 | UTRN | 144780278 | 144780324 | + | Homo sapiens | 46 |
| 88 | chr6 | UTRN | 144783879 | 144783933 | + | Homo sapiens | 54 |
| 89 | chr6 | UTRN | 144783933 | 144783995 | + | Homo sapiens | 62 |
| 90 | chr6 | UTRN | 144800953 | 144800999 | + | Homo sapiens | 46 |
| 91 | chr6 | UTRN | 144802780 | 144802822 | + | Homo sapiens | 42 |
| 92 | chr6 | UTRN | 144803427 | 144803473 | + | Homo sapiens | 46 |
| 93 | chr6 | UTRN | 144803714 | 144803742 | + | Homo sapiens | 28 |
| 94 | chr6 | UTRN | 144803774 | 144803849 | + | Homo sapiens | 75 |
| 95 | chr6 | UTRN | 144806673 | 144806714 | + | Homo sapiens | 41 |
| 96 | chr6 | UTRN | 144808684 | 144808739 | + | Homo sapiens | 55 |
| 97 | chr6 | UTRN | 144808739 | 144808803 | + | Homo sapiens | 64 |
| 98 | chr6 | UTRN | 144809834 | 144809881 | + | Homo sapiens | 47 |
| 99 | chr6 | UTRN | 144811266 | 144811310 | + | Homo sapiens | 44 |
| 100 | chr6 | UTRN | 144814476 | 144814522 | + | Homo sapiens | 46 |
| 101 | chr6 | UTRN | 144815156 | 144815199 | + | Homo sapiens | 43 |
| 102 | chr6 | UTRN | 144820469 | 144820529 | + | Homo sapiens | 60 |
| 103 | chr6 | UTRN | 144832193 | 144832243 | + | Homo sapiens | 50 |
| 104 | chr6 | UTRN | 144835120 | 144835166 | + | Homo sapiens | 46 |
| 105 | chr6 | UTRN | 144835862 | 144835907 | + | Homo sapiens | 45 |
| 106 | chr6 | UTRN | 144837423 | 144837465 | + | Homo sapiens | 42 |
| 107 | chr6 | UTRN | 144837943 | 144837989 | + | Homo sapiens | 46 |
| 108 | chr6 | UTRN | 144844263 | 144844304 | + | Homo sapiens | 41 |
| 109 | chr6 | UTRN | 144858722 | 144858798 | + | Homo sapiens | 76 |
| 110 | chr6 | UTRN | 144870778 | 144870817 | + | Homo sapiens | 39 |
| 111 | chr6 | UTRN | 144871113 | 144871154 | + | Homo sapiens | 41 |
| 112 | chr6 | UTRN | 144872123 | 144872165 | + | Homo sapiens | 42 |
| 113 | chr6 | UTRN | 144872599 | 144872669 | + | Homo sapiens | 70 |
| 114 | chr6 | UTRN | 144873625 | 144873662 | + | Homo sapiens | 37 |
| 115 | chr6 | UTRN | 144875136 | 144875182 | + | Homo sapiens | 46 |
| 116 | chr6 | UTRN | 144875895 | 144875938 | + | Homo sapiens | 43 |
| 117 | chr6 | UTRN | 144886315 | 144886378 | + | Homo sapiens | 63 |
| 118 | chr6 | UTRN | 144904604 | 144904645 | + | Homo sapiens | 41 |
| 119 | chr6 | UTRN | 144905276 | 144905300 | + | Homo sapiens | 24 |
| 120 | chr6 | UTRN | 144906334 | 144906358 | + | Homo sapiens | 24 |
| 121 | chr6 | UTRN | 144907025 | 144907055 | + | Homo sapiens | 30 |
| 122 | chr6 | UTRN | 144908864 | 144908908 | + | Homo sapiens | 44 |
| 123 | chr6 | UTRN | 144909055 | 144909078 | + | Homo sapiens | 23 |
| 124 | chr6 | UTRN | 144910040 | 144910085 | + | Homo sapiens | 45 |
| 125 | chr6 | UTRN | 144918990 | 144919013 | + | Homo sapiens | 23 |
| 126 | chr6 | UTRN | 144935390 | 144935427 | + | Homo sapiens | 37 |
| 127 | chr6 | UTRN | 144938199 | 144938248 | + | Homo sapiens | 49 |
| 128 | chr6 | UTRN | 144941446 | 144941489 | + | Homo sapiens | 43 |
| 129 | chr6 | UTRN | 144941551 | 144941596 | + | Homo sapiens | 45 |
| 130 | chr6 | UTRN | 144941700 | 144941748 | + | Homo sapiens | 48 |
| 131 | chr6 | UTRN | 144941856 | 144941912 | + | Homo sapiens | 56 |
| 132 | chr6 | UTRN | 144941912 | 144941988 | + | Homo sapiens | 76 |
| 133 | chr6 | UTRN | 144942080 | 144942136 | + | Homo sapiens | 56 |
| 134 | chr6 | UTRN | 144944667 | 144944712 | + | Homo sapiens | 45 |
| 135 | chr6 | UTRN | 144945160 | 144945207 | + | Homo sapiens | 47 |
| 136 | chr6 | UTRN | 144950918 | 144950987 | + | Homo sapiens | 69 |
| 137 | chr6 | UTRN | 144952595 | 144952650 | + | Homo sapiens | 55 |
| 138 | chr6 | UTRN | 144954758 | 144954804 | + | Homo sapiens | 46 |
| 139 | chr6 | UTRN | 144960952 | 144960996 | + | Homo sapiens | 44 |
| 140 | chr6 | UTRN | 144968386 | 144968438 | + | Homo sapiens | 52 |
| 141 | chr6 | UTRN | 144981668 | 144981709 | + | Homo sapiens | 41 |
| 142 | chr6 | UTRN | 144985026 | 144985072 | + | Homo sapiens | 46 |
| 143 | chr6 | UTRN | 144999637 | 144999686 | + | Homo sapiens | 49 |
| 144 | chr6 | UTRN | 144999750 | 144999785 | + | Homo sapiens | 35 |
| 145 | chr6 | UTRN | 144999904 | 144999956 | + | Homo sapiens | 52 |
| 146 | chr6 | UTRN | 145012224 | 145012561 | + | Homo sapiens | 337 |
| 147 | chr6 | UTRN | 145017897 | 145017969 | + | Homo sapiens | 72 |
| 148 | chr6 | UTRN | 145017978 | 145018021 | + | Homo sapiens | 43 |
| 149 | chr6 | UTRN | 145019229 | 145019261 | + | Homo sapiens | 32 |
| 150 | chr6 | UTRN | 145021232 | 145021278 | + | Homo sapiens | 46 |
| 151 | chr6 | UTRN | 145021336 | 145021382 | + | Homo sapiens | 46 |
| 152 | chr6 | UTRN | 145031285 | 145031331 | + | Homo sapiens | 46 |
| 153 | chr6 | UTRN | 145038424 | 145038467 | + | Homo sapiens | 43 |
| 154 | chr6 | UTRN | 145042706 | 145042751 | + | Homo sapiens | 45 |
| 155 | chr6 | UTRN | 145047775 | 145047820 | + | Homo sapiens | 45 |
| 156 | chr6 | UTRN | 145051549 | 145051592 | + | Homo sapiens | 43 |
| 157 | chr6 | UTRN | 145061899 | 145061941 | + | Homo sapiens | 42 |
| 158 | chr6 | UTRN | 145063569 | 145063615 | + | Homo sapiens | 46 |
| 159 | chr6 | UTRN | 145069445 | 145069497 | + | Homo sapiens | 52 |
| 160 | chr6 | UTRN | 145072959 | 145073005 | + | Homo sapiens | 46 |
| 161 | chr6 | UTRN | 145079115 | 145079162 | + | Homo sapiens | 47 |
| 162 | chr6 | UTRN | 145079204 | 145079271 | + | Homo sapiens | 67 |
| 163 | chr6 | UTRN | 145080780 | 145080816 | + | Homo sapiens | 36 |
| 164 | chr6 | UTRN | 145080843 | 145080885 | + | Homo sapiens | 42 |
| 165 | chr6 | UTRN | 145081884 | 145081930 | + | Homo sapiens | 46 |
| 166 | chr6 | UTRN | 145087734 | 145087827 | + | Homo sapiens | 93 |
| 167 | chr6 | UTRN | 145087827 | 145087911 | + | Homo sapiens | 84 |
| 168 | chr6 | UTRN | 145088046 | 145088124 | + | Homo sapiens | 78 |
| 169 | chr6 | UTRN | 145088210 | 145088245 | + | Homo sapiens | 35 |
| 170 | chr6 | UTRN | 145088678 | 145088723 | + | Homo sapiens | 45 |
| 171 | chr6 | UTRN | 145090281 | 145090327 | + | Homo sapiens | 46 |
| 172 | chr6 | UTRN | 145090884 | 145090934 | + | Homo sapiens | 50 |
| 173 | chr6 | UTRN | 145093542 | 145093591 | + | Homo sapiens | 49 |
| 174 | chr6 | UTRN | 145094155 | 145094199 | + | Homo sapiens | 44 |
| 175 | chr6 | UTRN | 145096714 | 145096756 | + | Homo sapiens | 42 |
| 176 | chr6 | UTRN | 145101237 | 145101284 | + | Homo sapiens | 47 |
| 177 | chr6 | UTRN | 145101598 | 145101642 | + | Homo sapiens | 44 |
| 178 | chr6 | UTRN | 145127145 | 145127194 | + | Homo sapiens | 49 |
| 179 | chr6 | UTRN | 145127467 | 145127507 | + | Homo sapiens | 40 |
| 180 | chr6 | UTRN | 145127866 | 145127929 | + | Homo sapiens | 63 |
| 181 | chr6 | UTRN | 145128105 | 145128160 | + | Homo sapiens | 55 |
| 182 | chr6 | UTRN | 145128415 | 145128460 | + | Homo sapiens | 45 |
| 183 | chr6 | UTRN | 145132350 | 145132397 | + | Homo sapiens | 47 |
| 184 | chr6 | UTRN | 145132917 | 145132974 | + | Homo sapiens | 57 |
| 185 | chr6 | UTRN | 145133779 | 145133802 | + | Homo sapiens | 23 |
| 186 | chr6 | UTRN | 145134097 | 145134169 | + | Homo sapiens | 72 |
| 187 | chr6 | UTRN | 145134835 | 145134881 | + | Homo sapiens | 46 |
| 188 | chr6 | UTRN | 145142034 | 145142135 | + | Homo sapiens | 101 |
| 189 | chr6 | UTRN | 145142629 | 145142676 | + | Homo sapiens | 47 |
| 190 | chr6 | UTRN | 145148750 | 145148806 | + | Homo sapiens | 56 |
| 191 | chr6 | UTRN | 145149936 | 145149984 | + | Homo sapiens | 48 |
| 192 | chr6 | UTRN | 145153900 | 145154300 | + | Homo sapiens | 400 |
| 193 | chr6 | UTRN | 145154735 | 145154782 | + | Homo sapiens | 47 |
| 194 | chr6 | UTRN | 145155438 | 145155468 | + | Homo sapiens | 30 |
| 195 | chr6 | UTRN | 145156972 | 145157019 | + | Homo sapiens | 47 |
| 196 | chr6 | UTRN | 145157440 | 145157555 | + | Homo sapiens | 115 |
| 197 | chr6 | UTRN | 145157566 | 145157621 | + | Homo sapiens | 55 |
| 198 | chr6 | UTRN | 145158149 | 145158194 | + | Homo sapiens | 45 |
| 199 | chr6 | UTRN | 145160356 | 145160409 | + | Homo sapiens | 53 |
| 200 | chr6 | UTRN | 145161881 | 145161927 | + | Homo sapiens | 46 |
| 201 | chr6 | UTRN | 145167107 | 145167151 | + | Homo sapiens | 44 |
| 202 | chr6 | UTRN | 145167205 | 145167243 | + | Homo sapiens | 38 |
| 203 | chr6 | UTRN | 145168292 | 145168344 | + | Homo sapiens | 52 |
| 204 | chr6 | UTRN | 145169033 | 145169085 | + | Homo sapiens | 52 |
| 205 | chr6 | UTRN | 145169156 | 145169202 | + | Homo sapiens | 46 |
| 206 | chr6 | UTRN | 145169270 | 145169315 | + | Homo sapiens | 45 |
| 207 | chr6 | UTRN | 145171814 | 145171863 | + | Homo sapiens | 49 |
| 208 | chr6 | UTRN | 145173631 | 145173718 | + | Homo sapiens | 87 |
| 209 | chr6 | UTRN | 144608489 | 144612535 | + | Homo sapiens | 4046 |
| 210 | chr6 | UTRN | 144610994 | 144615040 | + | Homo sapiens | 4046 |
| 211 | chr6 | UTRN | 144612120 | 144616162 | + | Homo sapiens | 4042 |
| 212 | chr6 | UTRN | 144612968 | 144617021 | + | Homo sapiens | 4053 |
| 213 | chr6 | UTRN | 144616862 | 144620901 | + | Homo sapiens | 4039 |
| 214 | chr6 | UTRN | 144619690 | 144623714 | + | Homo sapiens | 4024 |
| 215 | chr6 | UTRN | 144623028 | 144627096 | + | Homo sapiens | 4068 |
| 216 | chr6 | UTRN | 144623129 | 144627174 | + | Homo sapiens | 4045 |
| 217 | chr6 | UTRN | 144623319 | 144627379 | + | Homo sapiens | 4060 |
| 218 | chr6 | UTRN | 144626965 | 144631011 | + | Homo sapiens | 4046 |
| 219 | chr6 | UTRN | 144631818 | 144635869 | + | Homo sapiens | 4051 |
| 220 | chr6 | UTRN | 144631933 | 144635968 | + | Homo sapiens | 4035 |
| 221 | chr6 | UTRN | 144656267 | 144660302 | + | Homo sapiens | 4035 |
| 222 | chr6 | UTRN | 144683087 | 144687140 | + | Homo sapiens | 4053 |
| 223 | chr6 | UTRN | 144693039 | 144697063 | + | Homo sapiens | 4024 |
| 224 | chr6 | UTRN | 144697670 | 144701699 | + | Homo sapiens | 4029 |
| 225 | chr6 | UTRN | 144702043 | 144706398 | + | Homo sapiens | 4355 |
| 226 | chr6 | UTRN | 144704312 | 144708345 | + | Homo sapiens | 4033 |
| 227 | chr6 | UTRN | 144704654 | 144708704 | + | Homo sapiens | 4050 |
| 228 | chr6 | UTRN | 144719683 | 144723738 | + | Homo sapiens | 4055 |
| 229 | chr6 | UTRN | 144720580 | 144724627 | + | Homo sapiens | 4047 |
| 230 | chr6 | UTRN | 144722848 | 144726889 | + | Homo sapiens | 4041 |
| 231 | chr6 | UTRN | 144725897 | 144729947 | + | Homo sapiens | 4050 |
| 232 | chr6 | UTRN | 144744408 | 144748448 | + | Homo sapiens | 4040 |
| 233 | chr6 | UTRN | 144747102 | 144751152 | + | Homo sapiens | 4050 |
| 234 | chr6 | UTRN | 144747948 | 144752026 | + | Homo sapiens | 4078 |
| 235 | chr6 | UTRN | 144748728 | 144752789 | + | Homo sapiens | 4061 |
| 236 | chr6 | UTRN | 144748789 | 144752853 | + | Homo sapiens | 4064 |
| 237 | chr6 | UTRN | 144755162 | 144759214 | + | Homo sapiens | 4052 |
| 238 | chr6 | UTRN | 144755214 | 144759274 | + | Homo sapiens | 4060 |
| 239 | chr6 | UTRN | 144756752 | 144760807 | + | Homo sapiens | 4055 |
| 240 | chr6 | UTRN | 144756807 | 144760864 | + | Homo sapiens | 4057 |
| 241 | chr6 | UTRN | 144759513 | 144763579 | + | Homo sapiens | 4066 |
| 242 | chr6 | UTRN | 144763456 | 144767506 | + | Homo sapiens | 4050 |
| 243 | chr6 | UTRN | 144766420 | 144770461 | + | Homo sapiens | 4041 |
| 244 | chr6 | UTRN | 144766737 | 144770764 | + | Homo sapiens | 4027 |
| 245 | chr6 | UTRN | 144770549 | 144774627 | + | Homo sapiens | 4078 |
| 246 | chr6 | UTRN | 144772960 | 144777001 | + | Homo sapiens | 4041 |
| 247 | chr6 | UTRN | 144777914 | 144781960 | + | Homo sapiens | 4046 |
| 248 | chr6 | UTRN | 144778026 | 144782070 | + | Homo sapiens | 4044 |
| 249 | chr6 | UTRN | 144778278 | 144782324 | + | Homo sapiens | 4046 |
| 250 | chr6 | UTRN | 144781879 | 144785933 | + | Homo sapiens | 4054 |
| 251 | chr6 | UTRN | 144781933 | 144785995 | + | Homo sapiens | 4062 |
| 252 | chr6 | UTRN | 144798953 | 144802999 | + | Homo sapiens | 4046 |
| 253 | chr6 | UTRN | 144800780 | 144804822 | + | Homo sapiens | 4042 |
| 254 | chr6 | UTRN | 144801427 | 144805473 | + | Homo sapiens | 4046 |
| 255 | chr6 | UTRN | 144801714 | 144805742 | + | Homo sapiens | 4028 |
| 256 | chr6 | UTRN | 144801774 | 144805849 | + | Homo sapiens | 4075 |
| 257 | chr6 | UTRN | 144804673 | 144808714 | + | Homo sapiens | 4041 |
| 258 | chr6 | UTRN | 144806684 | 144810739 | + | Homo sapiens | 4055 |
| 259 | chr6 | UTRN | 144806739 | 144810803 | + | Homo sapiens | 4064 |
| 260 | chr6 | UTRN | 144807834 | 144811881 | + | Homo sapiens | 4047 |
| 261 | chr6 | UTRN | 144809266 | 144813310 | + | Homo sapiens | 4044 |
| 262 | chr6 | UTRN | 144812476 | 144816522 | + | Homo sapiens | 4046 |
| 263 | chr6 | UTRN | 144813156 | 144817199 | + | Homo sapiens | 4043 |
| 264 | chr6 | UTRN | 144818469 | 144822529 | + | Homo sapiens | 4060 |
| 265 | chr6 | UTRN | 144830193 | 144834243 | + | Homo sapiens | 4050 |
| 266 | chr6 | UTRN | 144833120 | 144837166 | + | Homo sapiens | 4046 |
| 267 | chr6 | UTRN | 144833862 | 144837907 | + | Homo sapiens | 4045 |
| 268 | chr6 | UTRN | 144835423 | 144839465 | + | Homo sapiens | 4042 |
| 269 | chr6 | UTRN | 144835943 | 144839989 | + | Homo sapiens | 4046 |
| 270 | chr6 | UTRN | 144842263 | 144846304 | + | Homo sapiens | 4041 |
| 271 | chr6 | UTRN | 144856722 | 144860798 | + | Homo sapiens | 4076 |
| 272 | chr6 | UTRN | 144868778 | 144872817 | + | Homo sapiens | 4039 |
| 273 | chr6 | UTRN | 144869113 | 144873154 | + | Homo sapiens | 4041 |
| 274 | chr6 | UTRN | 144870123 | 144874165 | + | Homo sapiens | 4042 |
| 275 | chr6 | UTRN | 144870599 | 144874669 | + | Homo sapiens | 4070 |
| 276 | chr6 | UTRN | 144871625 | 144875662 | + | Homo sapiens | 4037 |
| 277 | chr6 | UTRN | 144873136 | 144877182 | + | Homo sapiens | 4046 |
| 278 | chr6 | UTRN | 144873895 | 144877938 | + | Homo sapiens | 4043 |
| 279 | chr6 | UTRN | 144884315 | 144888378 | + | Homo sapiens | 4063 |
| 280 | chr6 | UTRN | 144902604 | 144906645 | + | Homo sapiens | 4041 |
| 281 | chr6 | UTRN | 144903276 | 144907300 | + | Homo sapiens | 4024 |
| 282 | chr6 | UTRN | 144904334 | 144908358 | + | Homo sapiens | 4024 |
| 283 | chr6 | UTRN | 144905025 | 144909055 | + | Homo sapiens | 4030 |
| 284 | chr6 | UTRN | 144906864 | 144910908 | + | Homo sapiens | 4044 |
| 285 | chr6 | UTRN | 144907055 | 144911078 | + | Homo sapiens | 4023 |
| 286 | chr6 | UTRN | 144908040 | 144912085 | + | Homo sapiens | 4045 |
| 287 | chr6 | UTRN | 144916990 | 144921013 | + | Homo sapiens | 4023 |
| 288 | chr6 | UTRN | 144933390 | 144937427 | + | Homo sapiens | 4037 |
| 289 | chr6 | UTRN | 144936199 | 144940248 | + | Homo sapiens | 4049 |
| 290 | chr6 | UTRN | 144939446 | 144943489 | + | Homo sapiens | 4043 |
| 291 | chr6 | UTRN | 144939551 | 144943596 | + | Homo sapiens | 4045 |
| 292 | chr6 | UTRN | 144939700 | 144943748 | + | Homo sapiens | 4048 |
| 293 | chr6 | UTRN | 144939856 | 144943912 | + | Homo sapiens | 4056 |
| 294 | chr6 | UTRN | 144939912 | 144943988 | + | Homo sapiens | 4076 |
| 295 | chr6 | UTRN | 144940080 | 144944136 | + | Homo sapiens | 4056 |
| 296 | chr6 | UTRN | 144942667 | 144946712 | + | Homo sapiens | 4045 |
| 297 | chr6 | UTRN | 144943160 | 144947207 | + | Homo sapiens | 4047 |
| 298 | chr6 | UTRN | 144948918 | 144952987 | + | Homo sapiens | 4069 |
| 299 | chr6 | UTRN | 144950595 | 144954650 | + | Homo sapiens | 4055 |
| 300 | chr6 | UTRN | 144952758 | 144956804 | + | Homo sapiens | 4046 |
| 301 | chr6 | UTRN | 144958952 | 144962996 | + | Homo sapiens | 4044 |
| 302 | chr6 | UTRN | 144966386 | 144970438 | + | Homo sapiens | 4052 |
| 303 | chr6 | UTRN | 144979668 | 144983709 | + | Homo sapiens | 4041 |
| 304 | chr6 | UTRN | 144983026 | 144987072 | + | Homo sapiens | 4046 |
| 305 | chr6 | UTRN | 144997637 | 145001686 | + | Homo sapiens | 4049 |
| 306 | chr6 | UTRN | 144997750 | 145001785 | + | Homo sapiens | 4035 |
| 307 | chr6 | UTRN | 144997904 | 145001956 | + | Homo sapiens | 4052 |
| 308 | chr6 | UTRN | 145010224 | 145014561 | + | Homo sapiens | 4337 |
| 309 | chr6 | UTRN | 145015897 | 145019969 | + | Homo sapiens | 4072 |
| 310 | chr6 | UTRN | 145015978 | 145020021 | + | Homo sapiens | 4043 |
| 311 | chr6 | UTRN | 145017229 | 145021261 | + | Homo sapiens | 4032 |
| 312 | chr6 | UTRN | 145019232 | 145023278 | + | Homo sapiens | 4046 |
| 313 | chr6 | UTRN | 145019336 | 145023382 | + | Homo sapiens | 4046 |
| 314 | chr6 | UTRN | 145029285 | 145033331 | + | Homo sapiens | 4046 |
| 315 | chr6 | UTRN | 145036424 | 145040467 | + | Homo sapiens | 4043 |
| 316 | chr6 | UTRN | 145040706 | 145044751 | + | Homo sapiens | 4045 |
| 317 | chr6 | UTRN | 145045775 | 145049820 | + | Homo sapiens | 4045 |
| 318 | chr6 | UTRN | 145049549 | 145053592 | + | Homo sapiens | 4043 |
| 319 | chr6 | UTRN | 145059899 | 145063941 | + | Homo sapiens | 4042 |
| 320 | chr6 | UTRN | 145061569 | 145065615 | + | Homo sapiens | 4046 |
| 321 | chr6 | UTRN | 145067445 | 145071497 | + | Homo sapiens | 4052 |
| 322 | chr6 | UTRN | 145070959 | 145075005 | + | Homo sapiens | 4046 |
| 323 | chr6 | UTRN | 145077115 | 145081162 | + | Homo sapiens | 4047 |
| 324 | chr6 | UTRN | 145077204 | 145081271 | + | Homo sapiens | 4067 |
| 325 | chr6 | UTRN | 145078780 | 145082816 | + | Homo sapiens | 4036 |
| 326 | chr6 | UTRN | 145078843 | 145082885 | + | Homo sapiens | 4042 |
| 327 | chr6 | UTRN | 145079884 | 145083930 | + | Homo sapiens | 4046 |
| 328 | chr6 | UTRN | 145085734 | 145089827 | + | Homo sapiens | 4093 |
| 329 | chr6 | UTRN | 145085827 | 145089911 | + | Homo sapiens | 4084 |
| 330 | chr6 | UTRN | 145086046 | 145090124 | + | Homo sapiens | 4078 |
| 331 | chr6 | UTRN | 145086210 | 145090245 | + | Homo sapiens | 4035 |
| 332 | chr6 | UTRN | 145086678 | 145090723 | + | Homo sapiens | 4045 |
| 333 | chr6 | UTRN | 145088281 | 145092327 | + | Homo sapiens | 4046 |
| 334 | chr6 | UTRN | 145088884 | 145092934 | + | Homo sapiens | 4050 |
| 335 | chr6 | UTRN | 145091542 | 145095591 | + | Homo sapiens | 4049 |
| 336 | chr6 | UTRN | 145092155 | 145096199 | + | Homo sapiens | 4044 |
| 337 | chr6 | UTRN | 145094714 | 145098756 | + | Homo sapiens | 4042 |
| 338 | chr6 | UTRN | 145099237 | 145103284 | + | Homo sapiens | 4047 |
| 339 | chr6 | UTRN | 145099598 | 145103642 | + | Homo sapiens | 4044 |
| 340 | chr6 | UTRN | 145125145 | 145129194 | + | Homo sapiens | 4049 |
| 341 | chr6 | UTRN | 145125467 | 145129507 | + | Homo sapiens | 4040 |
| 342 | chr6 | UTRN | 145125866 | 145129929 | + | Homo sapiens | 4063 |
| 343 | chr6 | UTRN | 145126105 | 145130160 | + | Homo sapiens | 4055 |
| 344 | chr6 | UTRN | 145126415 | 145130460 | + | Homo sapiens | 4045 |
| 345 | chr6 | UTRN | 145130350 | 145134397 | + | Homo sapiens | 4047 |
| 346 | chr6 | UTRN | 145130917 | 145134974 | + | Homo sapiens | 4057 |
| 347 | chr6 | UTRN | 145131779 | 145135802 | + | Homo sapiens | 4023 |
| 348 | chr6 | UTRN | 145132097 | 145136169 | + | Homo sapiens | 4072 |
| 349 | chr6 | UTRN | 145132835 | 145136881 | + | Homo sapiens | 4046 |
| 350 | chr6 | UTRN | 145140034 | 145144135 | + | Homo sapiens | 4101 |
| 351 | chr6 | UTRN | 145140629 | 145144676 | + | Homo sapiens | 4047 |
| 352 | chr6 | UTRN | 145146750 | 145150806 | + | Homo sapiens | 4056 |
| 353 | chr6 | UTRN | 145147936 | 145151984 | + | Homo sapiens | 4048 |
| 354 | chr6 | UTRN | 145151900 | 145156300 | + | Homo sapiens | 4400 |
| 355 | chr6 | UTRN | 145152735 | 145156782 | + | Homo sapiens | 4047 |
| 356 | chr6 | UTRN | 145153438 | 145157468 | + | Homo sapiens | 4030 |
| 357 | chr6 | UTRN | 145154972 | 145159019 | + | Homo sapiens | 4047 |
| 358 | chr6 | UTRN | 145155440 | 145159555 | + | Homo sapiens | 4115 |
| 359 | chr6 | UTRN | 145155566 | 145159621 | + | Homo sapiens | 4055 |
| 360 | chr6 | UTRN | 145156149 | 145160194 | + | Homo sapiens | 4045 |
| 361 | chr6 | UTRN | 145158356 | 145162409 | + | Homo sapiens | 4053 |
| 362 | chr6 | UTRN | 145159881 | 145163927 | + | Homo sapiens | 4046 |
| 363 | chr6 | UTRN | 145165107 | 145169151 | + | Homo sapiens | 4044 |
| 364 | chr6 | UTRN | 145165205 | 145169243 | + | Homo sapiens | 4038 |
| 365 | chr6 | UTRN | 145166292 | 145170344 | + | Homo sapiens | 4052 |
| 366 | chr6 | UTRN | 145167033 | 145171085 | + | Homo sapiens | 4052 |
| 367 | chr6 | UTRN | 145167156 | 145171202 | + | Homo sapiens | 4046 |
| 368 | chr6 | UTRN | 145167270 | 145171315 | + | Homo sapiens | 4045 |
| 369 | chr6 | UTRN | 145169814 | 145173863 | + | Homo sapiens | 4049 |
| 370 | chr6 | UTRN | 145171631 | 145175718 | + | Homo sapiens | 4087 |
| 371 | chr6 | UTRN | 144608031 | 144608073 | - | Homo sapiens | 42 |
| 372 | chr6 | UTRN | 144612926 | 144612972 | - | Homo sapiens | 46 |
| 373 | chr6 | UTRN | 144628552 | 144628595 | - | Homo sapiens | 43 |
| 374 | chr6 | UTRN | 144633946 | 144633970 | - | Homo sapiens | 24 |
| 375 | chr6 | UTRN | 144650739 | 144650804 | - | Homo sapiens | 65 |
| 376 | chr6 | UTRN | 144657045 | 144657093 | - | Homo sapiens | 48 |
| 377 | chr6 | UTRN | 144696995 | 144697041 | - | Homo sapiens | 46 |
| 378 | chr6 | UTRN | 144747612 | 144747674 | - | Homo sapiens | 62 |
| 379 | chr6 | UTRN | 144747879 | 144747925 | - | Homo sapiens | 46 |
| 380 | chr6 | UTRN | 144759816 | 144759863 | - | Homo sapiens | 47 |
| 381 | chr6 | UTRN | 144768238 | 144768312 | - | Homo sapiens | 74 |
| 382 | chr6 | UTRN | 144780036 | 144780082 | - | Homo sapiens | 46 |
| 383 | chr6 | UTRN | 144782886 | 144782935 | - | Homo sapiens | 49 |
| 384 | chr6 | UTRN | 144795766 | 144795789 | - | Homo sapiens | 23 |
| 385 | chr6 | UTRN | 144806556 | 144806601 | - | Homo sapiens | 45 |
| 386 | chr6 | UTRN | 144854365 | 144854401 | - | Homo sapiens | 36 |
| 387 | chr6 | UTRN | 144858769 | 144858809 | - | Homo sapiens | 40 |
| 388 | chr6 | UTRN | 144861763 | 144861805 | - | Homo sapiens | 42 |
| 389 | chr6 | UTRN | 144865560 | 144865594 | - | Homo sapiens | 34 |
| 390 | chr6 | UTRN | 144871095 | 144871118 | - | Homo sapiens | 23 |
| 391 | chr6 | UTRN | 144872146 | 144872179 | - | Homo sapiens | 33 |
| 392 | chr6 | UTRN | 144873792 | 144873815 | - | Homo sapiens | 23 |
| 393 | chr6 | UTRN | 144875726 | 144875775 | - | Homo sapiens | 49 |
| 394 | chr6 | UTRN | 144881389 | 144881429 | - | Homo sapiens | 40 |
| 395 | chr6 | UTRN | 144902992 | 144903093 | - | Homo sapiens | 101 |
| 396 | chr6 | UTRN | 144913242 | 144913292 | - | Homo sapiens | 50 |
| 397 | chr6 | UTRN | 144916606 | 144916629 | - | Homo sapiens | 23 |
| 398 | chr6 | UTRN | 144953033 | 144953075 | - | Homo sapiens | 42 |
| 399 | chr6 | UTRN | 144957938 | 144957985 | - | Homo sapiens | 47 |
| 400 | chr6 | UTRN | 144960849 | 144960900 | - | Homo sapiens | 51 |
| 401 | chr6 | UTRN | 144963737 | 144963802 | - | Homo sapiens | 65 |
| 402 | chr6 | UTRN | 144980957 | 144981000 | - | Homo sapiens | 43 |
| 403 | chr6 | UTRN | 144981226 | 144981271 | - | Homo sapiens | 45 |
| 404 | chr6 | UTRN | 144981350 | 144981396 | - | Homo sapiens | 46 |
| 405 | chr6 | UTRN | 144981507 | 144981542 | - | Homo sapiens | 35 |
| 406 | chr6 | UTRN | 144983660 | 144983707 | - | Homo sapiens | 47 |
| 407 | chr6 | UTRN | 145005066 | 145005095 | - | Homo sapiens | 29 |
| 408 | chr6 | UTRN | 145005500 | 145005548 | - | Homo sapiens | 48 |
| 409 | chr6 | UTRN | 145021339 | 145021384 | - | Homo sapiens | 45 |
| 410 | chr6 | UTRN | 145036068 | 145036136 | - | Homo sapiens | 68 |
| 411 | chr6 | UTRN | 145036766 | 145036820 | - | Homo sapiens | 54 |
| 412 | chr6 | UTRN | 145038552 | 145038606 | - | Homo sapiens | 54 |
| 413 | chr6 | UTRN | 145058056 | 145058096 | - | Homo sapiens | 40 |
| 414 | chr6 | UTRN | 145059402 | 145059450 | - | Homo sapiens | 48 |
| 415 | chr6 | UTRN | 145060834 | 145060905 | - | Homo sapiens | 71 |
| 416 | chr6 | UTRN | 145062448 | 145062475 | - | Homo sapiens | 27 |
| 417 | chr6 | UTRN | 145063125 | 145063160 | - | Homo sapiens | 35 |
| 418 | chr6 | UTRN | 145063273 | 145063302 | - | Homo sapiens | 29 |
| 419 | chr6 | UTRN | 145071318 | 145071359 | - | Homo sapiens | 41 |
| 420 | chr6 | UTRN | 145079495 | 145079543 | - | Homo sapiens | 48 |
| 421 | chr6 | UTRN | 145090227 | 145090266 | - | Homo sapiens | 39 |
| 422 | chr6 | UTRN | 145095420 | 145095465 | - | Homo sapiens | 45 |
| 423 | chr6 | UTRN | 145097191 | 145097232 | - | Homo sapiens | 41 |
| 424 | chr6 | UTRN | 145098097 | 145098128 | - | Homo sapiens | 31 |
| 425 | chr6 | UTRN | 145098960 | 145099005 | - | Homo sapiens | 45 |
| 426 | chr6 | UTRN | 145106983 | 145107024 | - | Homo sapiens | 41 |
| 427 | chr6 | UTRN | 145124174 | 145124220 | - | Homo sapiens | 46 |
| 428 | chr6 | UTRN | 145128278 | 145128325 | - | Homo sapiens | 47 |
| 429 | chr6 | UTRN | 145142105 | 145142152 | - | Homo sapiens | 47 |
| 430 | chr6 | UTRN | 145149926 | 145149972 | - | Homo sapiens | 46 |
| 431 | chr6 | UTRN | 145153110 | 145153155 | - | Homo sapiens | 45 |
| 432 | chr6 | UTRN | 145155586 | 145155641 | - | Homo sapiens | 55 |
| 433 | chr6 | UTRN | 145156956 | 145157020 | - | Homo sapiens | 64 |
| 434 | chr6 | UTRN | 145161886 | 145161931 | - | Homo sapiens | 45 |
| 435 | chr6 | UTRN | 145166490 | 145166527 | - | Homo sapiens | 37 |
| 436 | chr6 | UTRN | 145167701 | 145167736 | - | Homo sapiens | 35 |
| 437 | chr6 | UTRN | 145173585 | 145173627 | - | Homo sapiens | 42 |
| 438 | chr6 | UTRN | 144606031 | 144610073 | - | Homo sapiens | 4042 |
| 439 | chr6 | UTRN | 144610926 | 144614972 | - | Homo sapiens | 4046 |
| 440 | chr6 | UTRN | 144626552 | 144630595 | - | Homo sapiens | 4043 |
| 441 | chr6 | UTRN | 144631946 | 144635970 | - | Homo sapiens | 4024 |
| 442 | chr6 | UTRN | 144648739 | 144652804 | - | Homo sapiens | 4065 |
| 443 | chr6 | UTRN | 144655045 | 144659093 | - | Homo sapiens | 4048 |
| 444 | chr6 | UTRN | 144694995 | 144699041 | - | Homo sapiens | 4046 |
| 445 | chr6 | UTRN | 144745612 | 144749674 | - | Homo sapiens | 4062 |
| 446 | chr6 | UTRN | 144745879 | 144749925 | - | Homo sapiens | 4046 |
| 447 | chr6 | UTRN | 144757816 | 144761863 | - | Homo sapiens | 4047 |
| 448 | chr6 | UTRN | 144766238 | 144770312 | - | Homo sapiens | 4074 |
| 449 | chr6 | UTRN | 144778036 | 144782082 | - | Homo sapiens | 4046 |
| 450 | chr6 | UTRN | 144780886 | 144784935 | - | Homo sapiens | 4049 |
| 451 | chr6 | UTRN | 144793766 | 144797789 | - | Homo sapiens | 4023 |
| 452 | chr6 | UTRN | 144804556 | 144808601 | - | Homo sapiens | 4045 |
| 453 | chr6 | UTRN | 144852365 | 144856401 | - | Homo sapiens | 4036 |
| 454 | chr6 | UTRN | 144856769 | 144860809 | - | Homo sapiens | 4040 |
| 455 | chr6 | UTRN | 144859763 | 144863805 | - | Homo sapiens | 4042 |
| 456 | chr6 | UTRN | 144863560 | 144867594 | - | Homo sapiens | 4034 |
| 457 | chr6 | UTRN | 144869095 | 144873118 | - | Homo sapiens | 4023 |
| 458 | chr6 | UTRN | 144870146 | 144874179 | - | Homo sapiens | 4033 |
| 459 | chr6 | UTRN | 144871792 | 144875815 | - | Homo sapiens | 4023 |
| 460 | chr6 | UTRN | 144873726 | 144877775 | - | Homo sapiens | 4049 |
| 461 | chr6 | UTRN | 144879389 | 144883429 | - | Homo sapiens | 4040 |
| 462 | chr6 | UTRN | 144900992 | 144905093 | - | Homo sapiens | 4101 |
| 463 | chr6 | UTRN | 144911242 | 144915292 | - | Homo sapiens | 4050 |
| 464 | chr6 | UTRN | 144914606 | 144918629 | - | Homo sapiens | 4023 |
| 465 | chr6 | UTRN | 144951033 | 144955075 | - | Homo sapiens | 4042 |
| 466 | chr6 | UTRN | 144955938 | 144959985 | - | Homo sapiens | 4047 |
| 467 | chr6 | UTRN | 144958849 | 144962900 | - | Homo sapiens | 4051 |
| 468 | chr6 | UTRN | 144961737 | 144965802 | - | Homo sapiens | 4065 |
| 469 | chr6 | UTRN | 144978957 | 144983000 | - | Homo sapiens | 4043 |
| 470 | chr6 | UTRN | 144979226 | 144983271 | - | Homo sapiens | 4045 |
| 471 | chr6 | UTRN | 144979350 | 144983396 | - | Homo sapiens | 4046 |
| 472 | chr6 | UTRN | 144979507 | 144983542 | - | Homo sapiens | 4035 |
| 473 | chr6 | UTRN | 144981660 | 144985707 | - | Homo sapiens | 4047 |
| 474 | chr6 | UTRN | 145003066 | 145007095 | - | Homo sapiens | 4029 |
| 475 | chr6 | UTRN | 145003500 | 145007548 | - | Homo sapiens | 4048 |
| 476 | chr6 | UTRN | 145019339 | 145023384 | - | Homo sapiens | 4045 |
| 477 | chr6 | UTRN | 145034068 | 145038136 | - | Homo sapiens | 4068 |
| 478 | chr6 | UTRN | 145034766 | 145038820 | - | Homo sapiens | 4054 |
| 479 | chr6 | UTRN | 145036552 | 145040606 | - | Homo sapiens | 4054 |
| 480 | chr6 | UTRN | 145056056 | 145060096 | - | Homo sapiens | 4040 |
| 481 | chr6 | UTRN | 145057402 | 145061450 | - | Homo sapiens | 4048 |
| 482 | chr6 | UTRN | 145058834 | 145062905 | - | Homo sapiens | 4071 |
| 483 | chr6 | UTRN | 145060448 | 145064475 | - | Homo sapiens | 4027 |
| 484 | chr6 | UTRN | 145061125 | 145065160 | - | Homo sapiens | 4035 |
| 485 | chr6 | UTRN | 145061273 | 145065302 | - | Homo sapiens | 4029 |
| 486 | chr6 | UTRN | 145069318 | 145073359 | - | Homo sapiens | 4041 |
| 487 | chr6 | UTRN | 145077495 | 145081543 | - | Homo sapiens | 4048 |
| 488 | chr6 | UTRN | 145088227 | 145092266 | - | Homo sapiens | 4039 |
| 489 | chr6 | UTRN | 145093420 | 145097465 | - | Homo sapiens | 4045 |
| 490 | chr6 | UTRN | 145095191 | 145099232 | - | Homo sapiens | 4041 |
| 491 | chr6 | UTRN | 145096097 | 145100128 | - | Homo sapiens | 4031 |
| 492 | chr6 | UTRN | 145096960 | 145101005 | - | Homo sapiens | 4045 |
| 493 | chr6 | UTRN | 145104983 | 145109024 | - | Homo sapiens | 4041 |
| 494 | chr6 | UTRN | 145122174 | 145126220 | - | Homo sapiens | 4046 |
| 495 | chr6 | UTRN | 145126278 | 145130325 | - | Homo sapiens | 4047 |
| 496 | chr6 | UTRN | 145140105 | 145144152 | - | Homo sapiens | 4047 |
| 497 | chr6 | UTRN | 145147926 | 145151972 | - | Homo sapiens | 4046 |
| 498 | chr6 | UTRN | 145151110 | 145155155 | - | Homo sapiens | 4045 |
| 499 | chr6 | UTRN | 145153586 | 145157641 | - | Homo sapiens | 4055 |
| 500 | chr6 | UTRN | 145154956 | 145159020 | - | Homo sapiens | 4064 |
| 501 | chr6 | UTRN | 145159886 | 145163931 | - | Homo sapiens | 4045 |
| 502 | chr6 | UTRN | 145164490 | 145168527 | - | Homo sapiens | 4037 |
| 503 | chr6 | UTRN | 145165701 | 145169736 | - | Homo sapiens | 4035 |
| 504 | chr6 | UTRN | 145171585 | 145175627 | - | Homo sapiens | 4042 |
| 505 | chr11 | HBB | 5234695 | 5260301 | - | Homo sapiens | 25606 |
| 506 | chr11 | HBB | 5234695 | 5260301 | + | Homo sapiens | 25606 |
| 507 | chr11 | HBD | 5242058 | 5267858 | - | Homo sapiens | 25800 |
| 508 | chr11 | HBD | 5242058 | 5267858 | + | Homo sapiens | 25800 |
| 509 | chr11 | HBE1 | 5277579 | 5303373 | - | Homo sapiens | 25794 |
| 510 | chr11 | HBE1 | 5277579 | 5303373 | + | Homo sapiens | 25794 |
| 511 | chr11 | HBG1 | 5257501 | 5283087 | - | Homo sapiens | 25586 |
| 512 | chr11 | HBG1 | 5257501 | 5283087 | + | Homo sapiens | 25586 |
| 513 | chr11 | HBG2 | 5262420 | 5288011 | - | Homo sapiens | 25591 |
| 514 | chr11 | HBG2 | 5262420 | 5288011 | + | Homo sapiens | 25591 |
| 515 | chr7 | Hbb-b1 | 110949041 | 110974437 | - | Mus musculus | 25396 |
| 516 | chr7 | Hbb-b1 | 110949041 | 110974437 | + | Mus musculus | 25396 |
| 517 | chr7 | Hbb-bh1 | 110978151 | 111003676 | - | Mus musculus | 25525 |
| 518 | chr7 | Hbb-bh1 | 110978151 | 111003676 | + | Mus musculus | 25525 |
| 519 | chr7 | Hbb-y | 110988267 | 111013721 | - | Mus musculus | 25454 |
| 520 | chr7 | Hbb-y | 110988267 | 111013721 | + | Mus musculus | 25454 |
| 521 | chr11 | HBB/HBD | 5246366 | 5246414 | + | Homo sapiens | 48 |
| 522 | chr11 | HBB/HBD | 5244366 | 5248414 | + | Homo sapiens | 4048 |
| 523 | chr12 | ATP2A2 | 110707031 | 110800897 | + | Homo sapiens | 93866 |
| 524 | chr12 | ATP2A2 | 110707031 | 110800897 | - | Homo sapiens | 93866 |
| 525 | chr5 | Atp2a2 | 122891521 | 122964234 | - | Mus musculus | 72713 |
| 526 | chr5 | Atp2a2 | 122891521 | 122964234 | + | Mus musculus | 72713 |
| 527 | chr12 | ATP2A2 | 110719627 | 110719694 | + | Homo sapiens | 67 |
| 528 | chr12 | ATP2A2 | 110720529 | 110720579 | + | Homo sapiens | 50 |
| 529 | chr12 | ATP2A2 | 110721473 | 110721523 | + | Homo sapiens | 50 |
| 530 | chr12 | ATP2A2 | 110723314 | 110723392 | + | Homo sapiens | 78 |
| 531 | chr12 | ATP2A2 | 110725261 | 110725324 | + | Homo sapiens | 63 |
| 532 | chr12 | ATP2A2 | 110727087 | 110727134 | + | Homo sapiens | 47 |
| 533 | chr12 | ATP2A2 | 110729885 | 110729930 | + | Homo sapiens | 45 |
| 534 | chr12 | ATP2A2 | 110734433 | 110734479 | + | Homo sapiens | 46 |
| 535 | chr12 | ATP2A2 | 110764013 | 110764059 | + | Homo sapiens | 46 |
| 536 | chr12 | ATP2A2 | 110765378 | 110765425 | + | Homo sapiens | 47 |
| 537 | chr12 | ATP2A2 | 110765494 | 110765540 | + | Homo sapiens | 46 |
| 538 | chr12 | ATP2A2 | 110765734 | 110765819 | + | Homo sapiens | 85 |
| 539 | chr12 | ATP2A2 | 110770988 | 110771034 | + | Homo sapiens | 46 |
| 540 | chr12 | ATP2A2 | 110771832 | 110771877 | + | Homo sapiens | 45 |
| 541 | chr12 | ATP2A2 | 110777332 | 110777374 | + | Homo sapiens | 42 |
| 542 | chr12 | ATP2A2 | 110777482 | 110777528 | + | Homo sapiens | 46 |
| 543 | chr12 | ATP2A2 | 110778549 | 110778580 | + | Homo sapiens | 31 |
| 544 | chr12 | ATP2A2 | 110778678 | 110778748 | + | Homo sapiens | 70 |
| 545 | chr12 | ATP2A2 | 110780135 | 110780183 | + | Homo sapiens | 48 |
| 546 | chr12 | ATP2A2 | 110780320 | 110780386 | + | Homo sapiens | 66 |
| 547 | chr12 | ATP2A2 | 110781060 | 110781169 | + | Homo sapiens | 109 |
| 548 | chr12 | ATP2A2 | 110781169 | 110781235 | + | Homo sapiens | 66 |
| 549 | chr12 | ATP2A2 | 110782732 | 110782779 | + | Homo sapiens | 47 |
| 550 | chr12 | ATP2A2 | 110783060 | 110783131 | + | Homo sapiens | 71 |
| 551 | chr12 | ATP2A2 | 110783131 | 110783186 | + | Homo sapiens | 55 |
| 552 | chr12 | ATP2A2 | 110783143 | 110783843 | + | Homo sapiens | 700 |
| 553 | chr12 | ATP2A2 | 110783803 | 110783845 | + | Homo sapiens | 42 |
| 554 | chr12 | ATP2A2 | 110784061 | 110784090 | + | Homo sapiens | 29 |
| 555 | chr12 | ATP2A2 | 110784453 | 110784505 | + | Homo sapiens | 52 |
| 556 | chr12 | ATP2A2 | 110784577 | 110784623 | + | Homo sapiens | 46 |
| 557 | chr12 | ATP2A2 | 110784786 | 110784835 | + | Homo sapiens | 49 |
| 558 | chr12 | ATP2A2 | 110784919 | 110784969 | + | Homo sapiens | 50 |
| 559 | chr12 | ATP2A2 | 110788463 | 110788513 | + | Homo sapiens | 50 |
| 560 | chr12 | ATP2A2 | 110717627 | 110721694 | + | Homo sapiens | 4067 |
| 561 | chr12 | ATP2A2 | 110718529 | 110722579 | + | Homo sapiens | 4050 |
| 562 | chr12 | ATP2A2 | 110719473 | 110723523 | + | Homo sapiens | 4050 |
| 563 | chr12 | ATP2A2 | 110721314 | 110725392 | + | Homo sapiens | 4078 |
| 564 | chr12 | ATP2A2 | 110723261 | 110727324 | + | Homo sapiens | 4063 |
| 565 | chr12 | ATP2A2 | 110725087 | 110729134 | + | Homo sapiens | 4047 |
| 566 | chr12 | ATP2A2 | 110727885 | 110731930 | + | Homo sapiens | 4045 |
| 567 | chr12 | ATP2A2 | 110732433 | 110736479 | + | Homo sapiens | 4046 |
| 568 | chr12 | ATP2A2 | 110762013 | 110766059 | + | Homo sapiens | 4046 |
| 569 | chr12 | ATP2A2 | 110763378 | 110767425 | + | Homo sapiens | 4047 |
| 570 | chr12 | ATP2A2 | 110763494 | 110767540 | + | Homo sapiens | 4046 |
| 571 | chr12 | ATP2A2 | 110763734 | 110767819 | + | Homo sapiens | 4085 |
| 572 | chr12 | ATP2A2 | 110768988 | 110773034 | + | Homo sapiens | 4046 |
| 573 | chr12 | ATP2A2 | 110769832 | 110773877 | + | Homo sapiens | 4045 |
| 574 | chr12 | ATP2A2 | 110775332 | 110779374 | + | Homo sapiens | 4042 |
| 575 | chr12 | ATP2A2 | 110775482 | 110779528 | + | Homo sapiens | 4046 |
| 576 | chr12 | ATP2A2 | 110776549 | 110780580 | + | Homo sapiens | 4031 |
| 577 | chr12 | ATP2A2 | 110776678 | 110780748 | + | Homo sapiens | 4070 |
| 578 | chr12 | ATP2A2 | 110778135 | 110782183 | + | Homo sapiens | 4048 |
| 579 | chr12 | ATP2A2 | 110778320 | 110782386 | + | Homo sapiens | 4066 |
| 580 | chr12 | ATP2A2 | 110779060 | 110783169 | + | Homo sapiens | 4109 |
| 581 | chr12 | ATP2A2 | 110779169 | 110783235 | + | Homo sapiens | 4066 |
| 582 | chr12 | ATP2A2 | 110780732 | 110784779 | + | Homo sapiens | 4047 |
| 583 | chr12 | ATP2A2 | 110781060 | 110785131 | + | Homo sapiens | 4071 |
| 584 | chr12 | ATP2A2 | 110781131 | 110785186 | + | Homo sapiens | 4055 |
| 585 | chr12 | ATP2A2 | 110781143 | 110785843 | + | Homo sapiens | 4700 |
| 586 | chr12 | ATP2A2 | 110781803 | 110785845 | + | Homo sapiens | 4042 |
| 587 | chr12 | ATP2A2 | 110782061 | 110786090 | + | Homo sapiens | 4029 |
| 588 | chr12 | ATP2A2 | 110782453 | 110786505 | + | Homo sapiens | 4052 |
| 589 | chr12 | ATP2A2 | 110782577 | 110786623 | + | Homo sapiens | 4046 |
| 590 | chr12 | ATP2A2 | 110782786 | 110786835 | + | Homo sapiens | 4049 |
| 591 | chr12 | ATP2A2 | 110782919 | 110786969 | + | Homo sapiens | 4050 |
| 592 | chr12 | ATP2A2 | 110786463 | 110790513 | + | Homo sapiens | 4050 |
| 593 | chr12 | ATP2A2 | 110764239 | 110764284 | - | Homo sapiens | 45 |
| 594 | chr12 | ATP2A2 | 110771869 | 110771910 | - | Homo sapiens | 41 |
| 595 | chr12 | ATP2A2 | 110777311 | 110777357 | - | Homo sapiens | 46 |
| 596 | chr12 | ATP2A2 | 110778603 | 110778648 | - | Homo sapiens | 45 |
| 597 | chr12 | ATP2A2 | 110781134 | 110781180 | - | Homo sapiens | 46 |
| 598 | chr12 | ATP2A2 | 110783112 | 110783161 | - | Homo sapiens | 49 |
| 599 | chr12 | ATP2A2 | 110783116 | 110783161 | - | Homo sapiens | 45 |
| 600 | chr12 | ATP2A2 | 110784019 | 110784061 | - | Homo sapiens | 42 |
| 601 | chr12 | ATP2A2 | 110784142 | 110784184 | - | Homo sapiens | 42 |
| 602 | chr12 | ATP2A2 | 110762239 | 110766284 | - | Homo sapiens | 4045 |
| 603 | chr12 | ATP2A2 | 110769869 | 110773910 | - | Homo sapiens | 4041 |
| 604 | chr12 | ATP2A2 | 110775311 | 110779357 | - | Homo sapiens | 4046 |
| 605 | chr12 | ATP2A2 | 110776603 | 110780648 | - | Homo sapiens | 4045 |
| 606 | chr12 | ATP2A2 | 110779134 | 110783180 | - | Homo sapiens | 4046 |
| 607 | chr12 | ATP2A2 | 110781112 | 110785161 | - | Homo sapiens | 4049 |
| 608 | chr12 | ATP2A2 | 110781116 | 110785161 | - | Homo sapiens | 4045 |
| 609 | chr12 | ATP2A2 | 110782019 | 110786061 | - | Homo sapiens | 4042 |
| 610 | chr12 | ATP2A2 | 110782142 | 110786184 | - | Homo sapiens | 4042 |
| 611 | chr11 | APOA1 | 116694468 | 116720338 | - | Homo sapiens | 25870 |
| 612 | chr11 | APOA1 | 116694468 | 116720338 | + | Homo sapiens | 25870 |
| 613 | chr4 | Abca1 | 53031660 | 53184767 | - | Mus musculus | 153107 |
| 614 | chr4 | Abca1 | 53031660 | 53184767 | + | Mus musculus | 153107 |
| 615 | chr9 | ABCA1 | 107531283 | 107702527 | - | Homo sapiens | 171244 |
| 616 | chr9 | ABCA1 | 107531283 | 107702527 | + | Homo sapiens | 171244 |
| 617 | chr9 | Apoa1 | 46024712 | 46050549 | + | Mus musculus | 25837 |
| 618 | chr9 | Apoa1 | 46024712 | 46050549 | - | Mus musculus | 25837 |
| 619 | chr11 | APOA1 | 116703452 | 116703490 | - | Homo sapiens | 38 |
| 620 | chr11 | APOA1 | 116716006 | 116716043 | - | Homo sapiens | 37 |
| 621 | chr9 | ABCA1 | 107544853 | 107544898 | - | Homo sapiens | 45 |
| 622 | chr9 | ABCA1 | 107545317 | 107545373 | - | Homo sapiens | 56 |
| 623 | chr9 | ABCA1 | 107549175 | 107549221 | - | Homo sapiens | 46 |
| 624 | chr9 | ABCA1 | 107550274 | 107550320 | - | Homo sapiens | 46 |
| 625 | chr9 | ABCA1 | 107550704 | 107550746 | - | Homo sapiens | 42 |
| 626 | chr9 | ABCA1 | 107550769 | 107550815 | - | Homo sapiens | 46 |
| 627 | chr9 | ABCA1 | 107553424 | 107553449 | - | Homo sapiens | 25 |
| 628 | chr9 | ABCA1 | 107555135 | 107555180 | - | Homo sapiens | 45 |
| 629 | chr9 | ABCA1 | 107559478 | 107559514 | - | Homo sapiens | 36 |
| 630 | chr9 | ABCA1 | 107562144 | 107562189 | - | Homo sapiens | 45 |
| 631 | chr9 | ABCA1 | 107562811 | 107562856 | - | Homo sapiens | 45 |
| 632 | chr9 | ABCA1 | 107564383 | 107564429 | - | Homo sapiens | 46 |
| 633 | chr9 | ABCA1 | 107564542 | 107564589 | - | Homo sapiens | 47 |
| 634 | chr9 | ABCA1 | 107565573 | 107565597 | - | Homo sapiens | 24 |
| 635 | chr9 | ABCA1 | 107566955 | 107566997 | - | Homo sapiens | 42 |
| 636 | chr9 | ABCA1 | 107567799 | 107567822 | - | Homo sapiens | 23 |
| 637 | chr9 | ABCA1 | 107568594 | 107568642 | - | Homo sapiens | 48 |
| 638 | chr9 | ABCA1 | 107570966 | 107571012 | - | Homo sapiens | 46 |
| 639 | chr9 | ABCA1 | 107571761 | 107571807 | - | Homo sapiens | 46 |
| 640 | chr9 | ABCA1 | 107572492 | 107572538 | - | Homo sapiens | 46 |
| 641 | chr9 | ABCA1 | 107573100 | 107573151 | - | Homo sapiens | 51 |
| 642 | chr9 | ABCA1 | 107574024 | 107574054 | - | Homo sapiens | 30 |
| 643 | chr9 | ABCA1 | 107574852 | 107574906 | - | Homo sapiens | 54 |
| 644 | chr9 | ABCA1 | 107574906 | 107574968 | - | Homo sapiens | 62 |
| 645 | chr9 | ABCA1 | 107576399 | 107576466 | - | Homo sapiens | 67 |
| 646 | chr9 | ABCA1 | 107576708 | 107576754 | - | Homo sapiens | 46 |
| 647 | chr9 | ABCA1 | 107578270 | 107578349 | - | Homo sapiens | 79 |
| 648 | chr9 | ABCA1 | 107578369 | 107578447 | - | Homo sapiens | 78 |
| 649 | chr9 | ABCA1 | 107582257 | 107582303 | - | Homo sapiens | 46 |
| 650 | chr9 | ABCA1 | 107583704 | 107583756 | - | Homo sapiens | 52 |
| 651 | chr9 | ABCA1 | 107584821 | 107584867 | - | Homo sapiens | 46 |
| 652 | chr9 | ABCA1 | 107590239 | 107590277 | - | Homo sapiens | 38 |
| 653 | chr9 | ABCA1 | 107590789 | 107590834 | - | Homo sapiens | 45 |
| 654 | chr9 | ABCA1 | 107591342 | 107591392 | - | Homo sapiens | 50 |
| 655 | chr9 | ABCA1 | 107593256 | 107593298 | - | Homo sapiens | 42 |
| 656 | chr9 | ABCA1 | 107593396 | 107593433 | - | Homo sapiens | 37 |
| 657 | chr9 | ABCA1 | 107593922 | 107593967 | - | Homo sapiens | 45 |
| 658 | chr9 | ABCA1 | 107594977 | 107595047 | - | Homo sapiens | 70 |
| 659 | chr9 | ABCA1 | 107596375 | 107596423 | - | Homo sapiens | 48 |
| 660 | chr9 | ABCA1 | 107597784 | 107597829 | - | Homo sapiens | 45 |
| 661 | chr9 | ABCA1 | 107597907 | 107597985 | - | Homo sapiens | 78 |
| 662 | chr9 | ABCA1 | 107602215 | 107602260 | - | Homo sapiens | 45 |
| 663 | chr9 | ABCA1 | 107602653 | 107602701 | - | Homo sapiens | 48 |
| 664 | chr9 | ABCA1 | 107613685 | 107613749 | - | Homo sapiens | 64 |
| 665 | chr9 | ABCA1 | 107615130 | 107615165 | - | Homo sapiens | 35 |
| 666 | chr9 | ABCA1 | 107620268 | 107620352 | - | Homo sapiens | 84 |
| 667 | chr9 | ABCA1 | 107620400 | 107620445 | - | Homo sapiens | 45 |
| 668 | chr9 | ABCA1 | 107624149 | 107624175 | - | Homo sapiens | 26 |
| 669 | chr9 | ABCA1 | 107625349 | 107625393 | - | Homo sapiens | 44 |
| 670 | chr9 | ABCA1 | 107632152 | 107632259 | - | Homo sapiens | 107 |
| 671 | chr9 | ABCA1 | 107640145 | 107640188 | - | Homo sapiens | 43 |
| 672 | chr9 | ABCA1 | 107648733 | 107648779 | - | Homo sapiens | 46 |
| 673 | chr9 | ABCA1 | 107651606 | 107651651 | - | Homo sapiens | 45 |
| 674 | chr9 | ABCA1 | 107651838 | 107651884 | - | Homo sapiens | 46 |
| 675 | chr9 | ABCA1 | 107654768 | 107654818 | - | Homo sapiens | 50 |
| 676 | chr9 | ABCA1 | 107656823 | 107656846 | - | Homo sapiens | 23 |
| 677 | chr9 | ABCA1 | 107663411 | 107663449 | - | Homo sapiens | 38 |
| 678 | chr9 | ABCA1 | 107664297 | 107664344 | - | Homo sapiens | 47 |
| 679 | chr9 | ABCA1 | 107666346 | 107666399 | - | Homo sapiens | 53 |
| 680 | chr9 | ABCA1 | 107666418 | 107666515 | - | Homo sapiens | 97 |
| 681 | chr9 | ABCA1 | 107669296 | 107669353 | - | Homo sapiens | 57 |
| 682 | chr9 | ABCA1 | 107669453 | 107669501 | - | Homo sapiens | 48 |
| 683 | chr9 | ABCA1 | 107669654 | 107669754 | - | Homo sapiens | 100 |
| 684 | chr9 | ABCA1 | 107669789 | 107669837 | - | Homo sapiens | 48 |
| 685 | chr9 | ABCA1 | 107688863 | 107688904 | - | Homo sapiens | 41 |
| 686 | chr9 | ABCA1 | 107689641 | 107689696 | - | Homo sapiens | 55 |
| 687 | chr9 | ABCA1 | 107689892 | 107689938 | - | Homo sapiens | 46 |
| 688 | chr9 | ABCA1 | 107690078 | 107690126 | - | Homo sapiens | 48 |
| 689 | chr9 | ABCA1 | 107690345 | 107690386 | - | Homo sapiens | 41 |
| 690 | chrll | ABCA1 | 116701452 | 116705490 | - | Homo sapiens | 4038 |
| 691 | chrll | ABCA1 | 116714006 | 116718043 | - | Homo sapiens | 4037 |
| 692 | chr9 | ABCA1 | 107542853 | 107546898 | - | Homo sapiens | 4045 |
| 693 | chr9 | ABCA1 | 107543317 | 107547373 | - | Homo sapiens | 4056 |
| 694 | chr9 | ABCA1 | 107547175 | 107551221 | - | Homo sapiens | 4046 |
| 695 | chr9 | ABCA1 | 107548274 | 107552320 | - | Homo sapiens | 4046 |
| 696 | chr9 | ABCA1 | 107548704 | 107552746 | - | Homo sapiens | 4042 |
| 697 | chr9 | ABCA1 | 107548769 | 107552815 | - | Homo sapiens | 4046 |
| 698 | chr9 | ABCA1 | 107551424 | 107555449 | - | Homo sapiens | 4025 |
| 699 | chr9 | ABCA1 | 107553135 | 107557180 | - | Homo sapiens | 4045 |
| 700 | chr9 | ABCA1 | 107557478 | 107561514 | - | Homo sapiens | 4036 |
| 701 | chr9 | ABCA1 | 107560144 | 107564189 | - | Homo sapiens | 4045 |
| 702 | chr9 | ABCA1 | 107560811 | 107564856 | - | Homo sapiens | 4045 |
| 703 | chr9 | ABCA1 | 107562383 | 107566429 | - | Homo sapiens | 4046 |
| 704 | chr9 | ABCA1 | 107562542 | 107566589 | - | Homo sapiens | 4047 |
| 705 | chr9 | ABCA1 | 107563573 | 107567597 | - | Homo sapiens | 4024 |
| 706 | chr9 | ABCA1 | 107564955 | 107568997 | - | Homo sapiens | 4042 |
| 707 | chr9 | ABCA1 | 107565799 | 107569822 | - | Homo sapiens | 4023 |
| 708 | chr9 | ABCA1 | 107566594 | 107570642 | - | Homo sapiens | 4048 |
| 709 | chr9 | ABCA1 | 107568966 | 107573012 | - | Homo sapiens | 4046 |
| 710 | chr9 | ABCA1 | 107569761 | 107573807 | - | Homo sapiens | 4046 |
| 711 | chr9 | ABCA1 | 107570492 | 107574538 | - | Homo sapiens | 4046 |
| 712 | chr9 | ABCA1 | 107571100 | 107575151 | - | Homo sapiens | 4051 |
| 713 | chr9 | ABCA1 | 107572024 | 107576054 | - | Homo sapiens | 4030 |
| 714 | chr9 | ABCA1 | 107572852 | 107576906 | - | Homo sapiens | 4054 |
| 715 | chr9 | ABCA1 | 107572906 | 107576968 | - | Homo sapiens | 4062 |
| 716 | chr9 | ABCA1 | 107574399 | 107578466 | - | Homo sapiens | 4067 |
| 717 | chr9 | ABCA1 | 107574708 | 107578754 | - | Homo sapiens | 4046 |
| 718 | chr9 | ABCA1 | 107576270 | 107580349 | - | Homo sapiens | 4079 |
| 719 | chr9 | ABCA1 | 107576369 | 107580447 | - | Homo sapiens | 4078 |
| 720 | chr9 | ABCA1 | 107580257 | 107584303 | - | Homo sapiens | 4046 |
| 721 | chr9 | ABCA1 | 107581704 | 107585756 | - | Homo sapiens | 4052 |
| 722 | chr9 | ABCA1 | 107582821 | 107586867 | - | Homo sapiens | 4046 |
| 723 | chr9 | ABCA1 | 107588239 | 107592277 | - | Homo sapiens | 4038 |
| 724 | chr9 | ABCA1 | 107588789 | 107592834 | - | Homo sapiens | 4045 |
| 725 | chr9 | ABCA1 | 107589342 | 107593392 | - | Homo sapiens | 4050 |
| 726 | chr9 | ABCA1 | 107591256 | 107595298 | - | Homo sapiens | 4042 |
| 727 | chr9 | ABCA1 | 107591396 | 107595433 | - | Homo sapiens | 4037 |
| 728 | chr9 | ABCA1 | 107591922 | 107595967 | - | Homo sapiens | 4045 |
| 729 | chr9 | ABCA1 | 107592977 | 107597047 | - | Homo sapiens | 4070 |
| 730 | chr9 | ABCA1 | 107594375 | 107598423 | - | Homo sapiens | 4048 |
| 731 | chr9 | ABCA1 | 107595784 | 107599829 | - | Homo sapiens | 4045 |
| 732 | chr9 | ABCA1 | 107595907 | 107599985 | - | Homo sapiens | 4078 |
| 733 | chr9 | ABCA1 | 107600215 | 107604260 | - | Homo sapiens | 4045 |
| 734 | chr9 | ABCA1 | 107600653 | 107604701 | - | Homo sapiens | 4048 |
| 735 | chr9 | ABCA1 | 107611685 | 107615749 | - | Homo sapiens | 4064 |
| 736 | chr9 | ABCA1 | 107613130 | 107617165 | - | Homo sapiens | 4035 |
| 737 | chr9 | ABCA1 | 107618268 | 107622352 | - | Homo sapiens | 4084 |
| 738 | chr9 | ABCA1 | 107618400 | 107622445 | - | Homo sapiens | 4045 |
| 739 | chr9 | ABCA1 | 107622149 | 107626175 | - | Homo sapiens | 4026 |
| 740 | chr9 | ABCA1 | 107623349 | 107627393 | - | Homo sapiens | 4044 |
| 741 | chr9 | ABCA1 | 107630152 | 107634259 | - | Homo sapiens | 4107 |
| 742 | chr9 | ABCA1 | 107638145 | 107642188 | - | Homo sapiens | 4043 |
| 743 | chr9 | ABCA1 | 107646733 | 107650779 | - | Homo sapiens | 4046 |
| 744 | chr9 | ABCA1 | 107649606 | 107653651 | - | Homo sapiens | 4045 |
| 745 | chr9 | ABCA1 | 107649838 | 107653884 | - | Homo sapiens | 4046 |
| 746 | chr9 | ABCA1 | 107652768 | 107656818 | - | Homo sapiens | 4050 |
| 747 | chr9 | ABCA1 | 107654823 | 107658846 | - | Homo sapiens | 4023 |
| 748 | chr9 | ABCA1 | 107661411 | 107665449 | - | Homo sapiens | 4038 |
| 749 | chr9 | ABCA1 | 107662297 | 107666344 | - | Homo sapiens | 4047 |
| 750 | chr9 | ABCA1 | 107664346 | 107668399 | - | Homo sapiens | 4053 |
| 751 | chr9 | ABCA1 | 107664418 | 107668515 | - | Homo sapiens | 4097 |
| 752 | chr9 | ABCA1 | 107667296 | 107671353 | - | Homo sapiens | 4057 |
| 753 | chr9 | ABCA1 | 107667453 | 107671501 | - | Homo sapiens | 4048 |
| 754 | chr9 | ABCA1 | 107667654 | 107671754 | - | Homo sapiens | 4100 |
| 755 | chr9 | ABCA1 | 107667789 | 107671837 | - | Homo sapiens | 4048 |
| 756 | chr9 | ABCA1 | 107686863 | 107690904 | - | Homo sapiens | 4041 |
| 757 | chr9 | ABCA1 | 107687641 | 107691696 | - | Homo sapiens | 4055 |
| 758 | chr9 | ABCA1 | 107687892 | 107691938 | - | Homo sapiens | 4046 |
| 759 | chr9 | ABCA1 | 107688078 | 107692126 | - | Homo sapiens | 4048 |
| 760 | chr9 | ABCA1 | 107688345 | 107692386 | - | Homo sapiens | 4041 |
| 761 | chr11 | ABCA1 | 116707870 | 116707947 | + | Homo sapiens | 77 |
| 762 | chr11 | ABCA1 | 116714416 | 116714458 | + | Homo sapiens | 42 |
| 763 | chr11 | ABCA1 | 116714536 | 116714578 | + | Homo sapiens | 42 |
| 764 | chr11 | ABCA1 | 116714762 | 116714821 | + | Homo sapiens | 59 |
| 765 | chr9 | ABCA1 | 107535234 | 107535273 | + | Homo sapiens | 39 |
| 766 | chr9 | ABCA1 | 107544880 | 107544926 | + | Homo sapiens | 46 |
| 767 | chr9 | ABCA1 | 107547830 | 107547871 | + | Homo sapiens | 41 |
| 768 | chr9 | ABCA1 | 107555512 | 107555554 | + | Homo sapiens | 42 |
| 769 | chr9 | ABCA1 | 107565561 | 107565603 | + | Homo sapiens | 42 |
| 770 | chr9 | ABCA1 | 107573103 | 107573142 | + | Homo sapiens | 39 |
| 771 | chr9 | ABCA1 | 107599184 | 107599234 | + | Homo sapiens | 50 |
| 772 | chr9 | ABCA1 | 107601126 | 107601170 | + | Homo sapiens | 44 |
| 773 | chr9 | ABCA1 | 107607774 | 107607819 | + | Homo sapiens | 45 |
| 774 | chr9 | ABCA1 | 107613720 | 107613783 | + | Homo sapiens | 63 |
| 775 | chr9 | ABCA1 | 107623957 | 107624003 | + | Homo sapiens | 46 |
| 776 | chr9 | ABCA1 | 107630025 | 107630076 | + | Homo sapiens | 51 |
| 777 | chr9 | ABCA1 | 107631197 | 107631265 | + | Homo sapiens | 68 |
| 778 | chr9 | ABCA1 | 107633948 | 107633996 | + | Homo sapiens | 48 |
| 779 | chr9 | ABCA1 | 107648816 | 107648890 | + | Homo sapiens | 74 |
| 780 | chr9 | ABCA1 | 107650500 | 107650558 | + | Homo sapiens | 58 |
| 781 | chr9 | ABCA1 | 107665550 | 107665591 | + | Homo sapiens | 41 |
| 782 | chr9 | ABCA1 | 107666033 | 107666073 | + | Homo sapiens | 40 |
| 783 | chr11 | ABCA1 | 116705870 | 116709947 | + | Homo sapiens | 4077 |
| 784 | chr11 | ABCA1 | 116712416 | 116716458 | + | Homo sapiens | 4042 |
| 785 | chr11 | ABCA1 | 116712536 | 116716578 | + | Homo sapiens | 4042 |
| 786 | chr11 | ABCA1 | 116712762 | 116716821 | + | Homo sapiens | 4059 |
| 787 | chr9 | ABCA1 | 107533234 | 107537273 | + | Homo sapiens | 4039 |
| 788 | chr9 | ABCA1 | 107542880 | 107546926 | + | Homo sapiens | 4046 |
| 789 | chr9 | ABCA1 | 107545830 | 107549871 | + | Homo sapiens | 4041 |
| 790 | chr9 | ABCA1 | 107553512 | 107557554 | + | Homo sapiens | 4042 |
| 791 | chr9 | ABCA1 | 107563561 | 107567603 | + | Homo sapiens | 4042 |
| 792 | chr9 | ABCA1 | 107571103 | 107575142 | + | Homo sapiens | 4039 |
| 793 | chr9 | ABCA1 | 107597184 | 107601234 | + | Homo sapiens | 4050 |
| 794 | chr9 | ABCA1 | 107599126 | 107603170 | + | Homo sapiens | 4044 |
| 795 | chr9 | ABCA1 | 107605774 | 107609819 | + | Homo sapiens | 4045 |
| 796 | chr9 | ABCA1 | 107611720 | 107615783 | + | Homo sapiens | 4063 |
| 797 | chr9 | ABCA1 | 107621957 | 107626003 | + | Homo sapiens | 4046 |
| 798 | chr9 | ABCA1 | 107628025 | 107632076 | + | Homo sapiens | 4051 |
| 799 | chr9 | ABCA1 | 107629197 | 107633265 | + | Homo sapiens | 4068 |
| 800 | chr9 | ABCA1 | 107631948 | 107635996 | + | Homo sapiens | 4048 |
| 801 | chr9 | ABCA1 | 107646816 | 107650890 | + | Homo sapiens | 4074 |
| 802 | chr9 | ABCA1 | 107648500 | 107652558 | + | Homo sapiens | 4058 |
| 803 | chr9 | ABCA1 | 107663550 | 107667591 | + | Homo sapiens | 4041 |
| 804 | chr9 | ABCA1 | 107664033 | 107668073 | + | Homo sapiens | 4040 |
| 805 | chr10 | PTEN | 89611194 | 89740532 | + | Homo sapiens | 129338 |
| 806 | chr10 | PTEN | 89611194 | 89740532 | - | Homo sapiens | 129338 |
| 807 | chr19 | Pten | 32820066 | 32912650 | + | Mus musculus | 92584 |
| 808 | chr19 | Pten | 32820066 | 32912650 | - | Mus musculus | 92584 |
| 809 | chr10 | PTEN | 89624228 | 89624270 | + | Homo sapiens | 42 |
| 810 | chr10 | PTEN | 89624833 | 89624878 | + | Homo sapiens | 45 |
| 811 | chr10 | PTEN | 89624926 | 89624970 | + | Homo sapiens | 44 |
| 812 | chr10 | PTEN | 89625394 | 89625442 | + | Homo sapiens | 48 |
| 813 | chr10 | PTEN | 89625544 | 89625602 | + | Homo sapiens | 58 |
| 814 | chr10 | PTEN | 89625817 | 89625863 | + | Homo sapiens | 46 |
| 815 | chr10 | PTEN | 89625913 | 89625938 | + | Homo sapiens | 25 |
| 816 | chr10 | PTEN | 89625981 | 89626027 | + | Homo sapiens | 46 |
| 817 | chr10 | PTEN | 89626204 | 89626244 | + | Homo sapiens | 40 |
| 818 | chr10 | PTEN | 89626597 | 89626641 | + | Homo sapiens | 44 |
| 819 | chr10 | PTEN | 89626724 | 89626775 | + | Homo sapiens | 51 |
| 820 | chr10 | PTEN | 89626875 | 89626911 | + | Homo sapiens | 36 |
| 821 | chr10 | PTEN | 89627125 | 89627169 | + | Homo sapiens | 44 |
| 822 | chr10 | PTEN | 89628194 | 89628243 | + | Homo sapiens | 49 |
| 823 | chr10 | PTEN | 89630898 | 89630936 | + | Homo sapiens | 38 |
| 824 | chr10 | PTEN | 89633768 | 89633831 | + | Homo sapiens | 63 |
| 825 | chr10 | PTEN | 89637731 | 89637778 | + | Homo sapiens | 47 |
| 826 | chr10 | PTEN | 89655949 | 89655994 | + | Homo sapiens | 45 |
| 827 | chr10 | PTEN | 89685417 | 89685446 | + | Homo sapiens | 29 |
| 828 | chr10 | PTEN | 89686532 | 89686579 | + | Homo sapiens | 47 |
| 829 | chr10 | PTEN | 89686846 | 89686893 | + | Homo sapiens | 47 |
| 830 | chr10 | PTEN | 89690160 | 89690213 | + | Homo sapiens | 53 |
| 831 | chr10 | PTEN | 89691658 | 89691701 | + | Homo sapiens | 43 |
| 832 | chr10 | PTEN | 89692927 | 89692973 | + | Homo sapiens | 46 |
| 833 | chr10 | PTEN | 89693941 | 89693990 | + | Homo sapiens | 49 |
| 834 | chr10 | PTEN | 89695260 | 89695313 | + | Homo sapiens | 53 |
| 835 | chr10 | PTEN | 89695827 | 89695873 | + | Homo sapiens | 46 |
| 836 | chr10 | PTEN | 89697310 | 89697355 | + | Homo sapiens | 45 |
| 837 | chr10 | PTEN | 89698069 | 89698110 | + | Homo sapiens | 41 |
| 838 | chr10 | PTEN | 89698500 | 89698543 | + | Homo sapiens | 43 |
| 839 | chr10 | PTEN | 89698790 | 89698828 | + | Homo sapiens | 38 |
| 840 | chr10 | PTEN | 89699611 | 89699656 | + | Homo sapiens | 45 |
| 841 | chr10 | PTEN | 89700446 | 89700493 | + | Homo sapiens | 47 |
| 842 | chr10 | PTEN | 89700876 | 89700919 | + | Homo sapiens | 43 |
| 843 | chr10 | PTEN | 89701325 | 89701377 | + | Homo sapiens | 52 |
| 844 | chr10 | PTEN | 89701617 | 89701717 | + | Homo sapiens | 100 |
| 845 | chr10 | PTEN | 89701764 | 89701818 | + | Homo sapiens | 54 |
| 846 | chr10 | PTEN | 89701915 | 89701962 | + | Homo sapiens | 47 |
| 847 | chr10 | PTEN | 89712065 | 89712111 | + | Homo sapiens | 46 |
| 848 | chr10 | PTEN | 89712351 | 89712402 | + | Homo sapiens | 51 |
| 849 | chr10 | PTEN | 89712411 | 89712510 | + | Homo sapiens | 99 |
| 850 | chr10 | PTEN | 89714201 | 89714228 | + | Homo sapiens | 27 |
| 851 | chr10 | PTEN | 89717191 | 89717238 | + | Homo sapiens | 47 |
| 852 | chr10 | PTEN | 89720717 | 89720765 | + | Homo sapiens | 48 |
| 853 | chr10 | PTEN | 89723393 | 89723443 | + | Homo sapiens | 50 |
| 854 | chr10 | PTEN | 89725518 | 89725564 | + | Homo sapiens | 46 |
| 855 | chr10 | PTEN | 89725617 | 89725658 | + | Homo sapiens | 41 |
| 856 | chr10 | PTEN | 89725819 | 89725865 | + | Homo sapiens | 46 |
| 857 | chr10 | PTEN | 89726333 | 89726368 | + | Homo sapiens | 35 |
| 858 | chr10 | PTEN | 89726640 | 89726709 | + | Homo sapiens | 69 |
| 859 | chr10 | PTEN | 89727525 | 89727567 | + | Homo sapiens | 42 |
| 860 | chr10 | PTEN | 89727527 | 89727569 | + | Homo sapiens | 42 |
| 861 | chr10 | PTEN | 89728125 | 89728171 | + | Homo sapiens | 46 |
| 862 | chr10 | PTEN | 89728126 | 89728170 | + | Homo sapiens | 44 |
| 863 | chr10 | PTEN | 89728128 | 89728172 | + | Homo sapiens | 44 |
| 864 | chr10 | PTEN | 89730064 | 89730112 | + | Homo sapiens | 48 |
| 865 | chr10 | PTEN | 89730269 | 89730384 | + | Homo sapiens | 115 |
| 866 | chr10 | PTEN | 89622228 | 89626270 | + | Homo sapiens | 4042 |
| 867 | chr10 | PTEN | 89622833 | 89626878 | + | Homo sapiens | 4045 |
| 868 | chr10 | PTEN | 89622926 | 89626970 | + | Homo sapiens | 4044 |
| 869 | chr10 | PTEN | 89623394 | 89627442 | + | Homo sapiens | 4048 |
| 870 | chr10 | PTEN | 89623544 | 89627602 | + | Homo sapiens | 4058 |
| 871 | chr10 | PTEN | 89623817 | 89627863 | + | Homo sapiens | 4046 |
| 872 | chr10 | PTEN | 89623913 | 89627938 | + | Homo sapiens | 4025 |
| 873 | chr10 | PTEN | 89623981 | 89628027 | + | Homo sapiens | 4046 |
| 874 | chr10 | PTEN | 89624204 | 89628244 | + | Homo sapiens | 4040 |
| 875 | chr10 | PTEN | 89624597 | 89628641 | + | Homo sapiens | 4044 |
| 876 | chr10 | PTEN | 89624724 | 89628775 | + | Homo sapiens | 4051 |
| 877 | chr10 | PTEN | 89624875 | 89628911 | + | Homo sapiens | 4036 |
| 878 | chr10 | PTEN | 89625125 | 89629169 | + | Homo sapiens | 4044 |
| 879 | chr10 | PTEN | 89626194 | 89630243 | + | Homo sapiens | 4049 |
| 880 | chr10 | PTEN | 89628898 | 89632936 | + | Homo sapiens | 4038 |
| 881 | chr10 | PTEN | 89631768 | 89635831 | + | Homo sapiens | 4063 |
| 882 | chr10 | PTEN | 89635731 | 89639778 | + | Homo sapiens | 4047 |
| 883 | chr10 | PTEN | 89653949 | 89657994 | + | Homo sapiens | 4045 |
| 884 | chr10 | PTEN | 89683417 | 89687446 | + | Homo sapiens | 4029 |
| 885 | chr10 | PTEN | 89684532 | 89688579 | + | Homo sapiens | 4047 |
| 886 | chr10 | PTEN | 89684846 | 89688893 | + | Homo sapiens | 4047 |
| 887 | chr10 | PTEN | 89688160 | 89692213 | + | Homo sapiens | 4053 |
| 888 | chr10 | PTEN | 89689658 | 89693701 | + | Homo sapiens | 4043 |
| 889 | chr10 | PTEN | 89690927 | 89694973 | + | Homo sapiens | 4046 |
| 890 | chr10 | PTEN | 89691941 | 89695990 | + | Homo sapiens | 4049 |
| 891 | chr10 | PTEN | 89693260 | 89697313 | + | Homo sapiens | 4053 |
| 892 | chr10 | PTEN | 89693827 | 89697873 | + | Homo sapiens | 4046 |
| 893 | chr10 | PTEN | 89695310 | 89699355 | + | Homo sapiens | 4045 |
| 894 | chr10 | PTEN | 89696069 | 89700110 | + | Homo sapiens | 4041 |
| 895 | chr10 | PTEN | 89696500 | 89700543 | + | Homo sapiens | 4043 |
| 896 | chr10 | PTEN | 89696790 | 89700828 | + | Homo sapiens | 4038 |
| 897 | chr10 | PTEN | 89697611 | 89701656 | + | Homo sapiens | 4045 |
| 898 | chr10 | PTEN | 89698446 | 89702493 | + | Homo sapiens | 4047 |
| 899 | chr10 | PTEN | 89698876 | 89702919 | + | Homo sapiens | 4043 |
| 900 | chr10 | PTEN | 89699325 | 89703377 | + | Homo sapiens | 4052 |
| 901 | chr10 | PTEN | 89699617 | 89703717 | + | Homo sapiens | 4100 |
| 902 | chr10 | PTEN | 89699764 | 89703818 | + | Homo sapiens | 4054 |
| 903 | chr10 | PTEN | 89699915 | 89703962 | + | Homo sapiens | 4047 |
| 904 | chr10 | PTEN | 89710065 | 89714111 | + | Homo sapiens | 4046 |
| 905 | chr10 | PTEN | 89710351 | 89714402 | + | Homo sapiens | 4051 |
| 906 | chr10 | PTEN | 89710411 | 89714510 | + | Homo sapiens | 4099 |
| 907 | chr10 | PTEN | 89712201 | 89716228 | + | Homo sapiens | 4027 |
| 908 | chr10 | PTEN | 89715191 | 89719238 | + | Homo sapiens | 4047 |
| 909 | chr10 | PTEN | 89718717 | 89722765 | + | Homo sapiens | 4048 |
| 910 | chr10 | PTEN | 89721393 | 89725443 | + | Homo sapiens | 4050 |
| 911 | chr10 | PTEN | 89723518 | 89727564 | + | Homo sapiens | 4046 |
| 912 | chr10 | PTEN | 89723617 | 89727658 | + | Homo sapiens | 4041 |
| 913 | chr10 | PTEN | 89723819 | 89727865 | + | Homo sapiens | 4046 |
| 914 | chr10 | PTEN | 89724333 | 89728368 | + | Homo sapiens | 4035 |
| 915 | chr10 | PTEN | 89724640 | 89728709 | + | Homo sapiens | 4069 |
| 916 | chr10 | PTEN | 89725525 | 89729567 | + | Homo sapiens | 4042 |
| 917 | chr10 | PTEN | 89725527 | 89729569 | + | Homo sapiens | 4042 |
| 918 | chr10 | PTEN | 89726125 | 89730171 | + | Homo sapiens | 4046 |
| 919 | chr10 | PTEN | 89726126 | 89730170 | + | Homo sapiens | 4044 |
| 920 | chr10 | PTEN | 89726128 | 89730172 | + | Homo sapiens | 4044 |
| 921 | chr10 | PTEN | 89728064 | 89732112 | + | Homo sapiens | 4048 |
| 922 | chr10 | PTEN | 89728269 | 89732384 | + | Homo sapiens | 4115 |
| 923 | chr10 | PTEN | 89623576 | 89623622 | - | Homo sapiens | 46 |
| 924 | chr10 | PTEN | 89623906 | 89623956 | - | Homo sapiens | 50 |
| 925 | chr10 | PTEN | 89624031 | 89624073 | - | Homo sapiens | 42 |
| 926 | chr10 | PTEN | 89624202 | 89624247 | - | Homo sapiens | 45 |
| 927 | chr10 | PTEN | 89624760 | 89624805 | - | Homo sapiens | 45 |
| 928 | chr10 | PTEN | 89625073 | 89625113 | - | Homo sapiens | 40 |
| 929 | chr10 | PTEN | 89628887 | 89628953 | - | Homo sapiens | 66 |
| 930 | chr10 | PTEN | 89665539 | 89665573 | - | Homo sapiens | 34 |
| 931 | chr10 | PTEN | 89692964 | 89693006 | - | Homo sapiens | 42 |
| 932 | chr10 | PTEN | 89695528 | 89695586 | - | Homo sapiens | 58 |
| 933 | chr10 | PTEN | 89695765 | 89695876 | - | Homo sapiens | 111 |
| 934 | chr10 | PTEN | 89695889 | 89695911 | - | Homo sapiens | 22 |
| 935 | chr10 | PTEN | 89697361 | 89697418 | - | Homo sapiens | 57 |
| 936 | chr10 | PTEN | 89697767 | 89697812 | - | Homo sapiens | 45 |
| 937 | chr10 | PTEN | 89721856 | 89721896 | - | Homo sapiens | 40 |
| 938 | chr10 | PTEN | 89621576 | 89625622 | - | Homo sapiens | 4046 |
| 939 | chr10 | PTEN | 89621906 | 89625956 | - | Homo sapiens | 4050 |
| 940 | chr10 | PTEN | 89622031 | 89626073 | - | Homo sapiens | 4042 |
| 941 | chr10 | PTEN | 89622202 | 89626247 | - | Homo sapiens | 4045 |
| 942 | chr10 | PTEN | 89622760 | 89626805 | - | Homo sapiens | 4045 |
| 943 | chr10 | PTEN | 89623073 | 89627113 | - | Homo sapiens | 4040 |
| 944 | chr10 | PTEN | 89626887 | 89630953 | - | Homo sapiens | 4066 |
| 945 | chr10 | PTEN | 89663539 | 89667573 | - | Homo sapiens | 4034 |
| 946 | chr10 | PTEN | 89690964 | 89695006 | - | Homo sapiens | 4042 |
| 947 | chr10 | PTEN | 89693528 | 89697586 | - | Homo sapiens | 4058 |
| 948 | chr10 | PTEN | 89693765 | 89697876 | - | Homo sapiens | 4111 |
| 949 | chr10 | PTEN | 89693889 | 89697911 | - | Homo sapiens | 4022 |
| 950 | chr10 | PTEN | 89695361 | 89699418 | - | Homo sapiens | 4057 |
| 951 | chr10 | PTEN | 89695767 | 89699812 | - | Homo sapiens | 4045 |
| 952 | chr10 | PTEN | 89719856 | 89723896 | - | Homo sapiens | 4040 |
| 953 | chr11 | BDNF | 27664441 | 27693196 | - | Homo sapiens | 28755 |
| 954 | chr11 | BDNF | 27664441 | 27693196 | + | Homo sapiens | 28755 |
| 955 | chr11 | BDNF-AS1 | 27516398 | 27731718 | + | Homo sapiens | 215320 |
| 956 | chr11 | BDNF-AS1 | 27516398 | 27731718 | - | Homo sapiens | 215320 |
| 957 | chr2 | Bdnf | 109502856 | 109579200 | + | Mus musculus | 76344 |
| 958 | chr2 | Bdnf | 109502856 | 109579200 | - | Mus musculus | 76344 |
| 959 | chr11 | BDNF | 27678819 | 27678888 | - | Homo sapiens | 69 |
| 960 | chr11 | BDNF | 27679423 | 27679469 | - | Homo sapiens | 46 |
| 961 | chr11 | BDNF | 27679512 | 27679558 | - | Homo sapiens | 46 |
| 962 | chr11 | BDNF | 27679705 | 27679749 | - | Homo sapiens | 44 |
| 963 | chr11 | BDNF | 27686657 | 27686742 | - | Homo sapiens | 85 |
| 964 | chr11 | BDNF | 27718502 | 27718548 | - | Homo sapiens | 46 |
| 965 | chr11 | BDNF | 27719743 | 27719780 | - | Homo sapiens | 37 |
| 966 | chr11 | BDNF | 27721391 | 27721434 | - | Homo sapiens | 43 |
| 967 | chr11 | BDNF | 27676819 | 27680888 | - | Homo sapiens | 4069 |
| 968 | chr11 | BDNF | 27677423 | 27681469 | - | Homo sapiens | 4046 |
| 969 | chr11 | BDNF | 27677512 | 27681558 | - | Homo sapiens | 4046 |
| 970 | chr11 | BDNF | 27677705 | 27681749 | - | Homo sapiens | 4044 |
| 971 | chr11 | BDNF | 27684657 | 27688742 | - | Homo sapiens | 4085 |
| 972 | chr11 | BDNF | 27716502 | 27720548 | - | Homo sapiens | 4046 |
| 973 | chr11 | BDNF | 27717743 | 27721780 | - | Homo sapiens | 4037 |
| 974 | chr11 | BDNF | 27719391 | 27723434 | - | Homo sapiens | 4043 |
| 975 | chr11 | BDNF | 27739230 | 27739276 | - | Homo sapiens | 46 |
| 976 | chr11 | BDNF | 27741576 | 27741622 | - | Homo sapiens | 46 |
| 977 | chr11 | BDNF | 27742481 | 27742526 | - | Homo sapiens | 45 |
| 978 | chr11 | BDNF | 27742552 | 27742602 | - | Homo sapiens | 50 |
| 979 | chr11 | BDNF | 27737230 | 27741276 | - | Homo sapiens | 4046 |
| 980 | chr11 | BDNF | 27739576 | 27743622 | - | Homo sapiens | 4046 |
| 981 | chr11 | BDNF | 27740481 | 27744526 | - | Homo sapiens | 4045 |
| 982 | chr11 | BDNF | 27740552 | 27744602 | - | Homo sapiens | 4050 |
| 983 | chr11 | BDNF | 27518527 | 27518574 | - | Homo sapiens | 47 |
| 984 | chr11 | BDNF | 27518780 | 27518823 | - | Homo sapiens | 43 |
| 985 | chr11 | BDNF | 27518870 | 27518922 | - | Homo sapiens | 52 |
| 986 | chr11 | BDNF | 27519285 | 27519333 | - | Homo sapiens | 48 |
| 987 | chr11 | BDNF | 27520498 | 27520547 | - | Homo sapiens | 49 |
| 988 | chr11 | BDNF | 27520913 | 27520996 | - | Homo sapiens | 83 |
| 989 | chr11 | BDNF | 27521081 | 27521112 | - | Homo sapiens | 31 |
| 990 | chr11 | BDNF | 27523348 | 27523395 | - | Homo sapiens | 47 |
| 991 | chr11 | BDNF | 27523423 | 27523469 | - | Homo sapiens | 46 |
| 992 | chr11 | BDNF | 27516527 | 27520574 | - | Homo sapiens | 4047 |
| 993 | chr11 | BDNF | 27516780 | 27520823 | - | Homo sapiens | 4043 |
| 994 | chr11 | BDNF | 27516870 | 27520922 | - | Homo sapiens | 4052 |
| 995 | chr11 | BDNF | 27517285 | 27521333 | - | Homo sapiens | 4048 |
| 996 | chr11 | BDNF | 27518498 | 27522547 | - | Homo sapiens | 4049 |
| 997 | chr11 | BDNF | 27518913 | 27522996 | - | Homo sapiens | 4083 |
| 998 | chr11 | BDNF | 27519081 | 27523112 | - | Homo sapiens | 4031 |
| 999 | chr11 | BDNF | 27521348 | 27525395 | - | Homo sapiens | 4047 |
| 1000 | chr11 | BDNF | 27521423 | 27525469 | - | Homo sapiens | 4046 |
| 1001 | chr11 | BDNF | 27681917 | 27681964 | + | Homo sapiens | 47 |
| 1002 | chr11 | BDNF | 27697978 | 27698023 | + | Homo sapiens | 45 |
| 1003 | chr11 | BDNF | 27718599 | 27718680 | + | Homo sapiens | 81 |
| 1004 | chr11 | BDNF | 27679917 | 27683964 | + | Homo sapiens | 4047 |
| 1005 | chr11 | BDNF | 27695978 | 27700023 | + | Homo sapiens | 4045 |
| 1006 | chr11 | BDNF | 27716599 | 27720680 | + | Homo sapiens | 4081 |
| 1007 | chr11 | BDNF | 27523708 | 27523784 | + | Homo sapiens | 76 |
| 1008 | chr11 | BDNF | 27527959 | 27528009 | + | Homo sapiens | 50 |
| 1009 | chr11 | BDNF | 27528063 | 27528106 | + | Homo sapiens | 43 |
| 1010 | chr11 | BDNF | 27521708 | 27525784 | + | Homo sapiens | 4076 |
| 1011 | chr11 | BDNF | 27525959 | 27530009 | + | Homo sapiens | 4050 |
| 1012 | chr11 | BDNF | 27526063 | 27530106 | + | Homo sapiens | 4043 |
| 1013 | chr11 | BDNF | 27734836 | 27734884 | + | Homo sapiens | 48 |
| 1014 | chr11 | BDNF | 27740311 | 27740344 | + | Homo sapiens | 33 |
| 1015 | chr11 | BDNF | 27741786 | 27741828 | + | Homo sapiens | 42 |
| 1016 | chr11 | BDNF | 27742450 | 27742493 | + | Homo sapiens | 43 |
| 1017 | chr11 | BDNF | 27732836 | 27736884 | + | Homo sapiens | 4048 |
| 1018 | chr11 | BDNF | 27738311 | 27742344 | + | Homo sapiens | 4033 |
| 1019 | chr11 | BDNF | 27739786 | 27743828 | + | Homo sapiens | 4042 |
| 1020 | chr11 | BDNF | 27740450 | 27744493 | + | Homo sapiens | 4043 |
| 1021 | chr3 | ADIPOQ | 186548462 | 186588252 | + | Homo sapiens | 39790 |
| 1022 | chr3 | ADIPOQ | 186548462 | 186588252 | - | Homo sapiens | 39790 |
| 1023 | chr16 | Adipoq | 23134608 | 23170041 | + | Mus musculus | 35433 |
| 1024 | chr16 | Adipoq | 23134608 | 23170041 | - | Mus musculus | 35433 |
| 1025 | chr3 | ADIPOQ | 186566781 | 186566827 | + | Homo sapiens | 46 |
| 1026 | chr3 | ADIPOQ | 186571630 | 186571674 | + | Homo sapiens | 44 |
| 1027 | chr3 | ADIPOQ | 186564781 | 186568827 | + | Homo sapiens | 4046 |
| 1028 | chr3 | ADIPOQ | 186569630 | 186573674 | + | Homo sapiens | 4044 |
| 1029 | chr3 | ADIPOQ | 186572160 | 186572189 | - | Homo sapiens | 29 |
| 1030 | chr3 | ADIPOQ | 186570160 | 186574189 | - | Homo sapiens | 4029 |
| 1031 | chrX | MECP2 | 153275263 | 153375188 | - | Homo sapiens | 99925 |
| 1032 | chrX | MECP2 | 153275263 | 153375188 | + | Homo sapiens | 99925 |
| 1033 | chrX | Mecp2 | 71260160 | 71342932 | - | Homo sapiens | 82772 |
| 1034 | chrX | Mecp2 | 71260160 | 71342932 | + | Homo sapiens | 82772 |
| 1035 | chrX | MECP2 | 153278064 | 153278111 | - | Homo sapiens | 47 |
| 1036 | chrX | MECP2 | 153278111 | 153278156 | - | Homo sapiens | 45 |
| 1037 | chrX | MECP2 | 153278706 | 153278747 | - | Homo sapiens | 41 |
| 1038 | chrX | MECP2 | 153279512 | 153279556 | - | Homo sapiens | 44 |
| 1039 | chrX | MECP2 | 153279613 | 153279658 | - | Homo sapiens | 45 |
| 1040 | chrX | MECP2 | 153281486 | 153281531 | - | Homo sapiens | 45 |
| 1041 | chrX | MECP2 | 153283707 | 153283737 | - | Homo sapiens | 30 |
| 1042 | chrX | MECP2 | 153284059 | 153284105 | - | Homo sapiens | 46 |
| 1043 | chrX | MECP2 | 153287944 | 153287992 | - | Homo sapiens | 48 |
| 1044 | chrX | MECP2 | 153288681 | 153288722 | - | Homo sapiens | 41 |
| 1045 | chrX | MECP2 | 153290087 | 153290134 | - | Homo sapiens | 47 |
| 1046 | chrX | MECP2 | 153290216 | 153290263 | - | Homo sapiens | 47 |
| 1047 | chrX | MECP2 | 153290364 | 153290414 | - | Homo sapiens | 50 |
| 1048 | chrX | MECP2 | 153291585 | 153291633 | - | Homo sapiens | 48 |
| 1049 | chrX | MECP2 | 153292312 | 153292362 | - | Homo sapiens | 50 |
| 1050 | chrX | MECP2 | 153292731 | 153292774 | - | Homo sapiens | 43 |
| 1051 | chrX | MECP2 | 153293138 | 153293185 | - | Homo sapiens | 47 |
| 1052 | chrX | MECP2 | 153293331 | 153293377 | - | Homo sapiens | 46 |
| 1053 | chrX | MECP2 | 153293427 | 153293469 | - | Homo sapiens | 42 |
| 1054 | chrX | MECP2 | 153293568 | 153293614 | - | Homo sapiens | 46 |
| 1055 | chrX | MECP2 | 153293715 | 153293764 | - | Homo sapiens | 49 |
| 1056 | chrX | MECP2 | 153293792 | 153293878 | - | Homo sapiens | 86 |
| 1057 | chrX | MECP2 | 153293901 | 153293948 | - | Homo sapiens | 47 |
| 1058 | chrX | MECP2 | 153294420 | 153294467 | - | Homo sapiens | 47 |
| 1059 | chrX | MECP2 | 153297927 | 153297972 | - | Homo sapiens | 45 |
| 1060 | chrX | MECP2 | 153315466 | 153315571 | - | Homo sapiens | 105 |
| 1061 | chrX | MECP2 | 153343401 | 153343447 | - | Homo sapiens | 46 |
| 1062 | chrX | MECP2 | 153344298 | 153344339 | - | Homo sapiens | 41 |
| 1063 | chrX | MECP2 | 153348654 | 153348702 | - | Homo sapiens | 48 |
| 1064 | chrX | MECP2 | 153348997 | 153349021 | - | Homo sapiens | 24 |
| 1065 | chrX | MECP2 | 153349179 | 153349222 | - | Homo sapiens | 43 |
| 1066 | chrX | MECP2 | 153349694 | 153349734 | - | Homo sapiens | 40 |
| 1067 | chrX | MECP2 | 153350493 | 153350518 | - | Homo sapiens | 25 |
| 1068 | chrX | MECP2 | 153356667 | 153356713 | - | Homo sapiens | 46 |
| 1069 | chrX | MECP2 | 153356742 | 153356795 | - | Homo sapiens | 53 |
| 1070 | chrX | MECP2 | 153357047 | 153357106 | - | Homo sapiens | 59 |
| 1071 | chrX | MECP2 | 153357161 | 153357204 | - | Homo sapiens | 43 |
| 1072 | chrX | MECP2 | 153361085 | 153361163 | - | Homo sapiens | 78 |
| 1073 | chrX | MECP2 | 153361423 | 153361467 | - | Homo sapiens | 44 |
| 1074 | chrX | MECP2 | 153362464 | 153362527 | - | Homo sapiens | 63 |
| 1075 | chrX | MECP2 | 153276064 | 153280111 | - | Homo sapiens | 4047 |
| 1076 | chrX | MECP2 | 153276111 | 153280156 | - | Homo sapiens | 4045 |
| 1077 | chrX | MECP2 | 153276706 | 153280747 | - | Homo sapiens | 4041 |
| 1078 | chrX | MECP2 | 153277512 | 153281556 | - | Homo sapiens | 4044 |
| 1079 | chrX | MECP2 | 153277613 | 153281658 | - | Homo sapiens | 4045 |
| 1080 | chrX | MECP2 | 153279486 | 153283531 | - | Homo sapiens | 4045 |
| 1081 | chrX | MECP2 | 153281707 | 153285737 | - | Homo sapiens | 4030 |
| 1082 | chrX | MECP2 | 153282059 | 153286105 | - | Homo sapiens | 4046 |
| 1083 | chrX | MECP2 | 153285944 | 153289992 | - | Homo sapiens | 4048 |
| 1084 | chrX | MECP2 | 153286681 | 153290722 | - | Homo sapiens | 4041 |
| 1085 | chrX | MECP2 | 153288087 | 153292134 | - | Homo sapiens | 4047 |
| 1086 | chrX | MECP2 | 153288216 | 153292263 | - | Homo sapiens | 4047 |
| 1087 | chrX | MECP2 | 153288364 | 153292414 | - | Homo sapiens | 4050 |
| 1088 | chrX | MECP2 | 153289585 | 153293633 | - | Homo sapiens | 4048 |
| 1089 | chrX | MECP2 | 153290312 | 153294362 | - | Homo sapiens | 4050 |
| 1090 | chrX | MECP2 | 153290731 | 153294774 | - | Homo sapiens | 4043 |
| 1091 | chrX | MECP2 | 153291138 | 153295185 | - | Homo sapiens | 4047 |
| 1092 | chrX | MECP2 | 153291331 | 153295377 | - | Homo sapiens | 4046 |
| 1093 | chrX | MECP2 | 153291427 | 153295469 | - | Homo sapiens | 4042 |
| 1094 | chrX | MECP2 | 153291568 | 153295614 | - | Homo sapiens | 4046 |
| 1095 | chrX | MECP2 | 153291715 | 153295764 | - | Homo sapiens | 4049 |
| 1096 | chrX | MECP2 | 153291792 | 153295878 | - | Homo sapiens | 4086 |
| 1097 | chrX | MECP2 | 153291901 | 153295948 | - | Homo sapiens | 4047 |
| 1098 | chrX | MECP2 | 153292420 | 153296467 | - | Homo sapiens | 4047 |
| 1099 | chrX | MECP2 | 153295927 | 153299972 | - | Homo sapiens | 4045 |
| 1100 | chrX | MECP2 | 153313466 | 153317571 | - | Homo sapiens | 4105 |
| 1101 | chrX | MECP2 | 153341401 | 153345447 | - | Homo sapiens | 4046 |
| 1102 | chrX | MECP2 | 153342298 | 153346339 | - | Homo sapiens | 4041 |
| 1103 | chrX | MECP2 | 153346654 | 153350702 | - | Homo sapiens | 4048 |
| 1104 | chrX | MECP2 | 153346997 | 153351021 | - | Homo sapiens | 4024 |
| 1105 | chrX | MECP2 | 153347179 | 153351222 | - | Homo sapiens | 4043 |
| 1106 | chrX | MECP2 | 153347694 | 153351734 | - | Homo sapiens | 4040 |
| 1107 | chrX | MECP2 | 153348493 | 153352518 | - | Homo sapiens | 4025 |
| 1108 | chrX | MECP2 | 153354667 | 153358713 | - | Homo sapiens | 4046 |
| 1109 | chrX | MECP2 | 153354742 | 153358795 | - | Homo sapiens | 4053 |
| 1110 | chrX | MECP2 | 153355047 | 153359106 | - | Homo sapiens | 4059 |
| 1111 | chrX | MECP2 | 153355161 | 153359204 | - | Homo sapiens | 4043 |
| 1112 | chrX | MECP2 | 153359085 | 153363163 | - | Homo sapiens | 4078 |
| 1113 | chrX | MECP2 | 153359423 | 153363467 | - | Homo sapiens | 4044 |
| 1114 | chrX | MECP2 | 153360464 | 153364527 | - | Homo sapiens | 4063 |
| 1115 | chrX | MECP2 | 153279614 | 153279660 | + | Homo sapiens | 46 |
| 1116 | chrX | MECP2 | 153281662 | 153281720 | + | Homo sapiens | 58 |
| 1117 | chrX | MECP2 | 153281946 | 153281988 | + | Homo sapiens | 42 |
| 1118 | chrX | MECP2 | 153284367 | 153284448 | + | Homo sapiens | 81 |
| 1119 | chrX | MECP2 | 153284489 | 153284534 | + | Homo sapiens | 45 |
| 1120 | chrX | MECP2 | 153288786 | 153288832 | + | Homo sapiens | 46 |
| 1121 | chrX | MECP2 | 153289895 | 153289940 | + | Homo sapiens | 45 |
| 1122 | chrX | MECP2 | 153292315 | 153292365 | + | Homo sapiens | 50 |
| 1123 | chrX | MECP2 | 153292496 | 153292548 | + | Homo sapiens | 52 |
| 1124 | chrX | MECP2 | 153297642 | 153297688 | + | Homo sapiens | 46 |
| 1125 | chrX | MECP2 | 153297723 | 153297765 | + | Homo sapiens | 42 |
| 1126 | chrX | MECP2 | 153300816 | 153300879 | + | Homo sapiens | 63 |
| 1127 | chrX | MECP2 | 153315579 | 153315621 | + | Homo sapiens | 42 |
| 1128 | chrX | MECP2 | 153316595 | 153316640 | + | Homo sapiens | 45 |
| 1129 | chrX | MECP2 | 153348783 | 153348830 | + | Homo sapiens | 47 |
| 1130 | chrX | MECP2 | 153349199 | 153349250 | + | Homo sapiens | 51 |
| 1131 | chrX | MECP2 | 153358221 | 153358285 | + | Homo sapiens | 64 |
| 1132 | chrX | MECP2 | 153277614 | 153281660 | + | Homo sapiens | 4046 |
| 1133 | chrX | MECP2 | 153279662 | 153283720 | + | Homo sapiens | 4058 |
| 1134 | chrX | MECP2 | 153279946 | 153283988 | + | Homo sapiens | 4042 |
| 1135 | chrX | MECP2 | 153282367 | 153286448 | + | Homo sapiens | 4081 |
| 1136 | chrX | MECP2 | 153282489 | 153286534 | + | Homo sapiens | 4045 |
| 1137 | chrX | MECP2 | 153286786 | 153290832 | + | Homo sapiens | 4046 |
| 1138 | chrX | MECP2 | 153287895 | 153291940 | + | Homo sapiens | 4045 |
| 1139 | chrX | MECP2 | 153290315 | 153294365 | + | Homo sapiens | 4050 |
| 1140 | chrX | MECP2 | 153290496 | 153294548 | + | Homo sapiens | 4052 |
| 1141 | chrX | MECP2 | 153295642 | 153299688 | + | Homo sapiens | 4046 |
| 1142 | chrX | MECP2 | 153295723 | 153299765 | + | Homo sapiens | 4042 |
| 1143 | chrX | MECP2 | 153298816 | 153302879 | + | Homo sapiens | 4063 |
| 1144 | chrX | MECP2 | 153313579 | 153317621 | + | Homo sapiens | 4042 |
| 1145 | chrX | MECP2 | 153314595 | 153318640 | + | Homo sapiens | 4045 |
| 1146 | chrX | MECP2 | 153346783 | 153350830 | + | Homo sapiens | 4047 |
| 1147 | chrX | MECP2 | 153347199 | 153351250 | + | Homo sapiens | 4051 |
| 1148 | chrX | MECP2 | 153356221 | 153360285 | + | Homo sapiens | 4064 |
| 1149 | chrX | FOXP3 | 49094896 | 49133288 | - | Homo sapiens | 38392 |
| 1150 | chrX | FOXP3 | 49094896 | 49133288 | + | Homo sapiens | 38392 |
| 1151 | chrX | Foxp3 | 7567675 | 7607243 | + | Mus musculus | 39568 |
| 1152 | chrX | Foxp3 | 7567675 | 7607243 | - | Mus musculus | 39568 |
| 1153 | chrX | FOXP3 | 49091852 | 49146158 | + | Homo sapiens | 54306 |
| 1154 | chrX | FOXP3 | 49105387 | 49126985 | + | Homo sapiens | 21598 |
| 1155 | chrX | FOXP3 | 49105442 | 49121156 | + | Homo sapiens | 15714 |
| 1156 | chrX | FOXP3 | 49131266 | 49131313 | + | Homo sapiens | 47 |
| 1157 | chrX | FOXP3 | 49131123 | 49131172 | + | Homo sapiens | 49 |
| 1158 | chrX | FOXP3 | 49127994 | 49128033 | + | Homo sapiens | 39 |
| 1159 | chrX | FOXP3 | 49127843 | 49127890 | + | Homo sapiens | 47 |
| 1160 | chrX | FOXP3 | 49127628 | 49127670 | + | Homo sapiens | 42 |
| 1161 | chrX | FOXP3 | 49124798 | 49124897 | + | Homo sapiens | 99 |
| 1162 | chrX | FOXP3 | 49123918 | 49123965 | + | Homo sapiens | 47 |
| 1163 | chrX | FOXP3 | 49120701 | 49120753 | + | Homo sapiens | 52 |
| 1164 | chrX | FOXP3 | 49118531 | 49118555 | + | Homo sapiens | 24 |
| 1165 | chrX | FOXP3 | 49115652 | 49115685 | + | Homo sapiens | 33 |
| 1166 | chrX | FOXP3 | 49112995 | 49113044 | + | Homo sapiens | 49 |
| 1167 | chrX | FOXP3 | 49112863 | 49112906 | + | Homo sapiens | 43 |
| 1168 | chrX | FOXP3 | 49112637 | 49112717 | + | Homo sapiens | 80 |
| 1169 | chrX | FOXP3 | 49107522 | 49107575 | + | Homo sapiens | 53 |
| 1170 | chrX | FOXP3 | 49106607 | 49106653 | + | Homo sapiens | 46 |
| 1171 | chrX | FOXP3 | 49106128 | 49106175 | + | Homo sapiens | 47 |
| 1172 | chrX | FOXP3 | 49105839 | 49105886 | + | Homo sapiens | 47 |
| 1173 | chrX | FOXP3 | 49105669 | 49105701 | + | Homo sapiens | 32 |
| 1174 | chrX | FOXP3 | 49105241 | 49105285 | + | Homo sapiens | 44 |
| 1175 | chrX | FOXP3 | 49089852 | 49148158 | + | Homo sapiens | 58306 |
| 1176 | chrX | FOXP3 | 49103387 | 49128985 | + | Homo sapiens | 25598 |
| 1177 | chrX | FOXP3 | 49103442 | 49123156 | + | Homo sapiens | 19714 |
| 1178 | chrX | FOXP3 | 49129266 | 49133313 | + | Homo sapiens | 4047 |
| 1179 | chrX | FOXP3 | 49129123 | 49133172 | + | Homo sapiens | 4049 |
| 1180 | chrX | FOXP3 | 49125994 | 49130033 | + | Homo sapiens | 4039 |
| 1181 | chrX | FOXP3 | 49125843 | 49129890 | + | Homo sapiens | 4047 |
| 1182 | chrX | FOXP3 | 49125628 | 49129670 | + | Homo sapiens | 4042 |
| 1183 | chrX | FOXP3 | 49122798 | 49126897 | + | Homo sapiens | 4099 |
| 1184 | chrX | FOXP3 | 49121918 | 49125965 | + | Homo sapiens | 4047 |
| 1185 | chrX | FOXP3 | 49118701 | 49122753 | + | Homo sapiens | 4052 |
| 1186 | chrX | FOXP3 | 49116531 | 49120555 | + | Homo sapiens | 4024 |
| 1187 | chrX | FOXP3 | 49113652 | 49117685 | + | Homo sapiens | 4033 |
| 1188 | chrX | FOXP3 | 49110995 | 49115044 | + | Homo sapiens | 4049 |
| 1189 | chrX | FOXP3 | 49110863 | 49114906 | + | Homo sapiens | 4043 |
| 1190 | chrX | FOXP3 | 49110637 | 49114717 | + | Homo sapiens | 4080 |
| 1191 | chrX | FOXP3 | 49105522 | 49109575 | + | Homo sapiens | 4053 |
| 1192 | chrX | FOXP3 | 49104607 | 49108653 | + | Homo sapiens | 4046 |
| 1193 | chrX | FOXP3 | 49104128 | 49108175 | + | Homo sapiens | 4047 |
| 1194 | chrX | FOXP3 | 49103839 | 49107886 | + | Homo sapiens | 4047 |
| 1195 | chrX | FOXP3 | 49103669 | 49107701 | + | Homo sapiens | 4032 |
| 1196 | chrX | FOXP3 | 49103241 | 49107285 | + | Homo sapiens | 4044 |
| 1197 | chrX | FOXP3 | 49091852 | 49146158 | - | Homo sapiens | 54306 |
| 1198 | chrX | FOXP3 | 49105387 | 49126985 | - | Homo sapiens | 21598 |
| 1199 | chrX | FOXP3 | 49127432 | 49127481 | - | Homo sapiens | 49 |
| 1200 | chrX | FOXP3 | 49127343 | 49127398 | - | Homo sapiens | 55 |
| 1201 | chrX | FOXP3 | 49117756 | 49117794 | - | Homo sapiens | 38 |
| 1202 | chrX | FOXP3 | 49100610 | 49100635 | - | Homo sapiens | 25 |
| 1203 | chrX | FOXP3 | 49100129 | 49100194 | - | Homo sapiens | 65 |
| 1204 | chrX | FOXP3 | 49099553 | 49099595 | - | Homo sapiens | 42 |
| 1205 | chrX | FOXP3 | 49089852 | 49148158 | - | Homo sapiens | 58306 |
| 1206 | chrX | FOXP3 | 49103387 | 49128985 | - | Homo sapiens | 25598 |
| 1207 | chrX | FOXP3 | 49125432 | 49129481 | - | Homo sapiens | 4049 |
| 1208 | chrX | FOXP3 | 49125343 | 49129398 | - | Homo sapiens | 4055 |
| 1209 | chrX | FOXP3 | 49115756 | 49119794 | - | Homo sapiens | 4038 |
| 1210 | chrX | FOXP3 | 49098610 | 49102635 | - | Homo sapiens | 4025 |
| 1211 | chrX | FOXP3 | 49098129 | 49102194 | - | Homo sapiens | 4065 |
| 1212 | chrX | FOXP3 | 49097553 | 49101595 | - | Homo sapiens | 4042 |

**Table 2:** Imprinted regions hit by the expanded PRC2 transcriptome.

Intersection of the PRC2 transcriptome with imprinted gene coordinates (available online at geneimprint.com). The murine imprinted gene (i.e., an intersecting or nearby gene) targeted by the PRC2 binding transcript is shown in column 1. Column 1 also shows the chromosome strand of the murine imprinted gene ("+"sign indicates that the gene is transcribed from the top or plus strand, while "-" sign indicates that the PRC2 binding transcript is transcribed from the bottom or minus strand of the chromosome). The chromosome localization and nucleotide coordinates in mm9 of the PRC2 binding transcript are shown in column 2, as well as a "+"sign or "-" sign that indicates whether the PRC2 binding transcript is transcribed from the top strand (plus strand hit) or bottom strand (minus strand hit) of the chromosome. Column 3 displays the sequence identifiers of the mouse PRC2 binding transcript (i.e., the nucleotide sequence transcribed from the mouse chromosomal coordinates and strand of column 2, converted to RNAby replacing T with U). Column 4 shows the corresponding human gene name for the murine imprinted gene of column 1, obtained from the Mouse Genome Database (MGD), Mouse Genome Informatics, The Jackson Laboratory, Bar Harbor, Maine. World Wide Web (informatics.jax.org). Mouse to human LiftOver of the mouse chromosome coordinates in column 2, performed in the UCSC genome browser as described herein, generated the orthologous human chromosome coordinates which appear in Column 5. 50% conservation was used for LiftOver analysis. Additional human chromosome coordinates were generated by mapping of highly conserved or homologous regions from the mouse to human genome. Column 6 displays the sequence identifiers of the predicted human PRC2 binding transcript (i.e., the nucleotide sequence transcribed from the human chromosomal coordinates and strand of column 5, converted to RNAby replacing T with U). When the PRC2 interacting transcript is transcribed from the opposite strand compared to the imprinted reference gene in column 1, that implies that the PRC2 interacting RNAis complementary, or antisense strand ("opposite strand") in orientation, to the reference imprinted gene. Note that the PRC2 binding transcript need not be the reference imprinted gene itself, but a distinct transcript that overlaps in position.

### Table 3: Hexamers that are not seed sequences of human miRNAs

**APPENDIX I,** of U.S. provisional application 61/425, 174 filed on December 20, 2010 is a listing of a complete RIP seq dataset, showing all of the reads in the dataset. Appendix I is not attached hereto. The sequence reads in Appendix I come directly off the Illumina GA II genome analyzer and are in an orientation that is the reverse complement of the PRC2 binding transcript. Appendix I is a filtered subset of all of the reads after bioinformatic filtering removed adaptor/primer dimers, mitochondrial RNA, rRNA, homopolymers, reads with indeterminate nucleotides, and truncated reads (<15nt).

### DETAILED DESCRIPTION

Aspects of the invention provided by the claims relate to the discovery of polycomb repressive complex 2 (PRC2)-interacting RNAs. Polycomb repressive complex 2 (PRC2) is a histone methyltransferase and a known epigenetic regulator involved in silencing of genomic regions through methylation of histone H3. Among other functions, PRC2 interacts with long noncoding RNAs (IncRNAs), such as RepA and Xist, and Tsix, to catalyze trimethylation of histone H3-lysine27. PRC2 contains four subunits, Eed, Suz12, RbAp48, and Ezh2.

A method, referred to herein as "RNA immunoprecipitation (RIP)-seq," was used to identify a genome-wide pool of >100,000 polycomb repressive complex 2 (PRC2)-interacting RNAs in embryonic stem cells. A large number of transcripts occur within and around imprinted regions, oncogene and tumor suppressor loci, and stem-cell-related bivalent domains. Evidence for direct RNA-protein interactions, some via the Ezh2 subunit, was established. Further evidence was established that single stranded oligonucleotides designed to bind to these PRC2-interacting RNAs can successfully up-regulate gene expression in a variety of separate and independent examples, which is believed to result from inhibition of PRC2 mediated repression of these target genes. Thus, PRC2 complexes interact with a genome-wide family of RNAs, which may be used as therapeutic targets for human disease. In some embodiments, the sequences of RNA's that interact with PRC2 were between 40-60 nucleotides in length.

As used herein, the term "PRC2-associated region" refers to a region of a nucleic acid that comprises or encodes a sequence of nucleotides that interact directly or indirectly with a component of PRC2. A PRC2-associated region may be present in a RNA (*e.g.,* a long noncoding RNA (lncRNA)) that that interacts with a PRC2. A PRC2-associated region may be present in a DNA that encodes an RNA that interacts with a PRC2.

In some embodiments, a PRC2-associated region is a region of an RNA that crosslinks to a component of PRC2 in response to *in situ* ultraviolet irradiation of a cell that expresses the RNA, or a region of genomic DNA that encodes that RNA region. In some embodiments, a PRC2-associated region is a region of an RNA that immunoprecipitates with an antibody that targets a component of PRC2, or a region of genomic DNA that encodes that RNA region. In some embodiments, a PRC2-associated region is a region of an RNA that immunoprecipitates with an antibody that targets SUZ12, EED, EZH2 or RBBP4 (which are components of PRC2), or a region of genomic DNA that encodes that RNA region.

In some embodiments, a PRC2-associated region is a region of an RNA that is protected from nucleases (*e.g.,* RNases) in an RNA-immunoprecipitation assay that employs an antibody that targets a component of PRC2, or a region of genomic DNA that encodes that protected RNA region. In some embodiments, a PRC2-associated region is a region of an RNA that is protected from nucleases (*e.g.,* RNases) in an RNA-immunoprecipitation assay that employs an antibody that targets SUZ12, EED, EZH2 or RBBP4, or a region of genomic DNA that encodes that protected RNA region.

In some embodiments, a PRC2-associated region is a region of an RNA within which occur a relatively high frequency of sequence reads in a sequencing reaction of products of an RNA-immunoprecipitation assay that employs an antibody that targets a component of PRC2, or a region of genomic DNA that encodes that RNA region. In some embodiments, a PRC2-associated region is a region of an RNA within which occur a relatively high frequency of sequence reads in a sequencing reaction of products of an RNA-immunoprecipitation assay that employs an antibody that targets SUZ12, EED, EZH2 or RBBP4, or a region of genomic DNA that encodes that protected RNA region. In such embodiments, the PRC2-associated region may be referred to as a "peak."

In some embodiments, a PRC2-associated region comprises a sequence of 40 to 60 nucleotides that interact with PRC2 complex. In some embodiments, a PRC2-associated region comprises a sequence of 40 to 60 nucleotides that encode an RNA that interacts with PRC2. In some embodiments, a PRC2-associated region comprises a sequence of up to 5kb in length that comprises a sequence (*e.g.,* of 40 to 60 nucleotides) that interacts with PRC2. In some embodiments, a PRC2-associated region comprises a sequence of up to 5kb in length within which an RNA is encoded that has a sequence (*e.g.,* of 40 to 60 nucleotides) that is known to interact with PRC2. In some embodiments, a PRC2-associated region comprises a sequence of about 4kb in length that comprise a sequence (*e.g.,* of 40 to 60 nucleotides) that interacts with PRC2. In some embodiments, a PRC2-associated region comprises a sequence of about 4kb in length within which an RNA is encoded that includes a sequence (*e.g.,* of 40 to 60 nucleotides) that is known to interact with PRC2.

In some embodiments, a PRC2-associated region has a sequence as set forth in any one of sequences A1 to A193,049, B1 to B916,209, and B916,626 to B934,931.

In some embodiments, single stranded oligonucleotides are provided that specifically bind to, or are complementary to, a PRC2-associated region, for example, a nucleic acid having a sequence as set forth in sequences A1 to A193,049, B1 to B916,209, and B916,626 to B934,931. Without being bound by a theory of invention, these oligonucleotides are able to interfere with the binding of and function of PRC2, by preventing recruitment of PRC2 to a specific chromosomal locus. For example, data herein shows that a single administration of single stranded oligonucleotides designed to specifically bind a PRC2-associated region IncRNA can stably displace not only the lncRNA, but also the PRC2 that binds to the lncRNA, from binding chromatin. After displacement, the full complement of PRC2 is not recovered for up to 24 hours. Further, data provided herein support that IncRNA can recruit PRC2 in a *cis* fashion, repressing gene expression at or near the specific chromosomal locus from which the lncRNA was transcribed, thus making it possible to design oligonucleotides that inhibit the function of PRC2 and increase the expression of a specific target gene.

In further aspects of the invention, methods are provided for selecting a candidate oligonucleotide for activating expression of a target gene as further defined by the claims. The methods generally involve selecting as a candidate oligonucleotide, a single stranded oligonucleotide comprising a nucleotide sequence that is complementary to a PRC2-associated region (*e.g.,* a nucleotide sequence as set forth in sequences A1 to A 193,049, B1 to B916,209, and B916,626 to B934,931). In some embodiments, sets of oligonucleotides may be selected that are enriched (*e.g.,* compared with a random selection of oligonucleotides) in oligonucleotides that activate expression of a target gene.

In some embodiments, the single stranded oligonucleotide is provided for use in a method of modulating expression of a "gene targeted by the PRC2-binding RNA" (*e.g.,* an intersecting or nearby gene, as set forth in Tables 1-3), meaning a gene whose expression is regulated by the PRC2-binding RNA. The term "PRC2-binding RNA" or "RNA that binds PRC2" is used interchangeably with "PRC2-associated RNA" and "PRC2-interacting RNA", and refers to a lncRNA, RNA transcript or a PRC2-associated region thereof (*e.g.,* a Peak as described below) that binds PRC2, directly or indirectly. Such binding may be determined by immunoprecipitation techniques using antibodies to a component of the PRC2 complex, *e.g.* Ezh2. Sequences A1 to A193,049, B1 to B916,209, and B916,626 to B934,931 represent murine RNA sequences containing portions that have been experimentally determined to bind PRC2 using the RIP-seq method described herein, or human RNA sequences corresponding to these murine RNA sequences.

Such methods of modulating gene expression may be carried out *in vitro, ex vivo,* or *in vivo.* Table 8 of International Patent Application Publication WO/2012/065143 displays genes targeted by the PRC2-binding RNA; the sequence identifiers of the PRC2-binding RNA are set forth in the same row as the gene name. In some embodiments, a single stranded oligonucleotide is provided for use in a method of treating disease, e.g. a disease category as set forth in Table 9 of International Patent Application Publication WO/2012/065143 or Table 2. Table 2 of International Patent Application Publication WO/2012/087983, displays genes targeted by the PRC2-binding RNA; the sequence identifiers of the PRC2-binding RNA are set forth in the same row as the gene name. In some embodiments, a single stranded oligonucleotide is provided for use in a method of treating disease, *e.g.* a disease category as set forth in Table 3 of International Patent Application Publication WO/2012/087983 or Table 2. The treatment may involve modulating expression of a gene targeted by the PRC2-binding RNA, preferably upregulating gene expression. The single stranded oligonucleotide may be formulated as a sterile composition for parenteral administration. It is understood that any reference to uses of compounds throughout the description contemplates use of the compound in preparation of a pharmaceutical composition or medicament for use in the treatment of a disease. Thus, as one nonlimiting example, this aspect of the disclosure includes use of such single stranded oligonucleotides in the preparation of a medicament for use in the treatment of disease, wherein the treatment involves upregulating expression of a gene targeted by the PRC2-binding RNA.

### Method for Selecting Candidate Oligonucleotides for Activating Gene Expression

Methods are provided herein for selecting a candidate oligonucleotide for activating expression of a target gene. The target gene of interest may, for example, be a gene of Table 9 of International Patent Application Publication WO/2012/065143. The target gene of interest may, for example, be a gene of Table 3 of International Patent Application Publication WO/2012/087983. The target gene of interest may be FXN, SMN1, SMN2, SMNP, UTRN, HBB, HBD, HBE1, HBG1, HBG2, Hbb-bl, Hbb-bh1, Hbb-y, HBB/HBD, ATP2A2, APOA1, Abcal, PTEN, BDNF, BDNF-AS1, ADIPOQ, MECP2 or FOXP3. Accordingly, the candidate oligonucleotide may be complementary to a sequence selected from the sequences set forth in SEQ ID NOS: 1-1212.

Typically, the methods involve one or more steps aimed at identifying oligonucleotides that target a PRC2-associated region that is functionally related to the target gene, for example a PRC2-associated region of a IncRNA that regulates expression of the target gene by facilitating (*e.g.,* in a *cis*-regulatory manner) the recruitment of PRC2 to the target gene. Such oligonucleotides are expected to be candidates for activating expression of the target gene because of their ability to hybridize with the PRC2-associated region of a nucleic acid (*e.g.,* a lncRNA). In some embodiments, this hybridization event is understood to disrupt interaction of PRC2 with the nucleic acid (*e.g.,* a lncRNA) and as a result disrupt recruitment of PRC2 and its associated co-repressors (*e.g.,* chromatin remodeling factors) to the target gene locus.

Methods of selecting a candidate oligonucleotide may involve selecting a PRC2-associated region (*e.g.,* a nucleotide sequence as set forth in sequences A1 to A193,049, B1 to B916,209, and B916,626 to B934,931) that maps to a chromosomal position encompassing or in proximity to a target gene of interest. The PRC2-associated region may map to the strand of the chromosome comprising the sense strand of the target gene, in which case the candidate oligonucleotide is complementary to the sense strand of the target gene (*i.e.,* is antisense to the target gene). Alternatively, the PRC2-associated region may map to the strand of the first chromosome comprising the antisense strand of the target gene, in which case the oligonucleotide is complementary to the antisense strand (the template strand) of the target gene (*i.e.,* is sense to the target gene).

Methods for selecting a set of candidate oligonucleotides that is enriched in oligonucleotides that activate expression of a target gene may involve selecting one or more PRC2-associated regions that maps to a chromosomal position that encompasses or that is in proximity to the target gene and selecting a set of oligonucleotides, in which each oligonucleotide in the set comprises a nucleotide sequence that is complementary with the one or more PRC2-associated regions. As used herein, the phrase, "a set of oligonucleotides that is enriched in oligonucleotides that activate expression of a target gene" refers to a set of oligonucleotides that has a greater number of oligonucleotides that activate expression of a target gene compared with a random selection of oligonucleotides of the same physicochemical properties (*e.g.,* the same GC content, Tₘ, length *etc*.) as the enriched set.

The PRC2-associated region may map to a position in a chromosome between 50 kilobases upstream of a 5'-end of the target gene and 50 kilobases downstream of a 3'-end of the target gene. The PRC2-associated region may map to a position in a chromosome between 25 kilobases upstream of a 5'-end of the target gene and 25 kilobases downstream of a 3'-end of the target gene. The PRC2-associated region may map to a position in a chromosome between 12 kilobases upstream of a 5'-end of the target gene and 12 kilobases downstream of a 3'-end of the target gene. The PRC2-associated region may map to a position in a chromosome between 5 kilobases upstream of a 5'-end of the target gene and 5 kilobases downstream of a 3'-end of the target gene.

The genomic position of the selected PRC2-associated region relative to the target gene may vary. For example, the PRC2-associated region may be upstream of the 5' end of the target gene. The PRC2-associated region may be downstream of the 3' end of the target gene. The PRC2-associated region may be within an intron of the target gene. The PRC2-associated region may be within an exon of the target gene. The PRC2-associated region may traverse an intron-exon junction, a 5'-UTR-exon junction or a 3'-UTR-exon junction of the target gene.

The candidate oligonucleotide selection methods may generally also involve determining or identifying an appropriate nucleotide sequence that is complementary with the PRC2-associated region. This nucleotide sequence may be complementary with at least 6, at least 7, at least 8, at least 9, at least 10, at least 15 or more consecutive nucleotides of the PRC2-associated region.

The candidate oligonucleotide may comprise a sequence having the formula X-Y-Z, in which X is any nucleotide, Y is a nucleotide sequence of 6 nucleotides in length that is not a human seed sequence of a microRNA, and Z is a nucleotide sequence of varying length. In some embodiments X is anchored at the 5' end of the oligonucleotide. In some embodiments, when X is anchored at the 5' end of the oligonucleotide, the oligonucleotide does not have any nucleotides or nucleotide analogs linked 5' to X. In some embodiments, other compounds such as peptides or sterols may be linked at the 5' end in this embodiment as long as they are not nucleotides or nucleotide analogs. Candidate oligonucleotides that have these sequence characteristics are predicted to avoid the miRNA pathway. Therefore, in some embodiments, oligonucleotides having these sequence characteristics unlikely to have an unintended consequence of functioning in a cell as a miRNA molecule. The Y sequence may be a nucleotide sequence of 6 nucleotides in length set forth in Table 3.

The candidate oligonucleotide may have a sequence that does not contain guanosine nucleotide stretches (*e.g.,* 3 or more, 4 or more, 5 or more, 6 or more consecutive guanosine nucleotides). In some embodiments, oligonucleotides having guanosine nucleotide stretches have increased non-specific binding and/or off-target effects, compared with oligonucleotides that do not have guanosine nucleotide stretches.

The candidate oligonucleotide may be selected such that it has a sequence that has less than a threshold level of sequence identity with every sequence of nucleotides, of equivalent length, that map to a genomic position encompassing or in proximity to an off-target gene. For example, a candidate oligonucleotide may be designed to ensure that it does not have a sequence that maps to genomic positions encompassing or in proximity with all known genes (*e.g.,* all known protein coding genes) other than the target gene. In a similar embodiment, a candidate oligonucleotide may be designed to ensure that it does not have a sequence that maps to any other known PRC2-associated region (*e.g.,* a nucleotide sequence as set forth in sequences A1 to A193,049, B1 to B916,209, and B916,626 to B934,931), particularly PRC2-associated regions that are functionally related to any other known gene (*e.g.,* any other known protein coding gene). In either case, the candidate oligonucleotide is expected to have a reduced likelihood of having off-target effects. The threshold level of sequence identity may be 50%, 60%, 70%, 80%, 85%, 90%, 95% or 99% sequence identity.

The candidate oligonucleotide may be selected such that it has a sequence that is complementary to a PRC2-associated region that encodes an RNA that forms a secondary structure comprising at least two single stranded loops. In has been discovered that, in some embodiments, oligonucleotides that are complementary to a PRC2-associated region that encodes an RNA that forms a secondary structure comprising one or more single stranded loops (*e.g.,* at least two single stranded loops) have a greater likelihood of being active than a randomly selected oligonucleotide. In some cases, the secondary structure may comprise a double stranded stem between the at least two single stranded loops. Accordingly, the selection methods may involve selecting a sequence for the oligonucleotide such that the region of complementarity between the oligonucleotide and the PRC2-associated region is at a location of the PRC2 associated region that encodes at least a portion of at least one of the loops. In some cases, the selection methods may involve selecting a sequence for the oligonucleotide such that the region of complementarity between the oligonucleotide and the PRC2-associated region is at a location of the PRC2-associated region that encodes at least a portion of at least two of the loops. In some cases, the selection methods may involve selecting a sequence for the oligonucleotide such that the region of complementarity between the oligonucleotide and the PRC2-associated region is at a location of the PRC2 associated region that encodes at least a portion of the double stranded stem. In some embodiments, a PRC2-associated region (e.g., of an lncRNA) is identified (e.g., using RIP-Seq methodology or information derived therefrom). In some embodiments, the predicted secondary structure RNA (e.g., lncRNA) containing the PRC2-associated region is determined using RNA secondary structure prediction algorithms, e.g., RNAfold, mfold. In some embodiments, oligonucleotides are designed to target a region of the RNA that forms a secondary structure comprising one or more single stranded loop (e.g., at least two single stranded loops) structures which may comprise a double stranded stem between the at least two single stranded loops.

The candidate oligonucleotide may be selected such that it has a sequence that is has greater than 30% G-C content, greater than 40% G-C content, greater than 50% G-C content, greater than 60% G-C content, greater than 70% G-C content, or greater than 80% G-C content. In some embodiments in which the oligonucleotide is 8 to 10 nucleotides in length, all but 1, 2, 3, 4, or 5 of the nucleotides of the complementary sequence of the PRC2-associated region are cytosine or guanosine nucleotides.

The candidate oligonucleotide selection methods may also involve determining that the candidate oligonucleotide is complementary to a chromosome of a different species (*e.g.,* a mouse, rat, rabbit, goat, monkey, *etc*.) at a position that encompasses or that is in proximity to the homolog of the target gene. This enables the design of oligonucleotides that may be tested *in vivo* or *in vitro* for efficacy in multiple species (*e.g.,* human and mouse). This approach also facilitates development of clinical candidates for treating human disease by selecting a species in which an appropriate animal exists for the disease. The candidate oligonucleotide can be readily tested in the animal model.

Where the design and/or synthesis of a single stranded oligonucleotide involves design and/or synthesis of a sequence that is complementary to a nucleic acid or PRC2-associated region described by such sequence information, the skilled person is readily able to determine the complementary sequence, *e.g.,* through understanding of Watson Crick base pairing rules which form part of the common general knowledge in the field.

In some embodiments design and/or synthesis of a single stranded oligonucleotide involves manufacture of an oligonucleotide from starting materials by techniques known to those of skill in the art, where the synthesis may be based on a sequence of a PRC2-associated region, or portion thereof.

Methods of design and/or synthesis of a single stranded oligonucleotide may involve one or more of the steps of:
Identifying and/or selecting PRC2-associated region;
Designing a nucleic acid sequence having a desired degree of sequence identity or complementarity to a PRC2-associated region or a portion thereof;
Synthesizing a single stranded oligonucleotide to the designed sequence;
Purifying the synthesized single stranded oligonucleotide; and
Optionally mixing the synthesized single stranded oligonucleotide with at least one pharmaceutically acceptable diluent, carrier or excipient to form a pharmaceutical composition or medicament.

Single stranded oligonucleotides so designed and/or synthesized may be useful in method of modulating gene expression as described herein.

Preferably, single stranded oligonucleotides for use in the invention are synthesized chemically. Oligonucleotides used to practice this invention can be synthesized *in vitro* by well-known chemical synthesis techniques.

Oligonucleotides for use in the invention can be stabilized against nucleolytic degradation such as by the incorporation of a modification, *e.g.,* a nucleotide modification. For example, nucleic acid sequences for use in the invention include a phosphorothioate at least the first, second, or third internucleotide linkage at the 5' or 3' end of the nucleotide sequence. As another example, the nucleic acid sequence can include a 2'-modified nucleotide, *e.g.,* a 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2`-O-dimethylaminopropyl (2'-O-DMAP), 2'-O-dimethylaminoethyloxyethyl (2'-O-DMAEOE), or 2'-O--N-methylacetamido (2'-O--NMA). As another example, the nucleic acid sequence can include at least one 2'-O-methyl-modified nucleotide, and in some embodiments, all of the nucleotides include a 2'-O-methyl modification. In some embodiments, the nucleic acids are "locked," i.e., comprise nucleic acid analogues in which the ribose ring is "locked" by a methylene bridge connecting the 2'-O atom and the 4'-C atom.

It is understood that any of the modified chemistries or formats of single stranded oligonucleotides described herein can be combined with each other, and that one, two, three, four, five, or more different types of modifications can be included within the same molecule.

In some embodiments, the method may further comprise the steps of amplifying the synthesized single stranded oligonucleotide, and/or purifying the single stranded oligonucleotide (or amplified single stranded oligonucleotide), and/or sequencing the single stranded oligonucleotide so obtained.

As such, the process of preparing a single stranded oligonucleotide may be a process that is for use in the manufacture of a pharmaceutical composition or medicament for use in the treatment of disease, optionally wherein the treatment involves modulating expression of a gene associated with a PRC2-associated region.

In the methods described above a PRC2-associated region may be, or have been, identified, or obtained, by a method that involves identifying RNA that binds to PRC2.

Such methods may involve the following steps: providing a sample containing nuclear ribonucleic acids, contacting the sample with an agent that binds specifically to PRC2 or a subunit thereof, allowing complexes to form between the agent and protein in the sample, partitioning the complexes, synthesizing nucleic acid that is complementary to nucleic acid present in the complexes.

Where the single stranded oligonucleotide is based on a PRC2-associated region, or a portion of such a sequence, it may be based on information about that sequence, *e.g.,* sequence information available in written or electronic form, which may include sequence information contained in publicly available scientific publications or sequence databases.

### Single Stranded Oligonucleotides

In one aspect of the disclosure single stranded oligonucleotides complementary to the PRC2-associated regions are provided for modulating expression of target genes in a cell. In some embodiments, expression of target genes is upregulated or increased. In some embodiments, single stranded oligonucleotides complementary to these PRC2-associated regions inhibit the interaction of PRC2 with long RNA transcripts, resulting in reduced methylation of histone H3 and reduced gene inactivation, such that gene expression is upregulated or increased. In some embodiments, this interaction may be disrupted or inhibited due to a change in the structure of the long RNA that prevents or reduces binding to PRC2. The oligonucleotide may be selected using any of the methods disclosed herein for selecting a candidate oligonucleotide for activating expression of a target gene.

In some embodiments, the region of complementarity is complementary with at least 8 to 15, 8 to 30, 8 to 40, or 10 to 50, or 5 to 50, or 5 to 40 bases, *e.g.,* 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 consecutive nucleotides of a PRC2-associated region. In some embodiments, the region of complementarity is complementary with at least 8 consecutive nucleotides of a PRC2-associated region. In some embodiments the sequence of the single stranded oligonucleotide is based on an RNA sequence that binds to PRC2, or a portion thereof, said portion having a length of from 5 to 40 contiguous base pairs, or about 8 to 40 bases, or about 5 to 15, or about 5 to 30, or about 5 to 40 bases, or about 5 to 50 bases.

Any of the oligonucleotides disclosed herein may be linked to one or more other oligonucleotides disclosed herein by a cleavable linker.

Complementary, as the term is used in the art, refers to the capacity for precise pairing between two nucleotides. For example, if a nucleotide at a certain position of an oligonucleotide is capable of hydrogen bonding with a nucleotide at the same position of PRC2-associated region, then the single stranded nucleotide and PRC2-associated region are considered to be complementary to each other at that position. The single stranded nucleotide and PRC2-associated region are complementary to each other when a sufficient number of corresponding positions in each molecule are occupied by nucleotides that can hydrogen bond with each other through their bases. Thus, "complementary" is a term which is used to indicate a sufficient degree of complementarity or precise pairing such that stable and specific binding occurs between the single stranded nucleotide and PRC2-associated region. For example, if a base at one position of a single stranded nucleotide is capable of hydrogen bonding with a base at the corresponding position of a PRC2-associated region, then the bases are considered to be complementary to each other at that position. 100% complementarity is not required.

The single stranded oligonucleotide may be at least 80% complementary to (optionally one of at least 85%, 90%, 91%, 92%, 93%, 94%, 95%, 96%, 97%, 98%, 99% or 100% complementary to) the consecutive nucleotides of a PRC2-associated region. In some embodiments the single stranded oligonucleotide may contain 1, 2 or 3 base mismatches compared to the portion of the consecutive nucleotides of a PRC2-associated region. In some embodiments the single stranded oligonucleotide may have up to 3 mismatches over 15 bases, or up to 2 mismatches over 10 bases.

It is understood in the art that a complementary nucleotide sequence need not be 100% complementary to that of its target to be specifically hybridizable. In some embodiments, a complementary nucleic acid sequence for purposes of the present methods is specifically hybridizable when binding of the sequence to the target molecule (e.g., lncRNA) interferes with the normal function of the target (*e.g.,* lncRNA) to cause a loss of activity (*e.g.,* inhibiting PRC2-associated repression with consequent up-regulation of gene expression) and there is a sufficient degree of complementarity to avoid non-specific binding of the sequence to non-target sequences under conditions in which avoidance of non-specific binding is desired, *e.g.,* under physiological conditions in the case of *in vivo* assays or therapeutic treatment, and in the case of *in vitro* assays, under conditions in which the assays are performed under suitable conditions of stringency.

In some embodiments, the single stranded oligonucleotide is 7, 8, 9, 10, 11, 12, 13, 14, 15, 16, 17, 18, 19, 20, 21, 22, 23, 24, 25, 26, 27, 28, 29, 30, 35, 40, 45, 50 or more nucleotides in length. In an embodiment, the oligonucleotide is 8 to 30 nucleotides in length.

In some embodiments, the PRC2-associated region occurs on the same DNA strand as a gene sequence (sense). In some embodiments, the PRC2-associated region occurs on the opposite DNA strand as a gene sequence (anti-sense). Oligonucleotides complementary to a PRC2-associated region can bind either sense or anti-sense sequences. Base pairings may include both canonical Watson-Crick base pairing and non-Watson-Crick base pairing (*e.g.,* Wobble base pairing and Hoogsteen base pairing). It is understood that for complementary base pairings, adenosine-type bases (A) are complementary to thymidine-type bases (T) or uracil-type bases (U), that cytosine-type bases (C) are complementary to guanosine-type bases (G), and that universal bases such as 3-nitropyrrole or 5-nitroindole can hybridize to and are considered complementary to any A, C, U, or T. Inosine (I) has also been considered in the art to be a universal base and is considered complementary to any A, C, U or T.

In some embodiments, any one or more thymidine (T) nucleotides (or modified nucleotide thereof) or uridines (U) nucleotides (or a modified nucleotide thereof) in a sequence provided herein, including a sequence provided in the sequence listing, may be replaced with any other nucleotide suitable for base pairing (e.g., via a Watson-Crick base pair) with an adenosine nucleotide. In some embodiments, any one or more thymidine (T) nucleotides (or modified nucleotide thereof) or uridines (U) nucleotides (or a modified nucleotide thereof) in a sequence provided herein, including a sequence provided in the sequence listing, may be suitably replaced with a different pyrimidine nucleotide or vice versa. In some embodiments, any one or more thymidine (T) nucleotides (or modified nucleotide thereof) in a sequence provided herein, including a sequence provided in the sequence listing, may be suitably replaced with a uridine (U) nucleotide (or a modified nucleotide thereof) or vice versa. Inosine (I) has also been considered in the art to be a universal base and is considered complementary to any A, C, U or T.

Inosine (I) has also been considered in the art to be a universal base and is considered complementary to any A, C, U or T.

In some embodiments, GC content of the single stranded oligonucleotide may be between about 30-60 %. Contiguous runs of three or more Gs or Cs may not be preferable in some embodiments. Accordingly, in some embodiments, the oligonucleotide does not comprise a stretch of three or more guanosine nucleotides.

In some embodiments, the single stranded oligonucleotide specifically binds to, or is complementary to an RNA that is encoded in a genome (e.g., a human genome) as a single contiguous transcript (e.g., a non-spliced RNA). In some embodiments, the single stranded oligonucleotide specifically binds to, or is complementary to an RNA that is encoded in a genome (e.g., a human genome), in which the distance in the genome between the 5'end of the coding region of the RNA and the 3' end of the coding region of the RNA is less than 1 kb, less than 2 kb, less than 3 kb, less than 4 kb, less than 5 kb, less than 7 kb, less than 8 kb, less than 9 kb, less than 10 kb, or less than 20 kb.

It is to be understood that any oligonucleotide provided herein can be excluded. In some embodiments, a single stranded oligonucleotide is not complementary to any one or more of SEQ ID NOs: 1213 to 1226.

### Nucleotide Analogues

In some embodiments, the oligonucleotide may comprise at least one ribonucleotide, at least one deoxyribonucleotide, and/or at least one bridged nucleotide. In some embodiments, the oligonucleotide may comprise a bridged nucleotide, such as a LNA nucleotide, a cEt nucleotide or a ENA nucleotide analogue. Examples of such nucleotides are disclosed herein and known in the art. In some embodiments, the oligonucleotide comprises a nucleotide analog disclosed in one of the following United States Patent or Patent Application Publications: US 7,399,845, US 7,741,457, US 8,022,193, US 7,569,686, US 7,335,765, US 7,314,923, US 7,335,765, and US 7,816,333, US 20110009471. The oligonucleotide may have one or more 2' O-methyl nucleotides. The oligonucleotide may consist entirely of 2' O-methyl nucleotides.

Often the single stranded oligonucleotide has one or more nucleotide analogues. For example, the single stranded oligonucleotide may have at least one nucleotide analogue that results in an increase in Tₘ of the oligonucleotide in a range of 1°C, 2 °C, 3°C, 4 °C, or 5°C compared with an oligonucleotide that does not have the at least one nucleotide analogue. The single stranded oligonucleotide may have a plurality of nucleotide analogues that results in a total increase in Tₘ of the oligonucleotide in a range of 2 °C, 3 °C, 4 °C, 5 °C, 6 °C, 7 °C, 8 °C, 9 °C, 10 °C, 15 °C, 20 °C, 25 °C, 30 °C, 35 °C, 40 °C, 45 °C or more compared with an oligonucleotide that does not have the nucleotide analogue.

The oligonucleotide may be of up to 50 nucleotides in length in which 2 to 10, 2 to 15, 2 to 16, 2 to 17, 2 to 18, 2 to 19, 2 to 20, 2 to 25, 2 to 30, 2 to 40, 2 to 45, or more nucleotides of the oligonucleotide are nucleotide analogues. The oligonucleotide may be of 8 to 30 nucleotides in length in which 2 to 10, 2 to 15, 2 to 16, 2 to 17, 2 to 18, 2 to 19, 2 to 20, 2 to 25, 2 to 30 nucleotides of the oligonucleotide are nucleotide analogues.

The oligonucleotide may be of 8 to 15 nucleotides in length in which 2 to 4, 2 to 5, 2 to 6, 2 to 7, 2 to 8, 2 to 9, 2 to 10, 2 to 11, 2 to 12, 2 to 13, 2 to 14 nucleotides of the oligonucleotide are nucleotide analogues. Optionally, the oligonucleotides may have every nucleotide except 1, 2, 3, 4, 5, 6, 7, 8, 9, or 10 nucleotides modified.

The oligonucleotide may consist entirely of bridged nucleotides (e.g., LNA nucleotides, cEt nucleotides, ENA nucleotides). The oligonucleotide may comprise alternating deoxyribonucleotides and 2'-fluoro-deoxyribonucleotides. The oligonucleotide may comprise alternating deoxyribonucleotides and 2'-O-methyl nucleotides. The oligonucleotide may comprise alternating deoxyribonucleotides and ENA nucleotide analogues. The oligonucleotide may comprise alternating deoxyribonucleotides and LNA nucleotides. The oligonucleotide may comprise alternating LNA nucleotides and 2'-O-methyl nucleotides. The oligonucleotide may have a 5' nucleotide that is a bridged nucleotide (*e.g.,* a LNA nucleotide, cEt nucleotide, ENA nucleotide). The oligonucleotide may have a 5' nucleotide that is a deoxyribonucleotide.

The oligonucleotide may comprise deoxyribonucleotides flanked by at least one bridged nucleotide (*e.g.,* a LNA nucleotide, cEt nucleotide, ENA nucleotide) on each of the 5' and 3' ends of the deoxyribonucleotides. The oligonucleotide may comprise deoxyribonucleotides flanked by 1, 2, 3, 4, 5, 6, 7, 8 or more bridged nucleotides (*e.g.,* LNA nucleotides, cEt nucleotides, ENA nucleotides) on each of the 5' and 3' ends of the deoxyribonucleotides. The 3' position of the oligonucleotide may have a 3' hydroxyl group. The 3' position of the oligonucleotide may have a 3' thiophosphate.

The oligonucleotide may be conjugated with a label. For example, the oligonucleotide may be conjugated with a biotin moiety, cholesterol, Vitamin A, folate, sigma receptor ligands, aptamers, peptides, such as CPP, hydrophobic molecules, such as lipids, ASGPR or dynamic polyconjugates and variants thereof at its 5' or 3' end.

Preferably the single stranded oligonucleotide comprises one or more modifications comprising: a modified sugar moiety, and/or a modified internucleoside linkage, and/or a modified nucleotide and/or combinations thereof. It is not necessary for all positions in a given oligonucleotide to be uniformly modified, and in fact more than one of the modifications described herein may be incorporated in a single oligonucleotide or even at within a single nucleoside within an oligonucleotide.

In some embodiments, the single stranded oligonucleotides are chimeric oligonucleotides that contain two or more chemically distinct regions, each made up of at least one nucleotide. These oligonucleotides typically contain at least one region of modified nucleotides that confers one or more beneficial properties (such as, for example, increased nuclease resistance, increased uptake into cells, increased binding affinity for the target) and a region that is a substrate for enzymes capable of cleaving RNA:DNA or RNA:RNA hybrids. Chimeric single stranded oligonucleotides for use in the invention may be formed as composite structures of two or more oligonucleotides, modified oligonucleotides, oligonucleosides and/or oligonucleotide mimetics as described above. Such compounds have also been referred to in the art as hybrids or gapmers. Representative United States patents that teach the preparation of such hybrid structures comprise, but are not limited to, US patent nos. 5,013,830; 5,149,797; 5, 220,007; 5,256,775; 5,366,878; 5,403,711; 5,491,133; 5,565,350; 5,623,065; 5,652,355; 5,652,356; and 5,700,922.

In some embodiments, the single stranded oligonucleotide comprises at least one nucleotide modified at the 2' position of the sugar, e.g., a 2'-O-alkyl, 2'-O-alkyl-O-alkyl or 2'-fluoro-modified nucleotide. In other embodiments, RNA modifications include 2'-fluoro, 2'-amino and 2' O-methyl modifications on the ribose of pyrimidines, abasic residues or an inverted base at the 3' end of the RNA. Such modifications are routinely incorporated into oligonucleotides and these oligonucleotides have been shown to have a higher Tm (i.e., higher target binding affinity) than 2'-deoxyoligonucleotides against a given target.

A number of nucleotide and nucleoside modifications have been shown to make the oligonucleotide into which they are incorporated more resistant to nuclease digestion than the native oligodeoxynucleotide; these modified oligos survive intact for a longer time than unmodified oligonucleotides. Specific examples of modified oligonucleotides include those comprising modified backbones, for example, phosphorothioates, phosphotriesters, methyl phosphonates, short chain alkyl or cycloalkyl intersugar linkages or short chain heteroatomic or heterocyclic intersugar linkages. Examples are oligonucleotides with phosphorothioate backbones and those with heteroatom backbones, particularly CH2 -NH-O-CH2, CH,∼N(CH3)∼O∼CH2 (known as a methylene(methylimino) or MMI backbone], CH2 --O-N (CH3)-CH2, CH2 -N (CH3)-N (CH3)-CH2 and O-N (CH3)- CH2 -CH2 backbones, wherein the native phosphodiester backbone is represented as O- P-- O- CH,); amide backbones (see De Mesmaeker et al. Ace. Chem. Res. 1995, 28:366-374); morpholino backbone structures (see Summerton and Weller, U.S. Pat. No. 5,034,506); peptide nucleic acid (PNA) backbone (wherein the phosphodiester backbone of the oligonucleotide is replaced with a polyamide backbone, the nucleotides being bound directly or indirectly to the aza nitrogen atoms of the polyamide backbone, see Nielsen et al., Science 1991, 254, 1497). Phosphorus-containing linkages include, but are not limited to, phosphorothioates, chiral phosphorothioates, phosphorodithioates, phosphotriesters, aminoalkylphosphotriesters, methyl and other alkyl phosphonates comprising 3'alkylene phosphonates and chiral phosphonates, phosphinates, phosphoramidates comprising 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'; see US patent nos. 3,687,808; 4,469,863; 4,476,301; 5,023,243; 5, 177,196; 5,188,897; 5,264,423; 5,276,019; 5,278,302; 5,286,717; 5,321,131; 5,399,676; 5,405,939; 5,453,496; 5,455, 233; 5,466,677; 5,476,925; 5,519,126; 5,536,821; 5,541,306; 5,550,111; 5,563, 253; 5,571,799; 5,587,361; and 5,625,050.

Morpholino-based oligomeric compounds are described in Dwaine A. Braasch and David R. Corey, Biochemistry, 2002, 41(14), 4503-4510); Genesis, volume 30, issue 3, 2001; Heasman, J., Dev. Biol., 2002, 243, 209-214; Nasevicius et al., Nat. Genet., 2000, 26, 216-220; Lacerra et al., Proc. Natl. Acad. Sci., 2000, 97, 9591-9596; and U.S. Pat. No. 5,034,506, issued Jul. 23, 1991. In some embodiments, the morpholino-based oligomeric compound is a phosphorodiamidate morpholino oligomer (PMO) (*e.g.,* as described in Iverson, Curr. Opin. Mol. Ther., 3:235-238, 2001; and Wang et al., J. Gene Med., 12:354-364, 2010).

Cyclohexenyl nucleic acid oligonucleotide mimetics are described in Wang et al., J. Am. Chem. Soc., 2000, 122, 8595-8602.

Modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These comprise those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts; see US patent nos. 5,034,506; 5,166,315; 5,185,444; 5,214,134; 5,216,141; 5,235,033; 5,264, 562; 5, 264,564; 5,405,938; 5,434,257; 5,466,677; 5,470,967; 5,489,677; 5,541,307; 5,561,225; 5,596, 086; 5,602,240; 5,610,289; 5,602,240; 5,608,046; 5,610,289; 5,618,704; 5,623, 070; 5,663,312; 5,633,360; 5,677,437; and 5,677,439.

Modified oligonucleotides are also known that include oligonucleotides that are based on or constructed from arabinonucleotide or modified arabinonucleotide residues. Arabinonucleosides are stereoisomers of ribonucleosides, differing only in the configuration at the 2'-position of the sugar ring. In some embodiments, a 2'-arabino modification is 2'-F arabino. In some embodiments, the modified oligonucleotide is 2'-fluoro-D-arabinonucleic acid (FANA) (as described in, for example, Lon et al., Biochem., 41:3457-3467, 2002 and Min et al., Bioorg. Med. Chem. Lett., 12:2651-2654, 2002).

Similar modifications can also be made at other positions on the sugar, particularly the 3' position of the sugar on a 3' terminal nucleoside or in 2'-5' linked oligonucleotides and the 5' position of 5' terminal nucleotide.

PCT Publication No. WO 99/67378 discloses arabinonucleic acids (ANA) oligomers and their analogues for improved sequence specific inhibition of gene expression via association to complementary messenger RNA.

Other modifications include ethylene-bridged nucleic acids (ENAs) (*e.g.,* International Patent Publication No. WO 2005/042777, Morita et al., Nucleic Acid Res., Suppl 1:241-242, 2001; Surono et al., Hum. Gene Ther., 15:749-757, 2004; Koizumi, Curr. Opin. Mol. Ther., 8:144-149, 2006 and Horie et al., Nucleic Acids Symp. Ser (Oxf), 49:171-172, 2005). Preferred ENAs include, but are not limited to, 2'-O,4'-C-ethylene-bridged nucleic acids.

Examples of LNAs are described in WO/2008/043753 and include compounds of the following formula. 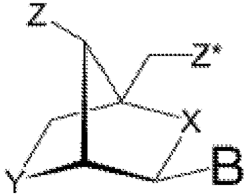
where X and Y are independently selected among the groups -O-,
-S-, -N(H)-, N(R)-, -CH2- or -CH- (if part of a double bond),
-CH₂-O-, -CH₂-S-, -CH₂-N(H)-, -CH₂-N(R)-, -CH₂-CH₂- or -CH₂-CH- (if part of a double bond),
-CH=CH-, where R is selected from hydrogen and C₁₋₄-alkyl; Z and Z* are independently selected among an internucleoside linkage, a terminal group or a protecting group; B constitutes a natural or non-natural nucleotide base moiety; and the asymmetric groups may be found in either orientation.

Preferably, the LNA used in the oligomer for use in the invention comprises at least one LNA unit according any of the formulas 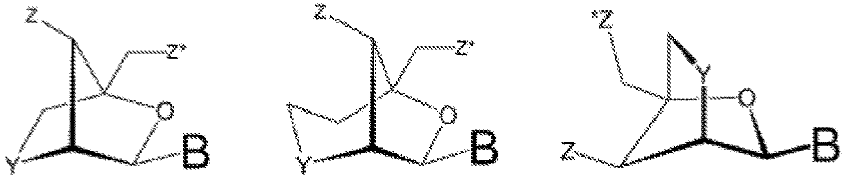 wherein Y is -O-, -S-, -NH-, or N(R^{H}); Z and Z* are independently selected among an internucleoside linkage, a terminal group or a protecting group; B constitutes a natural or non-natural nucleotide base moiety, and RH is selected from hydrogen and C₁₋₄-alkyl.

In some embodiments, the Locked Nucleic Acid (LNA) used in the oligomeric compound, such as an antisense oligonucleotide, for use in the invention comprises a Locked Nucleic Acid (LNA) unit according any of the formulas shown in Scheme 2 of PCT/DK2006/000512.

In some embodiments, the LNA used in the oligomer for use in the invention comprises internucleoside linkages selected from -O-P(O)₂-O-, -O-P(O,S)-O-, -0-P(S)₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R^{H})-O-, O-PO(OCH₃)-O-, -O-PO(NR^{H})-O-, -O-PO(OCH₂CH₂S-R)-O-, -O-PO(BH₃)-O-, -O-PO(NHR^{H})-O-, -O-P(O)₂-NR^{H}-, -NR^{H}-P(O)₂-O-, -NR^{H}-CO-O-, where R^{H} is selected from hydrogen and C₁₋₄-alkyl.

Certain examples of LNA units are shown in scheme 2:

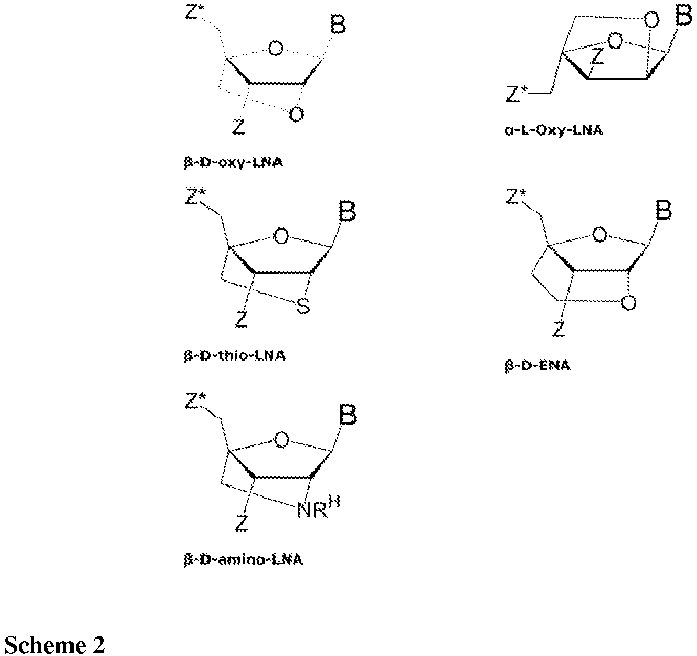

The term "thio-LNA" comprises a locked nucleotide in which at least one of X or Y in the general formula above is selected from S or -CH2-S-. Thio-LNA can be in both beta-D and alpha-L-configuration.

The term "amino-LNA" comprises a locked nucleotide in which at least one of X or Y in the general formula above is selected from -N(H)-, N(R)-, CH₂-N(H)-, and -CH₂-N(R)-where R is selected from hydrogen and C₁₋₄-alkyl. Amino-LNA can be in both beta-D and alpha-L-configuration.

The term "oxy-LNA" comprises a locked nucleotide in which at least one of X or Y in the general formula above represents -O- or -CH₂-O-. Oxy-LNA can be in both beta-D and alpha-L-configuration.

The term "ena-LNA" comprises a locked nucleotide in which Y in the general formula above is -CH₂-O- (where the oxygen atom of -CH₂-O- is attached to the 2'-position relative to the base B).

LNAs are described in additional detail herein.

One or more substituted sugar moieties can also be included, *e.g.,* one of the following at the 2' position: OH, SH, SCH₃, F, OCN, OCH₃ OCH₃, OCH₃ O(CH₂)n CH₃, O(CH₂)n NH₂ or O(CH₂)n CH₃ where n is from 1 to about 10; C1 to C10 lower alkyl, alkoxyalkoxy, substituted lower alkyl, alkaryl or aralkyl; Cl; Br; CN; CF3 ; OCF3; O-, S-, or N-alkyl; O-, S-, or N-alkenyl; SOCH3; SO2 CH3; ONO2; NO2; N3; NH2; heterocycloalkyl; heterocycloalkaryl; aminoalkylamino; polyalkylamino; substituted silyl; an RNA cleaving group; a reporter group; an intercalator; a group for improving the pharmacokinetic properties of an oligonucleotide; or a group for improving the pharmacodynamic properties of an oligonucleotide and other substituents having similar properties. An example modification includes 2'-methoxyethoxy [2'-O-CH₂CH₂OCH₃, also known as 2'-O-(2-methoxyethyl)] (Martin et al, HeIv. Chim. Acta, 1995, 78, 486). Other modifications include 2'-methoxy (2'-O-CH₃), 2'-propoxy (2'-OCH₂ CH₂CH₃) and 2'-fluoro (2'-F). Similar modifications may also be made at other positions on the oligonucleotide, particularly the 3' position of the sugar on the 3' terminal nucleotide and the 5' position of 5' terminal nucleotide. Oligonucleotides may also have sugar mimetics such as cyclobutyls in place of the pentofuranosyl group.

Single stranded oligonucleotides can also include, additionally or alternatively, nucleobase (often referred to in the art simply as "base") modifications or substitutions. As used herein, "unmodified" or "natural" nucleobases include adenine (A), guanine (G), thymine (T), cytosine (C) and uracil (U). Modified nucleobases include nucleobases found only infrequently or transiently in natural nucleic acids, *e.g.,* hypoxanthine, 6-methyladenine, 5-Me pyrimidines, particularly 5-methylcytosine (also referred to as 5-methyl-2' deoxycytosine and often referred to in the art as 5-Me-C), 5-hydroxymethylcytosine (HMC), glycosyl HMC and gentobiosyl HMC, isocytosine, pseudoisocytosine, as well as synthetic nucleobases, *e.g.,* 2-aminoadenine, 2-(methylamino)adenine, 2-(imidazolylalkyl)adenine, 2-(aminoalklyamino)adenine or other heterosubstituted alkyladenines, 2-thiouracil, 2-thiothymine, 5-bromouracil, 5-hydroxymethyluracil, 5-propynyluracil, 8-azaguanine, 7-deazaguanine, N6 (6-aminohexyl)adenine, 6-aminopurine, 2-aminopurine, 2-chloro-6-aminopurine and 2,6-diaminopurine or other diaminopurines. See, *e.g.,* Kornberg, "DNA Replication," W. H. Freeman & Co., San Francisco, 1980, pp75-77; and Gebeyehu, G., et al. Nucl. Acids Res., 15:4513 (1987)). A "universal" base known in the art, *e.g.,* inosine, can also be included. 5-Me-C substitutions have been shown to increase nucleic acid duplex stability by 0.6-1.2<0>C. (Sanghvi, in Crooke, and Lebleu, eds., Antisense Research and Applications, CRC Press, Boca Raton, 1993, pp. 276-278) and are example base substitutions.

It is not necessary for all positions in a given oligonucleotide to be uniformly modified, and in fact more than one of the modifications described herein may be incorporated in a single oligonucleotide or even at within a single nucleoside within an oligonucleotide.

In some embodiments, both a sugar and an internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, for example, an aminoethylglycine backbone. The nucleobases are retained and are bound directly or indirectly to aza nitrogen atoms of the amide portion of the backbone. Representative United States patents that teach the preparation of PNA compounds include, but are not limited to, US patent nos. 5,539,082; 5,714,331; and 5,719,262. Further teaching of PNA compounds can be found in Nielsen et al, Science, 1991, 254,1497-1500.

Single stranded oligonucleotides can also include one or more nucleobase (often referred to in the art simply as "base") modifications or substitutions. As used herein, "unmodified" or "natural" nucleobases comprise the purine bases adenine (A) and guanine (G), and the pyrimidine bases thymine (T), cytosine (C) and uracil (U). Modified nucleobases comprise other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudo-uracil), 4-thiouracil, 8-halo, 8-amino, 8-thiol, 8- thioalkyl, 8-hydroxyl and other 8-substituted adenines and guanines, 5-halo particularly 5- bromo, 5-trifluoromethyl and other 5-substituted uracils and cytosines, 7-methylquanine and 7-methyladenine, 8-azaguanine and 8-azaadenine, 7-deazaguanine and 7-deazaadenine and 3- deazaguanine and 3-deazaadenine.

Further, nucleobases comprise those disclosed in United States Patent No. 3,687,808, those disclosed in "The Concise Encyclopedia of Polymer Science And Engineering", pages 858-859, Kroschwitz, ed. John Wiley & Sons, 1990;, those disclosed by Englisch et al., Angewandle Chemie, International Edition, 1991, 30, page 613, and those disclosed by Sanghvi, Chapter 15, Antisense Research and Applications," pages 289- 302, Crooke, and Lebleu, eds., CRC Press, 1993. Certain of these nucleobases are particularly useful for increasing the binding affinity of the oligomeric compounds for use in the invention. These include 5-substituted pyrimidines, 6-azapyrimidines and N-2, N-6 and 0-6 substituted purines, comprising 2-aminopropyladenine, 5-propynyluracil and 5- propynylcytosine. 5-methylcytosine substitutions have been shown to increase nucleic acid duplex stability by 0.6-1.2<0>C (Sanghvi, et al., eds, "Antisense Research and Applications," CRC Press, Boca Raton, 1993, pp. 276-278) and are example base substitutions, even more particularly when combined with 2'-O-methoxyethyl sugar modifications. Modified nucleobases are described in US patent nos. 3,687,808, as well as 4,845,205; 5,130,302; 5,134,066; 5,175, 273; 5, 367,066; 5,432,272; 5,457,187; 5,459,255; 5,484,908; 5,502,177; 5,525,711; 5,552,540; 5,587,469; 5,596,091; 5,614,617; 5,750,692, and 5,681,941.

In some embodiments, the single stranded oligonucleotides are chemically linked to one or more moieties or conjugates that enhance the activity, cellular distribution, or cellular uptake of the oligonucleotide. For example, one or more single stranded oligonucleotides, of the same or different types, can be conjugated to each other; or single stranded oligonucleotides can be conjugated to targeting moieties with enhanced specificity for a cell type or tissue type. Such moieties include, but are not limited to, lipid moieties such as a cholesterol moiety (Letsinger et al., Proc. Natl. Acad. Sci. USA, 1989, 86, 6553-6556), cholic acid (Manoharan et al., Bioorg. Med. Chem. Let., 1994,4, 1053-1060), a thioether, e.g., hexyl-S- tritylthiol (Manoharan et al, Ann. N. Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, *e.g.,* dodecandiol or undecyl residues (Kabanov et al., FEBS Lett., 1990, 259, 327-330; Svinarchuk et al., Biochimie, 1993, 75, 49- 54), a phospholipid, *e.g.,* di-hexadecyl-rac-glycerol or triethylammonium 1,2-di-O-hexadecyl- rac-glycero-3-H-phosphonate (Manoharan et al., Tetrahedron Lett., 1995, 36, 3651-3654: Shea et al., Nucl. Acids Res., 1990, 18, 3777-3783), a polyamine or a polyethylene glycol chain (Mancharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-t oxycholesterol moiety (Crooke et al., J. Pharmacol. Exp. Ther., 1996, 277, 923-937). See also US patent nos. 4,828,979; 4,948,882; 5,218,105; 5,525,465; 5,541,313; 5,545,730; 5,552, 538; 5,578,717, 5,580,731; 5,580,731; 5,591,584; 5,109,124; 5,118,802; 5,138,045; 5,414,077; 5,486, 603; 5,512,439; 5,578,718; 5,608,046; 4,587,044; 4,605,735; 4,667,025; 4,762, 779; 4,789,737; 4,824,941; 4,835,263; 4,876,335; 4,904,582; 4,958,013; 5,082, 830; 5,112,963; 5,214,136; 5,082,830; 5,112,963; 5,214,136; 5, 245,022; 5,254,469; 5,258,506; 5,262,536; 5,272,250; 5,292,873; 5,317,098; 5,371,241, 5,391, 723; 5,416,203, 5,451,463; 5,510,475; 5,512,667; 5,514,785; 5, 565,552; 5,567,810; 5,574,142; 5,585,481; 5,587,371; 5,595,726; 5,597,696; 5,599,923; 5,599, 928 and 5,688,941,

These moieties or conjugates can include conjugate groups covalently bound to functional groups such as primary or secondary hydroxyl groups. Conjugate groups used in the invention include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugate groups include cholesterols, lipids, phospholipids, biotin, phenazine, folate, phenanthridine, anthraquinone, acridine, fluoresceins, rhodamines, coumarins, and dyes. Groups that enhance the pharmacodynamic properties, in the context of this invention, include groups that improve uptake, enhance resistance to degradation, and/or strengthen sequence-specific hybridization with the target nucleic acid. Groups that enhance the pharmacokinetic properties, in the context of this invention, include groups that improve uptake, distribution, metabolism or excretion of the compounds for use in the present invention. Representative conjugate groups are disclosed in International Patent Application No. PCT/US92/09196, filed Oct. 23, 1992, and U.S. Pat. No. 6,287,860. Conjugate moieties include, but are not limited to, lipid moieties such as a cholesterol moiety, cholic acid, a thioether, *e.g.,* hexyl-5-tritylthiol, a thiocholesterol, an aliphatic chain, *e.g.,* dodecandiol or undecyl residues, a phospholipid, *e.g.,* di-hexadecyl-rac- glycerol or triethylammonium 1,2-di-O-hexadecyl-rac-glycero-3-H-phosphonate, a polyamine or a polyethylene glycol chain, or adamantane acetic acid, a palmityl moiety, or an octadecylamine or hexylamino-carbonyl-oxy cholesterol moiety. See, *e.g.,* U.S. Pat. Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

In some embodiments, single stranded oligonucleotide modification include modification of the 5' or 3' end of the oligonucleotide. In some embodiments, the 3' end of the oligonucleotide comprises a hydroxyl group or a thiophosphate. It should be appreciated that additional molecules (*e.g.* a biotin moiety or a fluorophor) can be conjugated to the 5' or 3' end of the single stranded oligonucleotide. In some embodiments, the single stranded oligonucleotide comprises a biotin moiety conjugated to the 5' nucleotide.

In some embodiments, the single stranded oligonucleotide comprises locked nucleic acids (LNA), ENA modified nucleotides, 2'-O-methyl nucleotides, or 2'-fluoro-deoxyribonucleotides. In some embodiments, the single stranded oligonucleotide comprises alternating deoxyribonucleotides and 2'-fluoro-deoxyribonucleotides. In some embodiments, the single stranded oligonucleotide comprises alternating deoxyribonucleotides and 2'-O-methyl nucleotides. In some embodiments, the single stranded oligonucleotide comprises alternating deoxyribonucleotides and ENA modified nucleotides. In some embodiments, the single stranded oligonucleotide comprises alternating deoxyribonucleotides and locked nucleic acid nucleotides. In some embodiments, the single stranded oligonucleotide comprises alternating locked nucleic acid nucleotides and 2'-O-methyl nucleotides.

In some embodiments, the 5' nucleotide of the oligonucleotide is a deoxyribonucleotide. In some embodiments, the 5' nucleotide of the oligonucleotide is a locked nucleic acid nucleotide. In some embodiments, the nucleotides of the oligonucleotide comprise deoxyribonucleotides flanked by at least one locked nucleic acid nucleotide on each of the 5' and 3' ends of the deoxyribonucleotides. In some embodiments, the nucleotide at the 3' position of the oligonucleotide has a 3' hydroxyl group or a 3' thiophosphate.

In some embodiments, the single stranded oligonucleotide comprises phosphorothioate internucleotide linkages. In some embodiments, the single stranded oligonucleotide comprises phosphorothioate internucleotide linkages between at least two nucleotides. In some embodiments, the single stranded oligonucleotide comprises phosphorothioate internucleotide linkages between all nucleotides.

It should be appreciated that the single stranded oligonucleotide can have any combination of modifications as described herein.

The oligonucleotide may comprise a nucleotide sequence having one or more of the following modification patterns.
(a) (X)Xxxxxx, (X)xXxxxx, (X)xxXxxx, (X)xxxXxx, (X)xxxxXx and (X)xxxxxX,
(b) (X)XXxxxx, (X)XxXxxx, (X)XxxXxx, (X)XxxxXx, (X)XxxxxX, (X)xXXxxx, (X)xXxXxx, (X)xXxxXx, (X)xXxxxX, (X)xxXXxx, (X)xxXxXx, (X)xxXxxX, (X)xxxXXx, (X)xxxXxX and (X)xxxxXX,
(c) (X)XXXxxx, (X)xXXXxx, (X)xxXXXx, (X)xxxXXX, (X)XXxXxx, (X)XXxxXx, (X)XXxxxX, (X)xXXxXx, (X)xXXxxX, (X)xxXXxX, (X)XxXXxx, (X)XxxXXx (X)XxxxXX, (X)xXxXXx, (X)xXxxXX, (X)xxXxXX, (X)xXxXxX and (X)XxXxXx,
(d) (X)xxXXX, (X)xXxXXX, (X)xXXxXX, (X)xXXXxX, (X)xXXXXx, (X)XxxXXXX, (X)XxXxXX, (X)XxXXxX, (X)XxXXx, (X)XXxxXX, (X)XXxXxX, (X)XXxXXx, (X)XXXxxX, (X)XXXxXx, and (X)XXXXxx,
(e) (X)xXXXXX, (X)XxXXXX, (X)XXxXXX, (X)XXXxXX, (X)XXXXxX and (X)XXXXXx, and
(f) XXXXXX, XxXXXXX, XXxXXXX, XXXxXXX, XXXXxXX, XXXXXxX and XXXXXXx, in which "X" denotes a nucleotide analogue, (X) denotes an optional nucleotide analogue, and "x" denotes a DNA or RNA nucleotide unit. Each of the above listed patterns may appear one or more times within an oligonucleotide, alone or in combination with any of the other disclosed modification patterns.

### Further Features of Oligonucleotides

Evidence is provided herein that such oligonucleotides increased expression of mRNA corresponding to the gene by at least about 50% (i.e. 150% of normal or 1.5 fold), or by about 2 fold to about 5 fold. In some embodiments it is contemplated that expression may be increased by at least about 15 fold, 20 fold, 30 fold, 40 fold, 50 fold or 100 fold, or any range between any of the foregoing numbers. In other experiments, increased mRNA expression has been shown to correlate to increased protein expression.

The sequence identifiers outlined in Table 2 refer to sequences of RNAs that associate (binds) with PRC2 (*i.e.,* the RNA against which oligonucleotides would be directed) that are disclosed in International Patent Application Publication WO/2012/087983. Accordingly, each of the sequences comprise PRC2-associated regions. Each of (a) the reference genes described in the tables, (b) the PRC2 binding transcripts or Peaks (i.e., smaller regions of RNA that bind to PRC2) that target (modulate expression of) these genes, and (c) the oligonucleotides that specifically bind to, or are complementary to, the PRC2 binding transcripts or Peaks, may conveniently be grouped into any of these categories, represented by numbers in Table 3 of International Patent Application Publication WO/2012/087983 or represented by numbers in Table 9 of International Patent Application Publication WO/2012/065143 as follows: Diseases are marked by category numbers 11, 14, 15, 17, 21, 24, 26, 42, 44, 49, 58, 69, 82, 103, 119, 120, 126, 143, 163, 167, 172, 177, 182, 183, 184, 187, 191, 196, 200, 203, 204, 219, 220, 221, 227, 234, 239, 240, 244, 249, any one of 300-323, or any one of 400-643.

Other functional groups are marked by category numbers 10, 12, 13, 16, 18, 19, 20, 22, 23, 25, 27, 28, 29, 30, 31, 32, 33, 34, 36, 37, 38, 39, 40, 41, 43, 45, 46, 47, 48, 50, 51, 52, 54, 55, 56, 59, 60, 61, 62, 63, 64, 65, 66, 67, 68, 70, 71, 72, 73, 74, 75, 76, 77, 78, 79, 80, 81, 83, 84, 85, 86, 87, 88, 89, 90, 91, 92, 93, 94, 95, 96, 97, 98, 100, 101, 102, 104, 105, 106, 107, 108, 109, 110, 111, 112, 113, 114, 115, 116, 117, 118, 121, 122, 123, 124, 125, 127, 128, 129, 130, 131, 132, 133, 134, 135, 136, 137, 138, 139, 140, 141, 142, 144, 145, 146, 147, 148, 149, 150, 151, 152, 153, 154, 155, 156, 157, 158, 158, 160, 161, 162, 164, 165, 166, 168, 169, 170, 171, 173, 174, 175, 176, 178, 179, 180, 181, 185, 186, 188, 189, 190, 192, 193, 194, 195, 197, 198, 199, 201, 202, 205, 206, 207, 208, 209, 210, 211, 213, 215, 216, 217, 218, 222, 223, 224, 226, 228, 229, 230, 231, 232, 233, 235, 236, 237, 238, 241, 242, 243, 245, 246, 247, 248, 250, 251, 252, or 253.

### Category

| No. | Name |
|---|---|
| 10 | actin cytoskeleton organization |
| 11 | Acute myeloid leukemia |
| 12 | Adherens junction |
| 13 | Adipocytokine signaling pathway |
| 14 | aging |
| 15 | Alzheimer's disease |
| 16 | Amino sugar and nucleotide sugar metabolism |
| 17 | Amyotrophic lateral sclerosis (ALS) |
| 18 | angiogenesis |
| 19 | Apoptosis |
| 20 | Arginine and proline metabolism |
| 21 | Arrhythmogenic right ventricular cardiomyopathy (ARVC) |
| 22 | Axon guidance |
| 23 | B cell receptor signaling pathway |
| 24 | Basal cell carcinoma, also in category 644 |
| 25 | Basal transcription factors |
| 26 | Bladder cancer, also in category 644 |
| 27 | blood coagulation |
| 28 | blood vessel development |
| 29 | bone development |
| 30 | Calcium signaling pathway |
| 31 | Cardiac muscle contraction |
| 32 | cation channel activity |
| 33 | cell adhesion |
| 34 | cell cycle |
| 35 | Cell cycle |
| 36 | cell motion |
| 37 | cell surface receptor linked signal transduction |
| 38 | cellular response to stress |
| 39 | channel activity |
| 40 | Chemokine signaling pathway |
| 41 | cholesterol metabolic process |
| 42 | Chronic myeloid leukemia |
| 43 | Citrate cycle (TCA cycle) |
| 44 | Colorectal cancer |
| 45 | Complement and coagulation cascades |
| 46 | cytokine activity |
| 47 | cytoskeletal protein binding |
| 48 | cytosol |
| 49 | Dilated cardiomyopathy |
| 50 | DNA binding |
| 51 | DNA repair |
| 52 | DNA replication |
| 53 | DNA replication |
| 54 | Drug metabolism |
| 55 | embryonic morphogenesis |
| 56 | endocytosis |
| 57 | Endocytosis |
| 58 | Endometrial cancer |
| 59 | endoplasmic reticulum |
| 60 | ErbB signaling pathway |
| 61 | extracellular region |
| 62 | eye development |
| 63 | Fatty acid metabolism |
| 64 | Fructose and mannose metabolism |
| 65 | G-protein coupled receptor protein signaling pathway |
| 66 | gamete generation |
| 67 | Gap junction |
| 68 | gene silencing by miRNA |
| 69 | Glioma |
| 70 | glucose metabolic process |
| 71 | Glycolysis / Gluconeogenesis |
| 72 | Golgi apparatus |
| 73 | growth factor activity |
| 74 | GTPase regulator activity |
| 75 | heart development |
| 76 | Hedgehog signaling pathway |
| 77 | Hematopoietic cell lineage |
| 78 | hemopoiesis |
| 79 | hemopoietic or lymphoid organ development |
| 80 | histone modification |
| 81 | Huntington's disease |
| 82 | Hypertrophic cardiomyopathy (HCM) |
| 83 | immune response |
| 84 | immune system development |
| 85 | inflammatory response |
| 86 | Insulin signaling pathway |
| 87 | intracellular signaling cascade |
| 88 | ion channel activity |
| 89 | ion transport |
| 90 | Jak-STAT signaling pathway |
| 91 | learning or memory |
| 92 | leukocyte activation |
| 93 | Leukocyte transendothelial migration |
| 94 | limb development |
| 95 | locomotory behavior |
| 96 | Long-term potentiation |
| 97 | lung development |
| 98 | lysosome |
| 99 | Lysosome |
| 100 | MAPK signaling pathway |
| 101 | MAPKKK cascade |
| 102 | Melanogenesis |
| 103 | Melanoma |
| 104 | Mismatch repair |
| 105 | mitochondrion |
| 106 | mitochondrion organization |
| 107 | mTOR signaling pathway |
| 108 | muscle tissue development |
| 109 | ncRNA metabolic process |
| 110 | neuron development |
| 111 | Neurotrophin signaling pathway |
| 112 | Non-small cell lung cancer, also in category 644 |
| 113 | Notch signaling pathway |
| 114 | nucleolus |
| 115 | Oocyte meiosis |
| 116 | oxidation reduction |
| 117 | Oxidative phosphorylation |
| 118 | p53 signaling pathway |
| 119 | Pancreatic cancer, also in category 644 |
| 120 | Parkinson's disease |
| 121 | Pathways in cancer, also in category 644 |
| 122 | phosphatase activity |
| 123 | phosphoprotein phosphatase activity |
| 124 | positive regulation of cellular biosynthetic process |
| 125 | PPAR signaling pathway |
| 126 | Prostate cancer, also in category 644 |
| 127 | Proteasome |
| 128 | protein amino acid dephosphorylation |
| 129 | protein folding |
| 130 | protein kinase activity |
| 131 | protein serine/threonine kinase activity |
| 132 | Purine metabolism |
| 133 | Pyrimidine metabolism |
| 134 | Ras protein signal transduction |
| 135 | Regulation of actin cytoskeleton |
| 136 | Regulation of autophagy |
| 137 | regulation of cell death, also in category 644 |
| 138 | regulation of cell proliferation, also in category 644 |
| 139 | regulation of cell size |
| 140 | regulation of protein ubiquitination |
| 141 | regulation of Ras protein signal transduction |
| 142 | regulation of transcription |
| 143 | Renal cell carcinoma, also in category 644 |
| 144 | response to hypoxia |
| 145 | response to steroid hormone stimulus |
| 146 | response to virus |
| 147 | ribosome |
| 148 | RNA degradation |
| 149 | RNA processing |
| 150 | RNA splicing, via transesterification reactions |
| 151 | secretion |
| 152 | skeletal system development |
| 153 | skeletal system morphogenesis |
| 154 | Small cell lung cancer, also in category 644 |
| 155 | small GTPase regulator activity |
| 156 | spermatogenesis |
| 157 | Sphingolipid metabolism |
| 158 | spliceosome |
| 159 | Spliceosome |
| 160 | stem cell differentiation |
| 161 | Steroid biosynthesis |
| 162 | synapse |
| 163 | Systemic lupus erythematosus |
| 164 | T cell activation |
| 165 | T cell receptor signaling pathway |
| 166 | TGF-beta signaling pathway |
| 167 | Thyroid cancer, also in category 644 |
| 168 | Toll-like receptor signaling pathway |
| 169 | transcription activator activity |
| 170 | transcription factor activity |
| 171 | translation |
| 172 | Type II diabetes mellitus |
| 173 | Ubiquitin mediated proteolysis |
| 174 | Vascular smooth muscle contraction |
| 175 | vasculature development |
| 176 | VEGF signaling pathway |
| 177 | Viral myocarditis |
| 178 | Wnt signaling pathway |
| 179 | amino-acid biosynthesis |
| 180 | ank repeat |
| 181 | bromodomain |
| 182 | Cardiomyopathy |
| 183 | cataract |
| 184 | charcot-marie-tooth disease |
| 185 | cytokine |
| 186 | cytokine receptor |
| 187 | deafness |
| 188 | disease mutation |
| 189 | egf-like domain |
| 190 | endosome |
| 191 | epilepsy |
| 192 | glycoprotein |
| 193 | growth factor |
| 194 | Growth factor binding |
| 195 | growth factor receptor |
| 196 | Ichthyosis |
| 197 | Immunoglobulin domain |
| 198 | ionic channel |
| 199 | leucine-rich repeat |
| 200 | leukodystrophy |
| 201 | methylation |
| 202 | methyltransferase |
| 203 | neurodegeneration |
| 204 | neuropathy |
| 205 | nucleus |
| 206 | obesity |
| 207 | protein phosphatase |
| 208 | protein phosphatase inhibitor |
| 209 | Oncogene (including proto-oncogenes), also in category 644 |
| 210 | Secreted |
| 211 | serine/threonine-specific protein kinase |
| 212 | systemic lupus erythematosus |
| 213 | transmembrane |
| 214 | transmembrane protein |
| 215 | tumor suppressor, also in category 644 |
| 216 | tyro sine-protein kinase |
| 217 | ubl conjugation pathway |
| 218 | wd repeat |
| 300 | Downregulated in Bladder cancer, also in category 644 |
| 301 | Downregulated in Leukemia, also in category 644 |
| 302 | Downregulated in Brain cancer, also in category 644 |
| 303 | Downregulated in Breast cancer, also in category 644 |
| 304 | Downregulated in Cervical cancer, also in category 644 |
| 305 | Downregulated in Colon cancer, also in category 644 |
| 306 | Downregulated in Esophageal cancer, also in category 644 |
| 307 | Downregulated in Gastric cancer, also in category 644 |
| 308 | Downregulated in Head and Neck cancer, also in category 644 |
| 309 | Downregulated in Renal cancer, also in category 644 |
| 310 | Downregulated in Liver cancer, also in category 644 |
| 311 | Downregulated in Lung cancer, also in category 644 |
| 312 | Downregulated in Lymphoma, also in category 644 |
| 313 | Downregulated in Melanoma, also in category 644 |
| 314 | Downregulated in Multiple Myeloma, also in category 644 |
| 315 | Downregulated in Ovarian cancer, also in category 644 |
| 316 | Downregulated in Pancreatic cancer, also in category 644 |
| 317 | Downregulated in Prostate cancer, also in category 644 |
| 318 | Downregulated in Sarcoma, also in category 644 |
| 319 | Downregulated in Non-melanoma skin cancer, also in category 644 |
| 320 | Downregulated in Uterine cancer, also in category 644 |
| 321 | Downregulated in Mesothelioma, also in category 644 |
| 322 | Downregulated in Adrenal cancer, also in category 644 |
| 323 | Downregulated in Parathyroid cancer, also in category 644 |
| 400 | Upregulated in Clear cell sarcoma of kidney, also in category 644 |
| 401 | Upregulated in Acute lung injury |
| 402 | Upregulated in Acute megakaryoblastic leukemia, also in category 644 |
| 403 | Upregulated in Acute myelocytic leukemia, also in category 644 |
| 404 | Upregulated in Acute pancreatitis unspecified |
| 405 | Upregulated in Adenocarcinoma of esophagus, also in category 644 |
| 406 | Upregulated in Adenocarcinoma of lung, also in category 644 |
| 407 | Upregulated in Adenoma of small intestine, also in category 644 |
| 408 | Upregulated in Adenovirus infection |
| 409 | Upregulated in AIDS with encephalitis |
| 410 | Upregulated in Alcohol poisoning |
| 411 | Upregulated in Alexander disease |
| 412 | Upregulated in alpha-1-Antitrypsin deficiency |
| 413 | Upregulated in Alzheimer's disease |
| 414 | Upregulated in Anaplastic oligoastrocytoma, also in category 644 |
| 415 | Upregulated in Androgen insensitivity syndrome |
| 416 | Upregulated in Astrocytoma, also in category 644 |
| 417 | Upregulated in Atrophy - muscular |
| 418 | Upregulated in Autoimmune hepatitis |
| 419 | Upregulated in Bacterial infection |
| 420 | Upregulated in Barrett's esophagus |
| 421 | Upregulated in Carcinoma in situ of small intestin, also in category 644e |
| 422 | Upregulated in Cardiomyopathy |
| 423 | Upregulated in Chronic granulomatous disease |
| 424 | Upregulated in Chronic lymphocytic leukemia |
| 425 | Upregulated in Chronic obstructive airway disease |
| 426 | Upregulated in Chronic polyarticular juvenile rheumatoid arthritis |
| 427 | Upregulated in Cirrhosis of liver |
| 428 | Upregulated in Cocaine dependence |
| 429 | Upregulated in Complex dental caries |
| 430 | Upregulated in Crohn's disease |
| 431 | Upregulated in Decompensated cardiac failure |
| 432 | Upregulated in Dehydration |
| 433 | Upregulated in Dilated cardiomyopathy |
| 434 | Upregulated in Dilated cardiomyopathy secondary to viral myocarditis |
| 435 | Upregulated in Epithelial proliferation |
| 436 | Upregulated in Escherichia coli infection of the central nervous system |
| 437 | Upregulated in Essential thrombocythemia |
| 438 | Upregulated in Exhaustion due to excessive exertion |
| 439 | Upregulated in Familial hypophosphatemic bone disease |
| 440 | Upregulated in Fracture |
| 441 | Upregulated in Fracture of femur |
| 442 | Upregulated in Generalized ischemic myocardial dysfunction |
| 443 | Upregulated in Glioblastoma, also in category 644 |
| 444 | Upregulated in Hamman-Rich syndrome |
| 445 | Upregulated in Helicobacter pylori gastrointestinal tract infection |
| 446 | Upregulated in Hepatitis C |
| 447 | Upregulated in HIV infection |
| 448 | Upregulated in Huntington's disease |
| 449 | Upregulated in Hypercholesterolemia |
| 450 | Upregulated in Hypertrophy |
| 451 | Upregulated in Idiopathic thrombocytopenic purpura |
| 452 | Upregulated in Infection by Yersinia enterocolitica |
| 453 | Upregulated in Infertility due to azoospermia |
| 454 | Upregulated in Injury of heart |
| 455 | Upregulated in ISM - In situ melanoma of skin |
| 456 | Upregulated in Leber's amaurosis |
| 457 | Upregulated in Liver carcinoma, also in category 644 |
| 458 | Upregulated in Macular degeneration |
| 459 | Upregulated in Malignant lymphoma, also in category 644 |
| 460 | Upregulated in Malignant neoplasm of cervix uteri, also in category 644 |
| 461 | Upregulated in Malignant neoplasm of duodenum, also in category 644 |
| 462 | Upregulated in Malignant neoplasm of prostate, also in category 644 |
| 463 | Upregulated in Malignant neoplasm of stomach, also in category 644 |
| 464 | Upregulated in Malignant neoplasm of testis, also in category 644 |
| 465 | Upregulated in Malignant tumor of colon, also in category 644 |
| 466 | Upregulated in Multiple benign melanocytic nevi |
| 467 | Upregulated in Nephropathy - diabetic |
| 468 | Upregulated in Non-insulin dependent diabetes mellitus |
| 469 | Upregulated in Nutritional deficiency |
| 470 | Upregulated in Obstructive sleep apnea |
| 471 | Upregulated in Oligodendroglioma, also in category 644 |
| 472 | Upregulated in Papillary thyroid carcinoma, also in category 644 |
| 473 | Upregulated in Parkinson disease |
| 474 | Upregulated in Porcine nephropathy |
| 475 | Upregulated in Pre-eclampsia |
| 476 | Upregulated in Primary cardiomyopathy |
| 477 | Upregulated in Primary open angle glaucoma |
| 478 | Upregulated in Primary pulmonary hypoplasia |
| 479 | Upregulated in Pseudomonas infection |
| 480 | Upregulated in Pulmonary emphysema |
| 481 | Upregulated in Pulmonary hypertension |
| 482 | Upregulated in Renal disorder associated with type II diabetes mellitus |
| 483 | Upregulated in Retinal damage |
| 484 | Upregulated in Retinitis pigmentosa |
| 485 | Upregulated in Rheumatoid arthritis |
| 486 | Upregulated in Squamous cell carcinoma, also in category 644 |
| 487 | Upregulated in Squamous cell carcinoma of lung, also in category 644 |
| 488 | Upregulated in Status epilepticus |
| 489 | Upregulated in Systemic infection |
| 490 | Upregulated in Thrombocytopenia |
| 491 | Upregulated in Thymic carcinoma, also in category 644 |
| 492 | Upregulated in Transitional cell carcinoma, also in category 644 |
| 493 | Upregulated in Transitional cell carcinoma in situ, also in category 644 |
| 494 | Upregulated in Ulcerative colitis |
| 495 | Upregulated in Uterine fibroids |
| 496 | Upregulated in Ventilator-associated lung injury |
| 497 | Upregulated in Ventricular hypertrophy |
| 498 | Upregulated in Ventricular hypertrophy (& [left]) |
| 499 | Upregulated in Vitamin A deficiency |
| 500 | Downregulated in Clear cell sarcoma of kidney, also in category 644 |
| 501 | Downregulated in Acute lung injury |
| 502 | Downregulated in Acute megakaryoblastic leukemia, also in category 644 |
| 503 | Downregulated in Acute myelocytic leukemia, also in category 644 |
| 504 | Downregulated in Acute pancreatitis unspecified |
| 505 | Downregulated in Adenocarcinoma of esophagus, also in category 644 |
| 506 | Downregulated in Adenocarcinoma of lung, also in category 644 |
| 507 | Downregulated in Adenoma of small intestine, also in category 644 |
| 508 | Downregulated in Adenovirus infection |
| 509 | Downregulated in AIDS with encephalitis |
| 510 | Downregulated in Alcohol poisoning |
| 511 | Downregulated in Alexander disease |
| 512 | Downregulated in alpha-1-Antitrypsin deficiency |
| 513 | Downregulated in Alzheimer's disease |
| 514 | Downregulated in Anaplastic oligoastrocytoma |
| 515 | Downregulated in Androgen insensitivity syndrome |
| 516 | Downregulated in Astrocytoma, also in category 644 |
| 517 | Downregulated in Atrophy - muscular |
| 518 | Downregulated in Autoimmune hepatitis |
| 519 | Downregulated in Bacterial infection |
| 520 | Downregulated in Barrett's esophagus |
| 521 | Downregulated in Carcinoma in situ of small intestine, also in category 644 |
| 522 | Downregulated in Cardiomyopathy |
| 523 | Downregulated in Chronic granulomatous disease |
| 524 | Downregulated in Chronic lymphocytic leukemia |
| 525 | Downregulated in Chronic obstructive airway disease |
| 526 | Downregulated in Chronic polyarticular juvenile rheumatoid arthritis |
| 527 | Downregulated in Cirrhosis of liver |
| 528 | Downregulated in Cocaine dependence |
| 529 | Downregulated in Complex dental caries |
| 530 | Downregulated in Crohn's disease |
| 531 | Downregulated in Decompensated cardiac failure |
| 532 | Downregulated in Dehydration |
| 533 | Downregulated in Dilated cardiomyopathy |
| 534 | Downregulated in Dilated cardiomyopathy secondary to viral myocarditis |
| 535 | Downregulated in Epithelial proliferation |
| 536 | Downregulated in Escherichia coli infection of the central nervous system |
| 537 | Downregulated in Essential thrombocythemia |
| 538 | Downregulated in Exhaustion due to excessive exertion |
| 539 | Downregulated in Familial hypophosphatemic bone disease |
| 540 | Downregulated in Fracture |
| 541 | Downregulated in Fracture of femur |
| 542 | Downregulated in Generalized ischemic myocardial dysfunction |
| 543 | Downregulated in Glioblastoma, also in category 644 |
| 544 | Downregulated in Hamman-Rich syndrome |
| 545 | Downregulated in Helicobacter pylori gastrointestinal tract infection |
| 546 | Downregulated in Hepatitis C |
| 547 | Downregulated in HIV infection |
| 548 | Downregulated in Huntington's disease |
| 549 | Downregulated in Hypercholesterolemia |
| 550 | Downregulated in Hypertrophy |
| 551 | Downregulated in Idiopathic thrombocytopenic purpura |
| 552 | Downregulated in Infection by Yersinia enterocolitica |
| 553 | Downregulated in Infertility due to azoospermia |
| 554 | Downregulated in Injury of heart |
| 555 | Downregulated in ISM - In situ melanoma of skin, also in category 644 |
| 556 | Downregulated in Leber's amaurosis |
| 557 | Downregulated in Liver carcinoma, also in category 644 |
| 558 | Downregulated in Macular degeneration |
| 559 | Downregulated in Malignant lymphoma, also in category 644 |
| 560 | Downregulated in Malignant neoplasm of cervix uteri, also in category 644 |
| 561 | Downregulated in Malignant neoplasm of duodenum, also in category 644 |
| 562 | Downregulated in Malignant neoplasm of prostate, also in category 644 |
| 563 | Downregulated in Malignant neoplasm of stomach, also in category 644 |
| 564 | Downregulated in Malignant neoplasm of testis, also in category 644 |
| 565 | Downregulated in Malignant tumor of colon, also in category 644 |
| 566 | Downregulated in Multiple benign melanocytic nevi |
| 567 | Downregulated in Nephropathy - diabetic |
| 568 | Downregulated in Non-insulin dependent diabetes mellitus |
| 569 | Downregulated in Nutritional deficiency |
| 570 | Downregulated in Obstructive sleep apnea |
| 571 | Downregulated in Oligodendroglioma |
| 572 | Downregulated in Papillary thyroid carcinoma |
| 573 | Downregulated in Parkinson disease |
| 574 | Downregulated in Porcine nephropathy |
| 575 | Downregulated in Pre-eclampsia |
| 576 | Downregulated in Primary cardiomyopathy |
| 577 | Downregulated in Primary open angle glaucoma |
| 578 | Downregulated in Primary pulmonary hypoplasia |
| 579 | Downregulated in Pseudomonas infection |
| 580 | Downregulated in Pulmonary emphysema |
| 581 | Downregulated in Pulmonary hypertension |
| 582 | Downregulated in Renal disorder associated with type II diabetes mellitus |
| 583 | Downregulated in Retinal damage |
| 584 | Downregulated in Retinitis pigmentosa |
| 585 | Downregulated in Rheumatoid arthritis |
| 586 | Downregulated in Squamous cell carcinoma, also in category 644 |
| 587 | Downregulated in Squamous cell carcinoma of lung, also in category 644 |
| 588 | Downregulated in Status epilepticus |
| 589 | Downregulated in Systemic infection |
| 590 | Downregulated in Thrombocytopenia |
| 591 | Downregulated in Thymic carcinoma, also in category 644 |
| 592 | Downregulated in Transitional cell carcinoma, also in category 644 |
| 593 | Downregulated in Transitional cell carcinoma in situ, also in category 644 |
| 594 | Downregulated in Ulcerative colitis |
| 595 | Downregulated in Uterine fibroids |
| 596 | Downregulated in Ventilator-associated lung injury |
| 597 | Downregulated in Ventricular hypertrophy |
| 598 | Downregulated in Ventricular hypertrophy (& [left]) |
| 599 | Downregulated in Vitamin A deficiency |
| 600 | is associated with Bone diseases |
| 601 | is associated with Cancer diseases, also in category 644 |
| 602 | is associated with Cardiovascular diseases |
| 603 | is associated with Connective tissue disorder diseases |
| 604 | is associated with Dermatological diseases |
| 605 | is associated with Developmental diseases |
| 606 | is associated with Ear,Nose,Throat diseases |
| 607 | is associated with Endocrine diseases |
| 608 | is associated with Gastrointestinal diseases |
| 609 | is associated with Hematological diseases |
| 610 | is associated with Immunological diseases |
| 611 | is associated with Metabolic diseases |
| 612 | is associated with multiple diseases |
| 613 | is associated with Muscular diseases |
| 614 | is associated with Neurological diseases |
| 615 | is associated with Nutritional diseases |
| 616 | is associated with Ophthamological diseases |
| 617 | is associated with Other diseases |
| 618 | is associated with Psychiatric diseases |
| 619 | is associated with Renal diseases |
| 620 | is associated with Respiratory diseases |
| 621 | is associated with Skeletal diseases |
| 622 | is decreased in Bone diseases |
| 623 | is decreased in Cancer diseases, also in category 644 |
| 624 | is decreased in Cardiovascular diseases |
| 625 | is decreased in Connective tissue disorder diseases |
| 626 | is decreased in Dermatological diseases |
| 627 | is decreased in Developmental diseases |
| 628 | is decreased in Ear,Nose,Throat diseases |
| 629 | is decreased in Endocrine diseases |
| 630 | is decreased in Gastrointestinal diseases |
| 631 | is decreased in Hematological diseases |
| 632 | is decreased in Immunological diseases |
| 633 | is decreased in Metabolic diseases |
| 634 | is decreased in multiple diseases |
| 635 | is decreased in Muscular diseases |
| 636 | is decreased in Neurological diseases |
| 637 | is decreased in Nutritional diseases |
| 638 | is decreased in Ophthamological diseases |
| 639 | is decreased in Other diseases |
| 640 | is decreased in Psychiatric diseases |
| 641 | is decreased in Renal diseases |
| 642 | is decreased in Respiratory diseases |
| 643 | is decreased in Skeletal diseases |
| 644 | is involved in cancer |

Thus, in various aspects, the disclosure features oligonucleotides that specifically bind to any of the RNA sequences disclosed herein, for use in modulating expression of genes.In another aspect, the disclosure also features oligonucleotides that specifically bind, or are complementary, to any of the RNA sequences of sequences B47,408 to B616,428 [mouse Peaks] or B652,256 to B916,209 [human Peaks] or B916,626 to B934,761 - [longer region surrounding human Peaks], whether in the "opposite strand" or the "same strand" as a target gene (e.g., as indicated in Table 2 of International Patent Application Publication WO/2012/087983). In some embodiments, the oligonucleotide is provided for use in a method of modulating expression of a gene targeted by the PRC2 binding RNA (*e.g.,* an intersecting or nearby gene). Such methods may be carried out *in vitro, ex vivo,* or *in vivo.* In some embodiments, the oligonucleotide is provided for use in methods of treating disease. The treatments may involve modulating expression of a gene targeted by the PRC2 binding RNA, preferably upregulating gene expression. In some embodiments, the oligonucleotide is formulated as a sterile composition for parenteral administration. The reference genes targeted by these RNA sequences are set forth in Tables 2-3 and are grouped according to categories 1- 644 in Table 3 of International Patent Application Publication WO/2012/087983 or are imprinted genes set forth in Table 2. Thus, in one aspect the disclosure describes a group of oligonucleotides that specifically bind, or are complementary to, a group of RNA sequences, either transcripts or Peaks, in any one of categories 1-644. In particular, the disclosure features uses of such oligonucleotides to upregulate expression of any of the reference genes set forth in Tables 2, for use in treating a disease, disorder, condition or association described in any of the categories set forth in Table 3 of International Patent Application Publication WO/2012/087983 (*e.g.,* any one or more of category numbers 11, 14, 15, 17, 21, 24, 26, 42, 44, 49, 58, 69, 82, 103, 119, 120, 126, 143, 163, 167, 172, 177, 182, 183, 184, 187, 191, 196, 200, 203, 204, 212, 300 323, and/or 400-644).

By way of non-limiting example, category 45 (Complement and coagulation cascades) includes reference genes selected from the group consisting of A2M, SERPINC1, BDKRB1, BDKRB2, CFB, SERPING1, C1QA, C1QB, C1QC, C1R, CIS, C2, C3, C3AR1, C4A, C4B, C4BPA, C4BPB, C5, C5AR1, C6, C7, C8A, C8B, C9, CD59, CPB2, CR1, CR2, CD55, CFD, F2, F3, F5, F7, F8, F9, F10, F11, F12, F13A1, F13B, FGA, FGB, FGG, SERPIND1, CFH, CFI, KLKB1, KNG1, MBL2, CD46, SERPINE1, SERPINA1, PLAT, PLAU, PLAUR, PLG, SERPINF2, PROC, PROS1, MASP1, TFPI, THBD, VWF and/or MASP2.

In turn, each of A2M, SERPINC1, BDKRB1, BDKRB2, CFB, SERPING1, C1QA, C1QB, C1QC, C1R, CIS, C2, C3, C3AR1, C4A, C4B, C4BPA, C4BPB, C5, C5AR1, C6, C7, C8A, C8B, C9, CD59, CPB2, CR1, CR2, CD55, CFD, F2, F3, F5, F7, F8, F9, F10, F11, F12, F13A1, F13B, FGA, FGB, FGG, SERPIND1, CFH, CFI, KLKB1, KNG1, MBL2, CD46, SERPINE1, SERPINA1, PLAT, PLAU, PLAUR, PLG, SERPINF2, PROC, PROS1, MASP1, TFPI, THBD, VWF and/or MASP2 are targeted by PRC2-associated RNA having the sequence identifiers displayed in the applicable row of Table 2 of International Patent Application Publication WO/2012/087983. For example, F2 targeting sequences include sequences: B620037 [F], B620035 [4027], B790730 [4752], B4539 [2059], B341288 [ 3278], B4537 [4639] on the same strand as the coding gene, and sequences: B620036 [F], B790731 - [F], B4538 [F], B341286 [F], B341287 [F] on the opposite strand from the coding gene, according to Table 2 of International Patent Application Publication WO/2012/087983.

The group of oligonucleotides that specifically bind to, or are complementary to, any one of these sequences that are listed in Table 2 of International Patent Application Publication WO/2012/087983 as targeting refGenes A2M, SERPINC1, BDKRB1, BDKRB2, CFB, SERPING1, C1QA, C1QB, C1QC, C1R, C1S, C2, C3, C3AR1, C4A, C4B, C4BPA, C4BPB, C5, C5AR1, C6, C7, C8A, C8B, C9, CD59, CPB2, CR1, CR2, CD55, CFD, F2, F3, F5, F7, F8, F9, F10, F11, F12, F13A1, F13B, FGA, FGB, FGG, SERPIND1, CFH, CFI, KLKB1, KNG1, MBL2, CD46, SERPINE1, SERPINA1, PLAT, PLAU, PLAUR, PLG, SERPINF2, PROC, PROS1, MASP1, TFPI, THBD, VWF and/or MASP2 are contemplated for use in any of the compositions and methods described herein, including but not limited to use in treating a disease of category 45 (Complement and coagulation cascades), the treatment involving modulation of any of the refGenes A2M, SERPINC1, BDKRB1, BDKRB2, CFB, SERPING1, C1QA, C1QB, C1QC, C1R, C1S, C2, C3, C3AR1, C4A, C4B, C4BPA, C4BPB, C5, C5AR1, C6, C7, C8A, C8B, C9, CD59, CPB2, CR1, CR2, CD55, CFD, F2, F3, F5, F7, F8, F9, F10, F11, F12, F13A1, F13B, FGA, FGB, FGG, SERPIND1, CFH, CFI, KLKB1, KNG1, MBL2, CD46, SERPINE1, SERPNIA1, PLAT, PLAU, PLAUR, PLG, SERPINF2, PROC, PROS1, MASP1, TFPI, THBD, VWF and/or MASP2.

Similarly, oligonucleotides that specifically bind to, or are complementary to, genes in category 643 ("is decreased in Skeletal disease") are contemplated for use in any of the compositions and methods described herein, including but not limited to use in treating Skeletal disease. Oligonucleotides that specifically bind to, or are complementary to, genes in the categories that are also part of category 644 (involved in cancer) are contemplated for use in any of the compositions and methods described herein, including but not limited to use in treating cancer.

It is understood that oligonucleotides for use in the invention may be complementary to, or specifically bind to, Peaks, or non-Peak regions of transcripts disclosed herein, or regions adjacent to Peaks. In various aspects, the disclosure also features oligonucleotides that bind to the RNA sequence between two or more Peaks that correspond to chromosomal coordinates that are near each other, *e.g.,* within 100 bases, 200 bases, 300 bases, 400 bases, 500 bases, 1kb, or 2kb, of each other, and that are preferably associated with the same reference gene in Table 8 of International Patent Application Publication WO/2012/065143 or Table 2 of International Patent Application: PCT/US2011/65939. For example, the disclosure features oligonucleotides that specifically bind, or are complementary to, a fragment of any of the RNA transcripts of sequences A1 to A21582 or A191089 to A 193049 or B1 to B47,407 or B934,762 to B934,863[mouse transcripts] or B616,429 to B652,255 or B916,210 to B916,625 or B934,864 to B934,968 [human transcripts] or B916,626 to B934,761 [larger region surrounding human Peaks], said fragment about 2000, about 1750, about 1500, about 1250 nucleotides in length, or preferably about 1000, about 750, about 500, about 400, about 300 nucleotides in length, or more preferably about 200, about 150, or about 100 nucleotides in length, wherein the fragment of RNA comprises a stretch of at least five (5) consecutive nucleotides within any of sequences A124437 to A190716, or A 190934 to A191086, or A191087 [human Peaks], or sequences A21583 to A124436, or A190717 to 190933, or 191088 [mouse Peaks], or sequences B47,408 to B616,428 [mouse Peaks] or sequences B652,256 to B916,209 [human Peaks], or the reverse complement of any of the cDNA sequences of Appendix I of U.S. Prov. Appl. No. 61/425,174 filed on December 20, 2010. In exemplary embodiments the fragment of RNA comprises at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 consecutive nucleotides within any of sequences A124437 to A190716, or A190934 to A191086, or A191087 [human Peaks], or sequences A21583 to A124436, or A190717 to A190933, or A191088 [mouse Peaks], or sequences B47,408 to B616,428 [mouse Peaks] or sequences B652,256 to B916,209 [human Peaks], or the reverse complement of any of the cDNA sequences of Appendix I of U.S. Prov. Appl. No. 61/425,174 filed on December 20, 2010.

Thus, for example, this description includes oligonucleotides that bind to fragments about 2000, about 1750, about 1500, about 1250 nucleotides in length, or preferably about 1000, about 750, about 500, about 400, about 300 nucleotides in length, or more preferably about 200, about 150, or about 100 nucleotides in length, which are:
(a) fragments of any of sequences B1-B47407 [mouse transcripts] that comprise a stretch of at least five (5) consecutive nucleotides, or 6, 7, 8, 9 or 10 or more consecutive nucleotides, within any of sequences B47408-B616428 [mouse Peaks], preferably associated with the same reference gene in Table 2 of International Patent Application Publication WO/2012/087983;
(b) fragments of any of sequences B616429-B652255 [human transcripts] that comprise a stretch of at least five (5) consecutive nucleotides, or 6, 7, 8, 9 or 10 or more consecutive nucleotides, within any of sequences B652256-B916209 [human Peaks], preferably associated with the same reference gene in Table 2 of International Patent Application Publication WO/2012/087983;
(c) fragments of any of sequences B916626-B934761 - [longer regions around human Peaks] that comprise a stretch of at least five (5) consecutive nucleotides, or 6, 7, 8, 9 or 10 or more consecutive nucleotides, within any of sequences B652256-B916209 [human Peaks], preferably associated with the same reference gene in Table 2 of International Patent Application Publication WO/2012/087983;
(d) fragments of any of sequences B934762-B934863 [mouse imprinted transcripts] that encompass that comprise a stretch of at least five (5) consecutive nucleotides, or 6, 7, 8, 9 or 10 or more consecutive nucleotides, within any of sequences B47408-B616428 [mouse Peaks], preferably associated with the same reference gene in Table 2 of International Patent Application Publication WO/2012/087983 or Table 2;
(e) fragments of any of sequences B629991, B629992, B630983, B630984, B630990, B631003, B631004, B632396, B632397, B632402, B632403, B632419, B632422, B634959, B638303, B638304, B647595, B647596, B647597, B647598, B647601, B649028, and B934864-B934931 - [human imprinted transcripts] that comprise a stretch of at least five (5) consecutive nucleotides, or 6, 7, 8, 9 or 10 or more consecutive nucleotides, within any of sequences B652256-B916209 [human Peaks], preferably associated with the same reference gene in Table 2 of International Patent Application Publication WO/2012/087983 or Table 2;
(f) fragments of any of sequences B916210-B916625 [human transcripts] that comprise a stretch of at least five (5) consecutive nucleotides, or 6, 7, 8, 9 or 10 or more consecutive nucleotides, within any of sequences B652256-B916209 [human Peaks], preferably associated with the same reference gene in Table 2 of International Patent Application Publication WO/2012/087983; or
(g) fragments of any of sequences B934932-B934968 [human transcripts] that comprise a stretch of at least five (5) consecutive nucleotides, or 6, 7, 8, 9 or 10 or more consecutive nucleotides, within any of sequences B652256-B916209 [human Peaks], preferably associated with the same reference gene in Table 2 of International Patent Application Publication WO/2012/087983.

Thus, as noted above, the oligonucleotide can comprise or consist of a sequence of bases at least 80% complementary to at least 10, or 10-30 or 10-40 contiguous bases of the target RNA, or at least 80% complementary to at least 15, or 15-30, or 15-40 contiguous bases of the target RNA, or at least 80% complementary to at least 20, or 20-30, or 20-40 contiguous bases of the target RNA, or at least 80% complementary to at least 25, or 25-30, or 25-40 contiguous bases of the target RNA, or at least 80% complementary to at least 30, or 30-40 contiguous bases of the target RNA, or at least 80% complementary to at least 40 contiguous bases of the target RNA. Moreover, the oligonucleotide can comprise or consist of a sequence of bases at least 90% complementary to at least 5, or 5-30 or 5-40 or 8-40 contiguous bases of the target RNA, or at least 90% complementary to at least 10, or 10-30, or 10-40 contiguous bases of the target RNA, or at least 90% complementary to at least 15, or 15-30, or 15-40 contiguous bases of the target RNA, or at least 90% complementary to at least 20, or 20-30, or 20-40 contiguous bases of the target RNA, or at least 90% complementary to at least 25, or 25-30, or 25-40 contiguous bases of the target RNA, or at least 90% complementary to at least 30, or 30-40 contiguous bases of the target RNA, or at least 90% complementary to at least 40 contiguous bases of the target RNA. Similarly, the oligonucleotide can comprise or consist of a sequence of bases fully complementary to at least 5, 10, or 15 contiguous bases of the target RNA. It is understood that some additional non complementary bases may be included. It is understood that oligonucleotides that comprise such sequences of bases as described may also comprise other non-complementary bases. For example, an oligonucleotide can be 20 bases in total length but comprise a 15 base portion that is fully complementary to 15 bases of the target RNA. Similarly, an oligonucleotide can be 20 bases in total length but comprise a 15 base portion that is at least 80% complementary to 15 bases of the target RNA.

Complementarity can also be referenced in terms of the number of mismatches in complementary base pairing, as noted above. Thus, the oligonucleotide can comprise or consist of a sequence of bases with up to 3 mismatches over 10 contiguous bases of the target RNA, or up to 3 mismatches over 15 contiguous bases of the target RNA, or up to 3 mismatches over 20 contiguous bases of the target RNA, or up to 3 mismatches over 25 contiguous bases of the target RNA, or up to 3 mismatches over 30 contiguous bases of the target RNA. Similarly, the oligonucleotide can comprise or consist of a sequence of bases with up to 2 mismatches over 10 contiguous bases of the target RNA, or up to 2 mismatches over 15 contiguous bases of the target RNA, or up to 2 mismatches over 20 contiguous bases of the target RNA, or up to 2 mismatches over 25 contiguous bases of the target RNA, or up to 2 mismatches over 30 contiguous bases of the target RNA. Similarly, the oligonucleotide can comprise or consist of a sequence of bases with one mismatch over 10, 15, 20, 25 or 30 contiguous bases of the target RNA.

In some or any of the embodiments of oligonucleotides described herein (*e.g.,* in the summary, detailed description, or examples of embodiments) or the processes for designing or synthesizing them, the oligonucleotides may optionally exclude any one or more of the oligonucleotides as disclosed in any one or more of the following publications: as target HOTAIR RNA (Rinn et al., 2007), Tsix, RepA, or Xist RNAs ((Zhao et al., 2008) [sequences B936166 - B936170], or (Sarma et al., 2010) [sequences B936177 - B936186] or (Zhao et al., 2010) [sequences B936187 - B936188] or (Prasnath et al., 2005) [sequences B936173 - B936176]. or (Shamovsky et al., 2006) [sequence B936172] or (Mariner et al., 2008) [sequence B936171] or (Sunwoo et al., 2008) or (Bernard et al., 2010) [sequence B936189]; or as targeting short RNAs of 50 200 nt that are identified as candidate PRC2 regulators (Kanhere et al., 2010); or (Kuwabara et al., US 2005/0226848) [sequences B936190 - B936191] or (Li et al., US 2010/0210707) [sequences B936192 - B936227] or (Corey et al., 7,709,456) [sequences B936228 - B936245] or (Mattick et al., WO 2009/124341), or (Corey et al., US 2010/0273863) [sequences B936246 - B936265], or (Wahlstedt et al., US 2009/0258925) [sequences B935060 - B935126], or BACE: US 2009/0258925 [sequences B935060 - B935126]; ApoA1: US 2010/0105760 / EP235283 [sequences B935127 - B935299], P73, p53, PTEN, WO 2010/065787 A2 / EP2370582 [sequences B935300 - B935345]; SIRT1: WO 2010/065662 A2 / EP09831068 [sequences B935346 - B935392]; VEGF: WO 2010/065671 - A2 / EP2370581 - [sequences B935393 - B935403]; EPO: WO 2010/065792 A2 / EP09831152 [sequences B935404 - B935412]; BDNF: WO2010/093904 [sequences B935413 - B935423], DLK1: WO 2010/107740 [sequences B935424 - B935430]; NRF2/NFE2L2: WO 2010/107733 [sequences B935431 - - B935438]; GDNF: WO 2010/093906 [sequences B935439 - B935476]; SOX2, KLF4, Oct3A/B, "reprogramming factors: WO 2010/135329 [sequences B935477 - B935493]; Dystrophin: WO 2010/129861 - [sequences B935494 - B935525]; ABCA1, LCAT, LRP1, ApoE, LDLR, ApoA1: WO 2010/129799 [sequences B935526 - B935804]; HgF: WO 2010/127195 [sequences B935805 - B935809]; TTP/Zfp36: WO 2010/129746[sequences B935810 - B935824]; TFE3, IRS2: WO 2010/135695 [sequences B935825 - B935839]; RIG1, MDA5, IFNA1: WO 2010/138806 [sequences B935840 - B935878]; PON1: WO 2010/148065 [sequences B935879 - B935885]; Collagen: WO/2010/148050 [sequences B935886 - B935918]; Dyrk1A, Dscr1, "Down Syndrome Gene": WO/2010/151674 [sequences B935919 - B935942]; TNFR2: WO/2010/151671 - [sequences B935943 - B935951]; Insulin: WO/2011/017516 [sequences B935952 - B935963]; ADIPOQ: WO/2011/019815 [sequences B935964 - B935992]; CHIP: WO/2011/022606 [sequences B935993 - B936004]; ABCB1: WO/2011/025862 [sequences B936005 - B936014]; NEUROD1, EUROD1, HNF4A, MAFA, PDX, KX6, "Pancreatic development gene": WO/2011/085066 [sequences B936015 - B936054]; MBTPS1: WO/2011/084455 [sequences B936055 - B936059]; SHBG: WO/2011/085347 [sequences B936060 - B936075]; IRF8: WO/2011/082409 [sequences B936076 - B936080]; UCP2: WO/2011/079263 [sequences B936081 - - B936093]; HGF: WO/2011/079261 - [sequences B936094 - B936104]; GH: WO/2011/038205 [sequences B936105 - B936110]; IQGAP: WO/2011/031482 [sequences B936111 - - B936116]; NRF1: WO/2011/090740 [sequences B936117 - B936123]; P63: WO/2011/090741 - [sequences B936124 - B936128]; RNAseH1: WO/2011/091390 [sequences B936129 - B936140]; ALOX12B: WO/2011/097582 [sequences B936141 - - B936146]; PYCR1: WO/2011/103528 [sequences B936147 - B936151]; CSF3: WO/2011/123745 [sequences B936152 - B936157]; FGF21: WO/2011/127337 [sequences B936158 - B936165]; SIRTUIN (SIRT): WO2011/139387 [sequences B936266 - B936369 and B936408 - B936425]; PAR4: WO2011/143640 [sequences B936370 - B936376 and B936426]; LHX2: WO2011/146675 [sequences B936377 - B936388 and B936427 B936429]; BCL2L11: WO2011/146674 [sequences B936389 - B936398 and B936430 B936431]; MSRA: WO2011/150007 [sequences B936399 - B936405 and B936432]; ATOH1: WO2011/150005 [sequences B936406 - B936407 and B936433]. In some or any of the embodiments, optionally excluded from the invention are of oligonucleotides that specifically bind to, or are complementary to, any one or more of the following regions: Nucleotides 1 - 932 of sequence B935128; Nucleotides 1 - 1675 of sequence B935306; Nucleotides 1 - 518 of sequence B935307; Nucleotides 1 - 759 of sequence B935308; Nucleotides 1 - 25892 of sequence B935309; Nucleotides 1 - 279 of sequence B935310; Nucleotides 1 - 1982 of sequence B935311; Nucleotides 1 - 789 of sequence B935312; Nucleotides 1 - 467 of sequence B935313; Nucleotides 1 - 1028 of sequence B935347; Nucleotides 1 - 429 of sequence B935348; Nucleotides 1 - 156 of sequence B935349; Nucleotides 1 - 593 of sequence B935350; Nucleotides 1 - 643 of sequence B935395; Nucleotides 1 - 513 of sequence B935396; Nucleotides 1 - 156 of sequence B935406; Nucleotides 1 - 3175 of sequence B935414; Nucleotides 1 - 1347 of sequence B935426; Nucleotides 1 - 5808 of sequence B935433; Nucleotides 1 - 237 of sequence B935440; Nucleotides 1 - 1246 of sequence B935441; Nucleotides 1 - 684 of sequence B935442; Nucleotides 1 - 400 of sequence B935473; Nucleotides 1 - 619 of sequence B935474;Nucleotides 1 - 813 of sequence B935475; Nucleotides 1 - 993 of sequence B935480; Nucleotides 1 - 401 of sequence B935480; Nucleotides 1 - 493 of sequence B935481; Nucleotides 1 - 418 of sequence B935482; Nucleotides 1 - 378 of sequence B935496; Nucleotides 1 - 294 of sequence B935497; Nucleotides 1 - 686 of sequence B935498; Nucleotides 1 - 480 of sequence B935499; Nucleotides 1 - 501 of sequence B935500; Nucleotides 1 - 1299 of sequence B935533; Nucleotides 1 - 918 of sequence B935534; Nucleotides 1 - 1550 of sequence B935535; Nucleotides 1 - 329 of sequence B935536; Nucleotides 1 - 1826 of sequence B935537; Nucleotides 1 - 536 of sequence B935538; Nucleotides 1 - 551 of sequence B935539; Nucleotides 1 - 672 of sequence B935540; Nucleotides 1 - 616 of sequence B935541; Nucleotides 1 - 471 of sequence B935542; Nucleotides 1 - 707 of sequence B935543; Nucleotides 1 - 741 of sequence B935544; Nucleotides 1 - 346 of sequence B935545; Nucleotides 1 - 867 of sequence B935546; Nucleotides 1 - 563 of sequence B935547; Nucleotides 1 - 970 of sequence B935812; Nucleotides 1 - 1117 of sequence B935913; Nucleotides 1 - 297 of sequence B935814; Nucleotides 1 - 497 of sequence B935827; Nucleotides 1 - 1267 of sequence B935843; Nucleotides 1 - 586 of sequence B935844; Nucleotides 1 - 741 of sequence B935845; Nucleotides 1 - 251 of sequence B935846 ; Nucleotides 1 - 681 of sequence B935847; Nucleotides 1 - 580 of sequence B935848; Nucleotides 1 - 534 of sequence B935880; Nucleotides 1 - 387 of sequence B935889; Nucleotides 1 - 561 of sequence B935890; Nucleotides 1 - 335 of sequence B935891; Nucleotides 1 - 613 of sequence B935892; Nucleotides 1 - 177 of sequence B935893; Nucleotides 1 - 285 of sequence B935894; Nucleotides 1 - 3814 of sequence B935921; Nucleotides 1 - 633 of sequence B935922; Nucleotides 1 - 497 of sequence B935923 Nucleotides 1 - 545 of sequence B935924; Nucleotides 1 - 413 of sequence B935950; Nucleotides 1 - 413 of sequence B935951; Nucleotides 1 - 334 of sequence B935962; Nucleotides 1 - 582 of sequence B935963; Nucleotides 1 - 416 of sequence B935964; Nucleotides 1 - 3591 of sequence B935990; Nucleotides 1 - 875 of sequence B935991; Nucleotides 1 - 194 of sequence B935992; Nucleotides 1 - 2074 of sequence B936003; Nucleotides 1 - 1237 of sequence B936004; Nucleotides 1 - 4050 of sequence B936013; Nucleotides 1 - 1334 of sequence B936014; Nucleotides 1 - 1235 of sequence B936048; Nucleotides 1 - 17,964 of sequence B936049; Nucleotides 1 - 50,003 of sequence B936050; Nucleotides 1 - 486 of sequence B936051; Nucleotides 1 - 494 of sequence B936052; Nucleotides 1 - 1992 of sequence B936053; Nucleotides 1 - 1767 of sequence B936054; Nucleotides 1 - 1240 of sequence B936059; Nucleotides 1 - 3016 of sequence B936074; Nucleotides 1 - 1609 of sequence B936075; Nucleotides 1 - 312 of sequence B936080; Nucleotides 1 - 243 of sequence B936092; Nucleotides 1 - 802 of sequence B936093; Nucleotides 1 - 514 of sequence B936102; Nucleotides 1 - 936 of sequence B936103; Nucleotides 1 - 1075 of sequence B936104; Nucleotides 1 - 823 of sequence B936110; Nucleotides 1 - 979 of sequence B936116; Nucleotides 1 - 979 of sequence B936123; Nucleotides 1 - 288 of sequence B936128; Nucleotides 1 - 437 of sequence B936137; Nucleotides 1 - 278 of sequence B936138; Nucleotides 1 - 436 of sequence B936139; Nucleotides 1 - 1140 of sequence B936140; Nucleotides 1 - 2082 of sequence B936146; Nucleotides 1 - 380 of sequence B936151; Nucleotides 1 - 742 of sequence B936157; Nucleotides 1 - 4246 of sequence B936165; Nucleotides 1 - 1028 of sequence B936408; Nucleotides 1 - 429 of sequence B936409; Nucleotides 1 - 508 of sequence B936410; Nucleotides 1 - 593 of sequence B936411; Nucleotides 1 - 373 of sequence B936412; Nucleotides 1 - 1713 of sequence B936413; Nucleotides 1 - 660 of sequence B936414; Nucleotides 1 - 589 of sequence B936415; Nucleotides 1 - 726 of sequence B936416; Nucletides 1 - 320 of sequence B936417; Nucletides 1 - 616 of sequence B936418; Nucletides 1 - 492 of sequence B936419; Nucletides 1 - 428 of sequence B936420; Nucletides 1 - 4041 of sequence B936421; Nucletides 1 - 705 of sequence B936422; Nucletides 1 - 2714 of sequence B936423; Nucletides 1 - 1757 of sequence B936424; Nucletides 1 - 3647 of sequence B936425; Nucleotides 1 - 354 of sequence B936426; Nucleotides 1 - 2145 of sequence B936427, Nucleotides 1 - 606 of sequence B936428; Nucleotides 1 - 480 of sequence B936429; Nucleotides 1 - 3026 of sequence B936430; Nucleotides 1 - 1512 of sequence B936431; Nucleotides 1 - 3774 of sequence B936432; and Nucleotides 1 - 589 of sequence B936433. In some or any of the embodiments of the oligonucleotides described herein, or processes for designing or synthesizing them, the oligonucleotides will upregulate gene expression and may specifically bind or specifically hybridize or be complementary to the PRC2 binding RNA that is transcribed from the same strand as a protein coding reference gene. The oligonucleotide may bind to a region of the PRC2 binding RNA that originates within or overlaps an intron, exon, intron exon junction, 5' UTR, 3' UTR, a translation initiation region, or a translation termination region of a protein coding sense strand of a reference gene (refGene).

In some or any of the embodiments of oligonucleotides described herein, or processes for designing or synthesizing them, the oligonucleotides will upregulate gene expression and may specifically bind or specifically hybridize or be complementary to a PRC2 binding RNA that transcribed from the opposite strand (the antisense strand) of a protein coding reference gene. The oligonucleotide may bind to a region of the PRC2 binding RNA that originates within or overlaps an intron, exon, intron exon junction, 5' UTR, 3' UTR, a translation initiation region, or a translation termination region of a protein coding antisense strand of a reference gene

The oligonucleotides described herein may be modified, *e.g.,* comprise a modified sugar moiety, a modified internucleoside linkage, a modified nucleotide and/or combinations thereof. In addition, the oligonucleotides can exhibit one or more of the following properties: do not induce substantial cleavage or degradation of the target RNA; do not cause substantially complete cleavage or degradation of the target RNA; do not activate the RNAse H pathway; do not activate RISC; do not recruit any Argonaute family protein; are not cleaved by Dicer; do not mediate alternative splicing; are not immune stimulatory; are nuclease resistant; have improved cell uptake compared to unmodified oligonucleotides; are not toxic to cells or mammals; may have improved endosomal exit; do interfere with interaction of lncRNA with PRC2, preferably the Ezh2 subunit but optionally the Suz12, Eed, RbAp46/48 subunits or accessory factors such as Jarid2; do decrease histone H3 lysine27 methylation and/or do upregulate gene expression.

In some or any of the embodiments of oligonucleotides described herein, or processes for designing or synthesizing them, the oligonucleotides may optionally exclude those that bind DNA of a promoter region, as described in Kuwabara et al., US 2005/0226848 or Li et al., US 2010/0210707 or Corey et al., 7,709,456 or Mattick et al., WO 2009/124341, or those that bind DNA of a 3' UTR region, as described in Corey et al., US 2010/0273863.

Oligonucleotides that are designed to interact with RNA to modulate gene expression are a distinct subset of base sequences from those that are designed to bind a DNA target (*e.g.,* are complementary to the underlying genomic DNA sequence from which the RNA is transcribed).

### Methods for Modulating Gene Expression

In another aspect, the disclosure relates to methods for modulating gene expression in a cell, *e.g.,* a cancer cell, a stem cell, or other normal cell types for gene or epigenetic therapy. The cells can be *in vitro, ex vivo,* or *in vivo* (*e.g.,* in a subject who has cancer, *e.g.,* a tumor). In some embodiments, methods for modulating gene expression in a cell comprise delivering a single stranded oligonucleotide as described herein. In some embodiments, delivery of the single stranded oligonucleotide to the cell results in a level of expression of gene that is at least 5%, 10%, 20%, 30%, 40%, 50%, 60%, 70%, 80%, 90%, 100%, 200% or more greater than a level of expression of gene in a control cell to which the single stranded oligonucleotide has not been delivered. In certain embodiments, delivery of the single stranded oligonucleotide to the cell results in a level of expression of gene that is at least 50% greater than a level of expression of gene in a control cell to which the single stranded oligonucleotide has not been delivered.

In another aspect of the disclosure methods comprise administering to a subject (*e.g.* a human) a composition comprising a single stranded oligonucleotide as described herein to increase protein levels in the subject. In some embodiments, the increase in protein levels is at least 5%, 10%, 20%, 30%, 40%, 50%, 60%, 70%, 80%, 90%, 100%, 200%, or more, higher than the amount of a protein in the subject before administering.

As another example, to increase expression of a tumor suppressor in a cell, the methods include introducing into the cell a single stranded oligonucleotide that is sufficiently complementary to a PRC2-associated region (*e.g.,* of a long non-coding RNA) that maps to a genomic position encompassing or in proximity to a target gene (*e.g.,* a tumor suppressor as set forth in Table 2 of International Patent Application Publication WO/2012/087983, an imprinted gene in Table 2, and/or other growth-suppressing genes in Table 2 of International Patent Application Publication WO/2012/087983 (*e.g.*, Nkx2-1 or Titf-1, *e.g.,* in subjects with cancer, *e.g.,* lung adenocarcinoma patients)).

In another aspect of the disclosure provides methods of treating a condition (*e.g.,* cancer) associated with decreased levels of expression of a particular gene in a subject, the method comprising administering a single stranded oligonucleotide as described herein.

A subject can include a non-human mammal, *e.g.* mouse, rat, guinea pig, rabbit, cat, dog, goat, cow, or horse. In preferred embodiments, a subject is a human.

In some embodiments, specific cancers that can be treated using the methods described herein are listed in Table 3 of International Patent Application Publication WO/2012/087983, for example, and include, but are not limited to: breast, lung, prostate, CNS (*e.g.,* glioma), salivary gland, prostate, ovarian, and leukemias (*e.g.,* ALL, CML, or AML). Associations of these genes with a particular cancer are known in the art, *e.g.,* as described in Futreal et al., Nat Rev Cancer. 2004;4;177-83 (see, *e.g.,* Table 1); and The COSMIC (Catalogue of Somatic Mutations in Cancer) database and website, Bamford et al., Br J Cancer. 2004;91;355-8; see also Forbes et al., Curr Protoc Hum Genet. 2008;Chapter 10;Unit 10.11, and the COSMIC database, *e.g.,* v.50 (Nov. 30, 2010). It is understood that reference to any particular type of cancer in, for example, Table 3 of International Patent Application Publication WO/2012/087983, means that patients with other types of cancer, i.e., cancer in general, may be treated.

In addition, the methods described herein can be used for modulating (*e.g.,* enhancing or decreasing) pluripotency of a stem cell and to direct stem cells down specific differentiation pathways to make endoderm, mesoderm, ectoderm, and their developmental derivatives. To increase, maintain, or enhance pluripotency, the methods include introducing into the cell a single stranded oligonucleotide that specifically binds to, or is complementary to, a PRC2-associated region of a nucleic acid (*e.g.,* of any long non-coding RNA disclosed herein). Stem cells useful in the methods described herein include adult stem cells (*e.g.,* adult stem cells obtained from the inner ear, bone marrow, mesenchyme, skin, fat, liver, muscle, or blood of a subject, *e.g.,* the subject to be treated); embryonic stem cells, or stem cells obtained from a placenta or umbilical cord; progenitor cells (*e.g.,* progenitor cells derived from the inner ear, bone marrow, mesenchyme, skin, fat, liver, muscle, or blood); and induced pluripotent stem cells (*e.g.,* iPS cells).

In some embodiments, the methods described herein include administering a composition, *e.g.,* a sterile composition, comprising a single stranded oligonucleotide that is complementary to a PRC2-associated region of a nucleic acid (*e.g.,* of an IncRNA described herein, *e.g.,* as set forth in sequences A1 to A 193,049, B1 to B916,209, and B916,626 to B934,931). In some embodiments, the single stranded oligonucleotide comprises one or more modified nucleotides (*e.g*., a locked nucleic acid (LNA) molecule).

Single stranded oligonucleotides have been employed as therapeutic moieties in the treatment of disease states in animals, including humans. Single stranded oligonucleotides can be useful therapeutic modalities that can be configured to be useful in treatment regimes for the treatment of cells, tissues and animals, especially humans.

For therapeutics, an animal, preferably a human, suspected of having cancer is treated by administering single stranded oligonucleotide in accordance with this disclosure. For example, in one non-limiting embodiment, the methods comprise the step of administering to the animal in need of treatment, a therapeutically effective amount of a single stranded oligonucleotide as described herein.

### Formulation, Delivery, And Dosing

The oligonucleotides described herein can be formulated for administration to a subject. It should be understood that the formulations, compositions and methods can be practiced with any of the oligonucleotides disclosed herein.

The formulations may conveniently be presented in unit dosage form and may be prepared by any methods well known in the art of pharmacy. The amount of active ingredient (*e.g.,* an oligonucleotide or compound for use in the invention) which can be combined with a carrier material to produce a single dosage form will vary depending upon the host being treated, the particular mode of administration, *e.g.,* intradermal or inhalation. The amount of active ingredient which can be combined with a carrier material to produce a single dosage form will generally be that amount of the compound which produces a therapeutic effect, *e.g.* tumor regression.

Pharmaceutical formulations of this disclosure can be prepared according to any method known to the art for the manufacture of pharmaceuticals. Such formulations can contain sweetening agents, flavoring agents, coloring agents and preserving agents. A formulation can be admixtured with nontoxic pharmaceutically acceptable excipients which are suitable for manufacture. Formulations may comprise one or more diluents, emulsifiers, preservatives, buffers, excipients, etc. and may be provided in such forms as liquids, powders, emulsions, lyophilized powders, sprays, creams, lotions, controlled release formulations, tablets, pills, gels, on patches, in implants, etc.

A formulated single stranded oligonucleotide composition can assume a variety of states. In some examples, the composition is at least partially crystalline, uniformly crystalline, and/or anhydrous (*e.g.,* less than 80, 50, 30, 20, or 10% water). In another example, the single stranded oligonucleotide is in an aqueous phase, *e.g.,* in a solution that includes water. The aqueous phase or the crystalline compositions can, *e.g.,* be incorporated into a delivery vehicle, *e.g.,* a liposome (particularly for the aqueous phase) or a particle (*e.g.,* a microparticle as can be appropriate for a crystalline composition). Generally, the single stranded oligonucleotide composition is formulated in a manner that is compatible with the intended method of administration.

In some embodiments, the composition is prepared by at least one of the following methods: spray drying, lyophilization, vacuum drying, evaporation, fluid bed drying, or a combination of these techniques; or sonication with a lipid, freeze-drying, condensation and other self-assembly.

A single stranded oligonucleotide preparation can be formulated or administered (together or separately) in combination with another agent, *e.g.,* another therapeutic agent or an agent that stabilizes a single stranded oligonucleotide, *e.g.,* a protein that complexes with single stranded oligonucleotide. Still other agents include chelators, *e.g.,* EDTA (*e.g.,* to remove divalent cations such as Mg²⁺), salts, RNAse inhibitors (*e.g.,* a broad specificity RNAse inhibitor such as RNAsin) and so forth.

In one embodiment, the single stranded oligonucleotide preparation includes another single stranded oligonucleotide, *e.g.,* a second single stranded oligonucleotide that modulates expression of a second gene or a second single stranded oligonucleotide that modulates expression of the first gene. Still other preparation can include at least 3, 5, ten, twenty, fifty, or a hundred or more different single stranded oligonucleotide species. Such single stranded oligonucleotides can mediated gene expression with respect to a similar number of different genes.

In one embodiment, the single stranded oligonucleotide preparation includes at least a second therapeutic agent (*e.g.,* an agent other than an oligonucleotide). For example, *e.g.,* a single stranded oligonucleotide composition for the treatment of a cancer might further comprise a chemotherapeutic agent.

### Route of Delivery

A composition that includes a single stranded oligonucleotide can be delivered to a subject by a variety of routes. Exemplary routes include: intravenous, intradermal, topical, rectal, parenteral, anal, intravaginal, intranasal, pulmonary, ocular.

The single stranded oligonucleotide molecules for use in the invention can be incorporated into pharmaceutical compositions suitable for administration. Such compositions typically include one or more species of single stranded oligonucleotide and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The pharmaceutical compositions of the present disclosure may be administered in a number of ways depending upon whether local or systemic treatment is desired and upon the area to be treated. Administration may be topical (including ophthalmic, vaginal, rectal, intranasal, transdermal), oral or parenteral. Parenteral administration includes intravenous drip, subcutaneous, intraperitoneal or intramuscular injection, or intrathecal or intraventricular administration.

The route and site of administration may be chosen to enhance targeting. For example, to target muscle cells, intramuscular injection into the muscles of interest would be a logical choice. Lung cells might be targeted by administering the single stranded oligonucleotide in aerosol form. The vascular endothelial cells could be targeted by coating a balloon catheter with the single stranded oligonucleotide and mechanically introducing the oligonucleotide.

### Topical delivery

Topical administration refers to the delivery to a subject by contacting the formulation directly to a surface of the subject. The most common form of topical delivery is to the skin, but a composition disclosed herein can also be directly applied to other surfaces of the body, *e.g.,* to the eye, a mucous membrane, to surfaces of a body cavity or to an internal surface. As mentioned above, the most common topical delivery is to the skin. The term encompasses several routes of administration including, but not limited to, topical and transdermal. These modes of administration typically include penetration of the skin's permeability barrier and efficient delivery to the target tissue or stratum. Topical administration can be used as a means to penetrate the epidermis and dermis and ultimately achieve systemic delivery of the composition. Topical administration can also be used as a means to selectively deliver oligonucleotides to the epidermis or dermis of a subject, or to specific strata thereof, or to an underlying tissue.

Formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

Transdermal delivery is a valuable route for the administration of lipid soluble therapeutics. The dermis is more permeable than the epidermis and therefore absorption is much more rapid through abraded, burned or denuded skin. Inflammation and other physiologic conditions that increase blood flow to the skin also enhance transdermal adsorption. Absorption via this route may be enhanced by the use of an oily vehicle (inunction) or through the use of one or more penetration enhancers. Other effective ways to deliver a composition disclosed herein via the transdermal route include hydration of the skin and the use of controlled release topical patches. The transdermal route provides a potentially effective means to deliver a composition disclosed herein for systemic and/or local therapy. In addition, iontophoresis (transfer of ionic solutes through biological membranes under the influence of an electric field), phonophoresis or sonophoresis (use of ultrasound to enhance the absorption of various therapeutic agents across biological membranes, notably the skin and the cornea), and optimization of vehicle characteristics relative to dose position and retention at the site of administration may be useful methods for enhancing the transport of topically applied compositions across skin and mucosal sites.

### Oral or Nasal Delivery

Both the oral and nasal membranes offer advantages over other routes of administration. For example, oligonucleotides administered through these membranes may have a rapid onset of action, provide therapeutic plasma levels, avoid first pass effect of hepatic metabolism, and avoid exposure of the oligonucleotides to the hostile gastrointestinal (GI) environment. Additional advantages include easy access to the membrane sites so that the oligonucleotide can be applied, localized and removed easily.

In oral delivery, compositions can be targeted to a surface of the oral cavity, *e.g.,* to sublingual mucosa which includes the membrane of ventral surface of the tongue and the floor of the mouth or the buccal mucosa which constitutes the lining of the cheek. The sublingual mucosa is relatively permeable thus giving rapid absorption and acceptable bioavailability of many agents. Further, the sublingual mucosa is convenient, acceptable and easily accessible.

A pharmaceutical composition of single stranded oligonucleotide may also be administered to the buccal cavity of a human being by spraying into the cavity, without inhalation, from a metered dose spray dispenser, a mixed micellar pharmaceutical formulation as described above and a propellant. In one embodiment, the dispenser is first shaken prior to spraying the pharmaceutical formulation and propellant into the buccal cavity.

Compositions for oral administration include powders or granules, suspensions or solutions in water, syrups, slurries, emulsions, elixirs or non-aqueous media, tablets, capsules, lozenges, or troches. In the case of tablets, carriers that can be used include lactose, sodium citrate and salts of phosphoric acid. Various disintegrants such as starch, and lubricating agents such as magnesium stearate, sodium lauryl sulfate and talc, are commonly used in tablets. For oral administration in capsule form, useful diluents are lactose and high molecular weight polyethylene glycols. When aqueous suspensions are required for oral use, the nucleic acid compositions can be combined with emulsifying and suspending agents. If desired, certain sweetening and/or flavoring agents can be added.

### Parenteral Delivery

Parenteral administration includes intravenous drip, subcutaneous, intraperitoneal or intramuscular injection, intrathecal or intraventricular administration. In some embodiments, parental administration involves administration directly to the site of disease (*e.g.* injection into a tumor).

Formulations for parenteral administration may include sterile aqueous solutions which may also contain buffers, diluents and other suitable additives. Intraventricular injection may be facilitated by an intraventricular catheter, for example, attached to a reservoir. For intravenous use, the total concentration of solutes should be controlled to render the preparation isotonic.

### Ocular Delivery

Any of the single stranded oligonucleotides described herein can be administered to ocular tissue. For example, the compositions can be applied to the surface of the eye or nearby tissue, *e.g.,* the inside of the eyelid. For ocular administration, ointments or droppable liquids may be delivered by ocular delivery systems known to the art such as applicators or eye droppers. Such compositions can include mucomimetics such as hyaluronic acid, chondroitin sulfate, hydroxypropyl methylcellulose or poly(vinyl alcohol), preservatives such as sorbic acid, EDTA or benzylchronium chloride, and the usual quantities of diluents and/or carriers. The single stranded oligonucleotide can also be administered to the interior of the eye, and can be introduced by a needle or other delivery device which can introduce it to a selected area or structure.

### Pulmonary Delivery

Pulmonary delivery compositions can be delivered by inhalation by the patient of a dispersion so that the composition, preferably single stranded oligonucleotides, within the dispersion can reach the lung where it can be readily absorbed through the alveolar region directly into blood circulation. Pulmonary delivery can be effective both for systemic delivery and for localized delivery to treat diseases of the lungs.

Pulmonary delivery can be achieved by different approaches, including the use of nebulized, aerosolized, micellular and dry powder-based formulations. Delivery can be achieved with liquid nebulizers, aerosol-based inhalers, and dry powder dispersion devices. Metered-dose devices are preferred. One of the benefits of using an atomizer or inhaler is that the potential for contamination is minimized because the devices are self-contained. Dry powder dispersion devices, for example, deliver agents that may be readily formulated as dry powders. A single stranded oligonucleotide composition may be stably stored as lyophilized or spray-dried powders by itself or in combination with suitable powder carriers. The delivery of a composition for inhalation can be mediated by a dosing timing element which can include a timer, a dose counter, time measuring device, or a time indicator which when incorporated into the device enables dose tracking, compliance monitoring, and/or dose triggering to a patient during administration of the aerosol medicament.

The term "powder" means a composition that consists of finely dispersed solid particles that are free flowing and capable of being readily dispersed in an inhalation device and subsequently inhaled by a subject so that the particles reach the lungs to permit penetration into the alveoli. Thus, the powder is said to be "respirable." Preferably the average particle size is less than about 10 µm in diameter preferably with a relatively uniform spheroidal shape distribution. More preferably the diameter is less than about 7.5 µ m and most preferably less than about 5.0 µ m. Usually the particle size distribution is between about 0.1 µ m and about 5 µ m in diameter, particularly about 0.3 µ m to about 5 µ m.

The term "dry" means that the composition has a moisture content below about 10% by weight (% w) water, usually below about 5% w and preferably less it than about 3% w. A dry composition can be such that the particles are readily dispersible in an inhalation device to form an aerosol.

The term "therapeutically effective amount" is the amount of oligonucleotide present in the composition that is needed to provide the desired level of target gene expression in the subject to be treated to give the anticipated physiological response. The term "physiologically effective amount" is that amount delivered to a subject to give the desired palliative or curative effect. The term "pharmaceutically acceptable carrier" means that the carrier can be taken into the lungs with no significant adverse toxicological effects on the lungs.

The types of pharmaceutical excipients that are useful as carrier include stabilizers such as human serum albumin (HSA), bulking agents such as carbohydrates, amino acids and polypeptides; pH adjusters or buffers; salts such as sodium chloride; and the like. These carriers may be in a crystalline or amorphous form or may be a mixture of the two.

Suitable pH adjusters or buffers include organic salts prepared from organic acids and bases, such as sodium citrate, sodium ascorbate, and the like; sodium citrate is preferred. Pulmonary administration of a micellar single stranded oligonucleotide formulation may be achieved through metered dose spray devices with propellants such as tetrafluoroethane, heptafluoroethane, dimethylfluoropropane, tetrafluoropropane, butane, isobutane, dimethyl ether and other non-CFC and CFC propellants.

### Devices and Implants

Exemplary devices include devices which are introduced into the vasculature, *e.g.,* devices inserted into the lumen of a vascular tissue, or which devices themselves form a part of the vasculature, including stents, catheters, heart valves, and other vascular devices. These devices, *e.g.,* catheters or stents, can be placed in the vasculature of the lung, heart, or leg.

Other devices include non-vascular devices, *e.g.,* devices implanted in the peritoneum, or in organ or glandular tissue, *e.g.,* artificial organs. The device can release a therapeutic substance in addition to a single stranded oligonucleotide, *e.g.,* a device can release insulin.

In one embodiment, unit doses or measured doses of a composition that includes single stranded oligonucleotide are dispensed by an implanted device. The device can include a sensor that monitors a parameter within a subject. For example, the device can include pump, *e.g.,* and, optionally, associated electronics.

Tissue, *e.g.,* cells or organs can be treated with a single stranded oligonucleotide, ex vivo and then administered or implanted in a subject. The tissue can be autologous, allogeneic, or xenogeneic tissue. *E.g*., tissue can be treated to reduce graft v. host disease . In other embodiments, the tissue is allogeneic and the tissue is treated to treat a disorder characterized by unwanted gene expression in that tissue. *E.g.,* tissue, *e.g.,* hematopoietic cells, *e.g.,* bone marrow hematopoietic cells, can be treated to inhibit unwanted cell proliferation. Introduction of treated tissue, whether autologous or transplant, can be combined with other therapies. In some implementations, the single stranded oligonucleotide treated cells are insulated from other cells, *e.g.,* by a semi-permeable porous barrier that prevents the cells from leaving the implant, but enables molecules from the body to reach the cells and molecules produced by the cells to enter the body. In one embodiment, the porous barrier is formed from alginate.

In one embodiment, a contraceptive device is coated with or contains a single stranded oligonucleotide. Exemplary devices include condoms, diaphragms, IUD (implantable uterine devices, sponges, vaginal sheaths, and birth control devices.

### Dosage

In one aspect, the disclosure features a method of administering a single stranded oligonucleotide (e.g., as a compound or as a component of a composition) to a subject *(e.g.,* a human subject). In one embodiment, the unit dose is between about 10 mg and 25 mg per kg of bodyweight. In one embodiment, the unit dose is between about 1 mg and 100 mg per kg of bodyweight. In one embodiment, the unit dose is between about 0.1 mg and 500 mg per kg of bodyweight. In some embodiments, the unit dose is more than 0.001, 0.005, 0.01, 0.05, 0.1, 0.5, 1, 2, 5, 10, 25, 50 or 100 mg per kg of bodyweight.

The defined amount can be an amount effective to treat or prevent a disease or disorder, *e.g.,* a disease or disorder associated with the target gene. The unit dose, for example, can be administered by injection (*e.g.,* intravenous or intramuscular), an inhaled dose, or a topical application.

In some embodiments, the unit dose is administered daily. In some embodiments, less frequently than once a day, *e.g.,* less than every 2, 4, 8 or 30 days. In another embodiment, the unit dose is not administered with a frequency (*e.g.,* not a regular frequency). For example, the unit dose may be administered a single time. In some embodiments, the unit dose is administered more than once a day, *e.g.,* once an hour, two hours, four hours, eight hours, twelve hours, etc.

In one embodiment, a subject is administered an initial dose and one or more maintenance doses of a single stranded oligonucleotide. The maintenance dose or doses are generally lower than the initial dose, *e.g.,* one-half less of the initial dose. A maintenance regimen can include treating the subject with a dose or doses ranging from 0.0001 to 100 mg/kg of body weight per day, *e.g.,* 100, 10, 1, 0.1, 0.01, 0.001, or 0.0001 mg per kg of bodyweight per day. The maintenance doses may be administered no more than once every 1, 5, 10, or 30 days. Further, the treatment regimen may last for a period of time which will vary depending upon the nature of the particular disease, its severity and the overall condition of the patient. In some embodiments the dosage may be delivered no more than once per day, *e.g.,* no more than once per 24, 36, 48, or more hours, *e.g.,* no more than once for every 5 or 8 days. Following treatment, the patient can be monitored for changes in his condition and for alleviation of the symptoms of the disease state. The dosage of the oligonucleotide may either be increased in the event the patient does not respond significantly to current dosage levels, or the dose may be decreased if an alleviation of the symptoms of the disease state is observed, if the disease state has been ablated, or if undesired side-effects are observed.

The effective dose can be administered in a single dose or in two or more doses, as desired or considered appropriate under the specific circumstances. If desired to facilitate repeated or frequent infusions, implantation of a delivery device, *e.g.,* a pump, semi-permanent stent (*e.g.,* intravenous, intraperitoneal, intracisternal or intracapsular), or reservoir may be advisable.

In some embodiments, the oligonucleotide pharmaceutical composition includes a plurality of single stranded oligonucleotide species. In another embodiment, the single stranded oligonucleotide species has sequences that are non-overlapping and non-adjacent to another species with respect to a naturally occurring target sequence (*e.g.,* a PRC2-associated region). In another embodiment, the plurality of single stranded oligonucleotide species is specific for different PRC2-associated regions. In another embodiment, the single stranded oligonucleotide is allele specific.

In some cases, a patient is treated with a single stranded oligonucleotide in conjunction with other therapeutic modalities. For example, a patient being treated for cancer may be administered a single stranded oligonucleotide in conjunction with a chemotherapy.

Following successful treatment, it may be desirable to have the patient undergo maintenance therapy to prevent the recurrence of the disease state, wherein the compound for use in the invention is administered in maintenance doses, ranging from 0.0001 mg to 100 mg per kg of body weight.

The concentration of the single stranded oligonucleotide composition is an amount sufficient to be effective in treating or preventing a disorder or to regulate a physiological condition in humans. The concentration or amount of single stranded oligonucleotide administered will depend on the parameters determined for the agent and the method of administration, *e.g.* nasal, buccal, pulmonary. For example, nasal formulations may tend to require much lower concentrations of some ingredients in order to avoid irritation or burning of the nasal passages. It is sometimes desirable to dilute an oral formulation up to 10-100 times in order to provide a suitable nasal formulation.

Certain factors may influence the dosage required to effectively treat a subject, including but not limited to the severity of the disease or disorder, previous treatments, the general health and/or age of the subject, and other diseases present. Moreover, treatment of a subject with a therapeutically effective amount of a single stranded oligonucleotide can include a single treatment or, preferably, can include a series of treatments. It will also be appreciated that the effective dosage of a single stranded oligonucleotide used for treatment may increase or decrease over the course of a particular treatment. For example, the subject can be monitored after administering a single stranded oligonucleotide composition. Based on information from the monitoring, an additional amount of the single stranded oligonucleotide composition can be administered.

Dosing is dependent on severity and responsiveness of the disease condition to be treated, with the course of treatment lasting from several days to several months, or until a cure is effected or a diminution of disease state is achieved. Optimal dosing schedules can be calculated from measurements of target gene expression levels in the body of the patient. Persons of ordinary skill can easily determine optimum dosages, dosing methodologies and repetition rates. Optimum dosages may vary depending on the relative potency of individual compounds, and can generally be estimated based on EC50s found to be effective in in vitro and in vivo animal models. In some embodiments, the animal models include transgenic animals that express a human target gene. In another embodiment, the composition for testing includes a single stranded oligonucleotide that is complementary, at least in an internal region, to a sequence that is conserved between the target gene in the animal model and the target gene in a human.

In one embodiment, the administration of the single stranded oligonucleotide composition is parenteral, *e.g.* intravenous (*e.g.,* as a bolus or as a diffusible infusion), intradermal, intraperitoneal, intramuscular, intrathecal, intraventricular, intracranial, subcutaneous, transmucosal, buccal, sublingual, endoscopic, rectal, oral, vaginal, topical, pulmonary, intranasal, urethral or ocular. Administration can be provided by the subject or by another person, *e.g.,* a health care provider. The composition can be provided in measured doses or in a dispenser which delivers a metered dose. Selected modes of delivery are discussed in more detail below.

### Kits

In certain aspects of the disclosure kits are provided, comprising a container housing a composition comprising a single stranded oligonucleotide. In some embodiments, the composition is a pharmaceutical composition comprising a single stranded oligonucleotide and a pharmaceutically acceptable carrier. In some embodiments, the individual components of the pharmaceutical composition may be provided in one container. Alternatively, it may be desirable to provide the components of the pharmaceutical composition separately in two or more containers, *e.g.,* one container for single stranded oligonucleotides, and at least another for a carrier compound. The kit may be packaged in a number of different configurations such as one or more containers in a single box. The different components can be combined, *e.g.,* according to instructions provided with the kit. The components can be combined according to a method described herein, *e.g.,* to prepare and administer a pharmaceutical composition. The kit can also include a delivery device.

The present invention is defined in the claims, and is further illustrated by the following Examples, which in no way should be construed as further limiting.

### EXAMPLES

The invention is further described in the following examples, which do not limit the scope of the invention described in the claims.

### Materials and Methods

The following materials and methods were used in the Examples 1-7 set forth below.

### RIP-seq

RNA immunoprecipitation was performed using 10⁷ wildtype 16.7 (Lee and Lu, 1999) and Ezh2-/- ES cells. To construct RIP-seq libraries, cell nuclei were isolated, nuclear lysates were prepared, treated with 400 U/ml DNAse, and incubated with anti-Ezh2 antibodies (Active Motif) or control IgG (Cell Signaling Technology). RNA-protein complexes were immunoprecipitated with protein A agarose beads and RNA extracted using Trizol (Invitrogen). To preserve strand information, template switching was used for the library construction. 20-150 ng RNA and Adaptor! (5'-CTTTCCCTACACGACGCTCTTCCGATCTNNNNNN-3'; SEQ ID NO: 1277) were used for first-strand cDNA synthesis using Superscript II Reverse Transcription Kit (Invitrogen). Superscript II adds non-template CCC 3' overhangs, which were used to hybridize to Adaptor2-GGG template-switch primer (5'-CAAGCAGAAGACGGCATACGAGCTCTTCCGATCTGGG-3'; SEQ ID NO: 1278). During 1^{st}-strand cDNA synthesis, samples were incubated with adaptor1 at 20 °C for 10 min, followed by 37 °C for 10 min and 42 °C for 45 min. Denatured template switch primer was then added and each tube incubated for 30 min at 42 °C, followed by 75 °C for 15 min. Resulting cDNAs were amplified by forward (5'-AATGATACGGCGACCACCGAGATCTACACTCTTTCCCTACACGACGCTCTTCCGA TCT-3'; SEQ ID NO: 1279) and reverse (5'-CAAGCAGAAGACGGCATACGAGCTCTTCCGATCT-3'; SEQ ID NO: 1280) Illumina primers. PCR was performed by Phusion polymerase (BioRad) as follows: 98 °C for 30s, 20 - 24 cycles of [98°C 10s, 65°C 30s, 72°C 30s], and 72°C for 5 min. PCR products were loaded on 3% NuSieve gel for size-selection and 200-1,200 bp products were excised and extracted by QIAEX II Agarose Gel Extraction Kit (Qiagen). Minus-RT samples generally yielded no products. DNA concentrations were quantitated by PicoGreen. 5-10 ml of 2 - 20 nM cDNA samples were sequenced by the Sequencing Core Facility of the Dept. of Molecular Biology, MGH, on the Illumina GAII.

### Bioinformatic analysis

Except as noted below, all analyses were performed using C++ programs. Image processing and base calling were performed using the Illumina pipeline. 3' adaptor sequences were detected by crossmatch and matches of ≥5 bases were trimmed, homopolymer reads filtered, and reads matching the mitochondrial genome and ribosomal RNAs excluded from all subsequent analyses. Remaining sequences were then aligned to the mm9 mouse reference genome using shortQueryLookup (Batzoglou et al., 2002). Alignments with ≤1 error were retained. Because library construction and sequencing generate sequence from the opposite strand of the PRC2-bound RNA, in all further analysis, we treated each read as if it were reverse-complemented. To determine the correlation coefficients comparing the original a-Ezh2 RIP-seq library to its technical and biological replicates and also to RIP-seq of the Ezh2-/- control line the number of reads per gene was compared between two samples and, for each pair, the Pearson correlation was computed between the number of reads mapped to each refGene. That is, for each sample, a vector was created of counts of reads mapped to each refGene and computed the Pearson correlation between all pairs of vectors.

Locations of repetitive sequences in mm9 (RepeatMasker) were obtained from the UCSC Genome Browser database. The overlap of PRC2 transcriptome reads with these repeats was obtained by intersecting coordinates of RepeatMasker data with coordinates of read alignments. The UCSC transcriptome was used as general reference. To obtain a set of non-overlapping distinct transcribed regions, the UCSC transcriptome transcripts were sorted by start coordinate and merged overlapping transcripts on the same strand (joined UCSC transcriptome: 39,003 transcripts total). Read alignment coordinates were intersected with those of the merged UCSC transcripts to determine the number of UCSC transcripts present in the PRC2 transcriptome. Hits to the transcripts were converted to RPKM units, where the read count is 1/(n*K*M), and n is the number of alignments in the genome, K is the transcript length divided by 1,000, and M is the sequencing depth including only reads mapping to mm9 divided by 1,000,000. This normalization allows for comparisons between transcripts of differing lengths and between samples of differing sequencing depths.

To generate promoter maps, promoter regions were defined as -10,000 to +2000 bases relative to TSS (obtained from refGene catalog, UCSC Genome Browser,). Read counts overlapping promoter regions were plotted, except that the limit of 10 alignments was relaxed. For chromosomal alignments read numbers were computed for all non-overlapping consecutive 100 kb windows on each chromosome. Reads were normalized such that those mapping to n locations were counted as 1/n^{th} of a read at each location. Graphs were plotted using custom scripts written in R. A list of all enriched transcripts were found by comparing the RPKM scores on each strand for all transcripts in the WT and Ezh2-/- samples. Then their coordinates were intersected with coordinates of the feature of interest. Features not in NCBI37/mm9 mouse assembly coordinates were converted to those coordinates using UCSC's LiftOver utility. The liftOver utility effectively maps one genome to another, allowing rapid identification of regions of interest between successive assemblies of the same species or between two distinct species.

### RIP/qRT-PCR

Validation RIPs were performed, based on existing methods, using 5 ul of rabbit antimouse-Ezh2 antibodies (Active Motif) or normal rabbit IgG (Millipore). RIP was followed by quantitative, strand-specific RT-PCR using the ICYCLER IQ™ Real-time detection system (BioRad). Gene-specific PCR primer pairs are:
Malat-1: Forward 5'-GCCTTTTGTCACCTCACT-3' ; SEQ ID NO: 1281 Reverse 5'-CAAACTCACTGCAAGGTCTC-3'; SEQ ID NO: 1282
Malat1-as: Forward 5'-TACTGGGTCTGGATTCTCTG-3'; SEQ ID NO: 1283 Reverse 5'-CAGTTCCGTGGTCTTTAGTG-3'; SEQ ID NO: 1284
Foxn2-as: Forward5'-GGCTATGCTCATGCTGTAAC; SEQ ID NO: 1285 Reverse 5'-GTTACTGGCATCTTTCTCACA-3'; SEQ ID NO: 1286
Ly6e-as: Forward 5'-CCACACCGAGATTGAGATTG-3'; SEQ ID NO: 1287 Reverse 5'-GCCAGGAGAAAGACCATTAC-3'; SEQ ID NO: 1288
Bgn-as: Forward 5'-TGTGAACCCTTTCCTGGA-3'; SEQ ID NO: 1289 Reverse 5'-CTTCACAGGTCTCTAGCCA-3'; SEQ ID NO: 1290
Gtl2: Forward 5'- CGAGGACTTCACGCACAAC -3'; SEQ ID NO: 1291 Reverse 5'- TTACAGTTGGAGGGTCCTGG -3'; SEQ ID NO: 1292
Gtl2-as: Forward 5'-CACCCTGAACATCCAACA-3'; SEQ ID NO: 1293 Reverse 5'-CATCTGCTTTTCCTACCTGG-3' ; SEQ ID NO: 1294
Hapa1-upstream: Forward 5'-GGTCCAAAATCGGCAGT-3' ; SEQ ID NO: 1227
Reverse 5'-GTCCTCAAATCCCTACCAGA-3'; SEQ ID NO: 1228
Htr6-downstream: Forward 5'-ACACGGTCGTGAAGCTAGGTA-3'; SEQ ID NO: 1229
Reverse 5'-CAGTTGGAGTAGGCCATTCCC-3'; SEQ ID NO: 1230
Nespas/TR019501: Forward 5'-AGATGAGTCCAGGTGCTT-3'; SEQ ID NO: 1231
Reverse 5'-CAAGTCCAGAGTAGCCAAC-3'; SEQ ID NO: 1232

Xist-Forward 3F5 and -Reverse 2R primers have been described (Zhao et al., 2008). For strand-specific cDNA synthesis, the reverse primer was used, qPCR carried out with SYBR green (BioRad), and threshold crossings (Ct) recorded. Each value was normalized to input RNA levels.

### Northern blot analysis

5 µg of poly(A+) RNA were isolated from 16.7 ES cells, separated by 0.8% agarose gel containing formaldehyde, blotted onto Hybond-XL (GE Healthcare), and hybridized to probe using Ultrahyb (Ambion) at 42°C. Probes were generated using STRIP-EZ PCR kit (Ambion) and amplified from genomic DNA with:
Malat1-AS-F, 5'-TGGGCTATTTTTCCTTACTGG-3'; SEQ ID NO: 1233
Malat1-AS-R, 5'-GAGTCCCTTTGCTGTGCTG-3'; SEQ ID NO: 1234
(Gtl2) Meg3-F, 5'-GCGATAAAGGAAGACACATGC-3'; SEQ ID NO: 1235
Meg3-R, 5'-CCACTCCTTACTGGCTGCTC-3'; SEQ ID NO: 1236
Meg3 ds-F3, 5'- ATGAAGTCCATGGTGACAGAC-3'; SEQ ID NO: 1237
Meg3 ds-R2, 5'-ACGCTCTCGCATACACAATG-3'; SEQ ID NO: 1238
Rtl1-F, 5'-GTTGGGGATGAAGATGTCGT-3'; SEQ ID NO: 1239
Rtl1-R, 5'-GAGGCACAAGGGAAAATGAC-3'; SEQ ID NO: 1240
Nespas ds-F, 5'-TGGACTTGCTACCCAAAAGG-3'; SEQ ID NO: 1241
Nespas ds-R, 5'-CGATGTTGCCCAGTTATCAG-3'; SEQ ID NO: 1242
Bgn-AS-F, 5'-CAACTGACCTCATAAGCAGCAC-3'; SEQ ID NO: 1243
Bgn-AS-R, 5'-AGGCTGCTTTCTGCTTCACA-3'; SEQ ID NO: 1244
Htr6 up-F, 5'-ATACTGAAGTGCCCGGAGTG-3'; SEQ ID NO: 1245
Htr6 up-R, 5'-CAGGGGACAGACATCAGTGAG-3'; SEQ ID NO: 1246

### UV-crosslink RIP

UV-crosslink IP was performed using existing methods, except that transcripts in the RNA-protein complexes were not trimmed by RNAse treatment prior to RNA isolation in order to preserve full-length RNA for RT-PCR. Mouse ES cells were UV-irradiated at 254 nm, 400 mJ/cm² (using a Stratagene STRATALINKER), cell nuclei were lysed in RSB-TRITON buffer (10mM Tris-HCl, 100mM NaCl, 2.5 mM MgCl₂, 35 µg/mL digitonin, 0.5% triton X-100) with disruptive sonication. Nuclear lysates were pre-cleared with salmon sperm DNA/protein agarose beads for 1 hr at 4°C and incubated with antibodies overnight. RNA/antibody complexes were then precipitated with Protein A DYNABEADS (Invitrogen), washed first in a low-stringency buffer (1XPBS [150 mM NaCl], 0.1% SDS, 0.5% deoxycholate, 0.5% NP-40), then washed twice in a high-stringency, high-salt buffer (5XPBS [750 mM NaCl], 0.1% SDS, 0.5% deoxycholate, 0.5% NP-40), and treated with proteinase K. RNA was extracted using TRIZOL (Invitrogen) and RT-qPCR was performed as described above.

### Expression and purification of human PRC2 components

For expression of human PRC2 subunits, N-terminal flagged-tagged EZH2 and SUZ12 in pFastBac1 were expressed in Sf9 cells. For expression of the whole PRC2 complex, flag-tagged EZH2 was coexpressed with untagged SUZ12, EED, and RBAP48. Extracts were made by four freeze-thaw cycles in BC300 buffer (20mM HEPES pH 7.9, 300mM KCl, 0.2mM EDTA, 10% glycerol, 1mM DTT, 0.2mM PMSF, and complete protease inhibitors (Roche)) and bound to M2 beads for 4 h and washed with BC2000 before eluting in BC300 with 0.4mg/ml flag peptide. EZH2 and PRC2 were adjusted to 100mM KCl and loaded onto a HiTrap Heparin FF 1ml column and eluted with a 100-1000mM KCl gradient. Peak fractions were concentrated using Amicon ultra 10kDa MWCO concentrators (Millipore) and loaded onto a Superose 6 column equilibrated with BC300. Peak fractions were collected and concentrated. For SUZ12, the flag elution was concentrated and loaded onto a Superdex 200 column equilibrated with BC300.

### Electrophoretic mobility shifting assays (EMSA)

For RNA-EMSA, a 30 nt Hes-1 probe (∼270 bp downstream of TSS in an antisense direction) was used for gel shifts. RNA probes were radiolabeled with [γ-33p]ATP using T4 polynucleotide kinase (Ambion). Purified PRC2 proteins (1 µg) were incubated with labeled probe for 1hr at 4 C. RNA-protein complexes were separated on a 4% non-denaturing polyacrylamide gel in 0.5×TBE at 250 V at 4 °C for 1 h. Gels were dried and exposed to Kodak BioMax film.

### RNA pulldown assays

T7 promoter sequence was incorporated into forward primers for PCR products of RepA, Xist exon 1, and truncated Gtl2. Full-length Gtl2 was cloned into pYX-ASC and XistE1 into pEF1/V5/HisB (Invitrogen). Specific primer sequences were:
RepA-F: TAATACGACTCACTATAGGGAGAcccatcggggccacggatacctgtgtgtcc; SEQ ID NO: 1247
RepA-R: taataggtgaggtttcaatgatttacatcg; SEQ ID NO: 1248
Truncated-Gtl2-F:
   TAATACGACTCACTATAGGGAGATTCTGAGACACTGACCATGTGCCCAGTGCACC ; SEQ ID NO: 1249 Truncated-Gtl2-R: CGTCGTGGGTGGAGTCCTCGCGCTGGGCTTCC; SEQ ID NO: 1250
Xist E1-F: atgctctgtgtcctctatcaga; SEQ ID NO: 1251
Xist E1-R: gaagtcagtatggagggggt; SEQ ID NO: 1252

RNAs were then transcribed using the Mega Script T7 (Ambion), purified using Trizol, and slow-cooled to facilitate secondary structure formation. For pulldown assays, 3µg of Flag-PRC2 or Flag-GFP and 5 pmol of RNA supplemented with 20U RNAsin were incubated for 30 min on ice. 10µl of flag beads were added and incubated on a rotating wheel at 4°C for 1 hr. Beads were washed 3 times with 200 µl buffer containing 150mM KCl, 25mM Tris pH 7.4, 5mM EDTA, 0.5mM DTT, 0.5% NP40 and 1mM PMSF. RNA-protein complexes were eluted from flag beads by addition of 35µl of 0.2M-glycine pH2.5. Eluates were neutralized by addition of 1/10^{th} volume of 1M Tris pH 8.0 and analyzed by gel electrophoresis.

### Knockdown analysis and qRT-PCR

shRNA oligos were cloned into MISSION pLKO.1-puro (Sigma-Aldrich) vector and transfected into wild-type mouse ES cells by Lipofectamine 2000 (Invitrogen). After 10 days of puromycin selection, cells were collected and qRT-PCR was performed to confirm RNA knockdown. The corresponding scrambled sequence (MISSION Non-target shRNA) was used as a control (Scr). The shRNA oligos for Gtl2: (Top strand) 5' - CCG GGC AAG TGA GAG GAC ACA TAG GCT CGA GCC TAT GTG TCC TCT CAC TTG CTT TTT G - 3'; SEQ ID NO: 1253 (Bottom strand) 5' - AAT TCA AAA AGC AAG TGA GAG GAC ACA TAG GCT CGA GCC TAT GTG TCC TCT CAC TTG C - 3'; SEQ ID NO: 1254. qPCR primers for Gtl2 and Gtl2-as RNAs are as described above. Primers for Dlk1 RNAs: (Forward) 5' - ACG GGA AAT TCT GCG AAA TA -3'; SEQ ID NO: 1255 (Reverse) 5' - CTT TCC AGA GAA CCC AGG TG -3'; SEQ ID NO: 1256. Another Gtl2 shRNA was purchased from Open Biosystems (V2MM_97929). Ezh2 levels after knockdown with this shRNA were tested by qPCR. After testing multiple clones, we concluded that Gtl2 could be knocked down in early passage clones (50-70%), but knockdown clones were difficult to maintain in culture long-term.

### DNA ChIP and real-time PCR

ChIP was performed as described (Zhao et al., 2008). 5 µl of α-Ezh2 antibodies (Active Motif 39103), normal rabbit IgG (Upstate 12-370), and α -H3K27me3 (Upstate) were used per IP. Real-time PCR for ChIP DNA was performed at the *Gtl2*-proximal DMR with prGtl2F/prGtl2R, at the Gtl2-distal DMR with DMR-F/DMR-R, at the *Dlk1* promoter with prDlk1F/prDlk1R, and at the *Gapdh* promoter with prGAPDH-F/prGAPDH-R. Primer sequences are as follows:
proximal-DMR 5' - CATTACCACAGGGACCCCATTTT; SEQ ID NO: 1257
proximal-DMR 5' - GATACGGGGAATTTGGCATTGTT; SEQ ID NO: 1258
prDlklF 5' - CTGTCTGCATTTGACGGTGAAC; SEQ ID NO: 1259
prDlklR 5' - CTCCTCTCGCAGGTACCACAGT; SEQ ID NO: 1260
distal-DMR-F 5' - GCCGTAAAGATGACCACA; SEQ ID NO: 1261
distal-DMR-R 5' - GGAGAAACCCCTAAGCTGTA; SEQ ID NO: 1262
prGAPDH-F 5' - AGCATCCCTAGACCCGTACAGT; SEQ ID NO: 1263
prGAPDH-R 5' - GGGTTCCTATAAATACGGACTGC; SEQ ID NO: 1264
prActin-F 5' - GCA GGC CTA GTA ACC GAG ACA; SEQ ID NO: 1265
prActin-R 5' - AGT TTT GGC GAT GGG TGC T; SEQ ID NO: 1266

The following materials and methods were used in Examples 6-10 set forth below.

**LNA Nucleofection** - 2 X 10⁶ SV40T transformed MEFs were resuspended in 100µl of Mef nucleofector solution (Lonza). Cy3-labeled LNA molecules were added to a final concentration of 2µM. The cells were transfected using the T-20 program. 2 ml of culture medium was added to the cells and 100µl of this suspension was plated on one gelatinized 10 well slide per timepoint. LNA sequences were designed using Exiqon software (available at exiqon.com). Modified LNA bases were strategically introduced to maximize target affinity (Tm) while minimizing self-hybridization score. The LNA molecule sequences (from 5' to 3') were as follows:
LNA-Scr, GTGTAACACGTCTATACGCCCA; SEQ ID NO: 1267
LNA-C1, CACTGCATTTTAGCA; SEQ ID NO: 1268
LNA-C2, AAGTCAGTATGGAG; SEQ ID NO: 1269
LNA-B, AGGGGCTGGGGCTGG; SEQ ID NO: 1270
LNA-E, ATAGACACACAAAGCA; SEQ ID NO: 1271
LNA-F, AAAGCCCGCCAA; SEQ ID NO: 1272
LNA-4978, GCTAAATGCACACAGGG; SEQ ID NO: 1273
LNA-5205,CAGTGCAGAGGTTTTT; SEQ ID NO: 1274
LNA-726,TGCAATAACTCACAAAACCA; SEQ ID NO: 1275
LNA-3', ACCCACCCATCCACCCACCC; SEQ ID NO: 1276

**Real Time PCR** - Total RNA was extracted after nucleofection using Trizol (Invitrogen). Reverse transcriptase reaction was performed using the Superscript II kit and real time PCR performed on cDNA samples using icycler SYBR green chemistry (Biorad).

**ChIP** - Cells were fixed at various time points after nucleofection in 1% formaldehyde solution. Fixation was stopped by addition of glycine to 0.125M and ChIP was performed as described earlier (28) and quantitated by qPCR.

**Antibodies-** The antibodies for various epitopes were purchased as follows: H3K27me3, Active Motif 39535. Ezh2, Active Motif 39639 and BD Pharmingen 612666. For Immunostaining, H3K27me3 antibodies were used at 1:100 dilution and Ezh2 antibodies (BD Pharmingen) at 1:500. Alexa-Fluor secondary antibodies were from Invitrogen. For Western blots, Ezh2 antibodies (BD Pharmingen) were used at 1:2000 dilution. Actin antibody (Sigma A2066) was used at 1:5000 dilution.

**DNA FISH, RNA FISH, and Immunostaining-** Cells were grown on gelatinized glass slides or cytospun. RNA FISH, DNA FISH, serial RNA-DNA FISH, immunostaining, and immunoFISH were performed based on existing methods. Xist RNA FISH was performed using nick-translated pSx9-3 probe or an Xist riboprobe cocktail. pSx9-3 was used as probe for *Xist* DNA FISH. For metaphase spreads, colchicine was added to cells for 1 hr. Cells were trypsinized and resuspended in 3 ml of 0.056M KCl for 30 minutes at room temperature, centrifuged and resuspended in methanol:acetic acid (3:1) fixative. After several changes of fixative, cells were dropped on a chilled slide and processed for RNA or DNA FISH.

### Example 1. Capturing the PRC2 Transcriptome by RIP-seq

A method of capturing a genome-wide pool of RNA bound to PRC2 was developed by combining two existing methods native RIP and RNA-seq (this method is referred to herein as "RIP-seq"). Nuclear RNAs immunoprecipitated by α-Ezh2 antibodies were isolated from mouse ES cells and an Ezh2-/- control, cDNAs created using strand-specific adaptors, and those from 200-1,200 nt were purified and subjected to Illumina sequencing.

In pilot experiments, we performed RIP on 10⁷ ES cells and included several control RIPs to assess the specificity of α-Ezh2 pulldowns. In the wildtype pulldown and its technical and biological replicates, α-Ezh2 antibodies precipitated 70-170 ng of RNA from 107 ES cells and yielded a cDNA smear of >200 nt. Treatment with RNAses eliminated products in this size range and -RT samples yielded no products, suggesting that the immunoprecipitated material was indeed RNA. There was ∼10-fold less RNA in the Ezh2-/- pulldown (∼14 ng) and when wildtype cells were immunoprecipitated by IgG (∼24 ng). A 500-fold enrichment over a mock RIP control (no cells) was also observed. In the >200 nt size range, control RIPs (null cells, IgG pulldowns, mock) were even further depleted of RNA, as these samples were dominated by adaptor and primer dimers. Adaptor/primer dimers, rRNA, mitochondrial RNA, reads with <18 nt or indeterminate nucleotides, and homopolymer runs in excess of 15 bases were computationally filtered out. From an equivalent number of cells, control RIPs were significantly depleted of reads. In wildtype libraries, 231,880-1.2 million reads remained after filtering. By contrast, only 4,888 to 73,691 reads remained in controls. The overwhelming majority of transcripts in the controls were of spurious nature (adaptor/primer dimers, homopolymers, etc.). Therefore, wildtype RIPs exhibited substantial RNA enrichment and greater degrees of RNA complexity in comparison to control RIPs.

Approximately half of all reads in the wildtype libraries was represented three times or more. Even after removing duplicates to avoid potential PCR artifacts, the wildtype library contained 301,427 distinct reads (technical and biological replicates with 98,704 and 87,128, respectively), whereas control samples yielded only 1,050 (IgG) and 17,424 (null). The wildtype libraries were highly similar among each other, with correlation coefficients (CC) of 0.71-0.90, as compared to 0.27-0.01 when compared against Ezh2-/- and IgG controls, respectively. Reads mapping to repetitive elements of >10 copies/genome accounted for <20% of total wildtype reads, with simple repeats being most common and accounting for 85.714%, whereas LINEs, SINEs, and LTRs were relatively under-represented. Because reads with ≤10 alignments have greatest representation, we hereafter focus analysis on these reads (a cutoff of ≤10 retains genes with low-copy genomic duplications).

Genome distributions were examined by plotting distinct reads as a function of chromosome position . Alignments showed that PRC2-associated RNAs occurred on every chromosome in the wildtype libraries. Alignments for IgG and Ezh2-/- controls demonstrated few and sporadic reads. Therefore, our RIP-seq produced a specific and reproducible profile for the PRC2 transcriptome. A large number of wildtype reads hits the X-chromosome, and a zoom of the X-inactivation center showed that our positive controls - Tsix, RepA, and Xist RNAs - were each represented dozens of times . The high sensitivity of RIP-seq detection was suggested by representation of RepA and Xist, which are in aggregate expressed at <10 copies/ES cell. On the other hand, no hits occurred within other noncoding RNAs of the X-inactivation center. Thus, the RIP-seq technique was both sensitive and specific.

### Example 2. The PRC2 Transcriptome

To obtain saturating coverage, sequencing was scaled up and 31.9 million reads were obtained for the original wildtype sample and 36.4 million for its biological replicate. After removing duplicates and filtering 1,030,708 and 852,635 distinct reads of alignment ≤10 remained for each library, respectively. These reads were then combined with pilot wildtype reads for subsequent analyses (henceforth, WT library) and all analyses were performed using the Ezh2-/- library as control.

A strategy was designed based on the relative representation in the WT versus null libraries, reasoning that bona fide positives should be enriched in the WT. Genic representations were calculated using "reads per kilobase per million reads" (RPKM) as a means of normalizing for gene length and depth of sequencing, and then all 39,003 transcripts in the UCSC joined transcriptome were mapped to a scatterplot by their WT RPKM (x-axis) and their null RPKM (y-axis) values. Transcripts with zero or near-zero representation in both libraries accounted for the vast majority of datapoints [blue cloud at (0,0)]. Transcripts with nonzero x-values and a zero y-value indicated a population represented only in WT pulldowns. To determine an appropriate enrichment threshold, we performed an in silico subtraction. WT/null RPKM ratios were examined for the same calibrators. Xist/RepA scored 4.18/0, implying hundreds to thousands of representations in the WT library but none in the null. Tsix scored 10.35/3.27, Bsn pasr 0.95/0, and Kcnq1ot1 1.17/0. The negative controls scored low ratios, with Pax3- pasr at 0.11/0.26, Hey1-pasr 0.28/0, Hotair 0.25/0, Insl6 0.27/3.09, and Ccdc8 0.22/5.04. On this basis, a 3:1 enrichment ratio for RPKM(WT)/RPKM(null) and a minimum RPKM of 0.4 were called.

Transcript identification for the "PRC2 transcriptome" was based on the fact that there are ∼10-times more RNAs pulled down by EZH2 antibodies in the wildtype cell line than in the Ezh2-null line, indicating that the wildtype library is highly enriched for PRC2-associated transcripts and that no further in silico subtraction is necessary. Using this criterion, the size of the expanded PRC2 transcriptome is estimated at ∼57K RNAs.

### Example 3: Identification of PRC2-binding peaks (PRC2-associated regions) from Appendix I

In some or any embodiments, the region of an RNA to which a protein binding partner (*e.g.,* PRC2) binds is one of the exemplary locations on a target lncRNA to which a single stranded oligonucleotide is designed to hybridize. For example, these regions can be identified by reviewing the data in Appendix I and identifying regions that are enriched in the dataset; these regions are likely to include PRC2-binding sequences.

The sequence reads in Appendix I come directly off the Illumina GA-II genome analyzer and are in an orientation that is the reverse complement of the PRC2-binding transcript. Appendix I is a filtered subset of all of the reads after bioinformatic filtering removed adaptor/primer dimers, mitochondrial RNA, rRNA, homopolymers, reads with indeterminate nucleotides, and truncated reads (<15nt). They are likely to represent regions best protected from endogenous nucleases during RIP and subsequent RNA purification steps described in Example 1 above (a RIP-seq method) and thus represent candidate regions of RNA that bind to PRC2 or associated proteins or complexes. From Appendix I, reads were extracted corresponding to transcripts that are enriched 3:1 in WT vs. null [RPKM(WT)/RPKM(null)≥3.0] and with a minimal RPKM value of 0.4. Regions of the PRC2-binding transcripts with an uninterrupted pile-up of reads (peaks) were identified and considered candidate PRC2 contact regions within the RNA.

The sequence reads in Appendix I were used to generate sequence coverage on the reference genome using the Broad Institute's Arachne aligner, ShortQueryLookup, which is based on making a k-mer (K=12) dictionary of the reference genome and performing a local Smith-Waterman alignment on a read's candidate locations based on matching k-mer locations in the genome. The aligner does multiple placements. The best alignment is allowed to have at most one error and alignments that differ from the best alignment's number of errors by one are also accepted. The coverage is normalized by dividing by the number of places the read aligns (*e.g.* if a reads aligns to four places, 0.25 is added to each of the bases in the four places).

To obtain the target Peaks, the following methodology was used. A base-level mouse (mm9) coverage file of regions where the wild-type coverage of the transcriptome is enriched at least three-fold over the coverage of the Ezh2 -/- transcriptome and has a minimum RPKM coverage of at least 0.4 serves as the starting point. The coverage is strand-specific. Next, in non-overlapping consecutive windows of 100 bps in length, peak values and their locations are determined. Peak positions are then corrected for those peaks that are on the edge of a window that are determined to be on a side of a larger peak. Those peaks are moved to the top of the larger peak. Duplicate peak locations are then removed. Peaks positions that are on a plateau are moved to the center of the plateau. The coverage is then smoothed using a Gaussian kernel, (1/sqrt(2*sigma*pi))*exp(-t^2/(2*sigma)), where sigma = 5.0. Peak widths are then determined by locating the nearest position to the peak such that the smoothed coverage is less than or equal to one-third the maximum coverage. Adjacent peaks that overlap each other are resolved by placing a boundary between them at the midpoint between the peaks. Peaks are then output into a table with the position, width, the maximum amplitude, and the sum of unsmoothed coverage underneath the width of the peak. The corresponding nucleotide sequences of the mouse Peaks in mm9 (converted to RNA by replacing T with U) appear in sequences B47,408 to B616,428 [mouse Peaks]. Mouse-to-human LiftOver of the mouse chromosome coordinates and strand of these mouse Peaks was performed in the UCSC genome browser as described herein, to generate orthologous human chromosome coordinates. This process and LiftOver chains is known in the art. When the mouse coordinates (mm9) of each mouse Peak were converted to the corresponding human (hg19) coordinates, mapping percentages of 50, 65, 75, and 95 yielded essentially identical location and length results whenever a match occurred. Consequently, the 50% mapping parameter was used.

Each corresponding human Peak RNA sequence (i.e., the nucleotide sequence of the human chromosomal coordinates and strand, converted to RNA by replacing T with U) appear in sequences B652,256 to B916,209 [human Peaks]. Table 1 displays the mouse sequences and the corresponding human sequences. These human Peaks and the human PRC2 transcriptome (i.e. human sequences of PRC2-binding transcripts) were intersected with known genes from the NCBI database to identify genes targeted by the PRC2-binding RNA (i.e. an intersecting or nearby gene).

Table 2 of International Patent Application Publication WO/2012/087983shows the annotation of the mouse and human Peaks with the names of genes that were near or intersected with each Peak. The unique NCBI gene ID associated with the human gene (listed first) or mouse gene (listed second) appears in parentheses adjacent to the gene name. The degree of overlap between the Peak coordinates and the gene coordinates appears in square brackets. A positive number indicates the number of overlapping nucleotides between the two, and a negative number represents the size of the gap between the two (i.e. the number of nucleotides of distance between the two). For Peaks, an "F" within the square brackets indicates that the Peak coordinates fully overlap the gene coordinates. For transcripts, an "F" within the square brackets indicates that the transcript coordinates fully overlap the gene coordinates, or vice versa. The RNA transcript or Peak is "antisense" to the reference genes in the "Opposite Strand" column, while the RNA transcript or Peak is in the same "sense" orientation as the reference gene in the "Same Strand" column.

Bioinformatic analysis indicates that the average Peak is about 40-60 bases, which is an excellent size for initial design of single stranded oligonucleotides. Each of these Peaks is fully represented by the reverse-complement reads in Appendix I since it corresponds to a segment of overlapping reverse-complement reads from Appendix I. The Peaks can be found anywhere within the coding gene, and in either sense or antisense orientations. Peaks can also be found in the promoter/5'UTR regions, introns, internal exons, and 3'UTR and beyond. The analysis strongly suggests that the PRC2-interacting transcripts are not the protein-coding mRNA, but a distinct transcript or transcripts that overlap with the mRNA sequence. Many are novel RNAs not previously described.

Methods disclosed herein can be used to design a single stranded oligonucleotide that binds to target locations or segments with sufficient specificity, or are sufficiently complementary to the target RNA to give the desired effect. In some embodiments, the methods include using bioinformatics methods known in the art to identify regions of secondary structure, *e.g.,* one, two, or more stem-loop structures, or pseudoknots, and selecting those regions to target with a single stranded oligonucleotide.

Additional target segments 5-500 nucleotides in length, or about 5 to about 100 nucleotides in length, or about 2kb in length, comprising a stretch of at least five (5) consecutive nucleotides within the Peak, or immediately adjacent thereto, are considered to be suitable for targeting as well.

For each of the human Peaks that did not match a longer human transcript sequence, a longer 2kb fragment of surrounding human chromosomal sequence was identified, and appears in sequences B916,626-B934,761 [larger region surrounding human Peaks].

### Example 4. Evidence Supporting Direct Binding of RNA to PRC2 in or around the Peak regions

Experiments were carried out to test the idea that RNA identified using the criteria in Example 2 directly bind PRC2. *In vitro* biochemical analyses were performed using purified recombinant human PRC2 subunits, EED, EZH2, SUZ12, and RBAP48. The newly identified antisense RNA for Hes1 (a transcription factor in the Notch signaling pathway contains a double stem-loop structure, a motif also found in RepA. In an RNA electrophoretic mobility shift assay (EMSA), both the 28-nt RepA and 30-nt Hes1-as probes were shifted by PRC2, whereas RNAs derived from other regions of Xist (DsI, DsII) were not. Mutating the stem-loop structures reduced PRC2 binding. To determine which subunit of PRC2 binds Hes1-as, we performed EMSA using specific subunits. EZH2 strongly shifted wildtype but not mutated Hes1-as RNA, whereas neither SUZ12 nor EED shifted Hes1-as. The RNA-protein shift was always more discrete when whole PRC2 was used, suggesting that other subunits stabilize the interaction.

These results show that Hes1-as RNA directly and specifically interacts with PRC2 and Ezh2 is the RNA-binding subunit. Further evidence comes from the observation that the two of the greatest peaks within the Xist/Tsix locus localize to the Repeat A region, the 28-nt repeated motif known to directly interact with EZH2 on both the forward and reverse strands. RNA fragments derived from "Peaks" showed robust shifts with PRC2, whereas those mapping outside the "Peaks" shifted poorly. We therefore believe that many, if not all, of the identified "Peaks" of Table 2 of International Patent Application Publication WO/2012/087983 represent bona fide PRC2-interacting domains of the RNA. These results show that the Peaks, and likely adjacent regions, directly and specifically interact with PRC2 complex.

### Example 5. In vitro effect of single stranded oligonucleotides on upregulation of mRNA expression

### A. ApoE

Single stranded oligonucleotides were designed to target lncRNA in order to upregulate ApoE. The oligonucleotides were less than 16 bases in length and comprised unmodified DNA and multiple locked nucleic acid modified bases, all linked by phosphorothioate bonds. Transfection and data analysis were carried out briefly as follows.

RNA was harvested from the Hep 3B cells using Promega SV 96 Total RNA Isolation system omitting the DNAse step. In separate pilot experiments, 50 ng of RNA was determined to be sufficient template for the reverse transcriptase reaction. RNA harvested from the Hep3B cells was normalized so that 50ng of RNA was input to each reverse transcription reaction. For the few samples that were too dilute to reach this limit, the maximum input volume was added. Quantitative PCR evaluation was then completed.

A baseline level of ApoE mRNA expression was determined through quantitative PCR as outlined above. Baseline levels were also determined for mRNA of various housekeeping genes which are constitutively expressed. A "control" housekeeping gene with approximately the same level of baseline expression as ApoE mRNA was chosen for comparison purposes to ApoE.

Hep3B cells were seeded into each well of 24-well plates at a density of 25,000 cells per 500uL and transfections were performed with Lipofectamine and the single stranded oligonucleotides. Control wells contained Lipofectamine alone. At 48 hours post-transfection, approximately 200 uL of cell culture supernatants were stored at -80 C for ELISA. At 48 hours post-transfection, RNA was harvested from the Hep 3B cells and quantitative PCR was carried out as outlined above. The percent induction of ApoE mRNA expression by each single stranded oligonucleotide was determined by normalizing mRNA levels in the presence of the single stranded oligonucleotide to the mRNA levels in the presence of control (Lipofectamine alone). This was compared side-by-side with the increase in mRNA expression of the "control" housekeeping gene.

A total of 26 oligonucleotides tested were complementary to PRC2-binding RNA sequences identified according to Example 2 above. Of these 26 oligonucleotides, 7 upregulated apoE expression in human Hep3B cells, as indicated by increased ApoE mRNA levels relative to the "control" housekeeping gene.

The above procedure was repeated using human renal proximal tubule epithelial cells (RPTEC). Of the 26 oligonucleotides complementary to PRC2-binding RNA sequences identified according to Example 2 above, 5 increased ApoE mRNA levels in renal cells, relative to the "control" housekeeping gene. Levels increased by about 1.5 to about 5-fold over baseline expression.

In addition, of 11 oligonucleotides that are complementary to Peaks associated with apoE identified according to Example 3 above, 3 upregulated apoE expression.

Single stranded oligonucleotides as short as 8 nucleobases in length were demonstrated to upregulate gene expression.

### B. Nkx2-1

The experiments as described in Example 5A above were repeated for single stranded oligonucleotides designed to target lncRNA in order to upregulate Nkx2-1. A total of 13 oligonucleotides tested were complementary to a PRC2-binding RNA sequence identified according to Example 2 above. Of these 13 oligonucleotides, 3 upregulated Nkx2-1 expression as indicated by increased Nkx2-1 mRNA expression relative to baseline, although no "control" housekeeping gene could be matched with Nkx2-1 due to low levels of intrinsic expression. In addition, of 9 oligonucleotides that are complementary to Peaks associated with Nkx2-1 identified according to Example 3 above, 3 upregulated Nkx-21 expression.

### C. Brcal

The experiments as described in Example 5A above were repeated for single stranded oligonucleotides designed to target lncRNA in order to upregulate Brcal. A total of 30 oligonucleotides tested were complementary to two PRC2-binding RNA sequences identified according to Example 2 above. Of these 30 oligonucleotides, 5 oligonucleotides upregulated Brcal expression. Of these 30 oligonucleotides, 13 oligonucleotides were also complementary to Peaks associated with Brcal identified according to Example 3 above. Of these 13 oligonucleotides complementary to Peaks, 2 oligonucleotides upregulated Brcal expression. Levels increased by about 2 to about 3 fold over baseline expression.

### D. Smad7

The experiments as described in Example 5A above were repeated for single stranded oligonucleotides designed to target lncRNA as set forth in the sequence listing in order to upregulate Smad7, with the following exception: the kidney cell line RPTEC was used instead of HepB3. A total of 28 oligonucleotides tested were complementary to sequence B18602. Of these 28 oligonucleotides, 4 upregulated Smad7 expression. In addition, of 28 oligonucleotides that are complementary to Peaks in Table 2 of International Patent Application Publication WO/2012/087983 associated with Smad7, 4 upregulated Smad7 expression.

### E. SirT6

The experiments as described in Example 5A above were repeated for single stranded oligonucleotides designed to target lncRNA in order to upregulate SirT6. A total of 25 oligonucleotides tested were complementary to a PRC2-binding RNA sequence identified according to Example 2 above. Of these 25 oligonucleotides, 3 upregulated SirT6 expression. A total of 2 oligonucleotides tested were complementary to another PRC2-binding RNA sequence identified according to Example 2 above. Of these 2 oligonucleotides, 1 upregulated SirT6 expression. A total of 2 oligonucleotides tested were complementary to another PRC2-binding RNA sequence identified according to Example 2 above. Of these 2 oligonucleotides, neither upregulated SirT6 expression. Levels increased by 2 to 6 fold over baseline expression. In addition, of 6 oligonucleotides that are complementary to Peaks associated with SirT6 identified according to Example 3 above, 1 upregulated SirT6 expression.

### F. SerpinFl

The experiments as described in Example 5A above were repeated for single stranded oligonucleotides designed to target lncRNA in order to upregulate SerpinFl. A total of 38 oligonucleotides tested were complementary to two PRC2-binding RNA sequences identified according to Example 2 above. Of these 38 oligonucleotides, 3 upregulated SerpinFl expression. Levels increased by 1.2 to 2 fold over baseline expression. In addition, of 32 oligonucleotides that are complementary to Peaks associated with SerpinFl identified according to Example 3 above, 3 upregulated SerpinFl expression.

### G. KLF1

The experiments as described in Example 5A above were repeated for single stranded oligonucleotides designed to target lncRNA as set forth in Table 2 of International Patent Application Publication WO/2012/087983 in order to upregulate KLF1. A total of 30 oligonucleotides tested were complementary to sequences B15688 and B15689 in Table 2 of International Patent Application Publication WO/2012/087983. Of these 30 oligonucleotides, 15 upregulated KLF1 expression in human Hep3B cells, as indicated by increased KLF1 mRNA levels relative to the "control" housekeeping gene. In addition, of 2 oligonucleotides that are complementary to Peaks in Table 2 of International Patent Application Publication WO/2012/087983 associated with KLF1, 1 upregulated KLF1 expression. Levels increased by 2 to 50 fold over baseline expression.

### H. Rps19

The experiments as described in Example 5A above were repeated for single stranded oligonucleotides designed to target lncRNA as set forth in Table 2 of International Patent Application Publication WO/2012/087983 in order to upregulate rps19. A total of 30 oligonucleotides tested were complementary to sequences B630259 and B630260. Of these 30 oligonucleotides, 7 upregulated rps19 expression as indicated by increased rps19 mRNA expression relative to the "control" housekeeping gene. In addition, of 25 oligonucleotides that are complementary to Peaks in Table 2 of International Patent Application Publication WO/2012/087983 associated with rps19, 7 upregulated Rps19 expression. Levels increased by 1.2 to 1.6 fold over baseline expression.

### I. PTEN

The experiments as described in Example 5A above were repeated for single stranded oligonucleotides designed to target lncRNA as set forth in Table 2 of International Patent Application Publication WO/2012/087983 in order to upregulate PTEN. A total of 40 oligonucleotides tested were complementary to sequences B650,560 and B650,559 in Table 2 of International Patent Application Publication WO/2012/087983. Of these 40 oligonucleotides, 18 oligonucleotides upregulated PTEN expression. Of these 40 oligonucleotides, 31 were also complementary to Peaks in Table 2 of International Patent Application Publication WO/2012/087983 associated with PTEN. Of these 31 oligonucleotides complementary to Peaks, 11 oligonucleotides upregulated PTEN expression. Levels increased by about 1.5 to about 5 fold over baseline expression.

### J. EPO

The experiments as described in Example 5A above were repeated for single stranded oligonucleotides designed to target lncRNA as set forth in Table 2 of International Patent Application Publication WO/2012/087983 in order 111 to upregulate erythropoietin (EPO). A total of 13 tested oligonucleotides were complementary to sequences B932,189 or B932,190. Of these 13 oligonucleotides, 5 upregulated EPO expression. In addition, of 2 oligonucleotides that are complementary to Peaks in Table 2 of International Patent Application Publication WO/2012/087983 associated with EPO, 1 upregulated EPO expression. Levels increased by 4 fold over baseline expression.

An ELISA assay using a commercially available kit [DEP00, RnD Systems] was used according to the manufacturer's instructions to determine secreted protein present in cellular supernatant. Fold induction of protein was determined by normalizing protein levels induced by oligonucleotides to the protein levels induced by control (Lipofectamine alone). The data showed that of the 1 oligonucleotides tested that increased EPO mRNA expression, it demonstrated a corresponding EPO protein expression increase. 3 oligonucleotides complementary to sequences B14486 and B14487 (transcripts that overlap the mouse EPO gene) were tested in vivo for ability to upregulate mouse EPO expression.

In addition, two other oligos targeting downstream peak regions were tested as well. Of these, 4 oligonucleotides were complementary to Peak regions in Table 2 of International Patent Application Publication WO/2012/087983 associated with EPO. Male C57Bl6/J mice [6-8wks old and 20-25g] were administered subcutaneously a single injection of oligonucleotide, at a dose of either 10 mg/kg or 25 mg/kg in 100µl of sterile phosphate buffered saline.

At a time point 48 hours after injection, terminal blood samples were taken via cardiac puncture and assayed for levels of EPO protein using an ELISA assay [MEP00, RnD Systems] according to the manufacturer's instructions. Of the oligos tested that were complementary to sequence B14486 or B14487 or Peaks, one demonstrated a 5-fold induction and another demonstrated a 7-fold induction of EPO protein at a dose of 25 mg/kg. Of these two oligonucleotides that induced EPO protein expression in vivo, one is within 150 bases of (and the other is within 1500 bases of) and both are on the opposite strand as the mouse Peak that is sequence B461812. This mouse Peak corresponds to the human Peak of sequence B845472.

### K. BDNF

The experiments as described in Example 5A above were repeated for single stranded oligonucleotides designed to target lncRNA as set forth in Table 2 of International Patent Application Publication WO/2012/087983 in order to upregulate BDNF. A total of 21 oligonucleotides tested were complementary to sequences B620236 and B620237. Of these 21 oligonucleotides, 9 upregulated BDNF expression. A total of 2 oligonucleotides tested were complementary to sequence B 130,694. Of these 2 oligonucleotides, 1 upregulated BDNF expression. Levels increased by 1.5 to 6 fold over baseline expression. In addition, of 14 oligonucleotides that are complementary to Peaks in Table 2 of International Patent Application Publication WO/2012/087983 associated with BDNF, 6 upregulated BDNF expression. Levels increased by 2 to 7 fold over baseline expression.

### L. Granulin

The experiments as described in Example 5A above were repeated for single stranded oligonucleotides designed to target lncRNA as set forth in Table 2 of International Patent Application Publication WO/2012/087983 in order to upregulate Granulin. A total of 30 oligonucleotides tested were complementary to sequence B640164 and B192311. Of these 30 oligonucleotides, 6 upregulated Granulin expression as indicated by increased Granulin mRNA expression relative to the "control" housekeeping gene. In addition, of 22 oligonucleotides that are complementary to Peaks in Table 2 of International Patent Application Publication WO/2012/087983 associated with Granulin, 4 upregulated Granulin expression. Levels increased by 1.5 to 2 fold over baseline expression.

### M. KLF4

A total of 30 oligonucleotides tested were complementary to sequence B624099. Of these 30 oligonucleotides, 13 upregulated KLF1 expression in human Hep3B cells, as indicated by increased KLF4 mRNA levels relative to the "control" housekeeping gene. In addition, of 20 oligonucleotides that are complementary to Peaks in Table 2 of International Patent Application Publication WO/2012/087983 associated with KLF4, 10 upregulated KLF4 expression. Levels increased by 2 to 15 fold over baseline expression.

### N. Fvii (Factor VII)

The experiments as described in Example 5A above were repeated for single stranded oligonucleotides designed to target lncRNA as set forth in Table 2 of International Patent Application Publication WO/2012/087983 in order to upregulate Fvii. The oligonucleotides designed to target Fvii were about 20 bases in length and comprised modified DNA with a 2'-O- Me with full phosphorothioate linkage backbone. A total of 25 oligonucleotides tested were complementary to sequences B632564 and B632565 in Table 2 of International Patent Application Publication WO/2012/087983. Of these 25 oligonucleotides, 12 upregulated Fvii expression. Levels increased by 2- to 25 fold over baseline expression. In addition, of 25 oligonucleotides that are complementary to Peaks in Table 2 of International Patent Application Publication WO/2012/087983 associated with Fvii, 12 upregulated Fvii expression.

### Example 6. LNA Molecules Targeting Xist Repeat C rapidly displace Xist RNA from Xi

Repeat C was aligned using Geneious (Geneious v5.1, Available on the internet at geneious.com) and LNA molecules complementary to two regions with a high degree of inter-repeat conservation were synthesized. The first LNA molecule showed conservation in all 14 repeats (LNA-C1) and the second in 13 of 14 (LNA-C2). LNA molecules were nucleofected separately into transformed mouse embryonic fibroblasts (MEFs), and the cells were adhered onto slides and fixed *in situ* at various timepoints between 0 minutes (immediately after nucleofection) and 8 hours post-nucleofection. To examine effects on Xist RNA, RNA fluorescence *in situ* hybridization (FISH) was performed using Xist-specific probes. (MEF cells are tetraploid due to transformation; each tetraploid cell has two Xa and two Xi). In controls transfected with scrambled LNA molecules (LNA-Scr), robust Xist clouds were seen in 80-90% of cells at all timepoints. Intriguingly, introduction of either LNA-C1 or -C2 resulted in immediate loss of Xist RNA from Xi. Even at t=0 (cells fixed immediately, within seconds to minutes, after LNA introduction), ∼10% of nuclei displayed a loosening of the Xist RNA clusters, with the clusters appearing faint and diffuse. The percentage of nuclei with full Xist clouds continued to drop during the first hour and reached a minimum at t=60 minutes (21%, n=190). These findings indicate that LNA molecules disrupted Xist binding to chromatin as soon as they were introduced. However, the loss of Xist from Xi was transient, as pinpoints of Xist RNA typical of nascent transcripts seen in undifferentiated embryonic stem (ES) cells, became visible at t=3 hr. Full recovery of Xist clouds was not seen until 8-24 hr post-nucleofection (81% at 8 hr, n=117).

The next experiment addressed whether LNA molecules had similar effects in mouse ES cells an established *ex vivo* model which recapitulates XCI as the cells differentiate in culture. In the undifferentiated state, wildtype female ES cells express low levels of Xist RNA, visible as pinpoint signals by RNA FISH. By day 6 of differentiation, ∼40% of cells would normally have upregulated Xist RNA. When ES cells were nucleofected with LNA-C1 on day 6, Xist displacement occurred rapidly, reaching a maximum at 1 hr and recovering by 8 hr. Thus, LNA molecules were effective in ES cells as well as in somatic cells. These results contrasted sharply with those obtained from MEFs nucleofected with siRNAs or shRNAs toward the same region of Xist. Neither siRNAs nor shRNAs led to loss of Xist at the 1,3 or 24 hour timepoints, and partial decreases in Xist clouds occurred only at 48 hours (83%, n=84 at 1hr; 80%, n=106 at 24 hr). Thus, LNA molecules can be used efficiently to target long nuclear ncRNAs such as Xist with extremely rapid kinetics, much more rapid than the action of siRNAs or shRNAs, in multiple cell types.

To test the specificity of the LNA molecules, human 293 cells were nucleofected with the Repeat C LNA molecules. Sequence comparison between the mouse and human Xist/XIST revealed that the region targeted by LNA-C1 is conserved in 10 of 15 nt and is conserved in 10 of 14 nt for LNA-C2. Nucleofection of scrambled LNA molecules followed by XIST RNA FISH in human cells showed two normal XIST clouds in nearly all cells (92%, n=108). Similarly, nucleofection with either LNA-C1 or LNAC-2 did not change the XIST clouds (LNA-C1, 89%, n=126; LNA-C2, 85%, n=139). Thus, mouse Repeat C LNA molecules do not affect human XIST localization, suggesting that they function in a species-specific manner. To determine whether human Repeat C could displace human XIST, we nucleofected LNA molecules complementary to the human Repeat C into 293 cells, but observed no loss of XIST clouds (91%, n=103 at 1 hr; 87%, n=95 at 3 hr and 92%, n=85 at 8 hr). This finding indicated that, although Repeat C may play a role in humans, additional human elements function in RNA localization. Whereas mouse Repeat C occurs 14 times, the human repeat is present only once.

### Example 7. Xist RNA is displaced without transcript destabilization

Several mechanisms could explain the disappearance of Xist. LNA molecules could anneal to the complementary region and target Xist for degradation. Alternatively, hybridization to LNA molecules could displace Xist RNA from Xi without affecting the transcript stability. To distinguish between these possibilities, Xist levels were quantitated relative to Gadph levels (control) by qRT-PCR at different timepoints. At 1 hr when Xist clouds were no longer visible, Xist levels remained comparable to that seen in the scrambled control. Even at 3 and 8 hr, Xist levels did not change significantly. These results showed that displacement of Xist occurred without complete RNA degradation. Thus, LNA molecules function by blocking Xist interaction with chromatin rather than altering the RNA's stability.

The rapid displacement of Xist and the slow kinetics of recovery provided the opportunity to investigate several unanswered questions regarding Xist's mechanism of localization. To ask whether reappearance of Xist on Xi is due to relocalization of displaced Xist molecules or to coating by newly synthesized RNA, we performed time-course analysis in the presence of actinomycin D (ActD), an inhibitor of RNA polymerase II. Previous studies have shown that the half-life of Xist in the cell is approximately 4-6 hr. It was reasoned that treating cells with ActD for 0-8 hr would prevent new synthesis of Xist RNA during this timeframe and that, therefore, reappearance of Xist clouds would imply relocalization of displaced RNA back onto Xi. LNA molecules were introduced into cells and then the cells were allowed to recover in medium containing ActD. In the scrambled controls, Xist clouds were clearly visible at all time points without ActD. With ActD, Xist clouds were apparent in the 1 and 3 hr timepoints and were lost by 8 hr, consistent with a 4-6 hr half-life. In LNA-C1- or LNA-C2-treated samples allowed to recover without ActD, pinpoints of Xist were visible at 3 hr and Xist clouds were restored by the 8 hr timepoint. However, with ActD, Xist clouds were never restored, neither fully nor partially. Thus, Xist recovery after LNA molecule-mediated displacement from Xi is due to new RNA synthesis and not relocalization of the displaced transcript.

### Example 8. Xist RNA localizes near the X-inactivation center first

Taking further advantage of the rapid displacement and slow recovery, the longstanding question of whether Xist spreads in a piecemeal fashion or localizes simultaneously throughout Xi was asked. One hypothesis is that coating initiates near the *Xist* locus and proceeds to both ends of the chromosome through booster elements located along the X. Alternatively, coating can occur all at once through multiple X-linked seeding points which would promote local spreading. Xist localization on metaphase chromosomes was analyzed during the 3-8 hr period of recovery. In cells treated with scrambled LNA molecules, all metaphase chromosomes coated with Xist RNA showed a banded pattern similar to the heterogeneous patterns described in earlier works. By contrast, LNA-C1 treated cells gave intermediate patterns. At 1 hr, no metaphase chromosomes showed a coat of Xist RNA (0%, n= 41). At 3 hr when Xist RNA could be seen as a pinpoint in interphase cells, the predominant pattern was a combination of a single bright band in the middle of the metaphase chromosome together with a small number of very faint bands elsewhere on the X (52%, n=46). This result suggested that Xist RNA initially bound locally. To determine whether the strong RNA band was localized to the *Xist* region, Xist RNA FISH was carried out on non-denatured nuclei and followed with denaturation and hybridization to an *Xist* probe. Indeed, the focal RNA band observed at the 3-hr mark colocalized with the *Xist* region. At 5 hr, intermediate degrees of coating and intensities could be seen (68%, n= 38). At 8 hr, the predominant pattern was the whole-chromosome painting pattern typical of control cells (78%, n=38). In controls, intermediate patterns were not observed at any time. These findings argue that Xist RNA initially binds nearby, but seems to spread to the rest of Xi at the same time, within the temporal and spatial resolution of the FISH technique.

### Example 9. Xist RNA displacement is accompanied by loss of PRC2 localization

The pattern of Polycomb repressive complex 2 (PRC2) binding to Xi has been of considerable interest, as its Ezh2 subunit catalyzes trimethylation of Histone H3 at lysine 27 (H3K27me3). Several studies have shown that PRC2 localizes to Xi in an Xist-dependent manner, as deleting Xist in ES cells precludes PRC2 recruitment during differentiation and conditionally deleting Xist in MEF cells results in loss of PRC2 on Xi. However, the kinetics with which PRC2 is recruited to and lost from X are not known. Because Xist RNA directly recruits PRC2, it was asked whether LNA molecule-mediated displacement of Xist results in immediate loss of PRC2 by immunostaining for Ezh2 in MEFs after LNA molecule delivery. Upon treatment with the Repeat C LNA molecules, Ezh2 was rapidly lost. There was nearly perfect concordance between Xist and PRC2 loss. At 1 and 3 hr, Ezh2 foci were never observed in nuclei that had lost Xist and, conversely, were always observed in nuclei with restored Xist clouds. The loss of Ezh2 on Xi was due to Ezh2 protein turnover. Transient displacement of PRC2, however, does not lead to appreciable H3K27me3 loss within the 1-8 hr timeframe. Thus, PRC2's localization onto Xi absolutely depends on Xist RNA for both initial targeting and for stable association after XCI is established, but the H3K27me3 mark is stable in the short term when Xist and PRC2 are displaced.

Given this, it was asked whether LNA molecules affected gene silencing. At 3 hr when Xist was maximally displaced, RNA FISH was performed for Xist and either Pgk1 or Hprt, two X-linked genes subject to XCI. In control-nucleofected (LNA-Scr) cells, Xist clouds were observed from Xi and nascent Pgk1 or Hprt transcripts from Xa. Nucleofection with LNA-C1 and LNA-4978 did not change the expression pattern, as two foci of Pgk1 transcripts were still seen in 79% (n=39) of controls and 80% (n=36) of LNA-C1-treated cells, and two foci of Hprt RNA were seen in 84% (n=44) of controls and 79% (n=35) of LNA-C1-treated cells. Four foci of Pgk1 or Hprt transcripts were never seen. Thus, consistent with retention of H3K27me3, silencing was not disrupted by transient loss of Xist and PRC2.

### Example 10. A broader domain around Repeat C is required for Xist localization

The next experiments investigated other conserved repeats within Xist. As Repeat A has already been shown to be essential for targeting PRC2, the experiments focused on Repeats B, E, and F, and found tht Xist localization was not affected by targeting any repeat individually or in combination. Conserved unique regions of Xist were also tested, including LNA-726, LNA-4978 and LNA-5205, and LNA-3' (distal terminus of Xist). None affected Xist localization except for LNA-4978, which corresponds to a 15-nt element located 280 bp downstream of Repeat C. LNA-4978 induced effects similar to LNA-C1/C2 but differed by its slower kinetics. At 1 hr, Xist clouds were still visible but appeared faint and dispersed (78%, n=125). The number of clouds reached a minimum at 3 hr (25%, n=158). At 8 hr, Xist was visible as small pinpoints (39%, n=123). Recovery was not complete until the 24-hr timepoint. As for Repeat C LNA molecules, loss of Xist was not due to RNA turnover, as determined by qRT-PCR, and Ezh2 was displaced without affecting H3K27me3 or change in Ezh2 protein level. Therefore, Xist localization to chromatin involves a broader region encompass both Repeat C and a unique region directly downstream of the repeat.

To determine if the two motifs cooperate, LNA-4978 and LNA-C1 were nucleofected separately or together into MEFs. As expected, treating with LNA-C1 alone resulted in loss of Xist RNA clouds by 1 hr and recovery beginning at 3 hr, and treating with LNA-4978 showed loss and recovery at 3 hr and 8 hr, respectively. Treating with both LNA molecules expanded the window of Xist depletion: Loss of Xist RNA and Ezh2 was observed by 1 hr (as was the case for LNA-C1 alone) and recovery did not begin until the 8 hr timepoint (as was the case for LNA-4978 alone). Thus, the LNA molecule effects were additive, not synergistic, as the effects were not enhanced beyond the widening of the Xist-depleted time window.

### Example 11. Ezh2 recovery after LNA molecule nucleofection is slow but uniform along Xi

Finally, it was asked whether Ezh2 retargeting to Xi closely follows the piecemeal relocalization of Xist RNA during the recovery phase. Because PRC2 generally binds near promoters, Ezh2 localization at X-gene promoters was analyzed by quantitative chromatin immunoprecipitation (qChIP). Although female cells have two Xs and Ezh2 epitopes pulled down by the antibody could theoretically come from either Xa or Xi, evidence indicates that the vast bulk of Ezh2 and H3K27me3 is bound to Xi. Ezh2 was indeed enriched at promoters of genes that are silenced on Xi (*e.g., Xmr, Pgk1*), but not at promoters of genes (*e.g., Jarid1c*) that escape XCI. Then, MEF cells were nucleofected with LNA-C1 and performed qChIP using anti-Ezh2 antibodies between 1 and 24 hr. At t=1hr, Ezh2 levels decreased dramatically at all tested target gene promoters to background levels, indicating that depletion of promoter-bound Ezh2 closely followed Xist displacement along Xi. At the 3- and 8-hr points, there was a gradual, uniform increase in Ezh2 levels across all genes, with many genes appearing to have reached saturating amounts of Ezh2 by t=8hr. On promoters with the highest levels of Ezh2 at t=0hr, Ezh2 levels did not fully recover until 24 hr. Thus, ChIP pulldowns were expected to originate predominantly, if not nearly exclusively, from Xi. In contrast, Ezh2 levels at the *En1* control, a known autosomal PRC2 target, did not change significantly. Thus, Ezh2 levels fall and rise with similar kinetics throughout Xi. The loss of Xist RNA and Ezh2 binding between 1 and 8 hrs presents a window of opportunity during which cells could be reprogrammed to achieve novel epigenetic states.

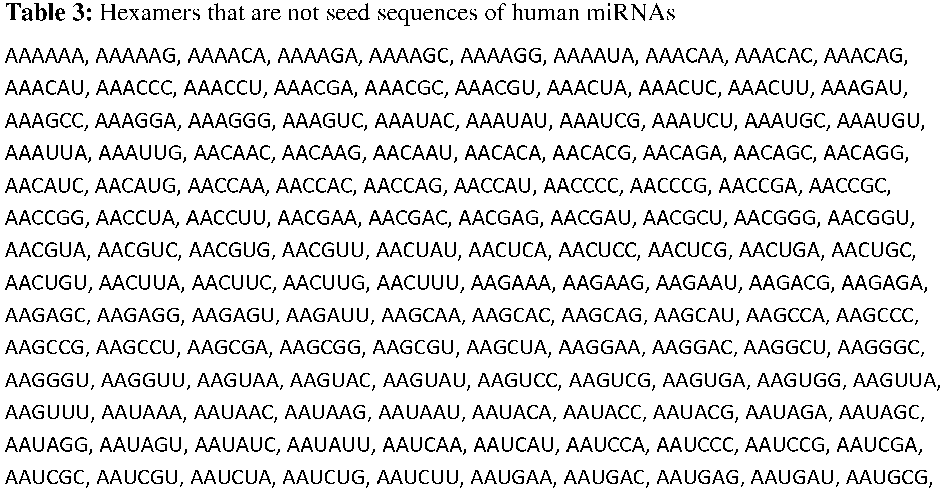

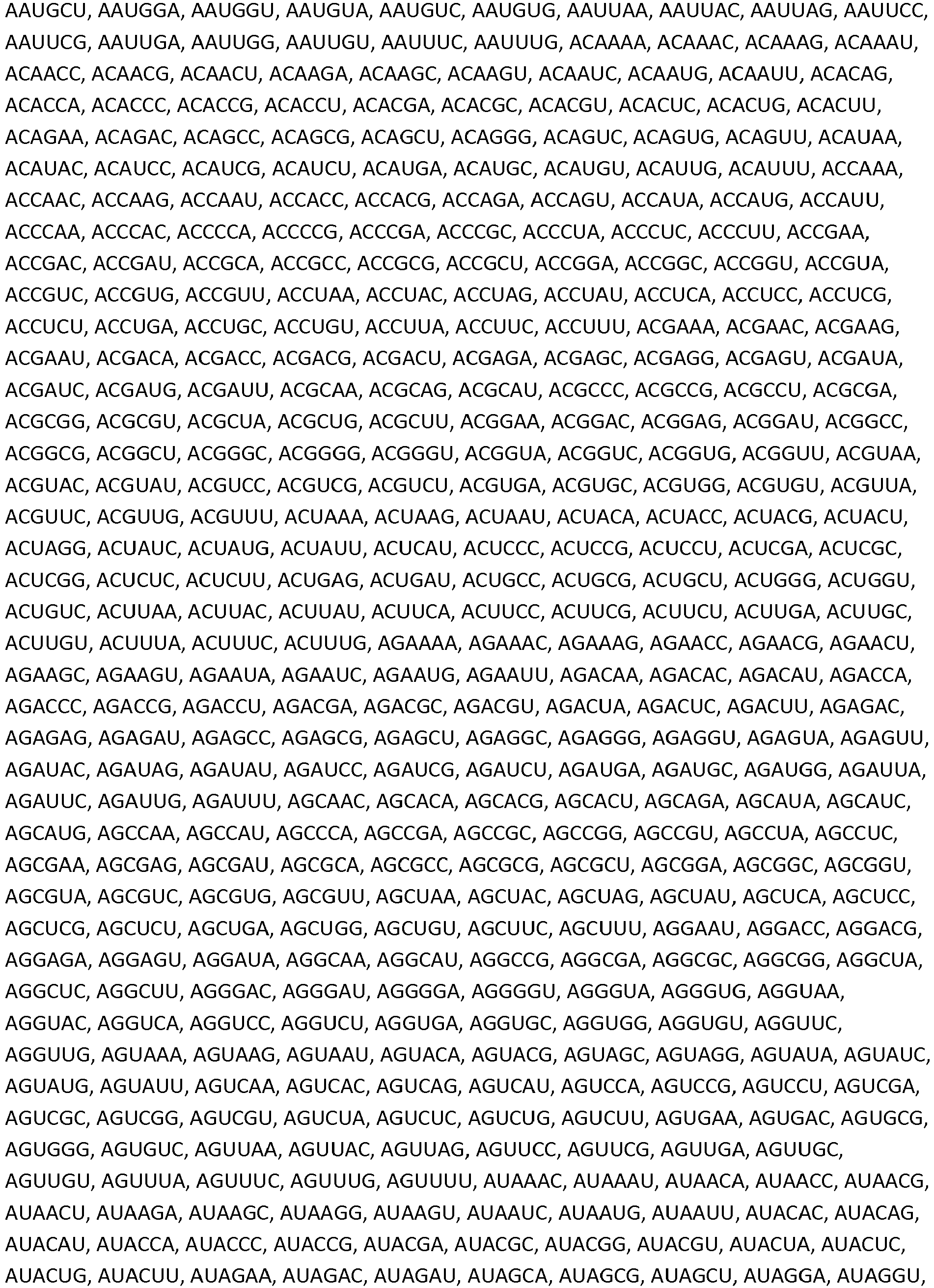

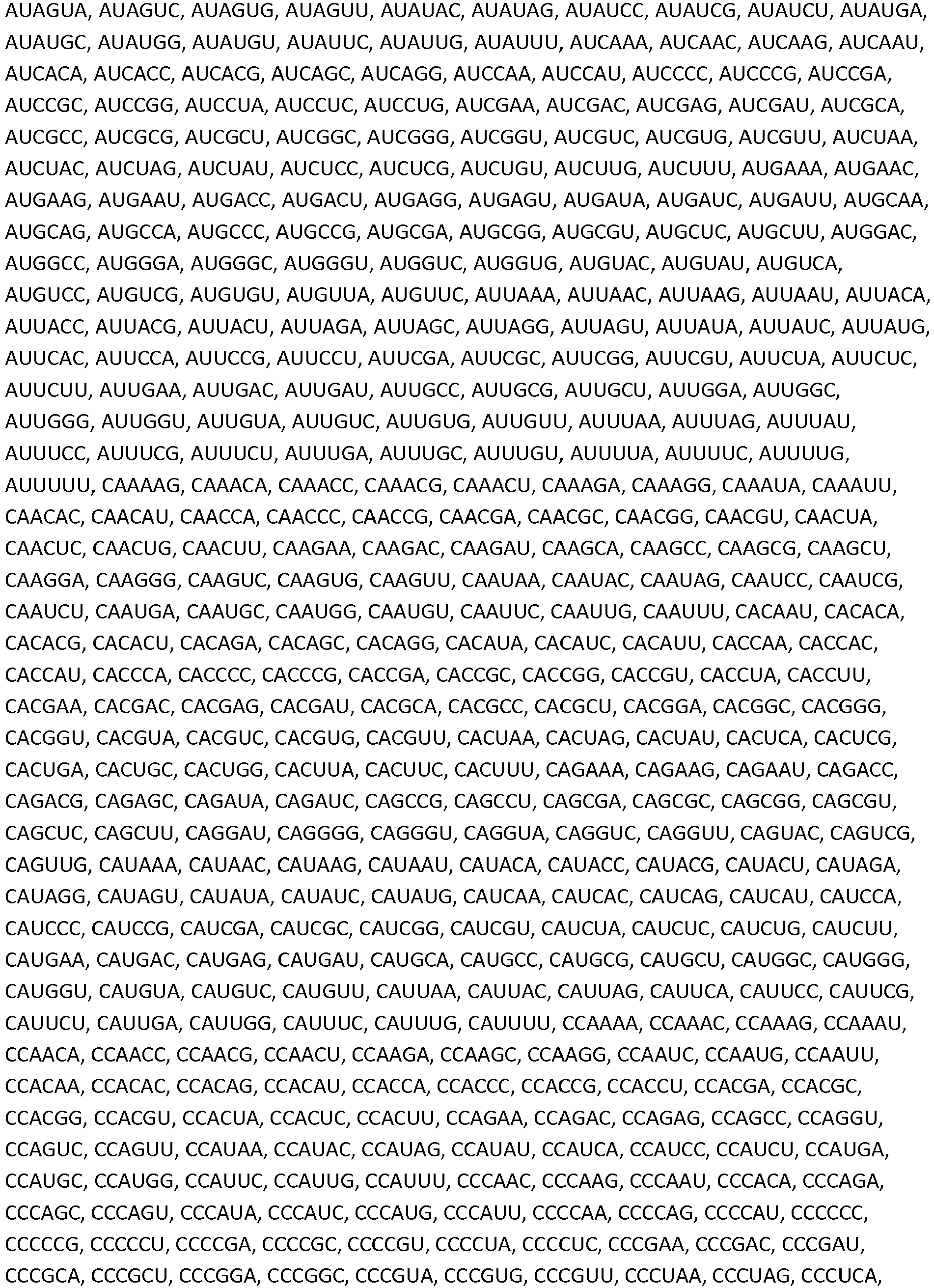

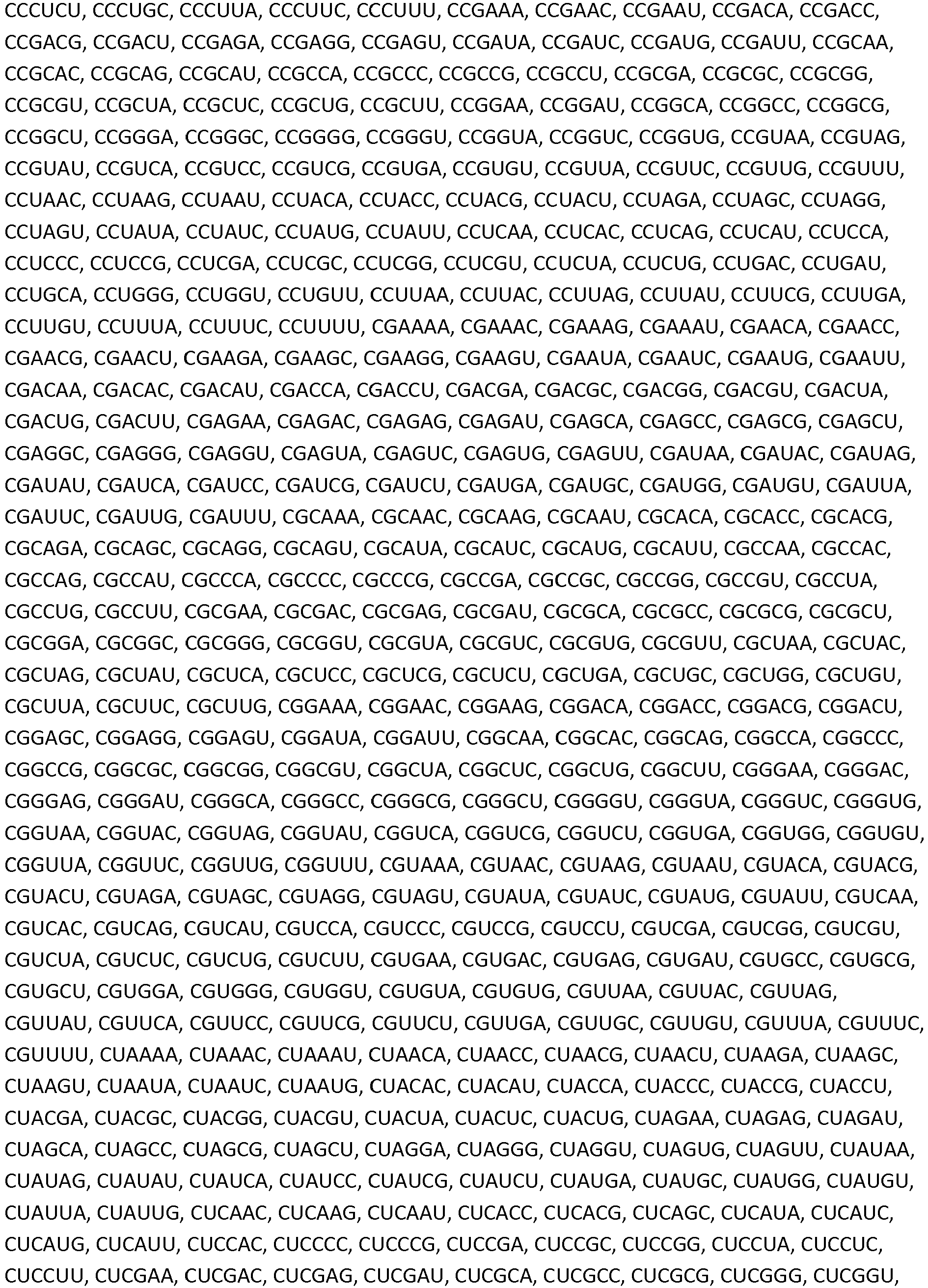

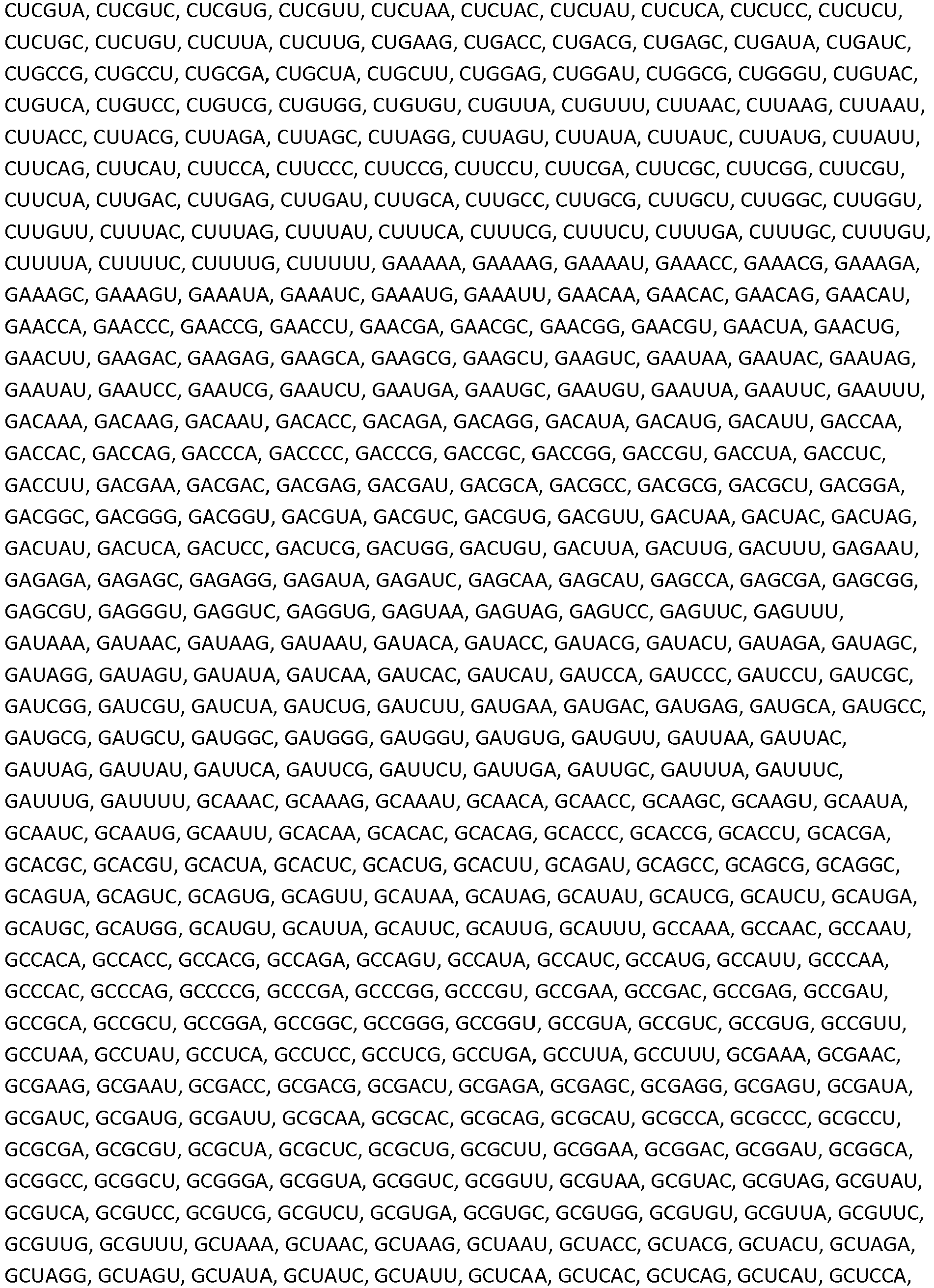

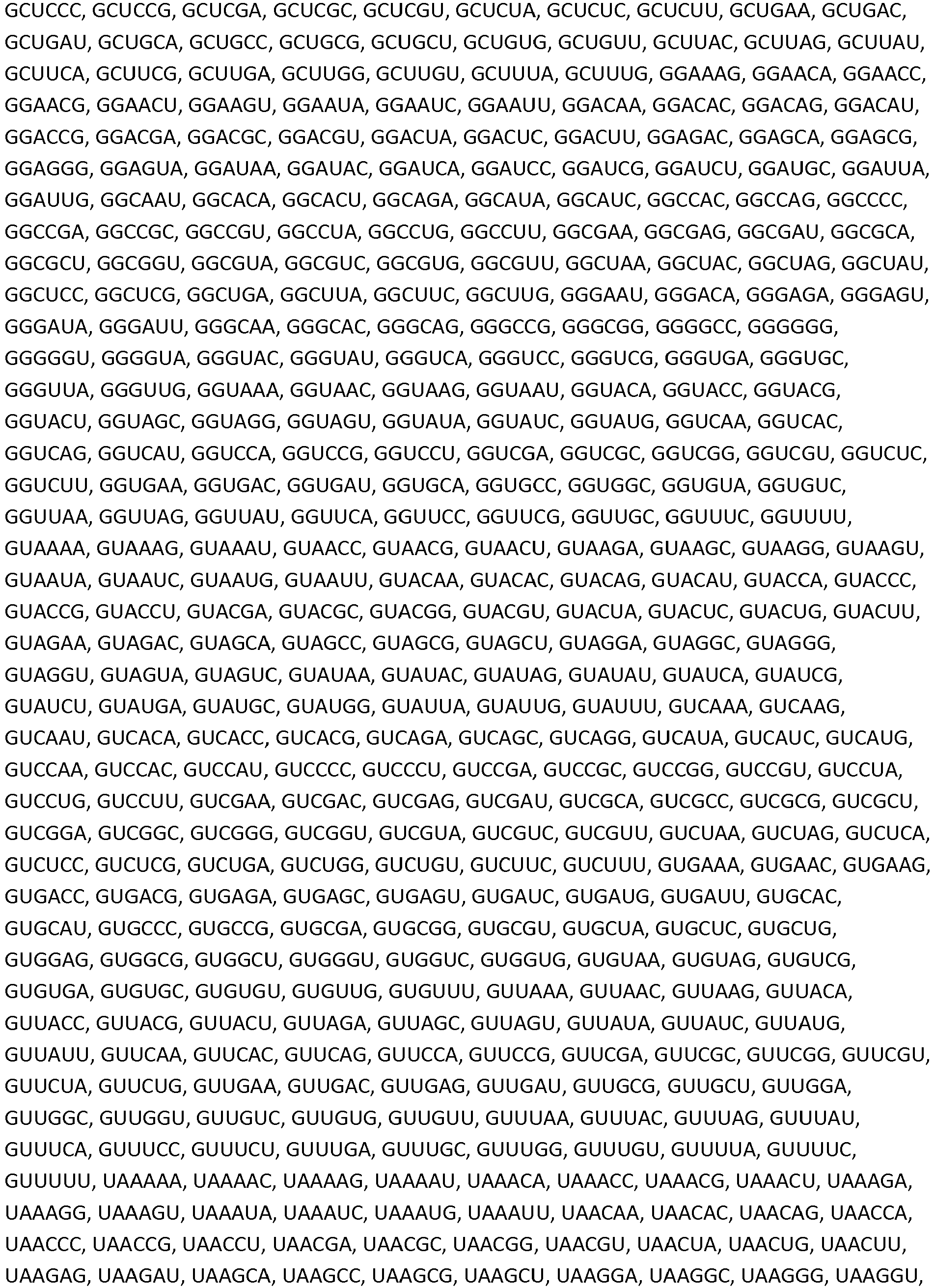

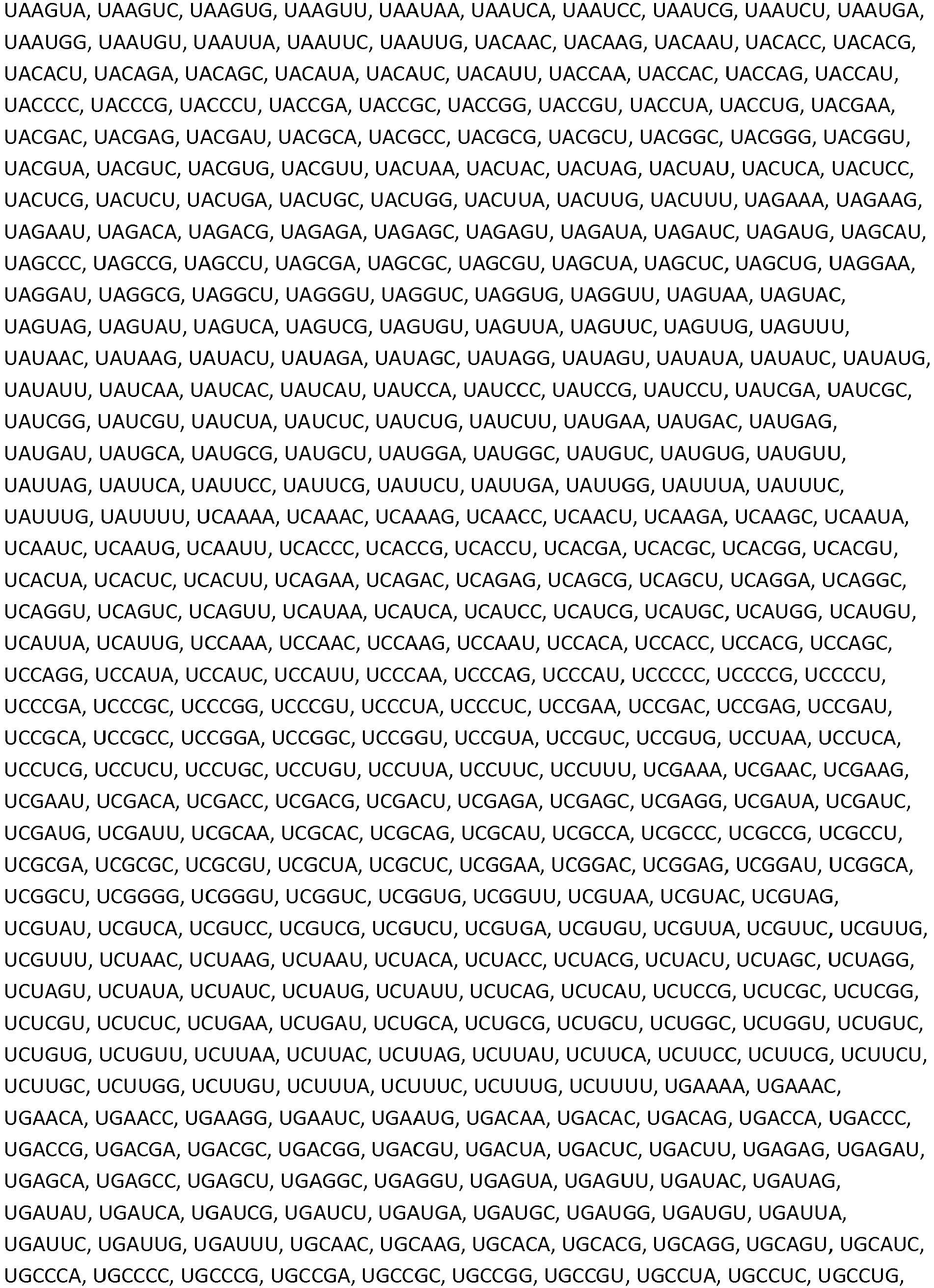

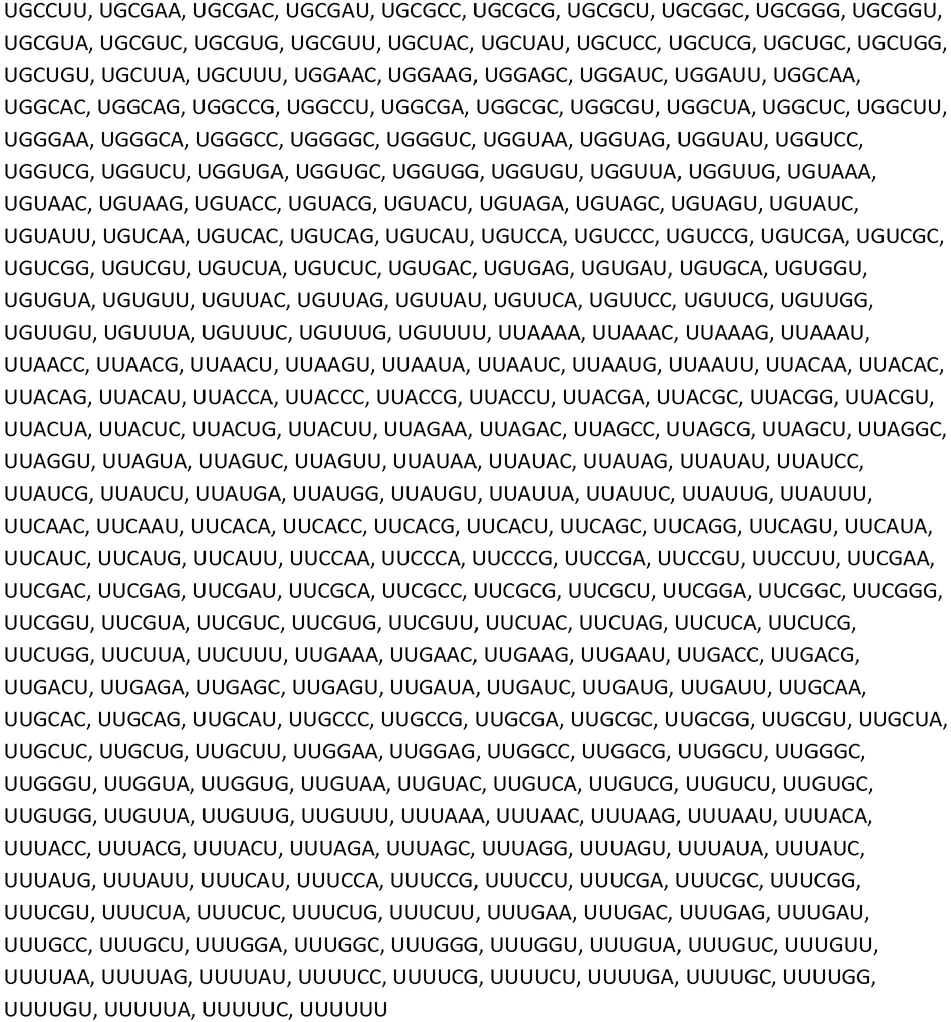

**Table 2: Imprinted regions hit by the expanded PRC2 transcriptome.**

| Imprinted gene targeted by PRC2-binding transcript and chromosome strand | mm9 coordinates and chromosome strand of PRC2-binding transcript | Sequence | MGI human gene name for mouse refGene | human liftOver coordinates (hg19) and chromosome strand of PRC2-binding transcript | Sequence |
|---|---|---|---|---|---|
| Wt1 (22431)+ | chr2:104956685-105023768+ | B934762 | WT1(7490)- | chr11:32400584-32466719- | B934864 |
| Wt1 (22431)+ | chr2: 104956685-105023768- | B934763 | WT1(7490)- | chr11:32400584-32466719+ | B934865 |
| Gatm(67092)- | chr2:122410207-122446997+ | B934764 | GATM(2628)- | chr15:45644412-45685240+ | B934866 |
| Gatm(67092)- | chr2:122410207-122446997- | B934765 | GATM(2628)- | chr15:45644412-45685240- | B934867 |
| L3mbt1(241764)+ | chr2:162759200-162810257+ | B934766 | | chr20:42088449-42184934+ | B934868 |
| L3mbtI(241764)+ | chr2:162759200-162810257- | B934767 | | chr20:42088449-42184934- | B934869 |
| Gnai3(14679)- | chr3:107900216-107959031+ | B934768 | GNAI3(2773) + | chr1:110081495-110150201- | B934870 |
| Gnai3(14679)- | chr3:107900216-107959031- | B934769 | GNAI3(2773) + | chr1:110081495-110150201+ | B934871 |
| Mkrn 1(54484)- | chr5:89257179-89278370+ | B934770 | MKRN 1(23608)- | chr7:140155983-140179369+ | B934905 |
| Mkrn 1(54484)- | chr5:89257179-89278370- | B934771 | MKRN 1(23608)- | chr7:140155983-140179369- | B934906 |
| Calcr(12311)- | chr6:3625733-3728615+ | B934772 | CALCR(799)- | chr7:93050410-93230834+ | B934872 |
| Calcr(12311)- | chr6:3625733-3728615- | B934773 | CALCR(799)- | chr7:93050410-93230834- | B934873 |
| Tfpi2(21789)- | chr6:3902594-3928353+ | B934774 | TFP 12(7980)- | chr7:93490853-93527640+ | B934874 |
| Tfpi2(21789)- | chr6:3902594-3928353- | B934775 | TFP 12(7980)- | chr7:93490853-93527640- | B934875 |
| Sgce(20392)- | chr6:4614349-4707098- | B934776 | SGCE(8910)- | chr7:94211984-94294870- | B934876 |
| Peg 10(170676)+ | chr6:4687379-4720475- | B934777 | PEG10(23089)+ | chr7:94275257-94299422- | B934877 |
| Pppl r9a(243725)+ | chr6:4843319-5125660+ | B934778 | PPP1R9A(55607)+ | chr7:94528605-94935514+ | B934878 |
| Pppl r9a(243725)+ | chr6:4843319-5125660- | B934779 | PPP1R9A(556 07)+ | chr7:94528605-94935514- | B934879 |
| Pon1(18979)- | chr6:5108104-5153823+ | B934780 | PON1(5444)- | chr7:94917863-94958087+ | B934880 |
| Pon1(18979)- | chr6:5108104-5153823- | B934781 | PON 1(5444)- | chr7:94917863-94958087- | B934881 |
| Pon3(269823)- | chr6:5160851-5216232+ | B934782 | PON3(5446)- | chr7:94989184-95025687+ | B934907 |
| Pon2(330260)- | chr6:5204623-5258372+ | B934783 | PON2(5445)- | chr7:95034174-95064384+ | B934908 |
| Pon2(330260)- | chr6:5204623-5258372- | B934784 | PON2(5445)- | chr7:95034174-95064384- | B934909 |
| Asb4(65255)+ | chr6:5323385-5393021+ | B934785 | ASB4(51666)+ | chr7:95094541-95179369+ | B934882 |
| Asb4(65255)+ | chr6:5323385-5393021- | B934786 | ASB4(51666)+ | chr7:95094541-95179369- | B934883 |
| Cpa4(71791)+ | chr6:30508375-30551746+ | B934787 | CPA4(51200)+ | chr7:129922767-129974483+ | B934884 |
| Cpa4(71791)+ | chr6:30508375-30551746- | B934788 | CPA4(51200)+ | chr7:129922767-129974483- | B934885 |
| Nap115(58243)- | chr6:58845227-58867058+ | B934789 | NAP1L5(266812)- | chr4:89617066-89619023+ | B934910 |
| Nap115(58243)- | chr6:58845227-58867058- | B934790 | NAP1L5(266812)- | chr4:89617066-89619023- | B934911 |
| Zim2(76637)- | chr7:6594458-6625116+ | B934791 | ZIM2(23619)- | chr19:57285923-57352097+ | B934912 |
| Zim2(76637)- | chr7:6594458-6625116- | B934792 | ZIM2(23619)- | chr19:57285923-57352097- | B934913 |
| Zim1 (22776)- | chr7:6618153-6659142+ | B934793 | | | |
| Zim1(22776)- | chr7:6618153-6659142- | B934794 | | | |
| Peg3(18616)- | chr7:6648670-6693129+ | B934795 | PEG3(5178)- | chr19:57319764-57358664+ | B934886 |
| Peg3(18616)- | chr7:6648670-6693129- | B934796 | PEG3(5178)- | chr19:57319764-57358664- | B934887 |
| Usp29(57775)+ | chr7:6673293-6929926+ | B934797 | USP29(57663)+ | chr19:57631509-57643293+ | B629991 |
| Usp29(57775)+ | chr7:6673293-6929926- | B934798 | USP29(57663)+ | chr19:57631509-57643293- | B629992 |
| Gabrg3(14407)- | chr7:63969611-64652167+ | B934799 | GABRG3(2567)+ | chr15:27216429-27778373- | B630983 |
| Gabrg3(14407)- | chr7:63969611-64652167- | B934800 | GABRG3(2567)+ | chr15:27216429-27778373+ | |
| Gabra5(110886)- | chr7:64653038-64775378+ | B934801 | GABRA5(2558)+ | chr15:27111866-27194357- | B934914 |
| Gabra5(110886)- | chr7:64653038-64775378- | B934802 | GABRA5(2558)+ | chr15:27111866-27194357+ | B934915 |
| Gabrb3(14402)+ | chr7:64835903-65094171+ | B934803 | GABRB3(2562)- | chr15:26788694-27018223- | B934916 |
| Gabrb3(14402)+ | chr7:64835903-65094171- | B934804 | GABRB3(2562)- | chr15:26788694-27018223+ | B934917 |
| Atp10a(11982)+ | chr7:65903701-66094160+ | B934805 | ATP 10A(57194)- | chr15:25923860-26108349- | B630990 |
| Snord116/Pwcr1 (64243)- | chr7:66921359-66941448+ | B934806 | | | |
| Snord116/Pwcr1 (64243)- | chr7:66921359-66941448- | B934807 | | | |
| Snrpn(20646)- | chr7:67117999-67159989+ | B934808 | SNRPN(6638)+ | chr15:25200135-25223729- | B934918 |
| Snrpn(20646)- | chr7:67117999-67159989- | B934809 | SNRPN(6638)+ | chr15:25200135-25223729+ | B934919 |
| Snurf(84704)- | chr7:67123487-67160009+ | B934810 | SNURF(8926)+ | chr15:25200135-25223729- | B934918 |
| Snurf(84704)- | chr7:67123487-67160009- | B934811 | SNURF(8926)+ | chr15:25200135-25223729+ | B934919 |
| Ndn(17984)+ | chr7:69483233-69504813- | B934812 | NDN(4692)- | chr15:23915288-23938997+ | B934888 |
| Magel2(27385)+ | chr7:69511864-69536525+ | B934813 | MAGEL2(54551)- | chr15:23888696-23892993- | B631003 |
| Magel2(27385)+ | chr7:69511864-69536525- | B934814 | MAGEL2(54551)- | chr15:23888696-23892993+ | B631004 |
| Mkrn3(22652)- | chr7:69552478-69575024+ | B934815 | MKRN3(7681)+ | chr15:23810454-23813166- | B934920 |
| Mkrn3(22652)- | chr7:69552478-69575024- | B934816 | MKRN3(7681)+ | chr15:23810454-23813166+ | B934921 |
| Peg12(27412)- | chr7:69596756-69619395+ | B934817 | | | |
| Peg12(27412)- | chr7:69596756-69619395- | B934818 | | | |
| Ins2(16334)- | chr7:149854565-149875612+ | B934819 | INS(3630)- | chr11:2181009-2182439+ | B632396 |
| Ins2(16334)- | chr7:149854565-149875612- | B934820 | INS(3630)- | chr11:2181009-2182439- | B632397 |
| Tspan32(27027)+ | chr7:150181595-150215548+ | B934821 | TSPAN32(10077)+ | chr11:2323243-2339430+ | B632402 |
| Tspan32(27027)+ | chr7:150181595-150215548- | B934822 | TSPAN32(10077)+ | chr11:2323243-2339430- | B632403 |
| S1c22a18(18400 )+ | chr7:150649693-150695226+ | B934823 | SLC22A18(5002)+ | chr11:2923512-2946476+ | B934922 |
| S1c22a18(18400 )+ | chr7:150649693-150695226- | B934824 | SLC22A18(5002)+ | chr11:2923512-2946476- | B934923 |
| PhIda2(22113)- | chr7:150677452-150698428+ | B934825 | PH LDA2(7262)- | chr11:2949503-2950650+ | B934924 |
| PhIda2(22113)- | chr7:150677452-150698428- | B934826 | PH LDA2(7262)- | chr11:2949503-2950650- | B934925 |
| Nap114(17955)- | chr7:150689483-150744994- | B934827 | NAP1L4(4676)- | chr11:2965660-3013607- | B632419 |
| Tnfrsf23(79201)- | chr7:150841711-150881776- | B934828 | | | |
| Osbp15(79196)- | chr7:150864666-150937867- | B934829 | OSBPL5(114879)- | chr11:3108346-3186582- | B632422 |
| Sdhd(66925)- | chr9:50394450-50421921+ | B934830 | SDHD(6392)+ | chr11:11195757 1-111966518- | B634959 |
| Rasgrf1 (19417)+ | chr9:89794612-89934638+ | B934831 | RASGRF1(5923)- | chr15:79252289-79383215- | B934926 |
| Rasgrf1(19417)+ | chr9:89794612-89934638- | B934832 | RASGRF1(5923)- | chr15:79252289-79383215+ | B934927 |
| Plagl 1 (22634)+ | chr10:12800714-12859693+ | B934833 | PLAGL1(5325)- | chr6:144257160-144341048- | B934889 |
| Ctnna3(216033)+ | chr10:62882845-64475689+ | B934834 | CTNNA3(29119)- | chr10:67679725-69425416- | B934928 |
| Ctnna3(216033)+ | chr10:62882845-64475689- | B934835 | CTNNA3(29119)- | chr10:67679725-69425416+ | B934929 |
| Dcn(13179)+ | chr10:96935000-96990784- | B934836 | DCN (1634)- | chr12:91539035-91576806+ | B934930 |
| Ddc(13195)- | chr11:11704105-11800403+ | B934837 | DDC(1644)- | chr7:50526134-50628768+ | B934931 |
| Grb10(14783)- | chr11:11820510-11947357+ | B934838 | GRB10(2887)- | chr7:50657755-50871312+ | B934890 |
| Grb10(14783)- | chr11:11820510-11947357- | B934839 | GRB10(2887)- | chr7:50657755-50871312- | |
| Commd 1(17846)- | chr11:22789727-22892283+ | B934840 | COMMD1(150684)+ | chr2:62132803-62363205- | B638303 |
| Commd 1(17846)- | chr11:22789727-22892283- | B934841 | COMMD150684)+ | chr2:62132803-62363205+ | B638304 |
| U2af(22185)+ | chr11:22862036-22884907+ | B934842 | | | |
| U2af(22185)+ | chr11:22862036-22884907- | B934843 | | | |
| M1r337/Mirn337(723843)+ | chr12:67749612-67769708+ | B934844 | | chr14:47524741-47544270+ | B934892 |
| M1r337/M1rn337(723843)+ | chr12:67749612-67769708- | B934845 | | chr14:47524741-47544270- | B934893 |
| DIk1 (13386)+ | chr12:11068143 2-110708900+ | B934846 | OLK1(8788)+ | chr14:10118369 0-101211352+ | B934894 |
| Meg3/Gt12(17263)+ | chr12:11077382 7-110809921- | B934847 | | chr14:10128776 2-101327347- | B934895 |
| 0103(107585)+ | chr12:11150744 2-111529304+ | B934848 | 0103(1735)+ | chr14:10201343 9-102036066+ | B934896 |
| Dio3(107585)+ | chr12:11150744 2-111529304- | B934849 | 0103(1735)+ | chr14:10201343 9-102036066- | B934897 |
| Htr2a(15558)+ | chr14:75030646-75116665+ | B934850 | HTR2A(3356)- | chr13:47401097-47479311- | B934898 |
| Htr2a(15558)+ | chr14:75030646-75116665- | B934851 | HTR2A(3356)- | chr13:47401097-47479311+ | B934899 |
| Kcnk9(223604)- | chr15:72335722-72389882+ | B934852 | KCNK9(51305)- | chr8:140621242-140723023+ | B934900 |
| Peg13(353342)- | chr15:72626029-72650753+ | B934853 | | chr8:141094733-141124284+ | B934901 |
| Peg13(353342)- | chr15:72626029-72650753- | B934854 | | chr8:141094733-141124284- | B934902 |
| S1c38a4(69354)- | chr15:96815253-96896386+ | B934855 | SLC38A4(55089)- | chr12:47116054-47237900+ | B934903 |
| S1c38a4(69354)- | chr15:96815253-96896386- | B934856 | SLC38A4(55089)- | chr12:47116054-47237900- | B934904 |
| S1c22a3(20519)- | chr17:12602837-12710569+ | B934857 | SLC22A3(6581)+ | chr6:160769425-160876014- | B647595 |
| SIc22a3(20519)- | chr17:12602837-12710569- | B934858 | SLC22A3(6581)+ | chr6:160769425-160876014+ | B647596 |
| S1c22a2(20518)+ | chr17:12767054-12831353+ | B934859 | SLC22A2(6582)- | chr6:160637794-160679963- | B647597 |
| S1c22a2(20518)+ | chr17:12767054-12831353- | B934860 | SLC22A2(6582)- | chr6:160637794-160679963+ | B647598 |
| Igf2r(16004)- | chr17:12865278-12972529+ | B934861 | IGF2R(3482)+ | chr6:160390131-160527583- | B647601 |
| Air/Airn(104103)+ | chr17:12931160-12954858+ | B934862 | | | |
| Impact(16210)+ | chr18:13120760-13161456+ | B934863 | IMPACT(55364)+ | chr18:22006609-22033494+ | B649028 |

The foregoing written specification is considered to be sufficient to enable one skilled in the art to practice the invention.

## Claims

1. A method for selecting a candidate oligonucleotide for activating expression of a target gene, the method comprising:
selecting a PRC2-associated region within a first nucleotide sequence, wherein the first nucleotide sequence maps to a position in a first chromosome between 50 kilobases upstream of a 5'-end of the target gene and 50 kilobases downstream of a 3'-end of the target gene, wherein the PRC2-associated region is a region of an RNA that is protected from a nuclease in an RNA-immunoprecipitation assay that employs an antibody that targets a component of PRC2;
determining a second nucleotide sequence that is complementary with at least 8 consecutive nucleotides of the PRC2-associated region; and
selecting as the candidate oligonucleotide, a single stranded oligonucleotide comprising the second nucleotide sequence, wherein the oligonucleotide has at least one of following features:
a) a sequence: 5'-X-Y-Z, wherein X is any nucleotide, Y is a nucleotide sequence of 6 nucleotides in length that is not a seed sequence of a human microRNA, and Z is a nucleotide sequence of 1 to 23 nucleotides in length, wherein X is anchored at the 5' end of the oligonucleotide;
b) a sequence that does not comprise three or more consecutive guanosine nucleotides;
c) a sequence that is complementary to a PRC2-associated region that encodes an RNA that forms a secondary structure comprising at least two single stranded loops; or
d) a sequence that has greater than 60% G-C content;
wherein the single stranded oligonucleotide is 8 to 30 nucleotides in length.

2. The method of claim 1, wherein the oligonucleotide has at least two of features a), b), c), and d).

3. The method of claim 1, wherein the oligonucleotide has at least three features of a), b), c) and d).

4. A method of selecting a set of oligonucleotides that is enriched in oligonucleotides that activate expression of a target gene, the method comprising:
selecting a PRC2-associated region within a first nucleotide sequence that maps to a position in a first chromosome between 50 kilobases upstream of a 5'-end of the target gene and 50 kilobases downstream of a 3'-end of the target gene, wherein the PRC2-associated region is a region of an RNA that is protected from a nuclease in an RNA-immunoprecipitation assay that employs an antibody that targets a component of PRC2;
selecting a set of oligonucleotides, wherein each oligonucleotide in the set comprises a second nucleotide sequence that is complementary with at least 8 consecutive nucleotides of the PRC2-associated region, and has at least one of the following features:
a) a sequence: 5'-X-Y-Z, wherein X is any nucleotide, Y is a nucleotide sequence of 6 nucleotides in length that is not a human seed sequence of a microRNA, and Z is a nucleotide sequence of 1 to 23 nucleotides in length, wherein X is anchored at the 5' end of the oligonucleotide;
b) a sequence that does not comprise three or more consecutive guanosine nucleotides;
c) a sequence that is complementary to a PRC2-associated region that encodes an RNA that forms a secondary structure comprising at least two single stranded loops; and/or
d) a sequence that has greater than 60% G-C content; and
wherein the set of oligonucleotides is enriched in oligonucleotides that activate expression of a target gene;
wherein each of the oligonucleotides is 8 to 30 nucleotides in length.

5. The method of claim 4, wherein each of the oligonucleotides in the set share at least two of features a), b), c), and d).

6. The method of claim 4, wherein each of the oligonucleotides in the set share at least three of features a), b), c), and d).

7. The method of any one of claims 1 to 6, wherein the first chromosome is a chromosome of a first species, and wherein the method further comprises
determining that the second nucleotide sequence is complementary to a second region of a second chromosome of a second species, the second region being located between 50 kilobases upstream of a 5'-end of a homolog of the target gene and 50 kilobases downstream of a 3'-end of the homolog of the target gene.

8. The method of any one of claims 1 to 7, wherein the first nucleotide sequence maps to the strand of the first chromosome comprising the sense strand of the target gene or the first nucleotide sequence maps to the strand of the first chromosome comprising the antisense strand of the target gene.

9. A single stranded oligonucleotide for use in the treatment of a disease, wherein the treatment involves upregulating expression of a gene targeted by the PRC2-binding RNA and wherein the single stranded oligonucleotide comprises a region of complementarity that is complementary with at least 8 consecutive nucleotides of a PRC2-associated region located in a first chromosome between 50 kilobases upstream of a 5'-end of a target gene and 50 kilobases downstream of a 3'-end of the target gene, wherein the PRC2-associated region is a region of an RNA that is protected from a nuclease in an RNA-immunoprecipitation assay that employs an antibody that targets a component of PRC2, and wherein the oligonucleotide has at least one of:
a) a sequence comprising 5'-X-Y-Z, wherein X is any nucleotide, Y is a nucleotide sequence of 6 nucleotides in length that is not a human seed sequence of a microRNA, and Z is a nucleotide sequence of 1 to 23 nucleotides in length;
b) a sequence that does not comprise three or more consecutive guanosine nucleotides;
c) a sequence that is complementary to a PRC2-associated region that encodes an RNA that forms a secondary structure comprising at least two single stranded loops; and/or
d) a sequence that has greater than 60% G-C content;
wherein the oligonucleotide is 8 to 30 nucleotides in length.

10. A single stranded oligonucleotide for use according to claim 9 wherein (a) the disease is cancer or a metabolic or respiratory disease or (b) the use comprises pulmonary delivery and, optionally, the disease is a disease of the lungs.

11. The single stranded oligonucleotide for use of claim 9 or 10, wherein the oligonucleotide has at least two of features a), b), c), and d).

12. The single stranded oligonucleotide for use of claim 9 or 10, wherein the oligonucleotide has at least three of features a), b), c), and d).

13. The single stranded oligonucleotide for use of any one of claims 9 to 12, wherein the at least 8 consecutive nucleotides of the PRC2-associated region in the strand of the chromosome comprising the sense strand of the target gene, or wherein the at least 8 consecutive nucleotides of the PRC2-associated region in the strand of the chromosome comprising the antisense strand of the target gene.

14. The method of any one of claims 1 to 8 or the single stranded oligonucleotide for use of any one of claims 9 to 13, wherein the PRC2-associated region
(a) is upstream of the 5' end of the target gene;
(b) is downstream of the 3' end of the target gene;
(c) is within an intron of the target gene;
(d) is within an exon of the target gene; or
(e) traverses an intron-exon junction, a 5'-UTR-exon junction or a 3'-UTR-exon junction of the target gene.

15. The single stranded oligonucleotide for use of any one of claims 9 to 14, wherein at least one nucleotide of the oligonucleotide is a nucleotide analogue.

16. The single stranded oligonucleotide for use of any one of claims 9 to 15,
wherein at least one nucleotide of the oligonucleotide comprises a 2' O-methyl; or
wherein the oligonucleotide comprises at least one ribonucleotide, at least one deoxyribonucleotide, or at least one bridged nucleotide, optionally wherein the bridge nucleotide is a LNA nucleotide, a cEt nucleotide or a ENA nucleotide analogue.

17. The single stranded oligonucleotide for use of claim 15, wherein the nucleotides of the oligonucleotide comprise:
a) alternating deoxyribonucleotides and 2'-fluoro-deoxyribonucleotides;
b) alternating deoxyribonucleotides and 2'-O-methyl nucleotides;
c) alternating deoxyribonucleotides and ENA nucleotide analogues;
d) alternating deoxyribonucleotides and LNA nucleotides; or
e) alternating LNA nucleotides and 2'-O-methyl nucleotides.

18. The single stranded oligonucleotide for use of any one of claims 9 to 17, further comprising phosphorothioate internucleotide linkages between at least two nucleotides, optionally comprising phosphorothioate internucleotide linkages between all nucleotides.

19. The single stranded oligonucleotide for use according to any of claims 9-18, wherein the treatment of a disease is for treating a condition associated with decreased levels of a target gene in a subject.

## Patentansprüche

1. Verfahren zum Auswählen eines Kandidaten-Oligonukleotids zum Aktivieren der Expression eines Ziel-Gens, wobei das Verfahren umfasst:
Auswählen einer PRC2-assoziierten Region innerhalb einer ersten Nukleotidsequenz, wobei die erste Nukleotidsequenz einer Position in einem ersten Chromosom zwischen 50 Kilobasen strangaufwärts von einem 5'-Ende des Ziel-Gens und 50 Kilobasen strangabwärts von einem 3'-Ende des Ziel-Gens zugeordnet ist (maps to), wobei die PRC2-assoziierte Region eine Region einer RNA ist, die vor einer Nuklease in einem RNA-Immunpräzipitationsassay, der einen Antikörper einsetzt, der auf eine Komponente von PRC2 zielt, geschützt ist;
Bestimmen einer zweiten Nukleotidsequenz, die komplementär zu wenigstens 8 aufeinanderfolgenden Nukleotiden der PRC2-assoziierten Region ist; und
Auswählen eines einzelsträngigen Oligonukleotids, welches die zweite Nukleotidsequenz umfasst, als das Kandidaten-Oligonukleotid, wobei das Oligonukleotid wenigstens eines der folgenden Merkmale aufweist:
a) eine Sequenz: 5'-X-Y-Z, wobei X irgendein Nukleotid ist, Y eine Nukleotidsequenz von 6 Nukleotiden Länge ist, die keine Seed-Sequenz einer humanen microRNA ist, und Z eine Nukleotidsequenz von 1 bis 23 Nukleotiden Länge ist, wobei X am 5'-Ende des Oligonukleotids verankert ist;
b) eine Sequenz, die nicht drei oder mehr aufeinanderfolgende Guanosin-Nukleotide umfasst;
c) eine Sequenz, die komplementär zu einer PRC2-assoziierten Region ist, die eine RNA codiert, die eine Sekundärstruktur bildet, die wenigstens zwei einzelsträngige Schleifen umfasst; oder
d) eine Sequenz, die einen G-C-Gehalt hat, der größer als 60% ist;
wobei das einzelsträngige Nukleotid 8 bis 30 Nukleotide lang ist.

2. Verfahren gemäß Anspruch 1, wobei das Oligonukleotid wenigstens zwei der Merkmale a), b), c) und d) aufweist.

3. Verfahren gemäß Anspruch 1, wobei das Oligonukleotid wenigstens drei Merkmale von a), b), c) und d) aufweist.

4. Verfahren zum Auswählen eines Satzes von Oligonukleotiden, der hinsichtlich Oligonukleotiden angereichert ist, die die Expression eines Ziel-Gens aktivieren, wobei das Verfahren umfasst:
Auswählen einer PRC2-assoziierten Region innerhalb einer ersten Nukleotidsequenz, die einer Position in einem ersten Chromosom zwischen 50 Kilobasen strangaufwärts von einem 5'-Ende des Ziel-Gens und 50 Kilobasen strangabwärts von einem 3'-Ende des Ziel-Gens zugeordnet ist (maps to), wobei die PRC2-assoziierte Region eine Region einer RNA ist, die vor einer Nuklease in einem RNA-Immunpräzipitationsassay, der einen Antikörper einsetzt, der auf eine Komponente von PRC2 zielt, geschützt ist;
Auswählen eines Satzes von Oligonukleotiden, wobei jedes Oligonukleotid in dem Satz eine zweite Nukleotidsequenz umfasst, die komplementär zu wenigstens 8 aufeinanderfolgenden Nukleotiden der PRC2-assoziierten Region ist und wenigstens eines der folgenden Merkmale aufweist:
a) eine Sequenz: 5'-X-Y-Z, wobei X irgendein Nukleotid ist, Y eine Nukleotidsequenz von 6 Nukleotiden Länge ist, die keine humane Seed-Sequenz einer microRNA ist, und Z eine Nukleotidsequenz von 1 bis 23 Nukleotiden Länge ist, wobei X am 5'-Ende des Oligonukleotids verankert ist;
b) eine Sequenz, die nicht drei oder mehr aufeinanderfolgende Guanosin-Nukleotide umfasst;
c) eine Sequenz, die komplementär zu einer PRC2-assoziierten Region ist, die eine RNA codiert, die eine Sekundärstruktur bildet, die wenigstens zwei einzelsträngige Schleifen umfasst; und/oder
d) eine Sequenz, die einen G-C-Gehalt hat, der größer als 60% ist; und
wobei der Satz von Oligonukleotiden hinsichtlich Oligonukleotiden angereichert ist, die die Expression eines Ziel-Gens aktivieren;
wobei jedes der Oligonukleotide 8 bis 30 Nukleotide lang ist.

5. Verfahren gemäß Anspruch 4, wobei jedes der Oligonukleotide in dem Satz wenigstens zwei der Merkmale a), b), c) und d) gemeinsam hat.

6. Verfahren gemäß Anspruch 4, wobei jedes der Oligonukleotide in dem Satz wenigstens drei der Merkmale a), b), c) und d) gemeinsam hat.

7. Verfahren gemäß irgendeinem der Ansprüche 1 bis 6, wobei das erste Chromosom ein Chromosom einer ersten Spezies ist und wobei das Verfahren weiterhin umfasst:
Bestimmen, dass die zweite Nukleotidsequenz komplementär zu einer zweiten Region eines zweiten Chromosoms einer zweiten Spezies ist, wobei die zweite Region zwischen 50 Kilobasen strangaufwärts von einem 5'-Ende eines Homologs des Ziel-Gens und 50 Kilobasen strangabwärts von einem 3'-Ende des Homologs des Ziel-Gens liegt.

8. Verfahren gemäß irgendeinem der Ansprüche 1 bis 7, wobei die erste Nukleotidsequenz dem Strang des ersten Chromosoms, welcher den Sense-Strang des Ziel-Gens umfasst, zugeordnet ist (maps to), oder die erste Nukleotidsequenz dem Strang des ersten Chromosoms, welcher den Antisense-Strang des Ziel-Gens umfasst, zugeordnet ist (maps to).

9. Einzelsträngiges Oligonukleotid zur Verwendung bei der Behandlung einer Erkrankung, wobei die Behandlung die Aufwärtsregulierung der Expression eines durch die PRC2-bindende RNA angezielten Gens involviert und wobei das einzelsträngige Oligonukleotid eine Komplementaritätsregion umfasst, die zu wenigstens 8 aufeinanderfolgenden Nukleotiden einer PRC2-assoziierten Region komplementär ist, die in einem ersten Chromosom zwischen 50 Kilobasen strangaufwärts von einem 5'-Ende eines Ziel-Gens und 50 Kilobasen strangabwärts von einem 3'-Ende des Ziel-Gens liegt, wobei die PRC2-assoziierte Region eine Region von RNA ist, die vor einer Nuklease in einem RNA-Immunpräzipitationsassay, der einen Antikörper einsetzt, welcher auf eine Komponente von PRC2 zielt, geschützt ist, und wobei das Oligonukleotid wenigstens eines der folgenden aufweist:
a) eine Sequenz, die 5'-X-Y-Z umfasst, wobei X irgendein Nukleotid ist, Y eine Nukleotidsequenz von 6 Nukleotiden Länge ist, die keine humane Seed-Sequenz einer microRNA ist, und Z eine Nukleotidsequenz von 1 bis 23 Nukleotiden Länge ist;
b) eine Sequenz, die nicht drei oder mehr aufeinanderfolgende Guanosin-Nukleotide umfasst;
c) eine Sequenz, die komplementär zu einer PRC2-assoziierten Region ist, die eine RNA codiert, die eine Sekundärstruktur bildet, welche wenigstens zwei einzelsträngige Schleifen umfasst, und/oder
d) eine Sequenz, die einen G-C-Gehalt hat, der größer als 60% ist;
wobei das Oligonukleotid 8 bis 30 Nukleotide lang ist.

10. Einzelsträngiges Oligonukleotid zur Verwendung gemäß Anspruch 9, wobei (a) die Erkrankung Krebs oder eine Stoffwechsel- oder Atemwegserkrankung ist, oder (b) die Verwendung pulmonale Zuführung umfasst, und die Erkrankung optional eine Erkrankung der Lungen ist.

11. Einzelsträngiges Oligonukleotid zur Verwendung gemäß Anspruch 9 oder 10, wobei das Oligonukleotid wenigstens zwei der Merkmale a), b), c) und d) aufweist.

12. Einzelsträngiges Oligonukleotid zur Verwendung gemäß Anspruch 9 oder 10, wobei das Oligonukleotid wenigstens drei der Merkmale a), b), c) und d) aufweist.

13. Einzelsträngiges Oligonukleotid zur Verwendung gemäß irgendeinem der Ansprüche 9 bis 12, wobei die wenigstens 8 aufeinanderfolgenden Nukleotide der PRC2-assoziierten Region in dem Strang des Chromosoms den Sense-Strang des Ziel-Gens umfassen, oder wobei die wenigstens 8 aufeinanderfolgenden Nukleotide der PRC2-assoziierten Region in dem Strang des Chromosoms den Antisense-Strang des Ziel-Gens umfassen.

14. Verfahren gemäß irgendeinem der Ansprüche 1 bis 8 oder einzelsträngiges Oligonukleotid zur Verwendung gemäß irgendeinem der Ansprüche 9 bis 13, wobei die PRC2-assoziierte Region
(a) strangaufwärts zu dem 5'-Ende des Ziel-Gens ist;
(b) strangabwärts zu dem 3'-Ende des Ziel-Gens ist;
(c) innerhalb eines Introns des Ziel-Gens ist;
(d) innerhalb eines Exons des Ziel-Gens ist; oder
(e) eine Intron-Exon-Verbindung, eine 5'-UTR-Exon-Verbindung oder eine 3'-UTR-Exon-Verbindung des Ziel-Gens kreuzt.

15. Einzelsträngiges Oligonukleotid zur Verwendung gemäß irgendeinem der Ansprüche 9 bis 14, wobei wenigstens ein Nukleotid des Oligonukleotids ein Nukleotid-Analogon ist.

16. Einzelsträngiges Oligonukleotid zur Verwendung gemäß irgendeinem der Ansprüche 9 bis 15,
wobei wenigstens ein Nukleotid des Oligonukleotids ein 2'-O-Methyl umfasst; oder
wobei das Oligonukleotid wenigstens ein Ribonukleotid, wenigstens ein Desoxyribonukleotid oder wenigstens ein gebrücktes Nukleotid (bridged nucleotide) umfasst, wobei optional das Brücken-Nukleotid ein LNA-Nukleotid, ein cEt-Nukleotid oder ein ENA-Nukleotid-Analogon ist.

17. Einzelsträngiges Oligonukleotid zur Verwendung gemäß Anspruch 15, wobei die Nukleotide des Oligonukleotids umfassen:
a) alternierende Desoxyribonukleotide und 2'-Fluor-Desoxyribonukleotide;
b) alternierende Desoxyribonukleotide und 2'-O-Methyl-Nukleotide;
c) alternierende Desoxyribonukleotide und ENA-Nukleotid-Analoga;
d) alternierende Desoxyribonukleotide und LNA-Nukleotide; oder
e) alternierende LNA-Nukleotide und 2'-O-Methyl-Nukleotide.

18. Einzelsträngiges Oligonukleotid zur Verwendung gemäß irgendeinem der Ansprüche 9 bis 17, weiterhin umfassend Phosphorthioat-Internukleotid-Verknüpfungen zwischen wenigstens zwei Nukleotiden, optional umfassend Phosphorthioat-Internukleotid-Verknüpfungen zwischen allen Nukleotiden.

19. Einzelsträngiges Oligonukleotid zur Verwendung gemäß irgendeinem der Ansprüche 9-18, wobei die Behandlung einer Erkrankung zur Behandlung eines mit verringerten Niveaus eines Ziel-Gens in einem Subjekt assoziierten Zustands ist.

## Revendications

1. Méthode de sélection d'un oligonucléotide candidat pour activer l'expression d'un gène cible, la méthode comprenant :
la sélection d'une région associée à PRC2 dans une première séquence de nucléotide, dans laquelle la première séquence de nucléotide cartographie une position dans un premier chromosome entre 50 kilobases en amont d'une fin 5' du gène cible et 50 kilobases en aval d'une fin 3' du gène cible, dans laquelle la région associée à PCR2 est une région d'un ARN qui est protégée d'une nucléase dans un test d'immunoprécipitation d'ARN qui emploie un anticorps ciblant un composant de PRC2 ;
la détermination d'une deuxième séquence de nucléotide qui est complémentaire avec au moins 8 nucléotides consécutifs de la région associée à PRC2 ; et
la sélection en tant qu'oligonucléotide candidat, d'un oligonucléotide à un seul brin comprenant la deuxième séquence de nucléotide, dans laquelle l'oligonucléotide a au moins l'une des caractéristiques suivantes :
a) une séquence : 5'-X-Y-Z, dans laquelle X est un nucléotide quelconque, Y est une séquence de nucléotide de 6 nucléotides en longueur qui n'est pas une séquence de base d'un micro ARN humain, et Z est une séquence de nucléotides de 1 à 23 nucléotide(s) en longueur, dans laquelle X est ancré à la fin 5' de l'oligonucléotide ;
b) une séquence qui ne comprend pas trois ou plus de trois nucléotides guanosine consécutifs ;
c) une séquence qui est complémentaire à une région associée à PRC2 qui encode un ARN qui forme une structure secondaire comprenant au moins deux boucles à un seul brin ; ou
d) une séquence qui une teneur en G-C supérieure à 60% ;
dans laquelle l'oligonucléotide à un seul brin est de 8 à 30 nucléotides en longueur.

2. La méthode de la revendication 1, dans laquelle l'oligonucléotide a au moins deux des caractéristiques a), b) c) et d).

3. La méthode de la revendication 1, dans laquelle l'oligonucléotide a au moins trois des caractéristiques a), b), c) et d).

4. Méthode de sélection d'un jeu d'oligonucléotides qui est enrichi en oligonucléotides qui active l'expression d'un gène cible, la méthode comprenant :
la sélection d'une région associée à PRC2 dans une première séquence de nucléotide qui cartographie une position dans un premier chromosome entre 50 kilobases en amont d'une fin 5' du gène cible et 50 kilobases en aval d'une fin 3' du gène cible, dans laquelle la région associée à PCR2 est une région d'un ARN qui est protégée d'une nucléase dans un test d'immunoprécipitation d'ARN qui emploie un anticorps ciblant un composant de PRC2 ;
la sélection d'un jeu d'oligonucléotides, dans laquelle chaque oligonucléotide dans le jeu comprend une deuxième séquence d'oligonucléotides qui est complémentaire avec au moins 8 nucléotides consécutifs de la région associée à PRC2, et au moins l'une des caractéristiques suivantes :
a) une séquence : 5'-X-Y-Z, dans laquelle X est un nucléotide quelconque, Y est une séquence de nucléotide de 6 nucléotides en longueur qui n'est pas une séquence de base d'un micro ARN humain, et Z est une séquence de nucléotides de 1 à 23 nucléotide(s) en longueur, dans laquelle X est ancré à la fin 5' de l'oligonucléotide ;
b) une séquence qui ne comprend pas trois ou plus de trois nucléotides guanosine consécutifs ;
c) une séquence qui est complémentaire à une région associée à PRC2 qui encode un ARN qui forme une structure secondaire comprenant au moins deux boucles à un seul brin ; ou
d) une séquence qui une teneur en G-C supérieure à 60% ;
dans laquelle l'oligonucléotide à un seul brin est de 8 à 30 nucléotides en longueur.

5. La méthode de la revendication 4, dans laquelle chacun des oligonucléotides dans le jeu partage au moins deux des caractéristiques a), b), c) et d).

6. La méthode de la revendication 4, dans laquelle chacun des oligonucléotides dans le jeu partage au moins trois des caractéristiques a), b), c) et d).

7. La méthode de l'une quelconque des revendications 1 à 6, dans laquelle le premier chromosome est un chromosome d'une première espèce, et dans laquelle la méthode comprend en outre
la détermination que la deuxième séquence de nucléotide est complémentaire à une deuxième région d'un deuxième chromosome d'une deuxième espèce, la deuxième région étant située entre 50 kilobases en amont d'une fin 5' d'un homologue du gène cible et 50 kilobases en aval d'une fin 3' de l'homologue du gène cible.

8. La méthode de l'une quelconque des revendications 1 à 7, dans laquelle la première séquence d'oligonucléotide cartographie le brin du premier chromosome comprenant le brin sens du gène cible ou la première séquence de nucléotide cartographie le brin du premier chromosome comprenant le brin antisens du gène cible.

9. Oligonucléotide à un seul brin pour l'utilisation dans le traitement dans une maladie, dans laquelle le traitement implique la régulation à la hausse de l'expression d'un gène ciblé par l'ARN se liant à PRC2 et dans laquelle l'oligonucléotide à un seul brin comprend une région de complémentarité qui est complémentaire avec au moins 8 nucléotides consécutifs d'une région associée à PRC2 située dans un premier chromosome entre 50 kilobases en amont d'une fin 5' d'un gène cible et 50 kilobases en aval d'une fin 3' du gène cible, dans laquelle la région associée à PRC2 est une région d'un ARN qui est protégée d'une nucléase dans un test d'immunoprécipitation d'ARN qui emploie un anticorps ciblant un composant de PRC2, et dans laquelle l'oligonucléotide a au moins l'un de :
a) une séquence : 5'-X-Y-Z, dans laquelle X est un nucléotide quelconque, Y est une séquence de nucléotide de 6 nucléotides en longueur qui n'est pas une séquence de base d'un micro ARN, et Z est une séquence de nucléotides de 1 à 23 nucléotide(s) en longueur ;
b) une séquence qui ne comprend pas trois ou plus de trois nucléotides guanosine consécutifs ;
c) une séquence qui est complémentaire à une région associée à PRC2 qui encode un ARN qui forme une structure secondaire comprenant au moins deux boucles à un seul brin ; et/ou
d) une séquence qui une teneur en G-C supérieure à 60% ;
dans laquelle l'oligonucléotide est de 8 à 30 nucléotides en longueur.

10. Oligonucléotide à un seul brin pour l'utilisation selon la revendication 9 dans laquelle (a) la maladie est un cancer ou un une maladie respiratoire ou métabolique ou (b) l'utilisation comprend l'administration pulmonaire et, optionnellement, la maladie est une maladie des poumons.

11. L'oligonucléotide à simple brin pour l'utilisation de la revendication 9 ou 10, dans laquelle l'oligonucléotide a au moins deux des caractéristiques a), b), c) et d).

12. L'oligonucléotide à simple brin pour l'utilisation de la revendication 9 ou 10, dans laquelle l'oligonucléotide a au moins trois des caractéristiques a), b), c) et d).

13. L'oligonucléotide à simple brin pour l'utilisation de l'une quelconque des revendications 9 à 12, dans laquelle les au moins 8 nucléotides consécutifs de la région associée à PRC2 dans le brin du chromosome comprenant le brin sens du gène cible, ou dans laquelle les au moins 8 nucléotides consécutifs de la région associée à PRC2 dans le brin du chromosome comprenant le brin antisens du gène cible.

14. La méthode de l'une quelconque des revendications 1 à 8 ou l'oligonucléotide à simple brin pour l'utilisation de l'une quelconque des revendications 9 à 13, dans laquelle la région associée à PRC2
(a) est en amont de la fin 5' du gène cible ;
(b) est en aval de la fin 3' du gène cible ;
(c) est au sein d'un intron du gène cible ;
(d) est au sein d'un exon du gène cible ; ou
(e) traverse une jonction intron-exon, une jonction exon-UTR-5' ou une jonction exon-UTR-3' du gène cible.

15. L'oligonucléotide à un seul brin pour l'utilisation de l'une quelconque des revendications 9 à 14, dans laquelle au moins un nucléotide de l'oligonucléotide est un analogue de nucléotide.

16. L'oligonucléotide à un seul brin pour l'utilisation de l'une quelconque des revendications 9 à 15,
dans laquelle au moins un nucléotide de l'oligonucléotide comprend un 2' O-méthyle ; ou
dans laquelle l'oligonucléotide comprend au moins un ribonucléotide, au moins un désoxyribonucléotide, ou au moins un nucléotide ponté, optionnellement dans lequel le nucléotide de pont est un nucléotide LNA, un nucléotide cEt ou un analogue de nucléotide ENA.

17. L'oligonucléotide à un seul brin pour l'utilisation de la revendication 15, dans laquelle les nucléotides de l'oligonucléotide comprennent :
a) une alternance de désoxyribonucléotides et de 2'-fluoro-déoxyribonucléotides ;
b) une alternance de désoxyribonucléotides et de 2'-O-méthyl nucléotides ;
c) une alternance de désoxyribonucléotides et d'analogues de nucléotides ENA ;
d) une alternance de désoxyribonucléotides et de nucléotides LNA ; ou
e) une alternance de nucléotides LNA et de 2'-O-méthyl nucléotides.

18. L'oligonucléotide à un seul brin pour l'utilisation de l'une quelconque des revendications 9 à 17, comprenant en outre des liaisons inter-nucléotides phosphorothioate entre au moins deux nucléotides, optionnellement comprenant des liaisons inter-nucléotides phosphorothioate entre tous les nucléotides.

19. L'oligonucléotide à un seul brin pour l'utilisation de l'une quelconque des revendications 9-18, dans laquelle le traitement d'une maladie est pour le traitement d'une affection associée avec des niveaux élevés d'un gène cible dans un sujet.
